# EUROPÄISCHE PATENTANMELDUNG

(11) **EP 0 861 670 A2**
(43) Veröffentlichungstag der Anmeldung: **02.09.1998**
(21) Anmeldenummer: 98250038.1
(22) Anmeldetag: 01.02.1998
(51) Int. Cl.: A61M 5/42

(54) **Punktionsspitze an Griffel oder Teststreifen zur Blutgewinnung aus der Haut einer Lebewesens, bevorzugte Verfahren ihrer Anwendungsweise und bevorzugte Verfahren ihrer Herstellung**

(30) Priorität: 01.02.1997 DE 19705091; 29.03.1997 EP 97250104; 23.05.1997 DE 19722852; 27.05.1997 DE 19722934; 01.06.1997 DE 19723730; 07.10.1997 DE 19745600; 04.11.1997 DE 19749965; 23.11.1997 DE 19754332; 30.12.1997 DE 19758402
(71) Anmelder: Wagner, Wolfgang, Dr.med., 13503 Berlin (DE)
(72) Erfinder: Wagner, Wolfgang, Dr.med., 13503 Berlin (DE)

(57) **Zusammenfassung**

Punktionsmittel mit Spitze in Verbindung mit einem Träger zur Blut und Gewebsflüssigkeitsgewinnung aus der Haut eines Lebewesens durch plötzliche Bewegung nach Eindringen unter die Hautoberfläche in Richtung etwa längs der Hautoberfläche zur Eröffnung von Blutgefäßen, welches vornehmlich nur eine Spitze aufweist mit höchstens spitzennaher Schneide, so daß die Hautverletzung infolge der Stichwunde mit höchstens minimaler Schnittwunde ganz kleingehalten werden mit minimaler Narbenbildung.

Es werden Anwendungsweisen unter Beeinflussung vor allem der Hautspannung in Nähe des Punktionsmittels angegeben, welche an bestimmte Stiftbewegungen und Vorkehrungen in der Umgebung des Punktionsstiftes gebunden sind, um den Wundspalt soweit zu eröffnen, wie für den Blutaustritt in eine Sammelkapillare oder auf ein Testfeld für chemische Reaktionen erforderlich ist, einschließlich konstruktiver Besonderheiten, welche Voraussetzung einer taschengängigen Formgebung sind, ohne welche eine Anwendung praktisch ausgeschlossen ist. Schließlich werden noch vorzugsweise elektrolytische Fertigungsverfahren für die Punktionsspitze geschildert.

## Beschreibung

### Stand der Technik

Die Erfindung bezieht sich auf das Gebiet der Medizintechnik, im engeren Sinne auf das der Diagnostik durch Blutgewinnung zu Zwecken qualitativer und vor allem quantitativer Stoffbestimmungen vor allem aus dem Blute, aber auch aus Gewebsflüssigkeit. Sie baut vor allem auf dem Stand der Technik auf, wie er aus den Offenlegungsschriften Euro 301165, eingereicht am 1.2.89, offengelegt am 10.3.88 und Euro 0798004 A1, eingereicht am 29.3.97, offengelegt am 1.10.97 sowie DE 3925940.4 A1,eingereicht am 3.8.89, offengelegt am 31.5.90 sowie aus DE 3806574.6, eingereicht am 26.2.88 hervorgeht. Es wurde darin ein Saug-Diagnostikgerät beschrieben, worin innerhalb einer Saugglocke die angehobene Hat von einer Lanzette durchbohrt wird, welche einem Punktionsgriffel angegossen ist, welcher auch das Testfeld für die Analyse enthält und die Leitungsbahnen zu einem Meßgerät.
Ein hier weiter entwickeltes wesentliches Merkmal war, daß die Lanzette seitlich in Schwingungen gesetzt wurde, bzw. bei Verwendung einer Kanüle diese in Rotation versetzt wurde, um Gewebe zu zerstören und damit eine Blutung zu erzielen. Es wurde auch schon die Vibration in eine einzige seitliche Schnittbewegung reduziert.
Ergänzt wurde dieser Technikstand inzwischen durch die Patenteinreichungen DE 19705091.3, eingereicht am 1.2.97, welche in innerer Priorität die Anmeldung DE 19613722.5 umfaßt, und DE 19722852.6, eingereicht am 23.5.97, DE 19722934.4, eingereicht am 27.5.97, DE 19723730.4 angemeldet am 1.6.97, DE 19745600.6, eingereicht am 7.10.97, DE 197 49 965.1 eingereicht am 4.11.97, DE 197 54 332.4 eingereicht am 23.11.97 und DE 197 58 402.0 eingereicht am 30.12.97, von denen die vier letzteren weitgehend in diese Anmeldung übernommen und die Priorität zusätzlich auch aus DE 197 05 091.3 eingereicht am 1.2.97 und aus EP 97250104.3 eingereicht am 29.3.97 beansprucht wird, außerdem von DE 197 22852.6, DE 197 22 934.4, DE 197 23 730.4 und 197 45 600.6.

### Aufgabenstellung

Die Erfindung macht sich zur Aufgabe, die Punktionseinrichtung zu verbessern und zwar sollte dem Hauptziel einer schmerzarmen bis schmerzfreien Punktion bei geringer Narbenbildung, also einem minimal-invasiven Vorgehen bei Handlichkeit hinsichtlich Größe - "vor allem durch Flachform" und Bedienung der Einrichtung unter Bedingungen von Sterilität und Wirtschaftlichkeit näher zu kommen. Es galt also die Punktionswunde zu verkleinern, dennoch den Austritt des Blutes aus dem Wundspalt zu ermöglichen und auch die mechanische Zugbelastung auch nicht durch die Punktion zerstörter Hautgefäße, etwa durch Unterdruckwirkung, herabzusetzen. Insbesondere sollte bei der Einstellbarkeit der auf die Haut einwirkenden Sogstärke in Rücksichtnahme auf unterschiedliche Blutungsneigung bei den Benutzern aufwendige und störanfällige Druckreglerventile vermieden werden.

### Lösung der gestellten Aufgabe

Während die übliche Muskelpunktion etwa an den Fingerbeeren breite Stichkanäle erfordert, um das Blut abfließen zu lassen, wurde die messerartig wirkende Flachlanzette und die mehr rundliche mit breiten und schrägen Schliffkanten, einer Kanüle ohne Lumen ähnelnd, durch einen feinen Stift mit Punktionsspitze ersetzt. Durch die ruckartige Querbewegung der Punktionsspitze kann der Gefäßplexus sicher angeschnitten werden. Im Gegensatz dazu trifft beim senkrechten Einstich bis auf das tieferliegenden Hautgefäßnetz (der 100µ-Kapillaren) die Punktionsspitze nur zufällig ein Gefäß und eröffnet es. Wegen der hohen Hautelastizität legt sich die Haut dem Punktionsstift eng an, so daß ein Blutaustritt erschwert wird. Die Haut weicht aber auch hinter der Spitze der Konizität des Stiftes aus und zieht sich später wieder zusammen, so daß nur eine minimale Narbe zurückbleibt. Es ist deshalb unzweckmäßig hinter der Punktionsspitze eine Schneide vorzusehen, die etwa über die Breite von höchstens einen Millimeter hinausginge; besser bleibt eine solche unter der Breite eines halben Millimeters. Von einer üblichen (Blutentnahme-)Lanzette, wie sie bisher bekannt und üblich war, kann dann nicht mehr gesprochen werden.
Eine wesentliche Voraussetzung der Anwendung eines feinen Punktionsstiftes sind Vorkehrungen zur Eröffnung oder Offenhaltung des Wundspaltes, damit das Blut unter Sogwirkung in das Testfeld oder in Sammelkapillare im Punktionsgriffel oder Teststreifen eintreten kann. Gelöst wird die Aufgabe vorzugsweise durch Schrägstellung des Punktionsstiftes oder Punktionsmittel nach dessen senkrechtem Eindringen in die Haut, wodurch Hornhaut und die tiefere noch von dem verhältnismäßig nur eine kurze Strecke, ungefähr drei Millimeter in den Gefäßplexusbereich vorstoßenden Spitzenbereich des Punktionsmittels erreichte Hautschichten den Wundspalt sperrend auseinander gedrängt werden. Dabei spielt der Spannungszustand der Haut, der ja von der in der Saugglocke einwirkenden Unterdruckhöhe abhängig ist eine bedeutsame Rolle, weil ja durch die Zugwirkung des Soges die Elastizitätsreserven der Haut lokal herabgesetzt werden. Auf Beispiele zur Verwirklichung dieser Schräglage des Schaftes des Punktionsstiftes wird später noch eingegangen.

Die Entwicklung eines maximalen Anfangssoges, um die doch individuell sehr verschieden dehnbare Haut (schon wegen Alterung und Fettschicht) möglichst hoch in die Saugglocke hineinzuziehen, ist von der Pumpengestaltung und deren Lage zur Saugglocke ab. So entfallen bei Montage der Saugglocke innerhalb eines Paltenbalges sogbeeinträchtigende Rohrverbindungen. Eine Kolben-Zylinderpumpe ist auf gleichem Raum wirksamer als ein Paltenbalg mit seinem Totraum.
Die gestellte Aufgabe wird im Bereich der Sogstärkeregulierung dadurch gelöst, daß die Federung zur Bewegung der Sogpumpe, insbesondere der Ausdehnung eines Paltenbalges, wenigstens teilweise nach außerhalb dieses Faltenbalges verlegt wird und dabei durch eine Art verstellbare Distanzverbindung zwischen dem bewegten Ende des Faltenbalges - es kann dies dessen (oberer) Deckel- oder (unterer) Bodenbereich sein (in diesem Falle mit beweglichem Schlauch- oder Rohranschluß zur Saugglocke hin) - die Ausdehnung des Faltenbalges bzw. die Betätigung der Vakuumpumpe verzögert mit der Wiederausdehnung der Feder und damit verkürzt bewirkt. Diese Verkürzung der Ausdehnungsstrecke des Paltenbalges oder einer Pumpenkolbenbewegung bedeutet aber eine einstellbar geringere Sogentwicklung, die durch Luftabsaugung aus der Saugglocke dort auf die Haut wirksam wird. Es ist dabei nicht wesentlich, ob es sich bei der Federung um Federn handelt, die um den Pumpenzylinder oder den Faltenbalg herum oder in dessen Nähe montiert sind oder um einen von einer Torsionsfeder betriebenen Drahtbügel in Art einer inversen Mausefalle oder der Mechanik eines Fahrradgepäckbügels (kräftig in der Bewegungsendphase). Wesentlich ist daß an einem Bügel oder Deckel eine ungefähr konstante Bewegungsstrecke einstellbar ist, nach welcher ein Raster für die Auslösung der seitlichen Ablenkung des Punktionsmittels oder zur Bewegung der Punktionsspitze überhaupt ausgelöst wird,
die eben nicht völlig an das Ausmaß der Pumpenentfaltung und Sogentwicklung gebunden ist.
Um den Innenraum des Pumpenzylinders oder des Paltenbalges zu nutzen kann dabei dort zusätzlich eine Druckfeder plaziert werden, welche der Einhaltung eines gewissen Enddruckes nach der Vergrößerung des Pumpenraumes zu sichern hilft.
Die Steuerung des Soges mittels verstellbarer Ausdehnungsbewegung des Pumpenraumes wurde schon für Saugin jektoren bereits ab 1974 beschrieben, aber meiner Erinnerung nach ohne diese Möglichkeit, die Garantie eines gewissen Unterdruckes in der Saugglocke durch die Pumpenraumvergrößerung vor der Injektionsauslösung von der absoluten Länge dieser Entfaltungsstrecke abzukoppeln.
Ein weiteres Problem stellte aus hygienischer Sicht der Einführungstrichter für die Teststreifen oder Punktionsgriffel dar, welcher bis nahe an den austretenden Blutstropfen herangeführt und dabei leicht verunreinigt wurde. Er wurde für den Fall des Schwingprinzipes für die Punktionsspitzen- oder Teststreifenbewegung dadurch gelöst, den Einführungstrichter in Wegfall geraten zu lassen. Dies wurde ermöglicht durch eine exakte Führung des Griffels oder Streifens im Einführungsschacht und verläßlich starre Struktur des Testreifens. Die Einführung des stark ver-kürzten Teststreifens - denn es galt ja die Saugglockenhöhe zu beschränken möglichst ohne die Punktionsspitze quer zur Teststreifenlänge anordnen zu müssen - ließ sich durch einen Verpackungsstreifen ermöglichen oder doch erleichtern, der sowohl das Einschieben des Teststreifens mit seinen Ableitungskontakten in den Schacht der Deckelbuchse gestattete, als auch das Wiederherausziehen des Streifens nach Gebrauch. Dieser Zangeneffekt wird seit Jahrzehnten bei Kanülen mit Plastikansatzkonus hinsichtlich von deren Schutzhülsen angewandt, wo dieselbe Problemlage gegeben ist.

Die Aufgabe wurde dadurch gelöst, daß zwei elastische Lamellen etwa aus Pappe oder vorzugsweise aus Plastik wenigstens den punktionsspitzennahen Teil des Teststreifens zwischen sich schließen können. Sie liegen innerhalb einer äußeren gemeinsamen Schutzhülle und sind mit einer gegen den Teststreifen auf wenigstens einer Lamelle vorspringenden Randleiste oder sonstigem Vorsprung vor einer Noppe oder sonstigem Vorsprung des Teststreifens gelagert. Bevorzugt kann auch eine verkürzte Rand- oder Rahmenleiste auf einen seitlichen, vorzugsweise abgerundeten oder angeschrägten Randvorsprung einer der beiden Außenkanten des Teststreifens treffen. Solche Hindernisse erlauben es, den Teststreifen mit dem spitzenabgewandten Ende in den Schacht der Buchse für die Signal-weiterleitung (etwa unter dem Saugglockendeckel) einzuschieben und dann die den Teststreifen einscheidenden Folienblätter, die an ihrem Ende zusammenhängen, wieder abzuziehen. Weist die längere Rahmenleiste eine hakenförmige Krümmung nach innen auf, so kann dieser Haken über den Randvorsprung des Teststreifens bei um 180 Grad gedrehtem Aufschieben des Folienblattpaares auf den Teststreifen nach dessen Gebrauch hinweggeschoben und der Teststreifen mit dem Folienblattpaar herausgezogen werden. Analog - aber ohne Achsendrehung - gelingt das Herausziehen des Teststreifens bei der ersteren Variante nach Überqueren der Noppe auf der Teststreifenoberfläche durch die gegen sie vorspringende Randleiste des Folienblattpaares. Schließlich ist eine Kante oder Leiste an einem der Folienblätter nicht erforderlich, wenn nach Überholen der Randleiste oder Noppe das ende des Teststreifens mit der Punktionsspitze tiefer in den festklemmenden Spalt zwischen den Folienblätter gezwängt und dort festgeklemmt wird, was durch ein Klebemittel dort noch unlösbar (für die Punktionsspitzenentsorgung) verstärkt werden kann. Es kann dabei auch eine Noppe auf einem der Folienblätter in ein Loch im Teststreifen eindringen; eine Anordnung, die ebenfalls auch ohne zusätzliche Klemmung dem Herausziehen des Teststreifens aus dem Schacht des Punktionsgerätes dienen kann.
Mittels der elektrolytischen Zersetzung kann nicht nur eine Stahlspitze, sondern auch eine Schneide auf einem kurzen Endteil eines Stahlstiftes gefertigt werden. Hierzu wird ein entsprechend langes Ende des Stahlstiftes spatenartig verbreitert etwa in einer Druckgußform in noch glühendem Zustand nach oder während der Strangextrusion ausgeformt und eine weitere größere am spitzenabgekehrten Stiftende aber mit seiner größten Breite um 90 Winkelgrade zum späteren Schneidgrat gedreht. Die richtige Winkelstellung der spitzennahen Punktionsschneide bei und nach der Stiftbefestigung auf dem Teststreifen oder Träger kann so gesichert werden.
Auch Stahlstifte ohne Schneide können auch vor der Befestigung am Teststreifenmaterial elektrolytisch zugespitzt und dann erst mit dem Teststreifen- oder Trägermaterial vereinigt werden. Es kann hierzu eine in ihren Einzelgliedern höhenverschiebliche Kette von Stiftaufnahmen für -etwa aus einem Stapelmagazin abgegebene - Stahlstifte über eine Richtleiste gezogen werden, die nach unten hin die Stiftenden aus den Aufnahmemulden herausstehen läßt. Die Stahlstifte werden nach Lagerung in ihren einseitig offenen Befestigungsmulden erhitzt und dehnen sich aus, was wiederum ein Herausfallen aus den Lagerungsmulden verhindert. Ohne stehende Bodenleiste wird die Kette dann über das Säurebad geführt, wo die Einzelglieder nach unten verschoben werden, so daß die Stiftenden in die Säure eintauchen. Über eine an seitlicher Keilführung angehobenen Querstifte an den Einzelglieder werden die angespitzten Stahlstifte angehoben, ehe sie das Säurebad verlassen. Noch erhitzt werden sie mit den Teststreifen oder dessen noch nicht ausgestanztem Folienband zusammengebracht und diesem nach Abkühlung diesem je einzeln ein- oder angeschmolzen. Die minimale Stärke der angespitzten Stahlstifte kann vermutlich nicht weit unter diejenige einer Kanüle herabgesetzt werden, da ansonsten bei der ruckartigen Seitverschiebung eine zu starke Verbiegung auftritt; andererseits ist aber die Bruchgefahr gegenüber einem Kanülensegment herabgesetzt. Die Lösung der Aufgabe, den Wundspalt für den Blutaustritt offenzuhalten, wurde weiter über den Mechanismus einer stärkeren Nachführung der Punktions-spitze nach der Ruckbewegung - beides tangential zur Hautoberfläche - erleichtert. Es kann nämlich eine Fixation der Haut in unmittelbarer Nähe der bewegten Punktionsspitze durch (wenigstens relativ zu dieser Spitze) unbewegte Vorsprünge aus diesem Teststreifenende vorgenommen werden. Hierzu können benachbarte oder dem Teststreifen angelagerte Zungen auf die Haut gesenkt werden, welche die bewegte Punktionsspitze klammerartig umrahmen. Für einen in kleiner Sektorbewegung ruckartig rotierenden Teststreifen mit exzentrischer Punktionsspitze genügt ein zentrischer Aufstützpunkt am Teststreifenende zur Hautfixierung. Eine Sonderlösung stellt das Einstechen zweier schräg gestellter Punktionsspitzen in äußerster Annäherung ihrer Spitzen in die Haut dar, welche durch einen Keil ruckartig auseinandergetrieben werden und dabei einen Hautschlitz durch Dehnung erzeugen.
Da die quere Schnittbewegung der Punktionsspitze in der Regel in einer zentralen Eindellung der in der Saugglocke hochgehobenen Haut erfolgt, werden von einer einfachen Punktionsspitze verschiedene Tiefenbezirke der Haut berührt. (Die Hautbeschaffenheit ist intra- und interindividuell so variabel, daß bei konstanter Punktionsspitzenlänge ansonsten das Anschneiden eines Blutkapillarkomplexes ausreichender Stärke nicht gewährleistet wäre).
Für die Rotationsspitze kann dieser Effekt über die Absenkung der Schafthalterung im Saugglockendach während der Drehung gesichert werden. Dabei kann die Halterung innerhalb der Saugglocke gegen diese - etwa über eine Stiftführung in einer Rillenführung - gegen die Haut abgesenkt (oder angehoben) werden. Es kann auch die Rotation von Feingewindesegmenten außerhalb auf der Saugglocke, die zugleich der Höhenverstellung der Punktionsspitze vor Benutzung dienen kann - durch eine zentrale Dichtung im Deckeldach der Saugglocke hindurch sich der Schachthalterung für den Test-streifen mitteilen. Es wurde die Möglichkeit aufgezeigt, den Druckgeber auch bei auf eine Rotationsspitze mittels durch die Saugglockenwandung gedichtet geschobenen Stößels einwirken zu lassen. Das Stößelende für die Schwingspitze erhielt vorzugsweise eine metallene Schutzhülse aufgeschoben, von der eine Zunge herabreicht, deren Kante auf den Teststreifen aufschlägt. Hierdurch kann die Stößelachse höher in die Saugglocke verlegt werden und so die in der Saugglocke aufsteigende Haut weniger behindern. Besagte Zunge wiederum kann in die von der Punktionsgriffel eindrückte Eindellung hineinragen. Die Schutzhülse kann zur Reinigung leicht abgezogen werden.
Eine letzte Sicherheit bietet die sog. Nachschnittaste für den Fall, daß das über eine Art Zwischenkabel von den Kontakten im Schacht unter dem Saugglockendeckel angeschlossene elektrische Meßgerät (etwa Glukometer) nach einer Punktionsspitzenverschiebung keinen Bluteintritt auf das Testfeld meldet. Varianten wurden nun auch zu dieser Nachschnittaste angegeben, die einen Überholmechanismus für die Federspannung im Druckgeberbereich für die Punktionsspitze vereinfachen oder vermeiden.

Für die Nachschnittaste wurde eine Keilführung zur Federspannung des Druckgebers vorgeschlagen, deren Überbrückung während der Wiederanhebung der Taste zur Vermeidung einer Abbremsung des Druckgebers durch die Mithochnahme einer die Keilschräge überbrückenden Hülse vermieden wurde.
Eine gestaffelte Schubeinstellung unter Doppelnutzung eines einzigen Stößels wird dadurch ermöglicht, daß eine Art Klapprahmen zwischen hintere und vordere Einstellschraube auf den Stößel herabgesenkt wird, welche für die Stößelbewegung in Richtung Punktionsspitze als Begrenzung dient. Der Klapprahmen wird aber von einer Zugverbindung zum Deckel der Saugpumpe nach dem Stößelschlag wieder angehoben, so daß die Stößelhemmung durch die hinterste Einstellschraube entfällt und diejenige für den Nachschnitt wirksam wird.
Eine Stößelrückführung ist durch das Vorbeischieben einer Rückstellfeder mit dem Schluß des Saugglockendeckels möglich. Es werden Möglichkeiten der Nachfederung des Stößels zur Hautschlitzerweiterung nach dem gefäßzerstörenden Schlag aufgezeigt.
Hinsichtlich der Auslösung der Saugpumpe wurde auf eine solche durch Auslöserhebung beim Aufsetzen der Saugglocke auf die Haut für Injektoren aus DE 2551993.1 angemeldet am 19.11.74 zurückgegriffen. Jedoch wurde der Zweipunkt oder Dreipunktauslöser vom Saugglockenrand auf die Gerätefläche unterhalb des Faltenbalges als Sauggpumpe - als unter den Einsatzort direkt - verlagert. Die gesamte Punktionseinrichtung wurde in einen einstellbar rückfedernden Rahmen gesetzt, um das Einziehen der Haut unter Sogwirkung am Saugglockenrand vorbei nicht von der Druckanwendung von Seiten des Patienten abhängig zu machen. Die visuelle oder meßoptische Hautkontrolle kann durch oder unter einem durchsichtigen Saugglockendeckel erfolgen. Es kann dafür auch eine Art Lochblende von unten in die Saugglocke eingeschoben werden, die auch zugleich der Fixierung der die Punktionsspitze umgebenden Haut dienen und zur Reinigung leicht herausgezogen werden kann.
Die unterhalb des Faltenbalges nach unten herausziehbaren Auslösestangen können aber auch als Teleskophülsen oder -schienen verlängert zur Hautdistanzierung für die visuelle Hautkontrolle eingesetzt werden. Es wird dann von der Nähe des Saugglockenrandes aus das Licht beispielsweise dreier Lämpchen auf das Hautzentrum unterhalb der Saugglocke gerichtet und damit die spätere Punktionsstelle sichtbar und kontrollierbar gemacht. Der Patient braucht dann das Gerät nur noch abzusenken und damit die Hautansaugung und letztlich die Hautpunktion auszulösen. Der Stromkreis für die Lichtversorgung wird zweckmäßig durch Kontaktschluß erst bei vollkommen ausgezogenen Auslösern geschlossen.
Für die Auslöser wurden beispielhaft zwei Mechanismen angegeben, welche eine Auslösung durch Anhebung der Auslösestangen nur erlauben, wenn die Auslösertangen zuvor ausgezogen waren. Die Druckfeder oder die Druckfedern für die Saugpumpe können aber bei Andruck des Gerätes (und auch der Auslösestangen) gespannt und ihre Deckelstangen eingerastet werden. Hierzu wird im einen Falle die Hülse mit dem Raster für die Deckelstangen der Saugpumpe nach dessen Auslösung über einen Federschieber, der durch einen Stift vom abgesenkten Deckel her aktiviert wird, zur Rasterverriegelung abgesenkt. Die Federspannung erfolgt aber wesentlich durch die Anhebung der Hülse mit dem Raster selbst gegen eine Keilführung am Federschieber in der Phase der Anhebung zur Entriegelung, ist also auslösestangenzentriert.

In der zweiten Lösung bewirkt die Absenkung des Deckelstiftes gegen die Keilschräge eines Federschiebers eine Anhebung der Hülse mit der Rastermulde für die Deckelstangen. Stoppt den Federschieber aber bis zur völligen Deckelabsenkung unter Entrasterung der Deckelstangen. Die Hülse mit dem Raster ist zugleich ein Schub-Torsionsglied, welches die Rastmulde bei seiner Anhebung von dem Schaltglied für die Rastnuten in den Deckelstangen wegdreht und diese deshalb verriegelt. In diesem verriegelten Zustand senken die im Fußteil drehbaren Auslösestangen mit und können ohne Auslösung der Saugpumpe abgesenkt werden. Bei ihrer Anhebung bewirken sie wieder eine Sektordrehung der Hülse mit der Rastermulde in deren Ausgangsposition, was bei maximaler Anhebung die Auslösung der Deckelstangen zur Folge hat.
Einfacher werden bei der Absenkung des zur Grundplatte rückgefederten Rahmen auf die Haut durch Verschiebung der Auslöserplatte Sperrung der Auslöserplatte dort die Verrasterungen mit den Hülsen- oder Stangenverbindungen zur Deckelplatte gelöst, so daß die Starke Druck- oder Torsionsfeder den Faltenbalg zur Sogerzeugung nach oben zieht und ausdehnt.
Da aber der Rahmen oder Kasten nach Spannung jener Feder unter Absenkung der Deckelplatte wieder hochfedern muß, ist eine Überholklinke erforderlich, die das zuläßt. Außerdem muß ein Sperrknopf, der gegen die Auslöserplatte gefedert ist diese bis kurz vor der Punktion festhalten. Die Auslösung dieser Sperre erfolgt zweckmäßig über eine Zugverbindung des Sperrknopfes zum Haltebügel hin bei dessen Aufrichtung in die Vertikale.
Es bleibt innerhalb der Punktionseinrichtung sowohl in den Zwischenräumen zwischen Auslösern und Faltenbalg als auch bei der möglichen Straffung der Konstruktion hinter der Saugglocke genügend Raum, um das Meßgerät in das Punktionsgerät völlig zu integrieren, so daß dann auch die Herstellung einer Steckerverbindung für die Signalleitungen zum Meßgerät entfällt.

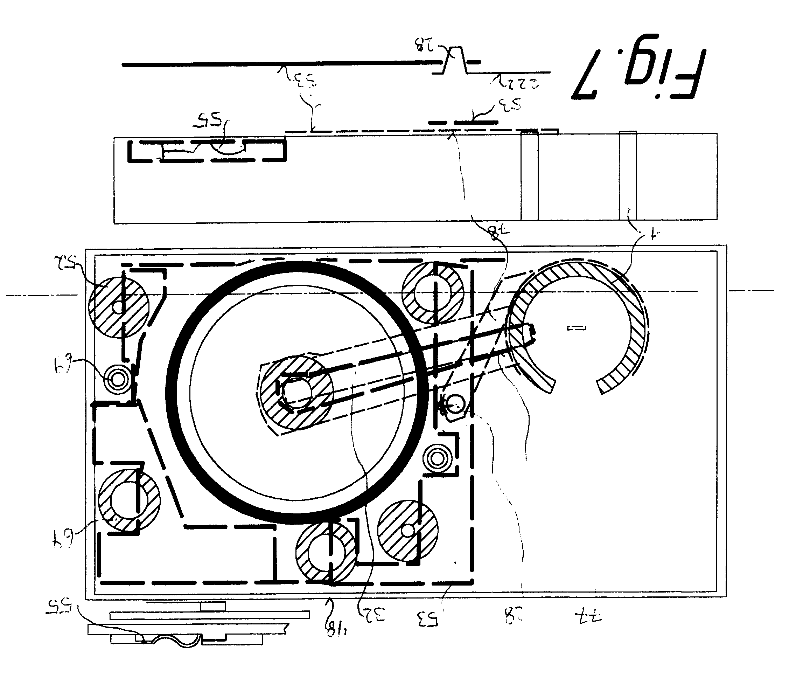

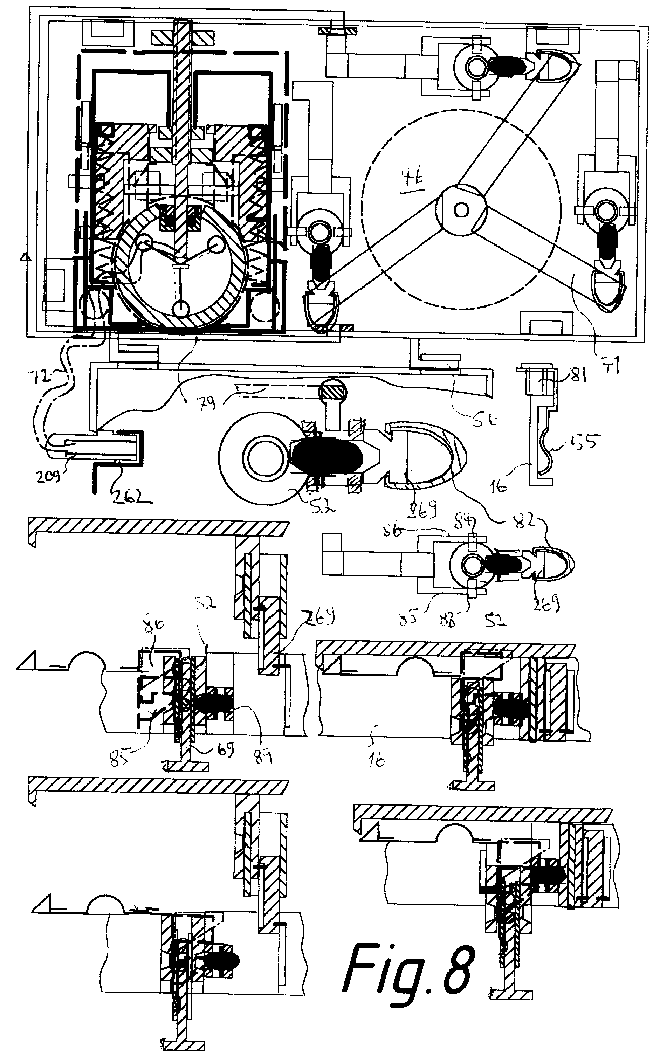

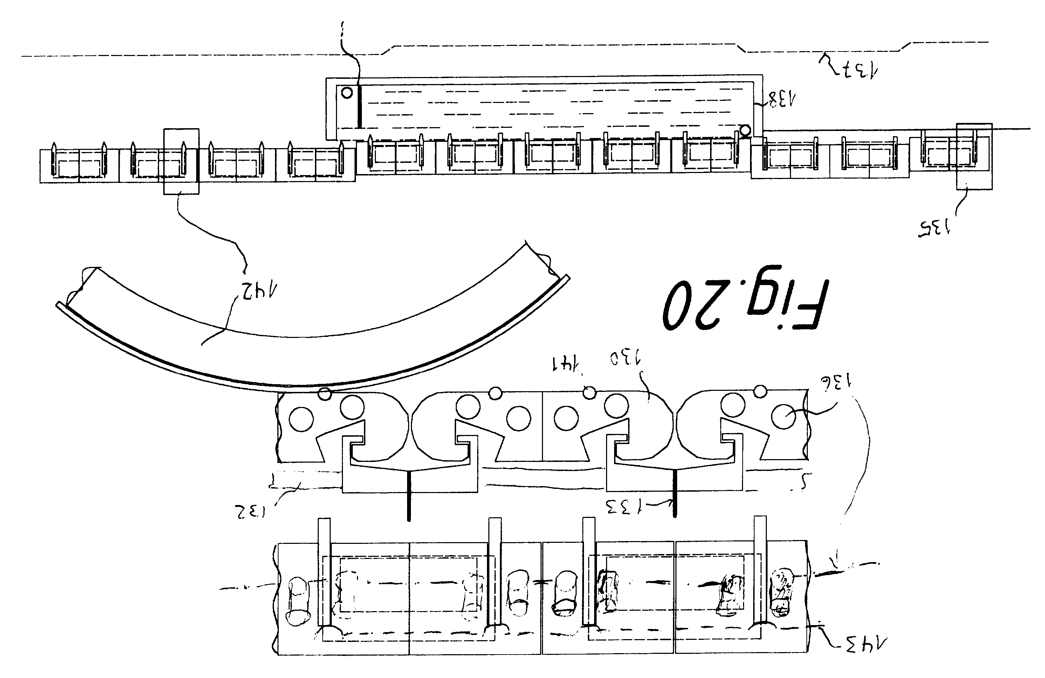

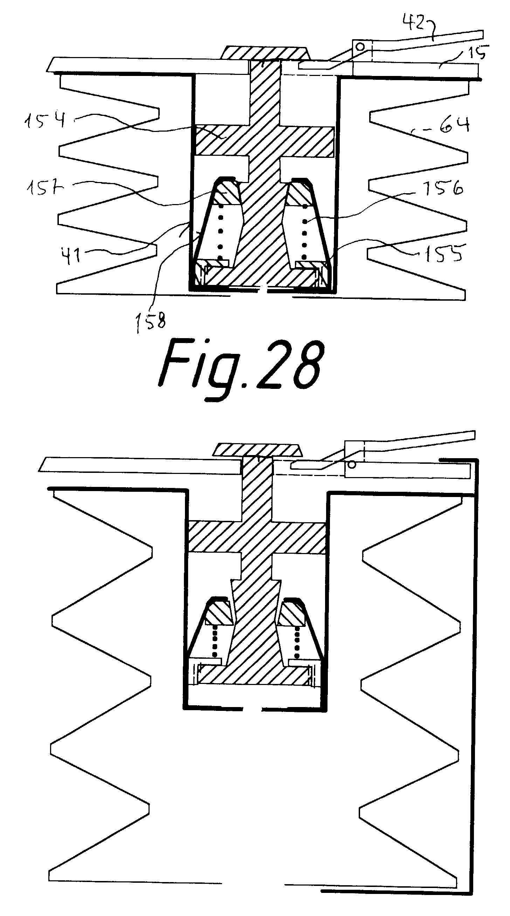

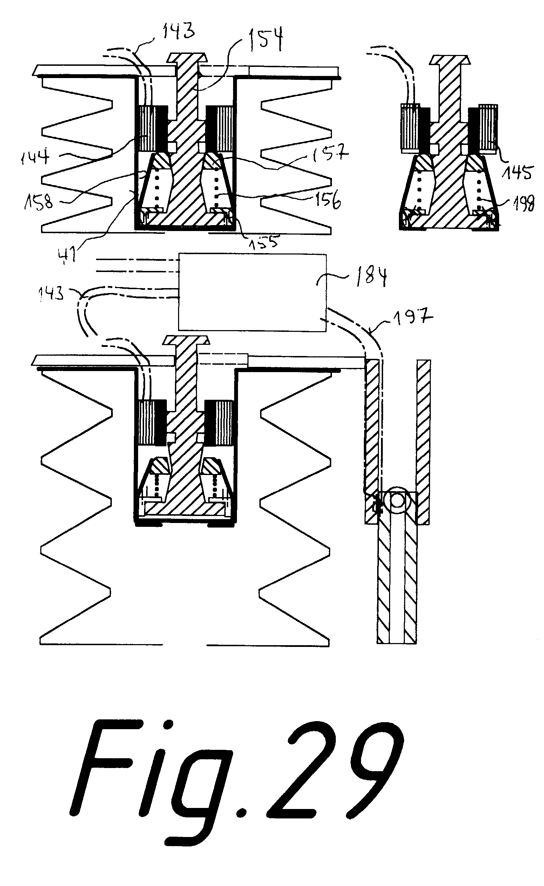

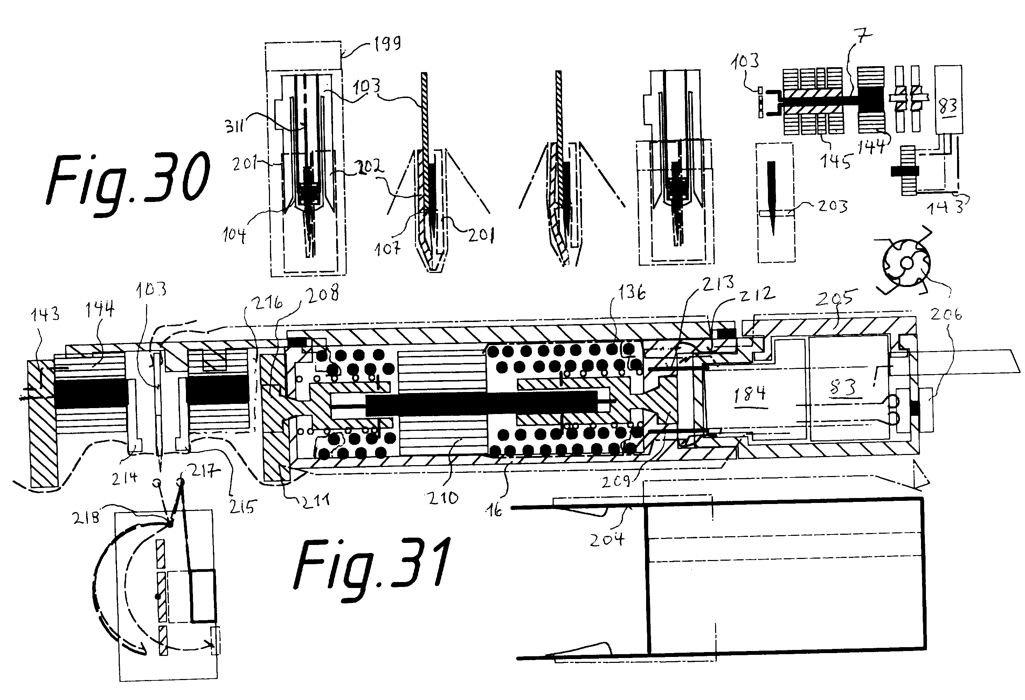

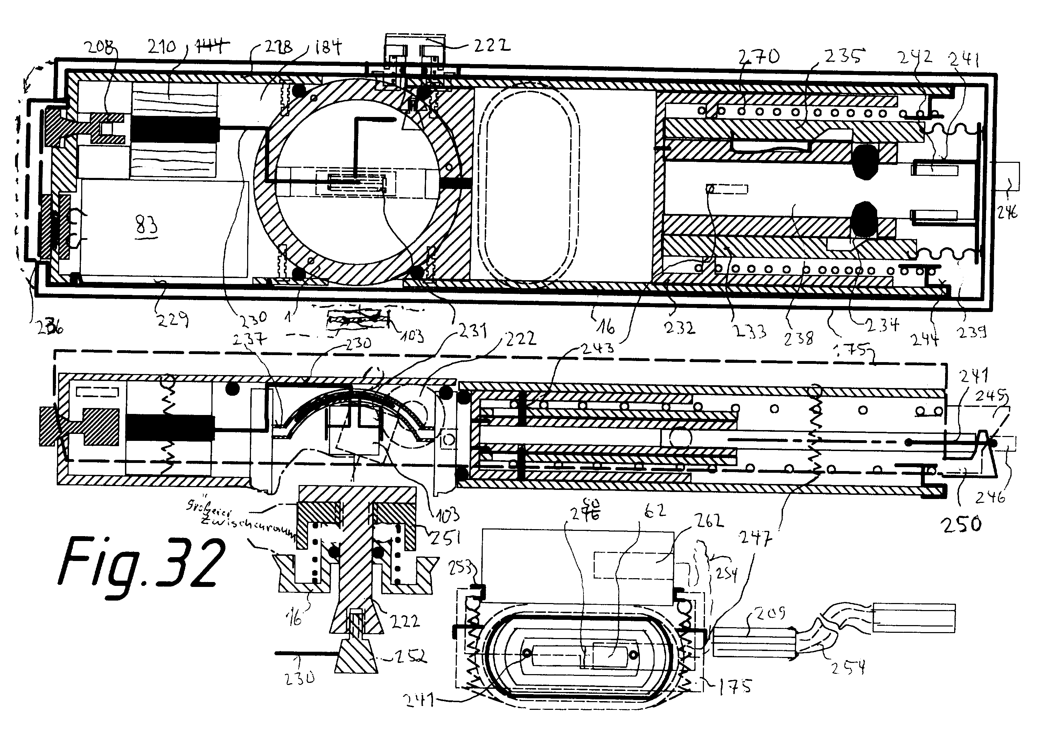

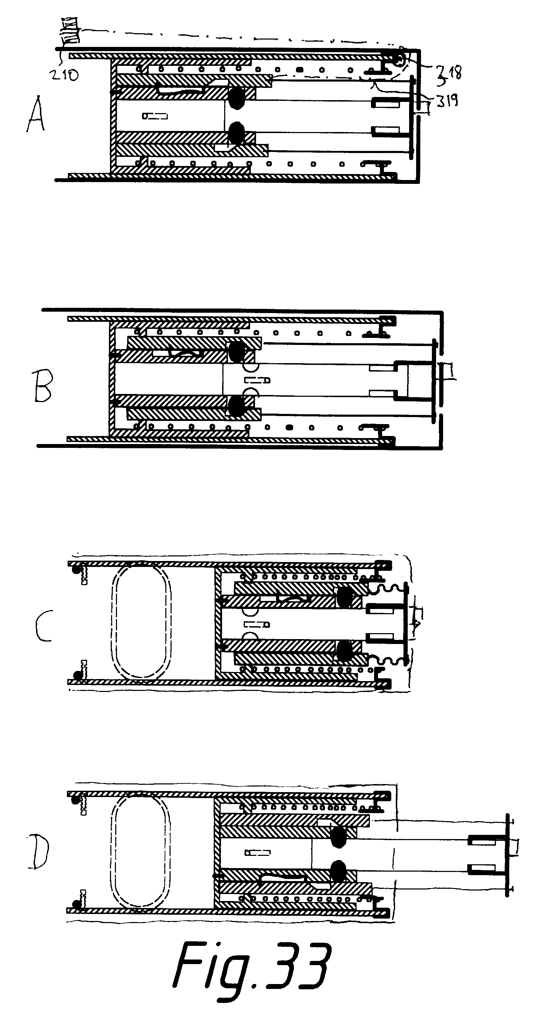

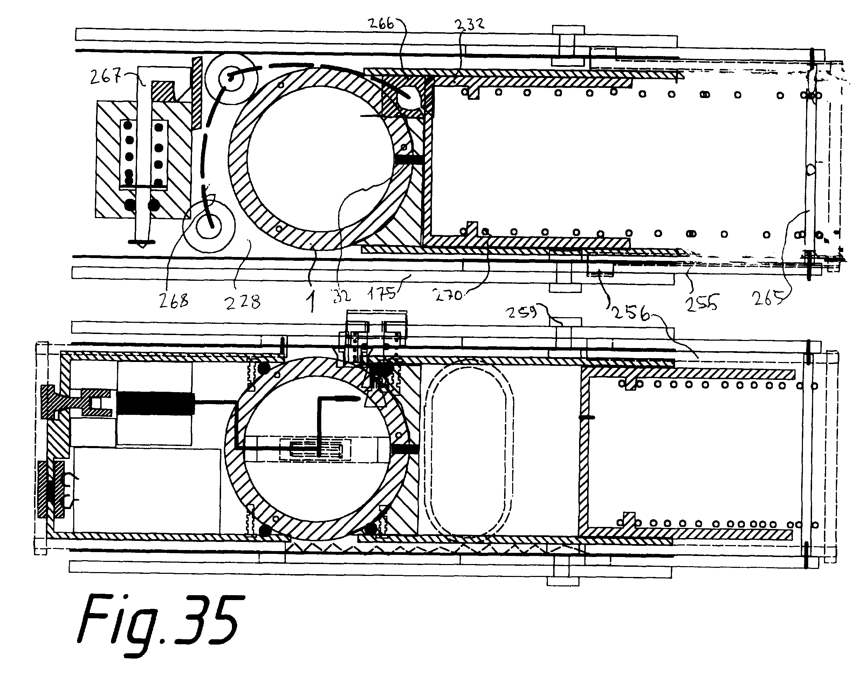

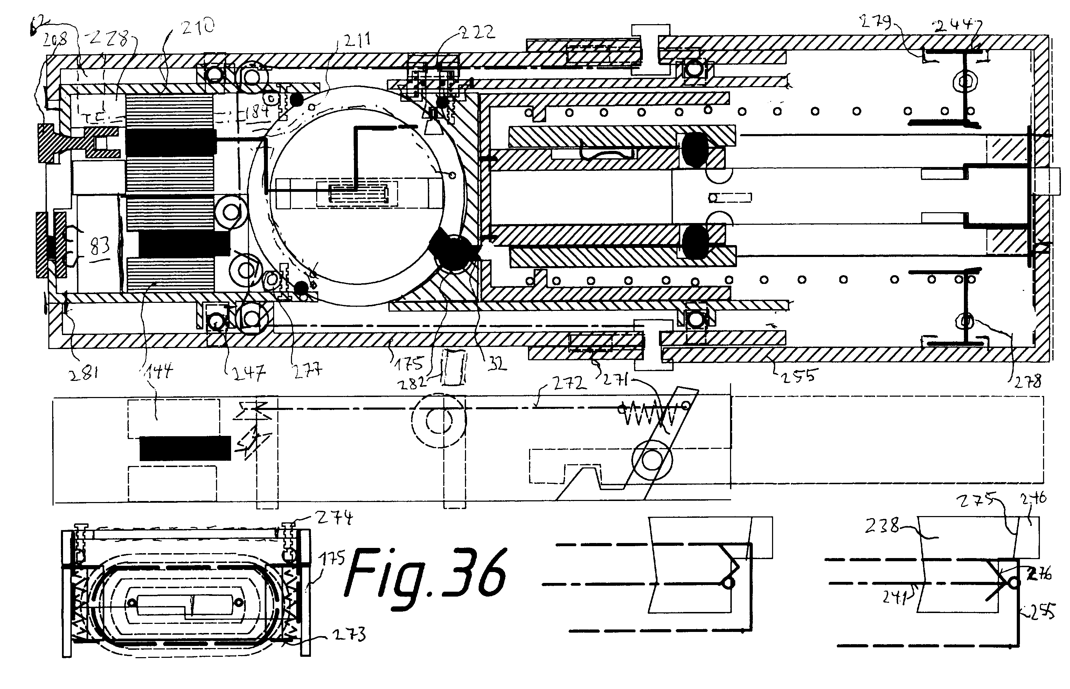

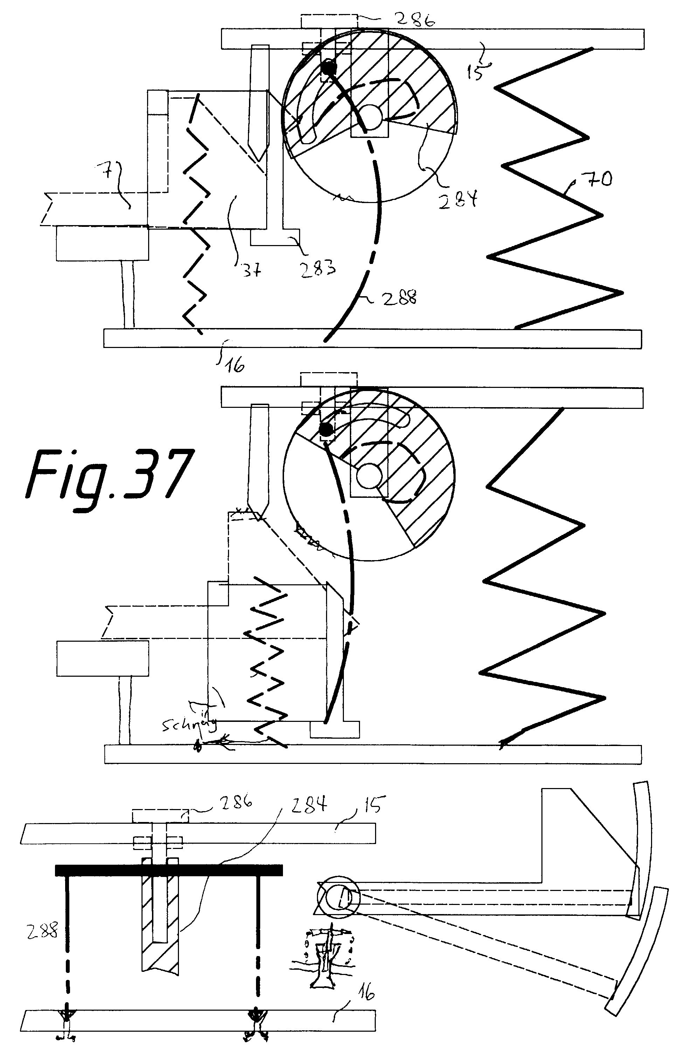

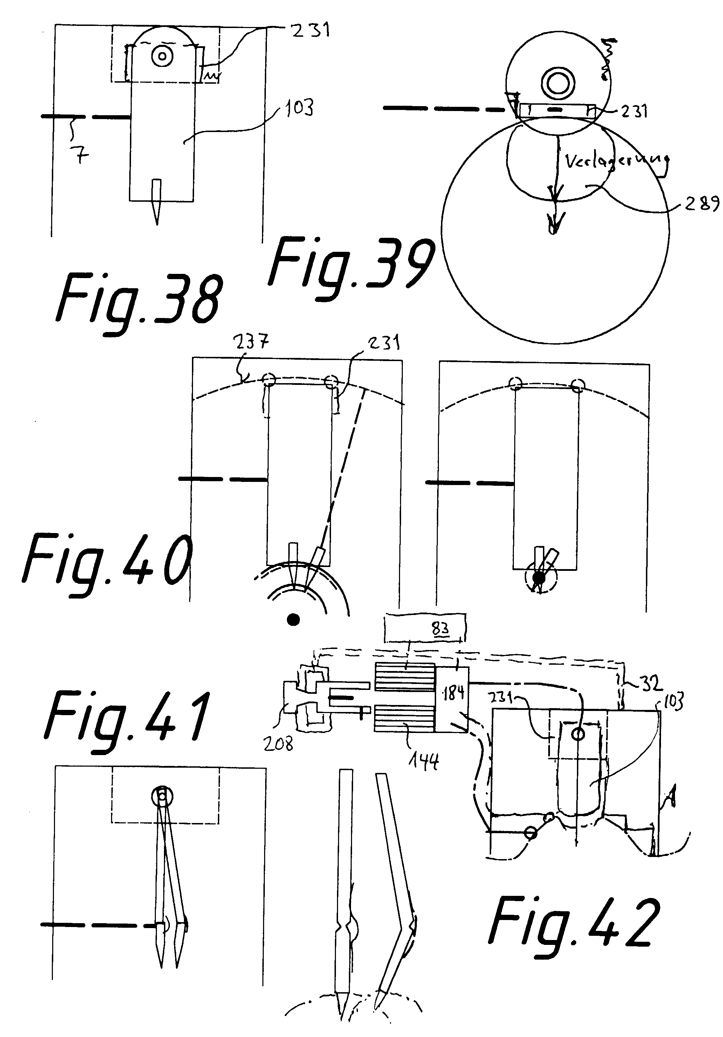

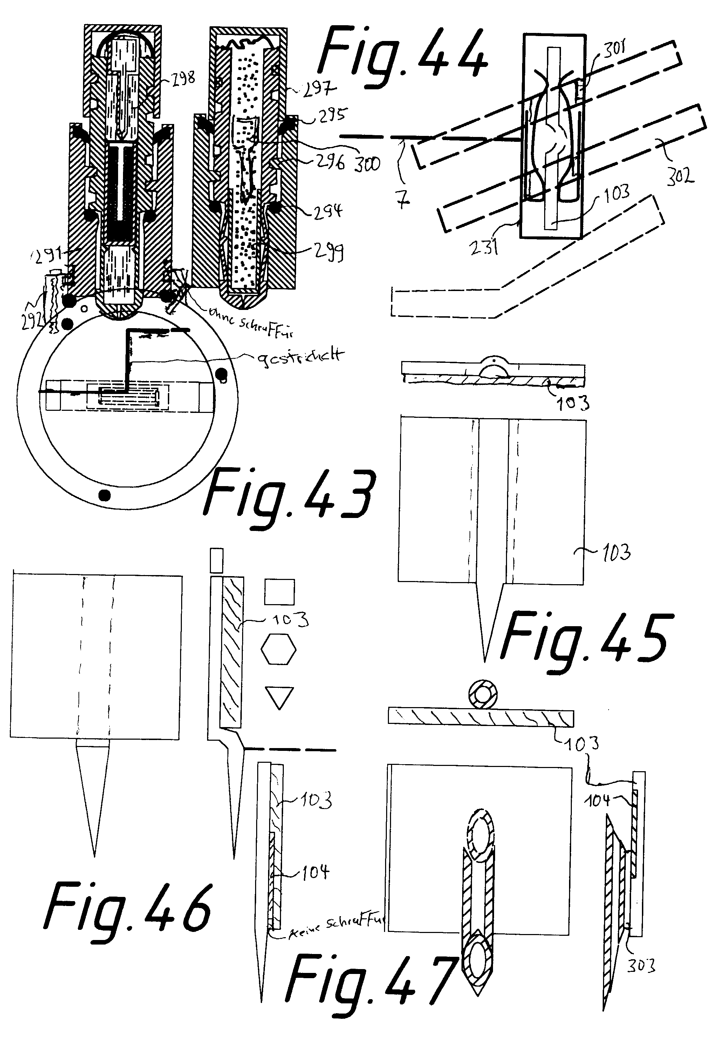

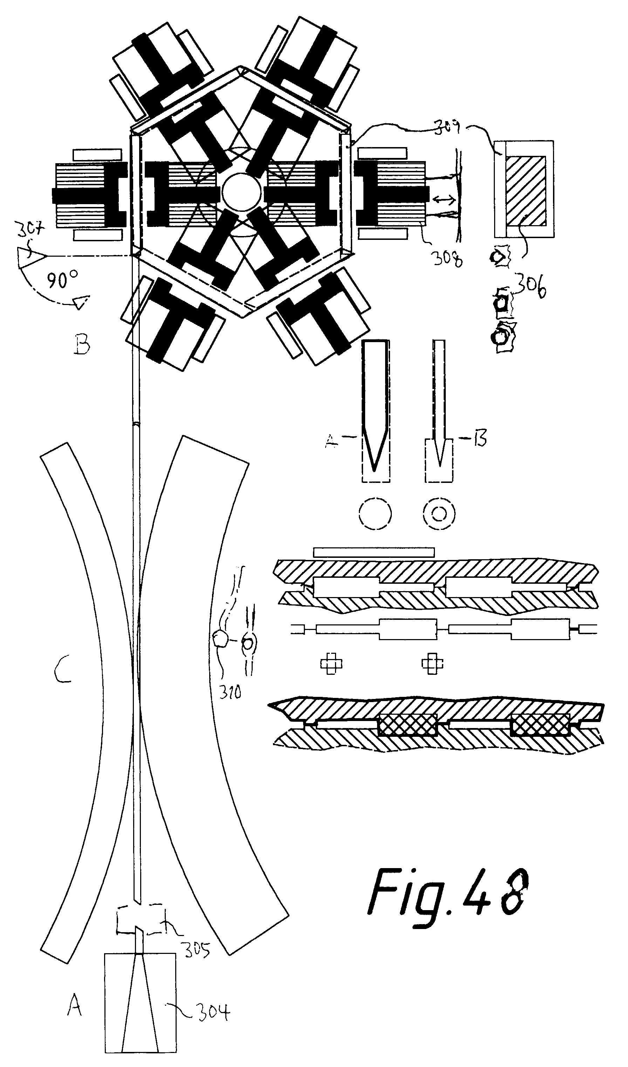

Die Hauptaufgabe der Sogregulierung kann auch für mechanische einrichtungen mit Auslösungssteuerung der federbetriebenen Punktionsspitzenbewegung mittels Stößels quer zur Haut über die Endbewegung der starken Druckfeder für die Saugpumpe wird auch so gelöst, daß der Auslöser selbst mit der Verkürzung der Pumpenausdehnungsstrecke verschoben wird. Dies wird am Beispiel einer Stößelauslösung über einen rückgefederten Rasterstift gezeigt, wobei die Rückfederung die Behinderung der Rückführung des Stößels verhindert. Die Auszugsstrecke des Rasthebels wird gemeinsam mit der Seil- oder Stangenverbindung der unter Federruck stehenden Deckelplatte unter Verschraubung verkürzt.
Dabei kann vorübergehend auch eine rückgefederte Ventilklappe geöffnet werden(vgl.Fig.37).
Es ist nämlich eine sehr nützliche Weiterentwicklung des Erfindungsgedankens, die volle Sogkraft der Einrichtung für das Einziehen der Haut in die Saugglocke zu nutzen, um diese beim Einstich der Punktionsspitze maximal gespannt zu halten und die Blutleere zu begünstigen. Für die darnach folgende Phase der Blutfülle dagegen wird dann Luft in die Saugglocke gelassen, um während dieser etwas längeren Phase bis zur Sättigung der Kapillaren oder des Testfeldes mit Blut die Blutkapillaren nicht zu strapazieren und Hauteinblutungen seltener zu machen.
Für den Fall der Verwendung eines Faltenbalges als Saugpumpe wird der zur Veringerung des Totraumes eingestülpte Topf mit der Deckelplatte von der starken Druckfeder gehoben. Es kann nun die Öffnung des einstellbar federbelasteten Ventiles innerhalb des Topfes während des Aufsteigens der Deckelplatte verhindert und so die etwaige Öffnung für die Luft zur Sogminderung verzögert werden. In einem federbetriebenen Beispiel wird der zentrale Ventilstößel von der Deckelplatte aus seinem Ventilsitz gehoben, dessen Ring er aber über eine Gegenkonizität verschließt. Der Topf des Faltenbalges wird mit letzterem über Zug am Ventilring gehoben, wobei sich die Zugwirkung über eine Membran über einer schwächeren einstellbaren Druckfeder auf den durchbohrten Topf auswirkt. Bei erreichtem Höchststand des Topfes wird die schwächere Druckfeder unter dem Ventilring wirksam und hebt diesen in die obere Konizität des Ventilstößels. Es stellt sich ein Verschlußzustand erst bei Gleichgewicht zwischen der Anspannung der schwächeren Druckfeder und Sogwirkung von unten ein(vgl.Fig.28). In einem zweiten Beispiel wird das Ventil im Topf mittels eines Elektromagneten während der Ausdehnung des Faltenbalges bis zu dessen voller Entfaltung verschlossen gehalten. Über elektrischen Kontaktschluß (oder Stromunterbrechung) bei diesem Hochstand der Deckelplatte erhält der Elektromagnet den Befehl zur Ventilöffnung (oder wird alternativ in seiner Funktion gestoppt, vgl.Fig.29). Das Belüftungsventil im Topf kann auch manuell unabhängig von der Federeinstellung zur Wiederbelüftung der Saugglocke geöffnet werden.
Wird ein Elektromagnet zur Betätigung eines Belüftungsventiles eingesetzt, so kann als Sogschalter für die Unterdruckmessung die Haut in der Saugglocke dienen, in dem wenigstens an zwei verschiedenen zwangsläufigen Berührungspunkten der Hautglocke mit Gehäuseteilen und/oder Punktionsspitze oder deren Trägermittel in einer Art Erdschaltung der Hautkuppenstand gemeldet wird. Die elektronische Steuerzentrale kann bei Meldung eines hauthochstandes den Elektromagneten zur Ventilöffnung anregen in Dauer- oder besser in Einzelimpulsen; vorteilhafterweise wird über die Punktionsspitze selbst der weiter erforderliche Hautkontakt der Steuerzentrale gemeldet und von dieser eventuell eine Störmeldung veranlaßt. Bei Unterschreitung eines gewissen Mindestsogpegels wird der Elektromagnet jedenfalls bis zur Erfolgsmeldung des Meßgerätes abgeschaltet und das Belüftungsventil geschlossen. Wird der Unterdruck durch Glühdrahtbeheizung des die Saugglocke umgebenden Gehäuses erzeugt, so kann ein einziger doppelläufiger Magnet sowohl das Ventil zur Saugglocke hin als auch zur Außenluft öffnen(vgl.Fig.31).
Für eine größere Sogkraft bei kleinerer Bauform wird eine liegende Kolbenpumpe vorgeschlagen. Neben der früher schon erwähnten Reihung dreier Kolben-Zylinderpumpen eignet sich eine einzige Kolben-Zylinderpumpe von ovalärer Formgebung besonders, weil dabei eine zentrale Sperrklinke für die Abstützung von Antriebsfedern für den Kolben genügend Platz finden kann(vgl.Fig.32). Ein Teleskophülsen- Schiebersystem erlaubt eine Baulängenherabsetzung. Aus einem zusammengeschobenen Zustand kann mit einer einzigen Zugbewegung auch die Rahmenkonstruktion zur federnden Hautauflage verlängert werden und in diesem Zustand einrasten(vgl.Fig.35,36). Um die Bedienung zu vereinfachen, ist in einem Spezialfall ein zusätzlicher Elektromagnet für die Entriegelung des Rahmens vorgesehen. Der doppelläufige Magnet für die quere Punktionsspitzenbewegung und für die Belüftung muß für ein feines Funktionsspiel zur Unterdruckregelung frei bleiben(vgl.Fig.42) Der federnde äußere Rahmen muß vor seiner Absenkung zur Punktion den zentralen Stempel gegen die starke Druckfeder für den Kolben festhalten, außerdem wird der den Stempel umgebende Schieberaster zur Kolbenauslösung herausgezogen. Der äußere Rahmen kann geradwandig aber auch gekrümmt konstruiert werden; das Meßgerät wird vorzugsweise oben in eine Schiene zwischen den Holmen des Rahmens eingeschoben; es kann aber auch seitlich, etwa in ein elastisches Band auf der Gegenseite der Stempelöffnung eingesteckt werden(vgl.Fig.32). Ein Zwischenkabel kann die elektrische Verbindung zwischen Punktionsgeräte- und Meßgeräteschacht herstellen, soweit das Meßgerät nicht ein- oder angebaut wird(vgl.Fig.31).
Als mechanische Variante einer Nachschnittaste sind zwei Konusschrauben vorgesehen, an denen ein Taster von der Aufnahme für den Teststreifen unter dem Saugglockendach gestuft Widerstand findet. Die Taste kann gegen eine Feder eine Strecke weit gedichtet eingeschoben werden, so daß ihre zweite (getrennt und gekoppelt tiefenverstellbare) Konusschräge für den Nachschnitt bei erfolgloser Erstbetätigung gegen den Taster gerichtet werden kann. Zugleich erfolgt elektrischer Kontaktschluß zur Magnetaktivierung.
Die Hautspalt- oder Schlitzerweiterung wird in einer Variante dadurch bewerkstelligt, daß der Teststreifen - jetzt schon zur quadratischen Platte verkürzt - in nach oben konvexem Bogen so bewegt wird, daß die Punktionsspitze eine kürzere Strecke bewegt wird als ihr Befestigungsende am Teststreifen - womöglich noch unter Bewegungsumkehr(vgl.Fig.34,40). Der Stößel kann auch auf einen Knick im unteren Teststreifen gerichtet sein, und eine Auslenkbewegung des teststreifennahen Teiles der Punktionsspitze bewirken, wobei die eigentliche Spitze etwas verzögert und eine kürzere Strecke bewegt wird (durch eine Sperre für einen festgelegten Abknickwinkel bedingt, vgl.Fig.41).
Die Punktionsspitze kann zwar punktförmig begrenzt werden, was für eine minimale Verletzung günstig ist, dabei ihr Schaft doch etwas rechteckig gegen die Schnittrichtung hin für eine größere Stabilität verbreitert sein. (Vor einer elektrolytischen Anspitzung ist der Spitzenbereich also quadratisch materialaufgefüllt,vgl.Fig.48 Mitte oben.) Um dabei eine Verdrehung zu einer Halteplatte hin zu vermeiden, kann für den Teststreifen eine Halterung vorgesehen werden, die selbst ein Gelenk besitzt und die Ausweichbewegung längs der Schmalseite des Teststreifens ermöglicht(vgl.Fig.38,39). Verschiedene Stiftquerschnitte (drei-, recht-, achteckig) werden angezeigt, dann die Formvariante einer Aufbiegung der Punktionsstiftbasis aus der Halteplatte heraus (vgl.Fig.46), eine Hohlrinnenbildung(vgl.Fig.45) zur Stablitätserhöhung und Blutaustrittserleichterung und schließlich die Beifügung eines Kanülensegments(vgl.Pig.47).
Ein Teststreifen kann nicht nur quer längs seiner Schmalseite, sondern -besonders nach der Ruckbewegung - auch seitlich bewegt werden und so den Wund-oder Hautspalt noch besser aufspreizen(vgl.Fig.44).
Die Wundspalterweiterung kann dadurch erreicht werden, daß der Teststreifen innerhalb seiner Aufnahmebuches in einer nach oben konvexen Schiene geführt wird, wodurch sich Punktionsspitze- und Basis auf anderen Kreisbahnen bewegen, die spitze auf kürzerer oder entgegengesetzt zur Basis. Besonders bei länglichem Stiftquerschnitt kann auch eine zusätzliche Querverschiebung, etwa die Kombination der Methode nach Pig.40 mit derjenigen nach Pig.44 zur Wundspalterweiterung zweckdienlich sein, auch die zusätzliche Kombination mit Teststreifenvorsprüngen gegen die Haut zu deren Bewegungsfixierung. Der Teststreifen kann mit seiner Aufnahmebuchse längs seiner Schmalseite geschwenkt oder - mittels eines Rotationssegmentes exzentrisch in der Saugglocke - gedreht werden.
Die Verpackung kann sich auch einer einzigen gespaltenen Scheidenlamelle bedienen, welche den Teststreifen innerhalb einer nur über dem Einsteckbereich abziehbaren äußeren Verpackungshülle schnabelartig umfaßt. Die Punktionsspitze selbst kann, durch tieferes Einschieben der Verpackungshülle über eine gegen den Teststreifen gerichtete Noppe auf der Lamelle hinweg durch ein Querband der Lamelle durchdringen und sich festkeilen, um den Abzug des Teststreifens aus dem Schacht und die Entsorgung zu begünstigen(vgl.Fig.30, links).
Die Funktion des oder der Elektromagnete kann variiert werden. so kann auf die äußeren Lappen eines Teststreifen - die der Hautfixierung dienen - eine von einem Rohr ausgehende Gabel unter Magneteinfluß wirken, während durch das Rohr hindurch ein zweiter Elektromagnet die eigentliche Punktionsstößel-Funktion ausübt. Es können auch verschiedene Magnetsegmente oder -wicklungsbereiche nacheinander oder verschieden stark mit Strom versorgt werden(vgl.Fig.30 rechts).
Bei einem Lösungsbeispiel wie in Fig.36 kann der zweite Elektromagnet auch doppelläufig gewählt werden und mit einem Auslösezug oder -schub für den Raster am Saugkolben eingesetzt werden. Die Pumpenauslösung kann dann über die Steuerzentrale mit der Erfüllung der Bedingung des Erdschlusses durch Hautberührung an den drei über den Saugglockenrand verteilten Schaltpunkte verknüpft werden.
Mit der Punktionseinrichtung kann als Variante auch ein Druckinjektor verbunden werden, wofür als Beispiel eine Einmal-Gasdruckpatrone mit Düse vorgestellt wird, welche mit einem gasbildenden Gemisch betrieben wird.
Bei der Herstellung und Fixierung von Punktionsspitzen können Haftpermanent- und Elektromagnete dem Transport dienen und die notwendigen Formen ausgewalzt werden(vgl.Fig.48).
Kreuzende Transportstraßen können die Stifte oder Bleche von der Extrusionsdüse aus nach Abkühlung und Abtrennung de Einzelstücke bis zur Aufwalzung des Plastik-Bandmaterials für den Teststreifen aufnehmen.
Es wurde zur Prioritätsfristwahrnehmung noch das Beispiel eines Punktionsgerätes für den Mehrfachgebrauch ohne Teststreifenwechsel aufgenommen, das in der Anwendung denselben Regeln der Unterdruckökonomie unterworfen werden könnte.Nicht nur die Unterdruckeinwirkung auf die Haut soll durch die Erfindung begrenzt werden; auch schon die Punktionswunde und -narbe soll möglichst gering gehalten werden. In EP 0 798 004 A1, veröffentlicht am 1.10.97, hatte ich bei Fig.20 bereits die herstellungs- und anwendungsmäßigen Vorteile erkannt, die darin liegen bei einer querbewegten Punktionsspitze auf eine weitere Schneide, die ja zudem nach der Bewegung ausgerichtet werden müßte, zu vermeiden. Mit Fig.33 wurde auch schon die Hinzufügung einer Metallspitze zum Teststreifen vorgeschlagen, in Fig.8 eine Kanüle speziell umgestaltet. Die Möglichkeiten einer Wundspaltverbreiterung durch eine Nachbewegung der Spitze wurde in Fig.35 erörtert, in Fig.40 wird das Blut über eine Art Kanüle (die aus verschiedenen Materialien bestehen konnte) in das Testfeld geleitet. In Pigur 19 von DE 196 13 722.5 wird bereits eine Punktionsspitze dem Teststreifen hinzugefügt; allerdings noch mit einem auch die Testfelder vereinigenden Rahmen. Eine zeichnerisch nicht besonders abgesetzte "Punktionsspitze im plastikummantelten Endteil" eines Teststreifens geht aus Fig.7 DE 197 23 730.4 hervor, die Punktionsspitze in Fig.6 aus DE 197 22 852.6 ist auch zeichnerisch deutlich vom Teststreifen abgegrenzt. Eine Punktions-spitze an einem Haltestahlblech findet sich an anderer Stelle bis zur Anklebung einer Stahlspitze mit DE 197 45 600.6.
Zur Wundspalterweiterung wird der Punktionsstift zusätzlich quer zu seiner Stoßbewegung gekippt, durch Knick im Teststreifen, dessen Führung in einer Kurvenschiene im Saugglockendach oder einfach durch Stöße gegen ein Drehgelenk der Aufnahmebuchse für den Teststreifen und damit weiter weg von der verunreinigenden Haut. Der Bewegungswinkel wird (eventuell veränderlich) festgelegt. Hautabspreizung kann auch von nicht oder gegenbewegten Fortsätzen des Teststreifens bewirkt werden, die neben der Punktionsspitze auf die Haut reichen, durch wenigstens zwei auseinander getriebene zunächst in eine einzige Spitze konvergierende Einzelnadeln und besonders wirksam quer zur Schnittrichtung, etwa durch die Hautdelle untergreifende Backen, welche den Hautbereich um die Punktionsspitze ausspannen. Es kann dadurch der Bereich, indem durch Blutaustritt aus unverletzten Gefäßen, Hautfleckungen auftreten, kleiner gehalten werden als bei starkem Sog über den gesamten Saugglockendurchmesser. Unschwer kann der die Backen auseinandertreibende Keil oder das Bewegungsglied über einen Elektromagneten gesteuert werden (vgl.Fig.30 rechts oben), wobei Meßimpulse aus dem analytischen Meßgerät von der elektronischen Steuerzentrale herangezogen werden. So kann das rechtzeitige Ausbleiben des Bluteintrittes auf das Testfeld - bei"Elite", vom kapillaren Schacht vorn neben der Punktionsnadel beginnend, - eine Verstärkung der Zugspannung auf die Haut auslösen.
Von besonderer Bedeutung ist die Narbenkleinheit eben für Patienten mit häufiger und regelmäßiger Anwendungsnotwendigkeit. Da das Gerät auch für die Blutgewinnung etwa für die Blutkörperchenzählung und Gerinnungsfaktorenbestimmung anwendbar ist, wird auch die Variante einer Wegwerfsaugglocke mit um ein Gelenk schwenkbarer Kanüle oder Punktionsspitze vorgeschlagen, wie als kleinen Trichter schon Gerhard Wagner für den Injektionsgebrauch beschrieb, nachdem ich meine diesbezügliche Anmeldung in München zurückgezogen hatte.
Besonders günstig hinsichtlich des Herstellungsaufwandes ist die Unterbringung der Saugglocke innerhalb eines Faltenbalges zur Sogerzeugung oder klinisch für den Mehrfachgebrauch die Kombination Wegwerfsaugglocke innerhalb einer Saugglocke mit Vakuumquelle, etwa einer Gasstrahlpumpe, welche mit einer Säure-Bikarbonatmischung oder Druckgaspatrone betrieben wird.

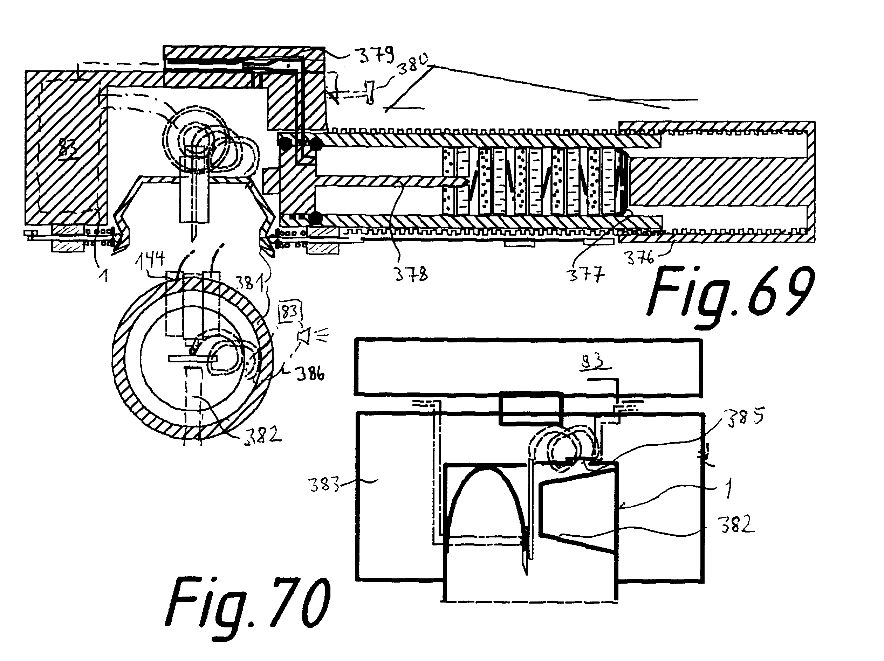

Möglich ist dies durch den Einsatz wenigstens eines Elektromagneten zur seitlichen Punktionsspitzenablenkung, wobei die elektrischen Zuleitungen keine Dichtungsprobleme bereiten. Wie in Fig.69 kann über Stromkreisschluß in der Signalleitung (386) durch Blut ein Signal für Wiederbelüftung gewonnen werden; hier zum Folienabriß an einer Wandlücke.
Da die Einrichtung taschengängig flach sein muß, kann es vorteilhaft sein, die Elektromagnete nicht in der Saugglocke, sondern im Sograum neben der Saugglocke anzuordnen. Ebenso kann für die sehr kurzen Teststreifen ein Spender geschaffen, ähnlich wie er jetzt von BAYER als DEX für Teststreifen ohne Punktionsspitzen herausgebracht wurde. Der Spender wird dann mit der Saugglocke in Kontakt gebracht und von unten der Teststreifen nach oben in seine Aufnahmebuche gedrückt. In den Spender zurückgeholt würde der Teststreifen mit Punktionsspitze allerdings erst in einem zweiten Akt nach der Hautpunktion.
Die Schrägstellung des Punktionsstiftes zur Hautspalterweiterung bedingt allerdings, daß die Stiftlänge größer gewählt werden muß, insbesondere dann, wenn die Schrägstellung bereits erfolgt, ehe die Spitze Blutkapillaren zerstört hat. Wird eine Punktionsspitze im Rotationsverfahren quer zur Haut bewegt, so ist es zweckmäßig, daß sie während ihrer Sektordrehung die Höhe wechselt, sich also auf einer Spiralbahn bewegt. Auf diese Weise erreicht sie mit höherer Wahrscheinlichkeit das Netz mit den größeren Blutkapillaren. Ansonsten würde die Spitze sich nämlich auf dem selben Niveau mit der Haut bewegen. Dagegen bewegt sich der schwingende Punktionsstift radiär zur Hautkuppe oder Hautdelle. Es ist zu berücksichtigen, daß im Gegensatz zur Wandstarre der Kanüle als Rohr, ein Stift den Biegekräften stärker verformend nachgibt. Deshalb fiel die Wahl auf eine Stahlspitze. Die Spitzenerzeugung hat preiswert zu erfolgen.

Die gestellte Aufgabe wird dadurch gelöst, daß eine Stahlspitze, vornehmlich aus VA-Stahl, Verwendung findet und dem Endabschnitt des Teststreifens inkorporiert oder ihm angeheftet wird.
Der feine Stift, der mindestens drei Millimeter unter die Hautoberfläche hinabreichen muß, um mit seiner Spitze dort ein breiteres Gefäßgeflecht zu erreichen, kann mittels Strangpresse gewonnen, aber auch in Formen gegossen werden. Er wird bei der vorteilhaften Erzeugung mittels Strangpresse in entsprechende Stücke - etwa durch Laser - (etwa lichtoptisch) abgemessen und dann abgeteilt. Dem Ende des Teststreifens vorzugsweise im Mittelbereich des reaktiven Testfeldes mit Meßreagentien wird er entweder in dieses Testfeld eingebettet, zwischen Testfeldern eingebracht, wenn etwa zwei oder mehrere vorhanden sind. Der Stahlstift, der in seiner breitesten, in die Haut eindringenden Ausdehnung möglichst einen Millimeter an Durchmesser nicht überschreiten soll (erwünscht ist ein Durchmesser um 0,5 Millimeter und weniger), kann auch an einen fertigen, handelsüblichen Teststreifen angeklebt werden. Allerdings weicht die elastische Haut hinter der Punktionsspitze auch einem etwas dickeren Stift gut aus, zumal eine maximale Hautstraffung im Moment des Eindringens in die Haut noch nicht vorliegt. Der Stift kann auch mittels einer Haftfolie auf dem Teststreifen befestigt werden. Die Spitze kann nach üblicher Fertigungsmethode wie bei der Kanülenherstellung angeschliffen werden; sehr vorteilhaft aber ist die elektrolytische Spitzenausziehung etwa in einem Bad mit ca. 20%iger Schwefelsäure und bei einer Stromstärke von ca. 0,1 Ampere Gleichstrom, wobei der Stahlstift die Anode darstellt (mit dem Pluspol einer Stromquelle verbunden wird), während das schwache Elektrolytbad mit dem negativen Pol verbunden wird. Die Stromeinwirkung für die Zersetzung der Stahlspitze beträgt - je nach Stärke des Stahlstiftes - nur einige Sekunden bis höchstens zwei Minuten.
Es kann eine große Zahl von Teststreifen, nicht nur in Blöcken nach Art der heutigen Laborroboter oder Manipulatoren in das Säurebad eingebracht werden, nachdem die Stifte bereits mit dem Teststreifen verbunden sein können. Es kann auch auf einem Rad eine Fertigung auf einem Band erfolgen, wobei auf dem Trägerpapier - dem Polyäthylen beispielsweise aufgewalzt sein kann - oder einem Kunststoffträger in einer Art Stempel die Testfelder und auch die Leitungsbahnen für die Signalabnahme aufgedruckt werden. Das Fertigungsband ist so angeordnet, daß es auf seiner Transportstrecke in ein Säurebad eintaucht, zumindest die Stahlstiftenden. Der fertige Stift kann auch in eine Art Hohlrinne am Ende des Testfeldes eingedrückt werden oder aber in eine vorgesehene Röhre gesteckt, wie sie im Gußverfahren etwa aus Kunststoff gebildet werden kann.
Vorteilhafterweise kann das Verfahren, den Punktionsstift nach der ruckartigen Bewegung, besonders bei Hautfixierung in seiner Umgebung, noch eine Strecke nachzuschieben mit einer Schrägstellung des Stiftes kombiniert werden, um so die Elastizität der Haut lokal zu erschöpfen und den Wundspalt zu öffnen. Die Stichverletzung soll die Schnittverletzung hautschonend ersetzen oder doch überwiegen. Nur eine flache Geräteform kommt bei Soganwendung in Frage, weshalb Kabelübertragung von Meßsignalen, verkürzte Teststreifen, zweckmäßige Druckgeber und Federanordnungen für die Saugpumpe und deren möglicher Ersatz durch eine Gasstrahlpumpe, etwa mit chemischen Treibsätzen gespeist, die Aufgabenstellung abrunden. In Anlehnung an den Hinweis auf die Möglichkeit einer Lanzettenanwendung ohne Saugglocke, wurde diese Modellvorstellung erweitert und durch eine Vorrichtung zur Hautfaltenstraffung bereichert.

### Kurze Beschreibung der Zeichnungen

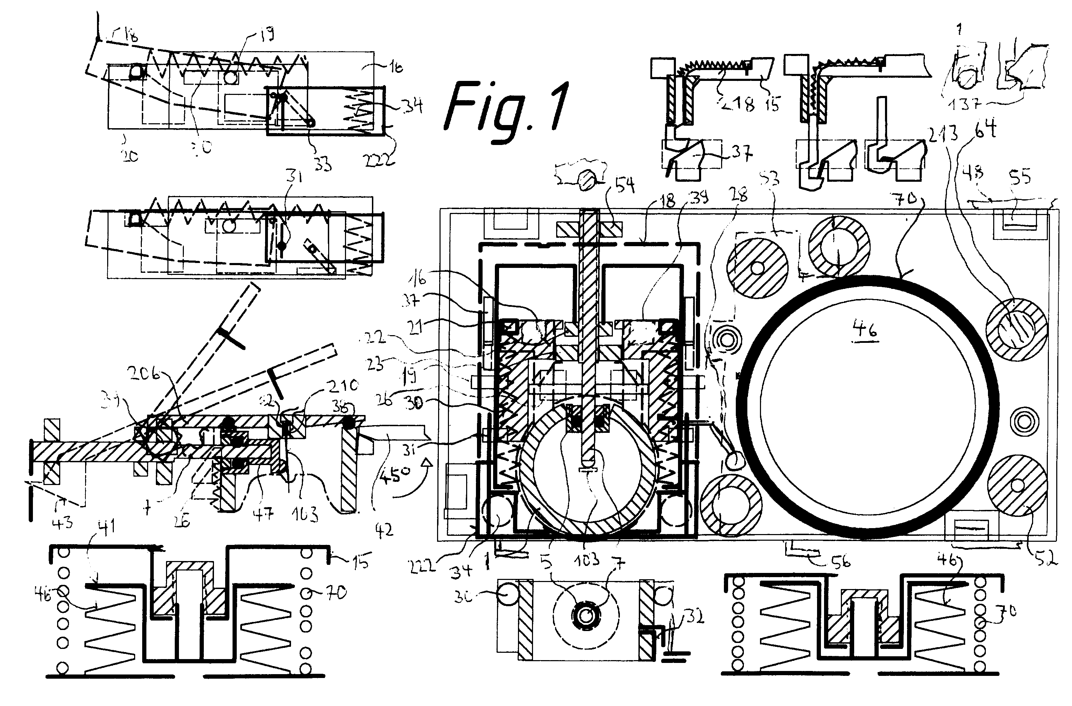

Die Figur 1 zeigt in natürlicher Größe in der Mitte rechts einen Horizontal- oder Querschnitt durch eine Punktionseinrichtung. Rechts oben leicht vergrößert das Detail eines Druckgeberauslösers im Längsschnitt in drei Funktionsstadien. Unten rechts ein schematisierter Längsschnitt links durch die Saugglocke, rechts durch den Faltenbalg der Saugpumpe. Links oben zwei schematisierte Darstellungen der Kraftübertragungs- und Steuerungsschieber in zwei Funktionsstadien. Darunter, links von der Saugglocke im Querschnitt, ein Längsschnitt durch die Saugglocke, darunter ein solcher durch den Faltenbalg in ausgedehntem Zustand. Rechts davon ein Längsschnittdetail durch die Saugglocke, und ganz rechts unten durch den Faltenbalg in zusammengezogenem Zustand. Rechts oben Längsschnittdetails durch den Auslösemechanismus für den Druckgeber für die Querbewegung der Punktionsspitze, ganz rechts Quer- und Längsschnittdetail durch eine Variante.

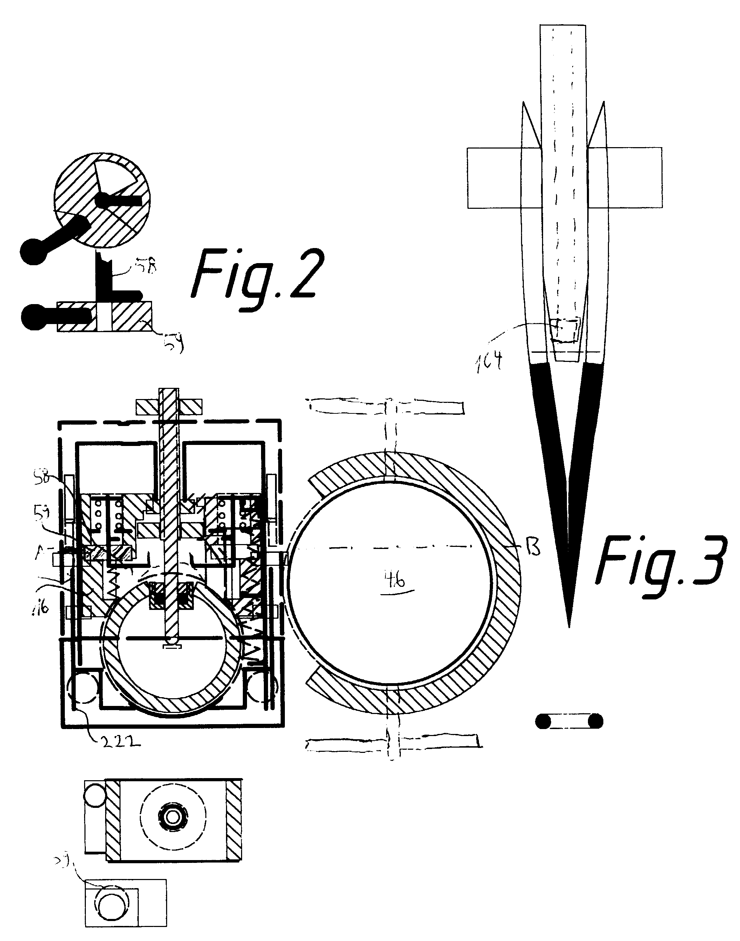

Die Figur 2 bezieht sich auf eine Einrichtung wie in Fig.1 . wobei als Variante für eine wundspaltsperrende Weiterbewegung der Punktionsspitze über eine gebremste Stößelbewegung eine besondere Vorrichtung vorgesehen ist. Im Maßstab 2 : 1 ist oben ein Vertikalschnitt- und darunter ein Querschnittsdetail längs der Schnittlinie A - B des unteren Querschnittes durch den Hauptteil der Einrichtung dargestellt. Auf dem Längsschnittdetail ganz unten kommt die leichte Exzentrizität zwischen Schaltrolle(58) und Druckfeder zur Darstellung.

Die Figur 3 zeigt im Maßstab von etwa 10 : 1 im schematischen Längsschnitt eine Sonderform eines Teststreifenendes mit zwei Punktionsnadeln am Ende.

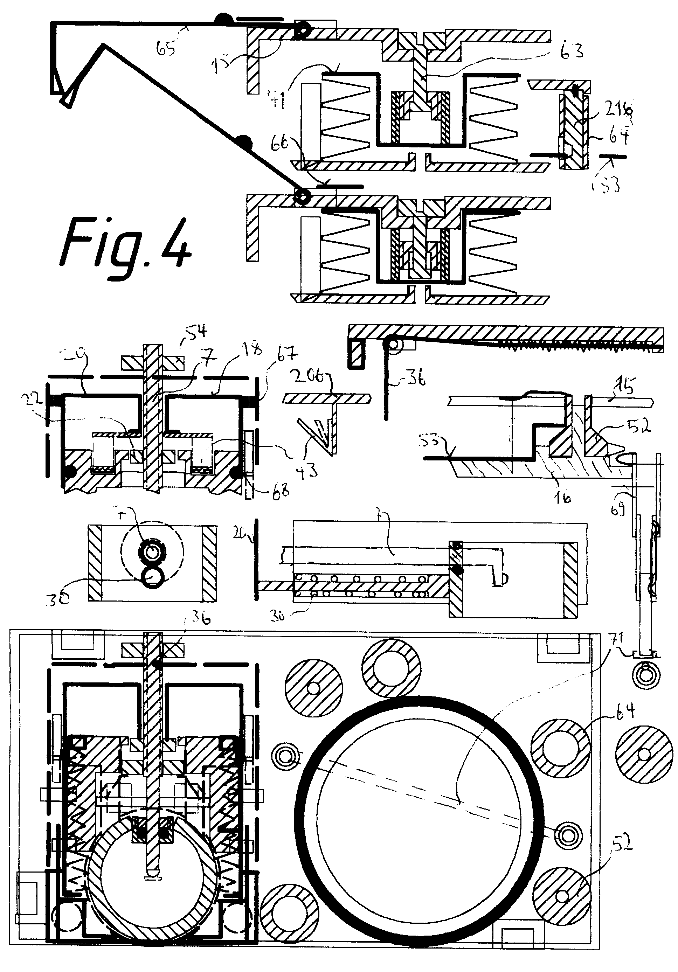

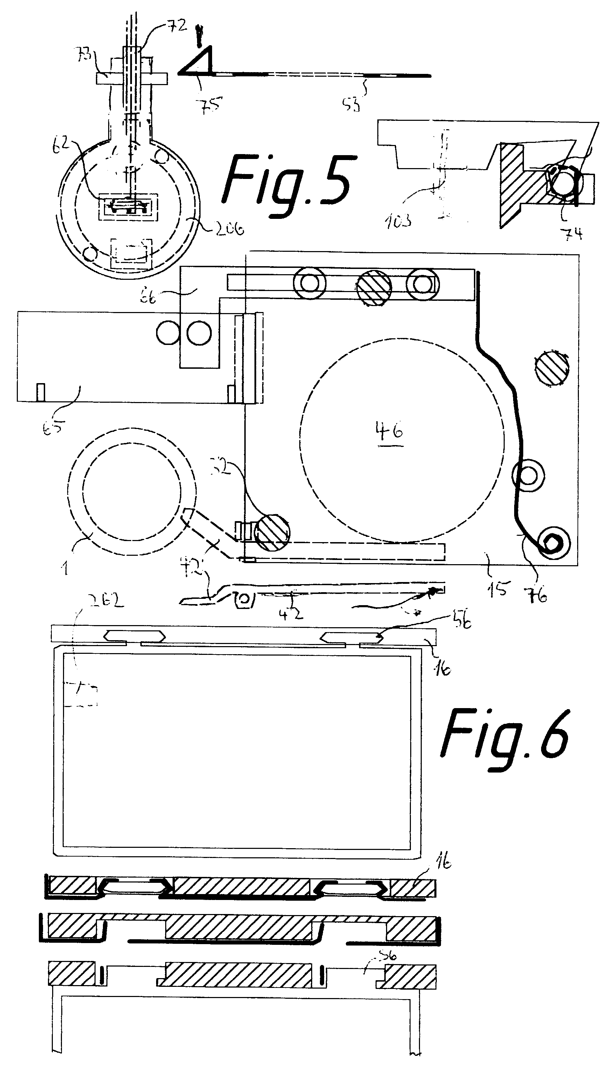

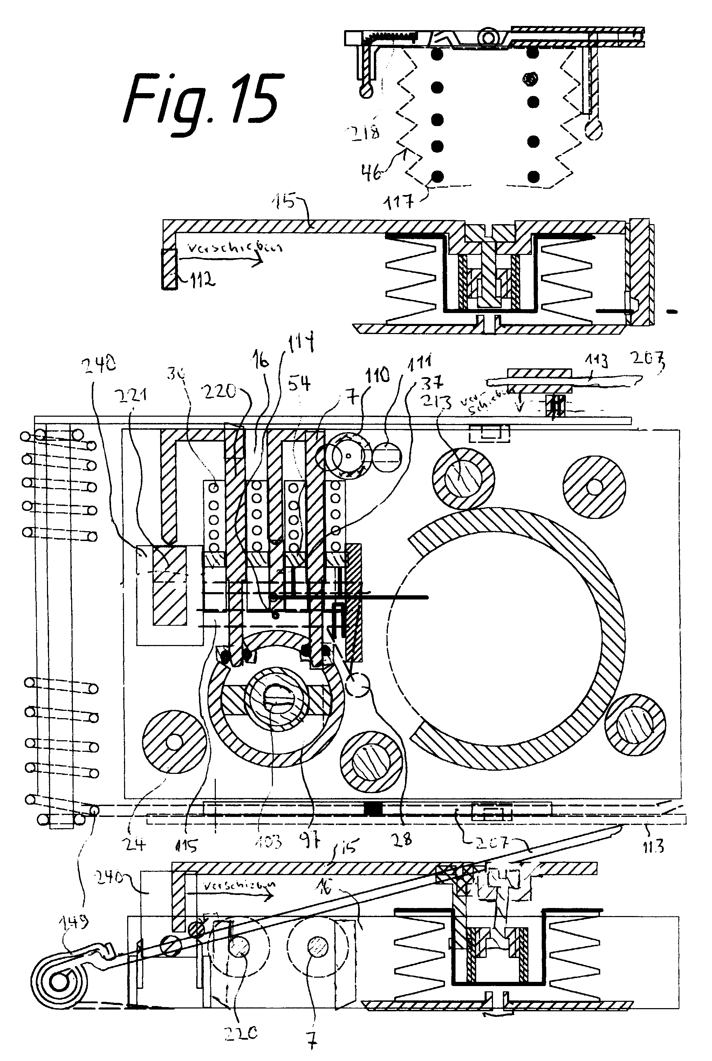

Die Figur 4 zeigt in natürlicher Größe über dem Querschnitt der Vorrichtung, der demjenigen in Fig.1 entspricht und der Orientierung dienen soll, oben in zwei Funktionsstadien Längsschnitte durch den Faltenbalg. Dieser wird durch einen (nicht dargestellten) Drahtbügel von einer Torsionfeder angehoben(vgl.Fig.15 unten). Linksseitig wird die Deckelplatte(15)durch die Scharnierklappe(65) verlängert.
Die untere Abbildung zeigt die Scharnierklappe nach Rückzug des Schiebers(66) von der Noppe der Scharnierklappe um ihr Scharnier hochgeklappt, um den Klappdeckel der Saugglocke vorbeizulassen(vgl.Fig.5).
In der Mitte links ist über einem Längsschnittdetail der Saugglocke ein der saugglockenabgewandte Teil des Querschnittes (unten) wiederholt und zugleich abgewandelt.
Rechts (und mittig) wird ein Vertikalschnittsdetail durch den Bereich von Stößel und Druckfeder(30) gezeigt.
Darüber befindet sich noch in einem Längsschnittdetail die Darstellung der Zugsaite(36), die hier über eine Rolle der Klappe (18,vgl.Querschnitt unten) entgegen geführt wird und sich unter dem Deckel in eine Zugfeder fortsetzt.
Darunter bis ganz nach rechts unten reichend das Längsschnittdetail durch einen Startmechanismus für die Sogauslösung. Unten ein Querschnitt durch die Einrichtung, rechts oben davon ein Längsschnittdetail und darunter ein Querschnitt durch eine Teleskopstange für die Sogauslösung.

Die Figur 5 zeigt links oben in natürlicher Größe den Klappdeckel zu einer Einrichtung nach Fig.1 in einer Draufsicht in Projektion auf den Grundriß der Saugglocke in der Mitte links.
Rechts des Querschnittsdetails eine Seitenansicht mit Aufbruch, um die Lage des Teststreifens(103) und die Torsionsfeder(74) für den Deckelschluß zu zeigen. Der Keil aus einer Federblechumbiegung an der Auslöserplatte(53) wird im Längsschnittdetail in der Mitte oben verdeutlicht. Das Querschnittsdetail (obere Mitte) entspricht einer Aufsicht auf die Deckelplatte(15).

Die Figur 6 zeigt Varianten der Befestigung eines Meßgerätes an der Punktionseinrichtung nach Fig.1.
Oben ist in einer Aufsicht ein Befestigungsrahmen, in welchen ein Meßgerät mit Schacht(262) für das Einschieben einer Trägerlamelle mit den Signalkontakten eingezeichnet.
Darunter zwei Detailvarianten lösbarer Rahmenverbindungen im Längsschnitt.

Die Figur 7 zeigt oben ein Querschnittsdetail, unten ein Längsschnittdetail durch eine Einrichtung nach Fig.1 in natürlicher Größe. Es soll Lage und Form der Auslöserplatte (53) verdeutlicht werden. Auf dem Längsschnittdetail ist zu erkennen, daß eine Brücke der Auslöserplatte(53) unterhalb des Bodenblechs hindurch geführt wird. Auch wird die Lage der von der Auslöserplatte ausgehenden Federzunge (55) verdeutlicht. Ganz unten die Darstellung wie der von der Drucktaste(222) - nach einer Auslöservariante - ausgehende Sperrknopf(28) sperrend in eine Loch der Auslöserplatte(53) hineinragt.

Die Figur 8 zeigt in natürlicher Größe oben einen Querschnitt durch eine Einrichtung nach Fig.1 bei Ergänzung durch eine Vorrichtung für die visuelle Hautkontrolle und einer Variation des Auslösers für die Saugpumpe; es wird auch noch die Verbindung zum Meßgerät dargestellt. In der Mitte im Maßstab 2 : 1 die Verbindung zwischen Auslöser(52) und Teleskopschienen(269).
Dicht darüber ein Längsschnittdetail der schlüsselochähnlichen Verriegelung für den in die Senkrechte hochschwenkbaren Haltebügel (79). Rechts wird nochmals die Lage der Federzunge(55), die vom Federblechkastenrahmen entspringt(vgl.Fig.1) wiedergegeben.Auf der unteren Bildhälfte Längsschnittdetail zur Erläuterung der Funktionsstadien des Auslösemechanismus wiedergegeben.

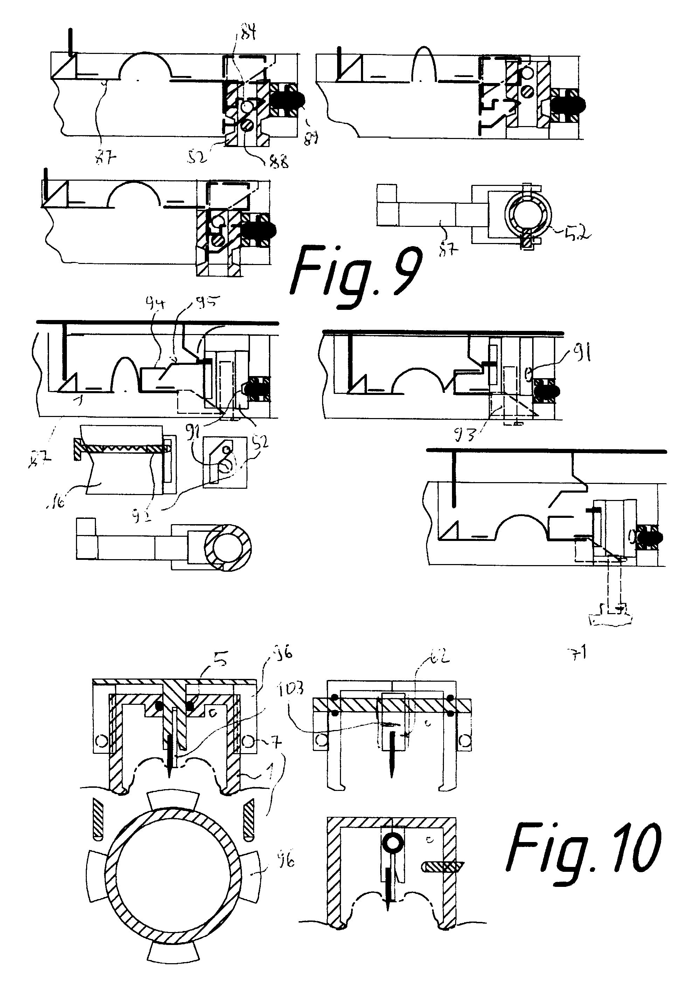

Die Figur 9 zeigt wiederum in Längsschnittdetails in natürlicher Größe eine Variante des Auslösemechanismus zu Fig.8 unten mit verschiedenen Funktionsstadien.

Die Figur 10 zeigt die Möglichkeit auf, die Punktionsspitzen-bewegung quer zur Haut nach Einstich in dieselbe durch das Aufprallen des Stößels außerhalb der Saugglocke auf den Test-streifen zu übertragen. und dies sowohl bei einer Spitzenbewegung nach dem Rotations- (linksseitig abgebildet) wie nach dem Schwingverfahren (rechtsseitig abgebildet).
In natürlicher Größe wird oben links ein Längs, und unten links ein Querschnitt durch das Detail einer Saugglocke gegeben.

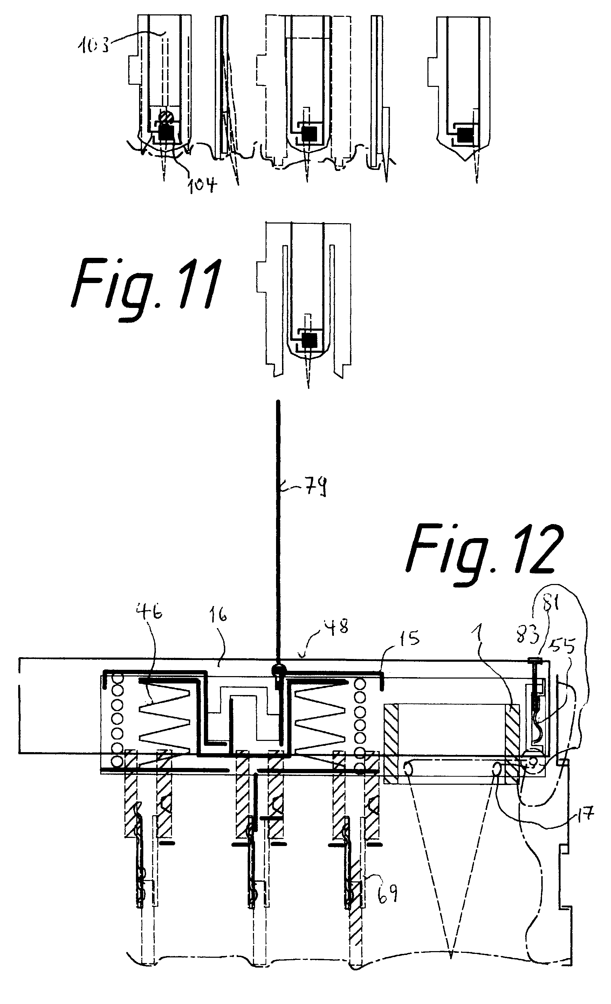

Die Figur 11 zeigt im Maßstab 2 : 1 (bei weit überzeichneter Stahlstiftbreite) oben zwei Draufsichten auf einen Teststreifen(103) mit Testfeld(104) und Leitungsbahnen bei in die Haut (strichpunktiert) eingestochener Punktionsspitze.
Rechts davon eine Seitenansicht. Die linke Draufsicht entspricht dem Stadium darnach, die rechte demjenigen vor dem Aufprall der Stößelkuppe.
Auf der Seitenansicht erkennt man, der Stahlstift an einer vorderen mittleren Lamelle des Teststreifens befestigt ist, größtenteils hinter dem Testfeld liegen und mittels des Stößels(7) nach rechts bewegt. Rechts oben ist die Draufsicht auf einen Test-streifen mit beigefügter Punktionsspitze für den Rotationsgebrauch dargestellt. Unten Mitte die Variante eines solchen Teststreifens. Siehe Anmerkung Seite 160 unten.

Die Figur 12 gibt stärker schematisiert eine Längschnittsübersichtsdarstellung zur Demonstration einer visuellen Hautkontrolle im Maßstab 1 : 1 .

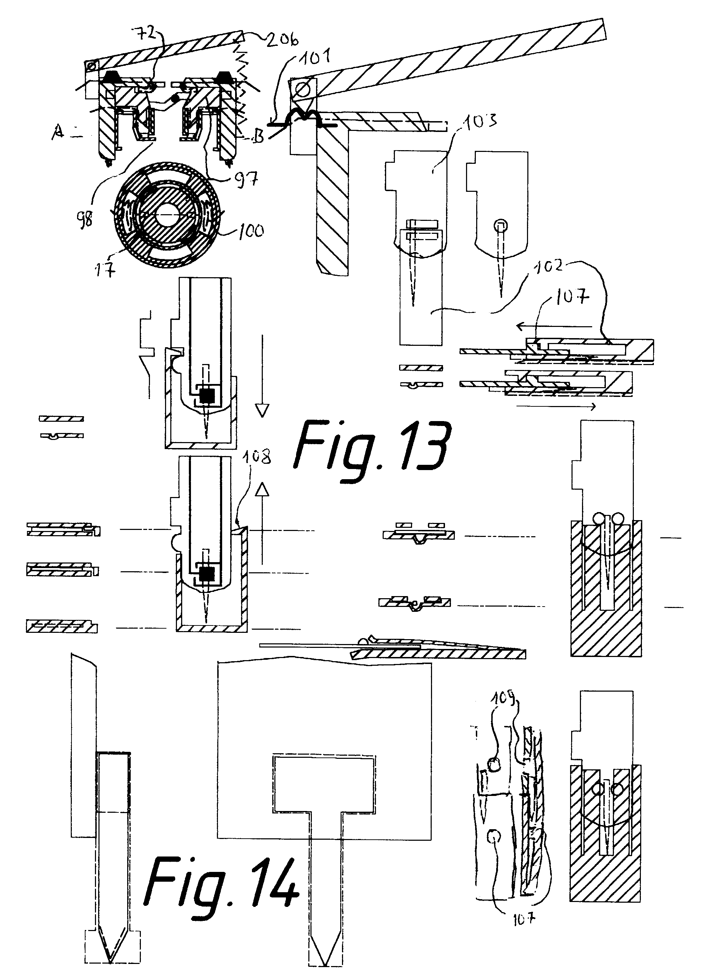

Die Figur 13 gibt links oben in natürlicher Größe, oben in einem Längschnittdetails eine Saugglocke mit einer Vorrichtung zur Punktionsspitzenrotation wieder; darunter in eine Draufsicht auf eine perforierte Lochplatte(98), wie sie von unten in die Saugglocke eingeschoben werden kann. Die Schnittlinie für den Querschnitt verläuft längs der Linie A - B des Längsschnittes. Rechts ist im Maßstab 2 : 1 ein Längsschnittdetail der linken Hälfte einer Saugglocke dargestellt. Darstellungen von Test-streifen(103) in Aufsicht im Maßstab 4 : 1 mit wiederum in der Breite stark überzeichnetem Stahlstift mit Punktionsspitze zeigen zugleich deren Verpackung an.
Darunter linksseitig wird eine ähnliche Verpackungslösung dargestellt, zum Längsschnitt linksseitig Querschnitte und oben eine Konturvariante des Teststreifens. Eine weitere Teststreifen-Verpackungsvariante ganz rechts , oben vor und unten nach dem Zusammenschieben, oben links mit Querschnittdarstellungen längs der Schnittlinien des Längsschnittes. Rechts der Mitte unten eine weitere Variante in Querschnitt und Längsschnitt (rechts davon).

Die Figur 14 gibt im Maßstab von etwa 10 : 1 das Ende eines Teststreifens(103) mit einem Stahlstift wieder, der auf Stoß mit dem Teststreifen verklebt ist und zunächst die gestrichelte Kontur aufweist. Linksseitig ist eine Seitenansicht, rechts eine Draufsicht gegeben.

die Figur 15 zeigt in der Mitte in natürlicher Größe und leicht nach unten verschoben den Querschnitt durch eine Punktionseinrichtung nach dem Rotationsprinzip. Darunter ist ein Längsschnitt wiedergegeben (bei Aufprojektion des Drahtbügels für die Anhebung des Paltenbalges mit der Deckelplatte), oben zwei Faltenbälge mit Teilen der Deckelplatte als Längsschnittdetail. Das Längsschnittdetail rechts oben zeigt einen Faltenbalg in erweitertem, der darunter im komprimierten Zustand und erläutert die Sogregulation wie in Fig.4.

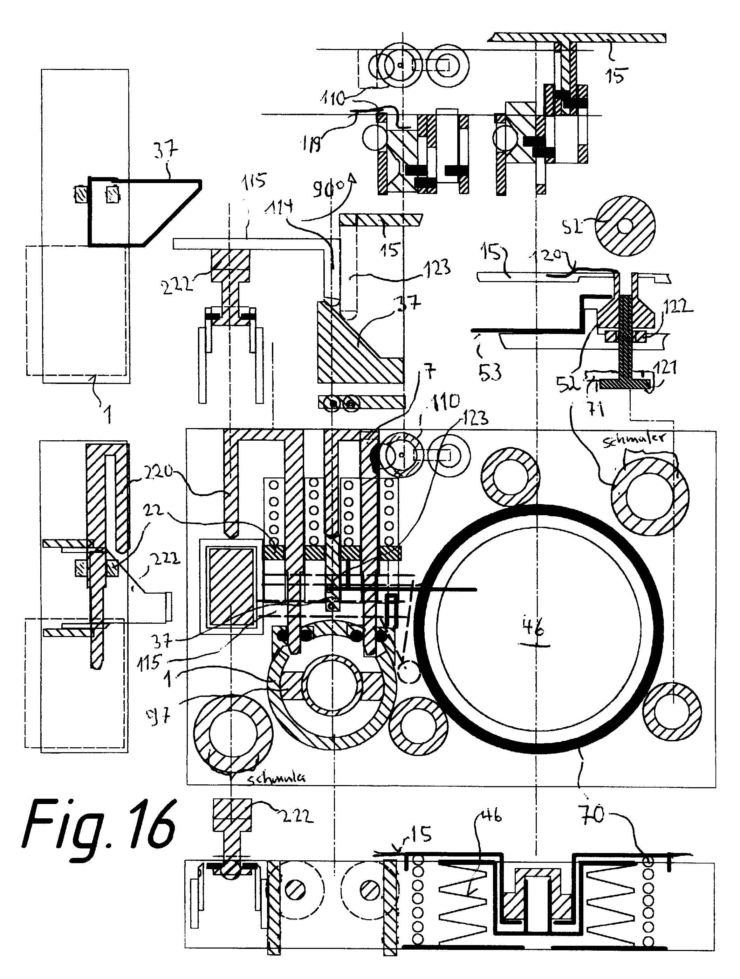

Die Figur 16 wiederholt zur Lageübersicht den Querschnitt der Fig.15 und erläutert einige Funktionselemente für die Auslösefunktionen derselben.
Zur Lageorientierung wird der Querschnitt der Figur 15 wiederholt. Ganz oben ist ein Längsschnittdetail durch den Auslöser nach Fig.15 für die seitlichen Schnittbewegung mit der feststehenden Rasterhülse(110) wiedergegeben.
Mit dem linken Längschnittdetail wird das Stadium der Verriegelung demonstriert, mit dem rechten das der Entriegelung.
Das Querschnittsdetail über dem linken Längsschnittdetail entspricht der Darstellung im Gesamtquerschnitt unten.
Rechts unter dem linken beschriebenen Längsschnittdetail wird in einem Längsschnittdetail einer der beiden Auslöser(52) unter einem Querschnitt durch denselben gezeigt, welche der Auslösung der Sogpumpe dienen.

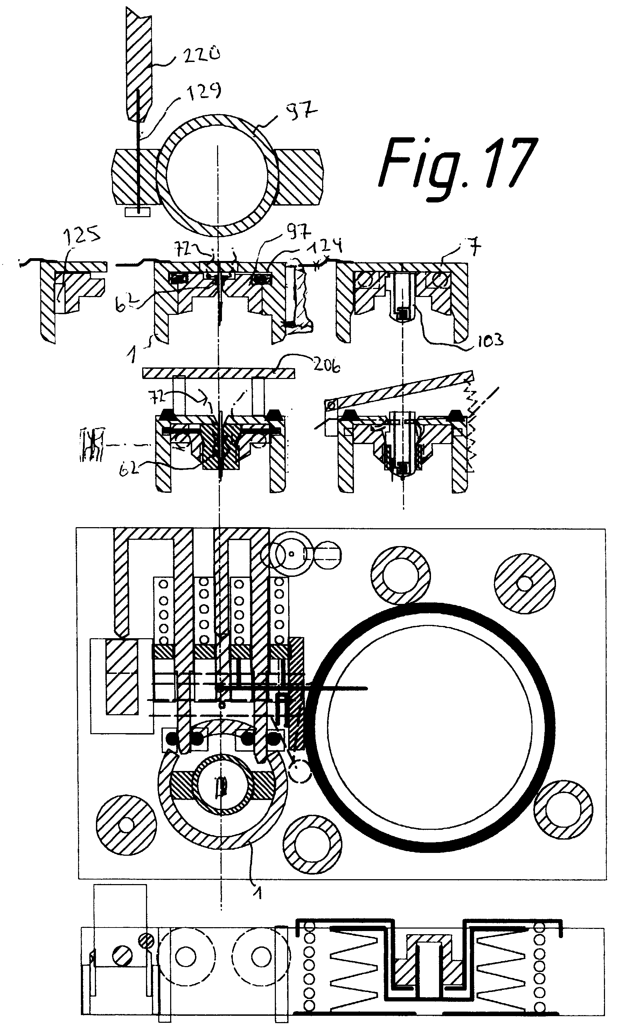

Die Figur 17 zeigt über dem Gesamtgrundriß der Fig.15 ( mit dem Längsschnitt durch die Saugglocke in natürlicher Größe darunter) oben links ein Querschnittsdetail durch den Rotoreinschub und Endteil eines Schieberbalkens im Maßstab 2 : 1 Saugglocke(1) und darunter zwei weitere Längsschnittdetails durch eine Saugglocke mit Klappdeckel in natürlicher Größe(vgl.Fig.13, Längsschnittdetail links oben). Die Reihe mit den Längsschnittdetails entsprechen einer Variante der Teststreifenbefestigung.

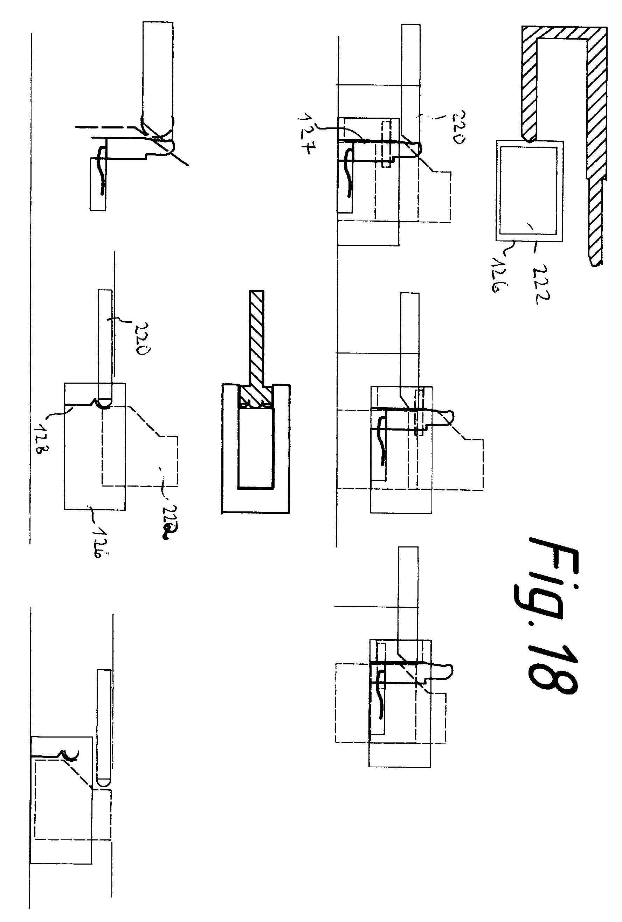

Die Figur 18 zeigt schematisch unterhalb eines Querschnittdetails links(vgl.Gesamtquerschnitt der Fig.15) schematisiert drei Funktionsstadien der Drucktastenbetätigung in Längsschnitten.

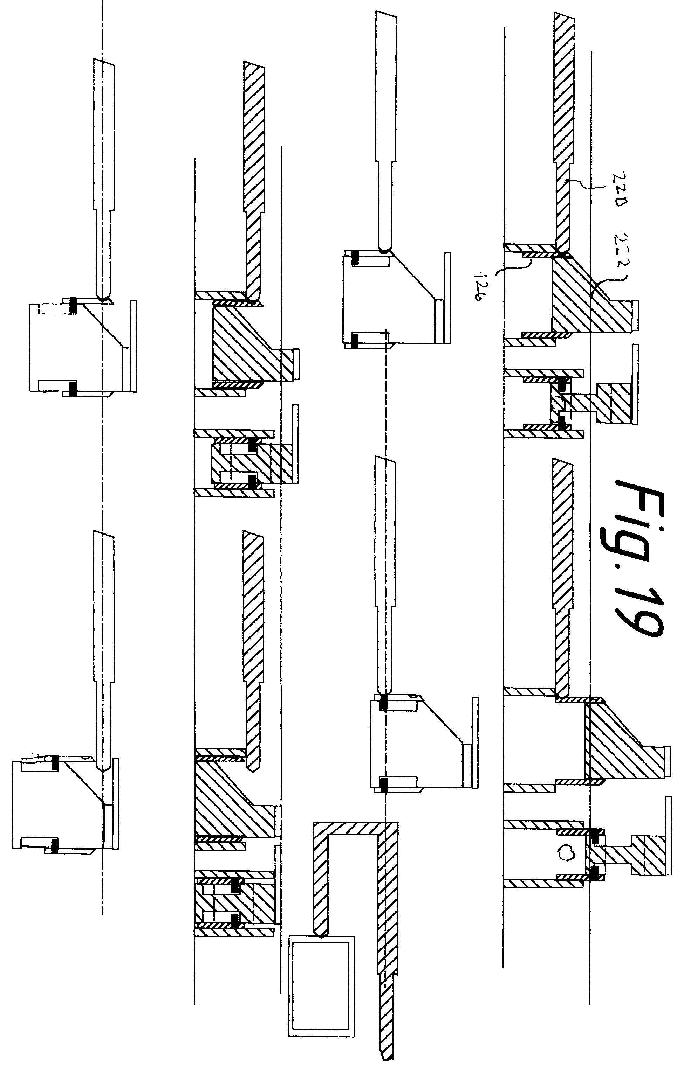

Die Figur 19 stellt eine bevorzugte Lösung der bereits in Fig.18 behandelten Lösung für eine Nachschnittaste dar. Es wird jeweils rechts neben einem Längsschnitt ein Querschnitt in verschiedenen Funktionsstadien und in einer oberen (schraffierten) und unteren Variante gezeigt.

Die Figur 20 zeigt unten im Maßstab von etwa 2: 1 bei Übertreibung der Stahlstiftbreite einen schematisierten Ausschnitt aus einer Herstellungskette für die elektrolytische Anspitzung und Montage von Punktionsspitzen auf Testsstreifen in Seitenansicht bzw. (das Elektrolytbad betreffend) im Längsschnitt. Darüber einige Kettenglieder und die Nahtstelle zur Teststreifenzuführung im Maßstab 10 : 1 oben in einer Seitenansicht, unten in einem Querschnitt.

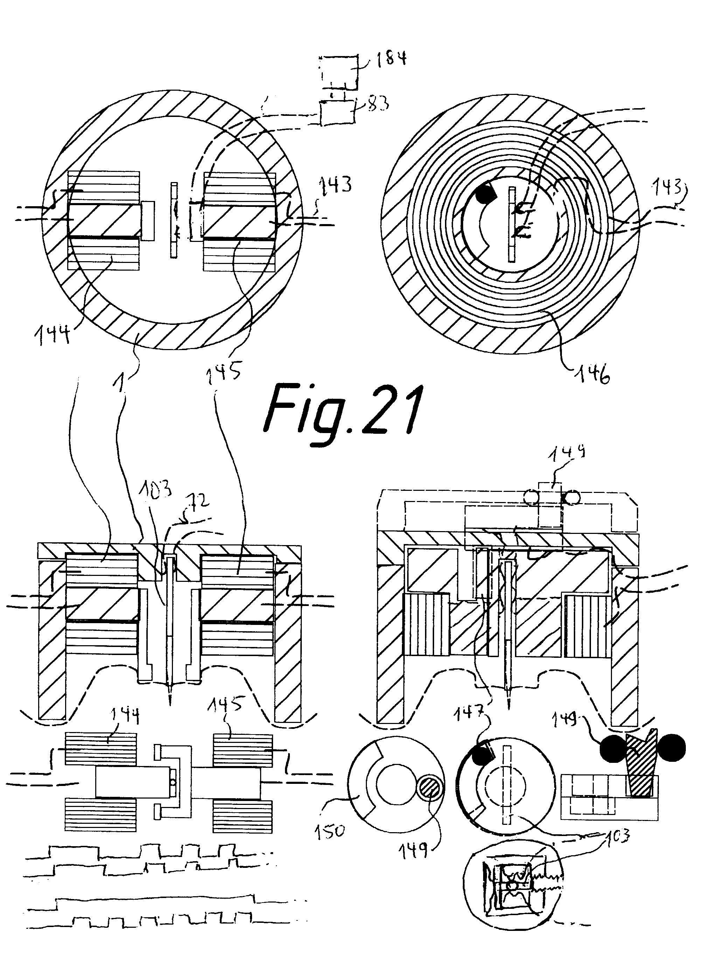

Die Figur 21 gibt oben in einem Quer- und unten im Längsschnitt im Maßstab 2 : 1 eine Lösung für die Punktionsspitzenbewegung quer zur Haut an, bei welcher Elektromagnete innerhalb der Saugglocke eingesetzt werden. Links wird ein Beispiel für die Schwingmethode gegeben, rechts eine für Rotation.
Unten ist - unter Weglassung der Saugglocke - ein Querschnittsdetail mit einer Variante der Magnetfunktionsausgestaltung angegeben, und zwar im Erregungszustand beider Magnete. Die beiden Doppelprofillinienpaare geben eine Impulsfolge der Magnete wieder

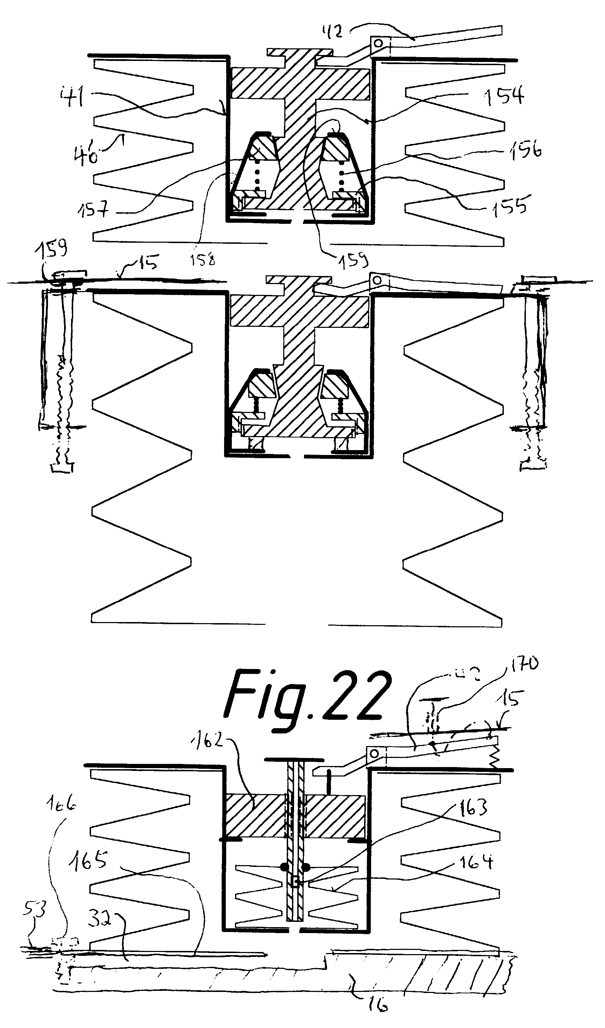

Die Figur 22 bringt im Maßstab 2 : 1 im Längsschnitt zwei Beispiele für eine Sogstärkenregelung mittels Ventiles im Topf des Faltenbalges, das zugleich der Wiederbelüftung der Saugglocke dienen kann. Das erste, obere Beispiel zeigt den Faltenbalg in zwei Ausdehnungsstadien.

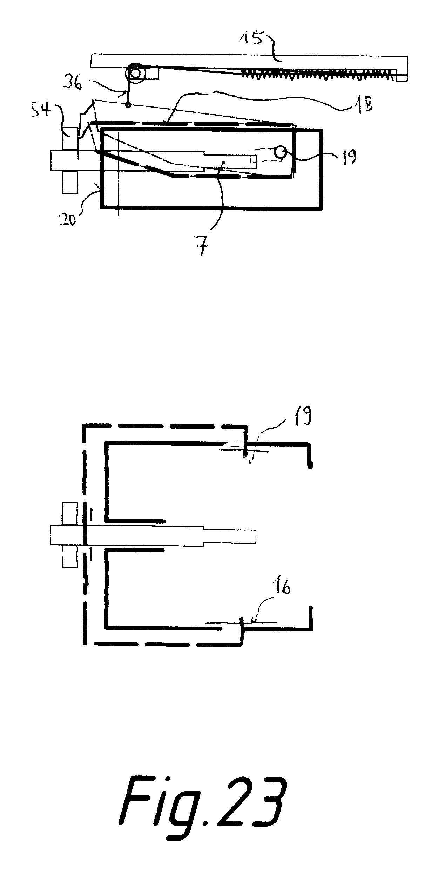

Die Figur 23 ist ein Nachtrag zur Einrichtungsvariante nach Fig.1 unter dem Schwingprinzip für die Punktionsspitze.
Sie verdeutlicht, wie nach dem Aufprall des Stößels(7) auf den Teststreifen innerhalb der Saugglocke(beides nicht dargestellt) ein langsameres Weiterrücken des Stößels zur Hautspalterweiterung erzielt werden kann. Auf dem schematischen Längschnittdetail oben in natürlicher Größe erkennt man die Einstellschraube(54). Das Querschnittsdetail unten zeigt die Befestigung der Drehachse (19) für die Klappe in der Grundplatte(16) und das die Fensterlücke für die Verschiebestrecke des Schieberahmens.

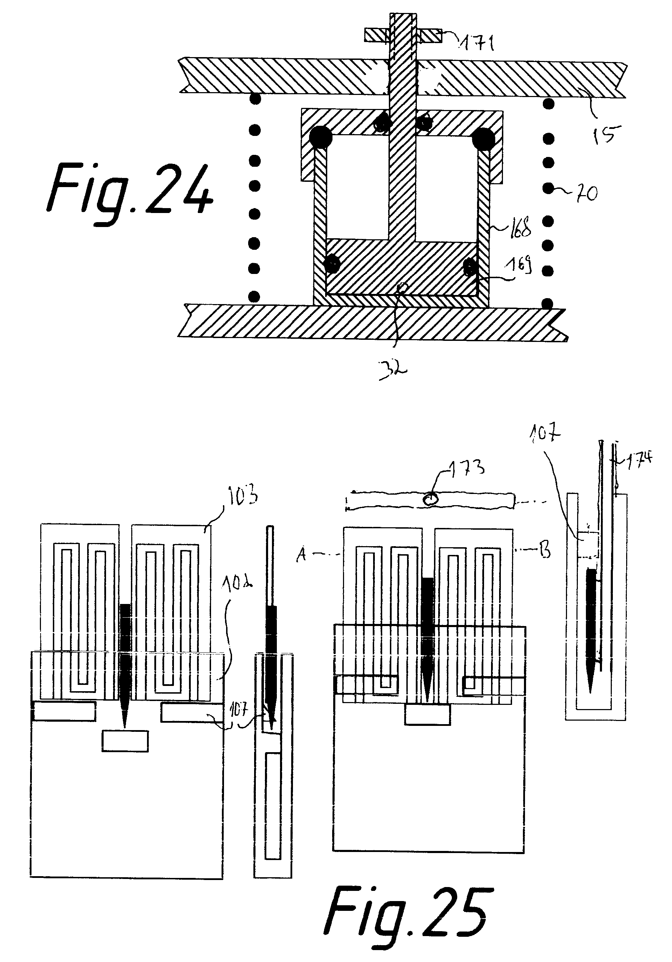

Die Figur 24 zeigt schematisch einen Längsschnitt durch eine Zylinder-Kolbenpumpe mit Vorlaufmöglichkeit der Deckplatte zur Sogregulierung.

Die Figur 25 zeigt im Maßstab von etwa 10 : 1 links eine Draufsicht und rechts einen Längsschnitt durch die Mitte der Draufsicht auf einen Teststreifen mit beigehefteter Punktionsspitze(schwarz). Der Vertikalschnitt ganz links zeigt eine über den Punktions-spit-zenbereich zur Entfernung des Teststreifens mit der Kapillare(174) vorgeschobene Folienscheide. Rechts das Stadium nach Gebrauch (das Blut in den Kapillaren ist nicht eingezeichnet) und nach Einschub der Folienscheide. Darüber ein Querschnitt längs der Schnittlinie A - B.

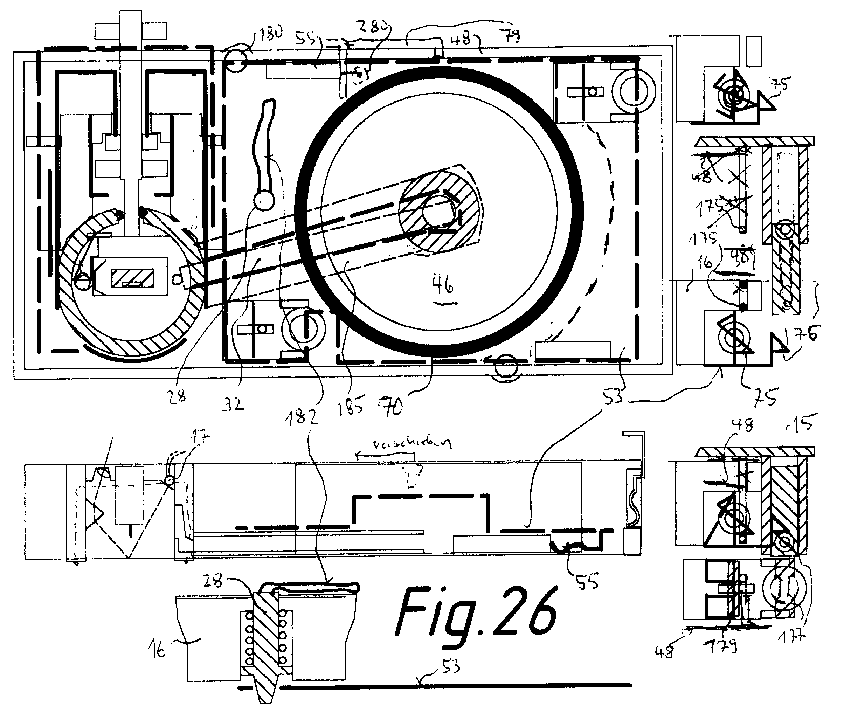

Die Figur 26 lehnt sich an Fig.7 an und bringt eine weitere Lösung für die Sogauslösung im Zusammenhang mit dem Problem der Spannung der starken Druckfeder (nicht dargestellt) für den Faltenbalg. Oben wird ein Querschnitt wiedergegeben, darunter ein Längsschnitt und rechts Längschnittdetail aus der Randzone des Querschntts mit schematischer Darstellung des Auslösemechanismus mittels des Federblechkastenrahmens. Ganz unten noch das Längsschnittdetail durch den Sperrknopf für die Auslöserplattenbewegung.
Die paarig angelegte Federzunge(55) wird auf dem Längsschnitt in ihrer Funktion verständlich gemacht. Rechts in Vertikalschnittdetails, ganz unten im Querschnitt, die Erläuterung der Überholklinke für Spannung der starken Feder unter dem Faltenbalg und später dessen Auslösung über Senkung des Blechkastenrahmens. Das untere der Längschnittdetails rechts gibt des Zustand bei Anspannung der großen Druckfeder nach Senkung der Deckelplatte(15) wieder. Unten ein Längsschnittdetail, da den Sperrknopf(28) in der Auslöserplatte(53) zeigt.

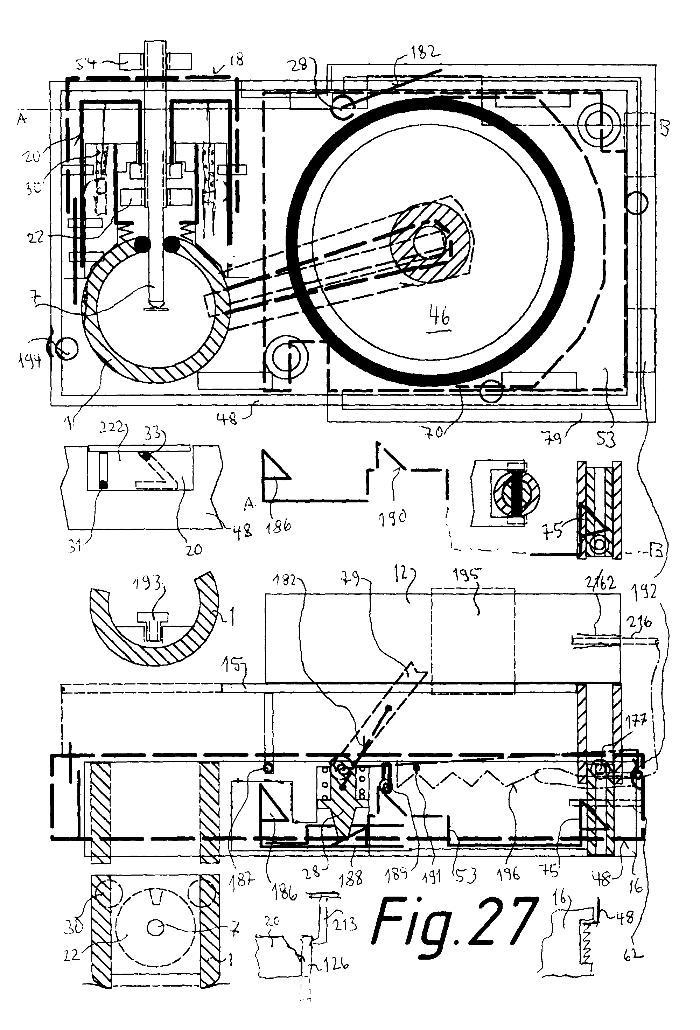

Die Figur 27 entspricht weitgehend in der oberen Querschnittdarstellung in natürlicher Größe der Fig.26.
Links unter dem Querschnitt ein Vertikalschnittdetail durch Nachschnittaste, rechts davon die Aufbiegung der Auslöserplatte, ganz rechts der Sperraster für die Deckelplattenabsenkung innerhalb der Teleskoprohre, mit links im Querschnitt erkennbarem Querstift. Unten ein Längsschnitt mit dem als Rechteck symbolisch aufgesetzten Meßgerät und strichpunktieren Leitungsverbindungen zur Punktionseinrichtung. Auf dem Längsschnittdetail links unten durch die Saugglocke die Lageverdeutlichung des Stößels(7),rechts davon der Vertikalschnitt durch die Nachschnittaste und ganz rechts ein Vertikalschnittdetail zur Verdeutlichung der Abfederung des Federblechkastenrahmens.

Die Figur 28 lehnt sich konstruktiv eng an die Fig.22 an. Es handelt sich wiederum um zwei Längsschnitte verschiedener Funktionsphasen eines Druckregelungsventiles innerhalb des Topfes eines Faltenbalges(64) als Saugpumpe im Maßstab 2 : 1.

Die Figur 29 zeigt ebenfalls in zwei Funktionsstadien im Längsschnitt, aber in natürlicher Größe eine Vorrichtung für die Lösung der gleichen Aufgabe mittels eines Elektrotauchmagnets(144).

Die Figur 30 behandelt das Verpackungsproblem eines Teststreifens mit Punktionsspitze ähnlich wie die Fig.11 und 13. Der Maßstab ist 4 : 1 . Die Punktionsspitze ist wiederum stark überzeichnet. Links eine Draufsicht, rechts davon ein Längsschnitt. Es folgen nach rechts ein Längsschnitt und eine Draufsicht einer Lösungsvariante, ganz rechts dann noch eine Verdeutlichung dafür, daß die Punktionsspitze in einer Querleiste der Verpackungsfolie steckt. Ganz rechts oben ein speuzieller Elektromagnet im Längsschnitt und das Schema seiner Stromversorgung.

Die Figur 31 zeigt ein Beispiel für eine Punktionseinrichtung, bei welcher Heizdrähte als Saugpumpe Verwendung finden. Die Darstellung erfolgt im Längsschnitt im Maßstab 2 : 1.
Links unten im Maßstab 2 : 1 eine Darstellung einer Rückstellfeder für die seitlichen Lappen eines Teststreifens in der Saugglocke im Querschnitt. Rechts davon die Aufsteckhülse für ein Meßgerät. Rechts über dem Längsschnitt das kleine Vertikalschnittdetail des äußeren Schaltknopfes.

Die Figur 32 zeigt oben in einem Quer- oder Horizontalschnitt darunter in einem Längsschnitt im Maßstab 2 : 1 eine Einrichtung unter Verwendung einer ovalären Kolben-Zylinder-Pumpe. Unten ist im Maßstab 4 : 1 der Querschnitt durch eine Nachschnittvorrichtung verdeutlicht. Rechts davon ein Vertikalschnitt mit aufgeschobenem Meßgerät und ganz rechts die Draufsicht auf ein Verbindungskabel, jeweils im Maßstab 2 : 1 .

Die Figur 33 gibt in Querschnittsdetail im Maßstab 1 : 1 vier Funktionsstadien A - D einer Einrichtung nach Fig.32 wieder.

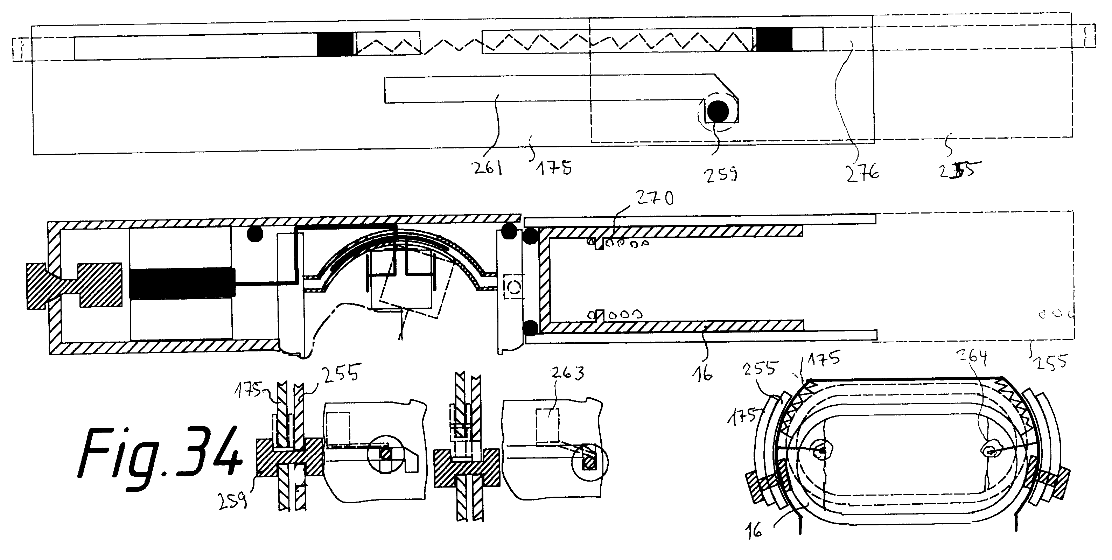

Die Figur 34 gibt oben die schematische Seitenansicht auf und darunter einen Längsschnitt durch eine Einrichtung ähnlich wie die in Fig.32 und 33 beschriebene. Der Zylinder oder die Grundplatte wurden aber wesentlich verkürzt durch eine Schiebekonstruktion. Die gebogene Schienenführung in der Saugglocke demonstriert einen Mechanismus zur Wundspalteröffnung.
Links unten wird das Verrastern der Auszugsschiene gegen den Federzug im Kolben in zwei Funktionsstadien erläutert, jeweils links im Vertikalschnitt und rechts in einen Längsschnitt längs der Außenkante der Punktionseinrichtung. Auf einem Vertikalschnitt rechts wird unter Weglassen der Zugfeder und des Kolbens der Zylinder als Grundplatte(16) gezeigt, welcher durch eine Art Federblechkastenrahmen gehalten wird.

Die Figur 35 zeigt wiederum im Maßstab 2 : 1 die Variante der Einrichtung nach Fig.32,33 in der Art, wie in Fig.34 ausgeführt. Es handelt sich um Querschnittsdetails in zwei verschiedenen Funktionsstadien und in verschiedener Ausstattung des Saugglockenbereiches.

Die Figur 36 gibt noch einmal einen Gesamtquerschnitt (oben) und eine Seitenansicht längs der Seitenwand einer Einrichtung nach den Fig.33-35 mit einer weiteren Variation der Verrasterung der Rahmenteile. Links unten ein Querschnitt mit einer Variante für den Federblechrahmen, rechts zwei Funktionsdetails für die Stempelsperrung und die Rasterlösung im Kolbenbereich im Längsschnitt.

Die Figur 37 zeigt schematisiert im Maßstab von etwa 3 : 1 im Längsschnitt eine Auslösevorrichtung für den Stößel(7) einer mechanischen Einrichtung etwa nach Fig.27.
Das Detail links unten im Vertikalschnitt zeigt, wie die Stellschrauben(286) über den Querstift(schwarz) nicht nur das Bewegungsmaß im Sektorschlitz des Radsegments(284) beschränkt sondern auch die Höhenbewegung der Deckelplatte(15) durch einen paarigen Seilzug(288) begrenzt. Auch wird der Unterdruck über ein Ventil mit Seilzug reguliert. Rechts eine Hebelvariante für den Auslöser der Saugpumpe in zwei Funktionsstadien.

Die Figur 38 zeigt im Maßstab von 2 : 1 schematisch im Längsschnitt einen Teststreifen(103) mit Punktionsspitze in einer schwenkbaren Aufnahmebuchse.

Die Figur 39 zeigt im Maßstab 2 : 1 im Querschnitt die Möglichkeit auf, einen Stößel(7) auf eine exzentrisch unter dem Saugglockendach um eine Achse drehbaren Scheibe(289) - etwa an einer Segmentkerbe - einwirken zu lassen.

Die Figur 40 zeigt schematisch im Maßstab 2 : 1 im Längsschnitt die Möglichkeit auf, durch eine Pührung der Aufnahmebuchse (231) mit dem Teststreifen längs einer nach oben konvex unter dem Saugglockendach gebogenen Schiene(237) Bewegungsausmaß und -Richtung zwischen Punktionsspitze und Schaftbasis zu verändern.

Die Figur 41 bringt in einem schematischen Längsschnitt im Maßstab 2 : 1 eine Lösung, bei welcher der Teststreifen das Gelenk(290) aufweist, um welches er knickbar ist. Der Knickwinkel wird beispielsweise dadurch beschränkt und festgelegt, so daß wiederum eine Schrägstellung des Punktionsstiftes erfolgt.

Die Figur 42 bringt in einem schematischen Längsschnitt durch die Saugglocke(1) im Maßstab von etwa 1 : 1 die bevorzugte Lösung einer Steuerung des Elektromagneten(144) für das Sitzventil(208) über die als drei Ringe eingezeichneten Erdkontakte innerhalb der Saugglocke.

Die Figur 43 zeigt in einem schematischen Längsschnitt im Maßstab 2 : 1 durch eine Saugglocke aus einer Punktionseinrichtung mit der Möglichkeit zum Anschluß eines Injektors, hier ein Einmalinjektor mit Treibsatz.

Die Figur 44 zeigt schematisch in einem Querschnitt im Maßstab von etwa 4 : 1 eine Möglichkeit auf, die Punktionsspitze schräg zur Einwirkungsrichtung des Stößels(7) zur Hautspalterweiterung durch denselben zu bewegen.

Die Figur 45 zeigt oben in einem Querschnitt und unten in einer Draufsicht im Maßstab von etwa 4 : 1 einen Teststreifen(103) mit angeschweißter Stahlfolienplatte und Aushöhlung in einer Hohlrinne in Richtung auf die Punktionsspitze.

Die Figur 46 bringt in einer Draufsicht und rechts davon in einem Vertikalschnitt im Maßstab 4 : 1 eine Punktionsspitzenvariante, bei welcher zur Stabilisierung gegen die Stoßbewegung der Punktionsspitzenschaft an der Stelle des Austritts aus dem Teststreifenende bogig ausgebildet ist.

Die Figur 47 zeigt links in einem Längs und rechts in einem Vertikalschnitt im Maßstab von 4 : 1 die Anschweißung oder Anklebung eines Kanülensegmentes an den Teststreifen(103) mit dem Testfeld (104).

Die Figur 48 zeigt im Maßstab von etwa 4 : 1 ergänzende Einzelheiten zum Herstellungsprozeß des Teststreifens mit Punktions-spitze. Oben der Längsschnitt durch eine sechseckige, drehbare Trommel mit Elektromagneten. Rechts davon ein Längsschnitt durch ein Transportglied mit Permanentmagnet zur Stiftfixierung, darunter kleine Querschnittsdetail durch die Hohlrinne mit Stift. Darunter mehr mittig Längs- und Vertikalschnitt durch einen ovalären Stift mit Formgebung im Spitzenbereich vor dem Elektrolytbad. Unten die Querschnitt längs der Schnittlinie A - B. Links in der Mitte bis unten ein Walzenpaar über einer Extrusionsdüse für Stahl, rechts daneben zwei Aufrollungen zur Demonstration von Profilgebungen für die Walzenoberfläche mit kleinen Querschnitten von der Stiftrinne dazwischen.

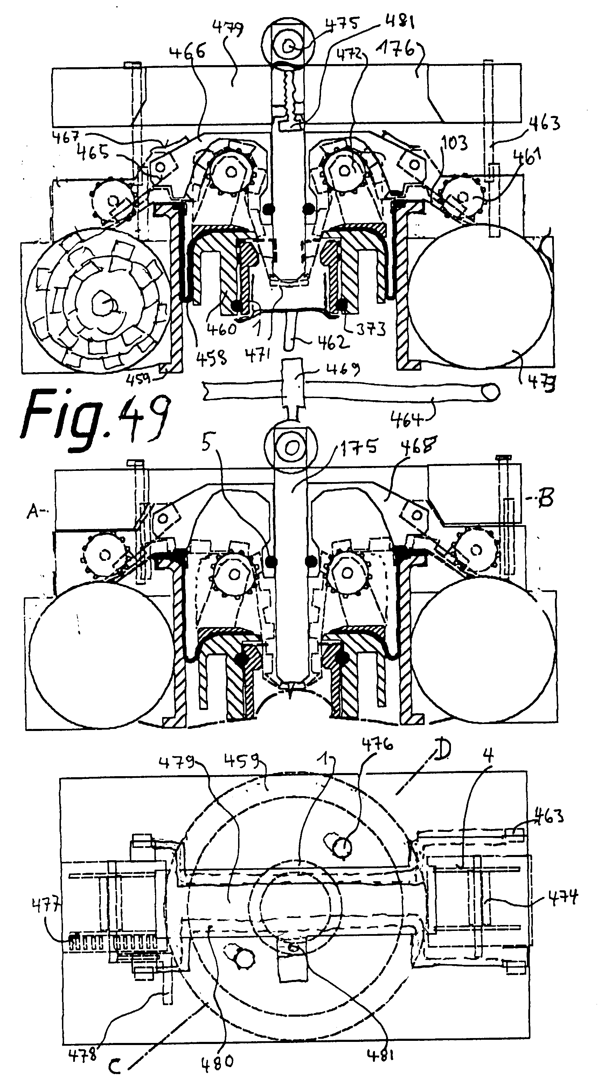

Die Figur 49 zeigt in natürlicher Größe oben und in der Mitte im Längsschnitt und unten im Querschnitt in Höhe der Schnittlinie A - B des mittleren Längsschnittes das Beispiel einer Punktionseinrichtung ohne die Notwendigkeit eines manuellen Teststreifenwechsels.

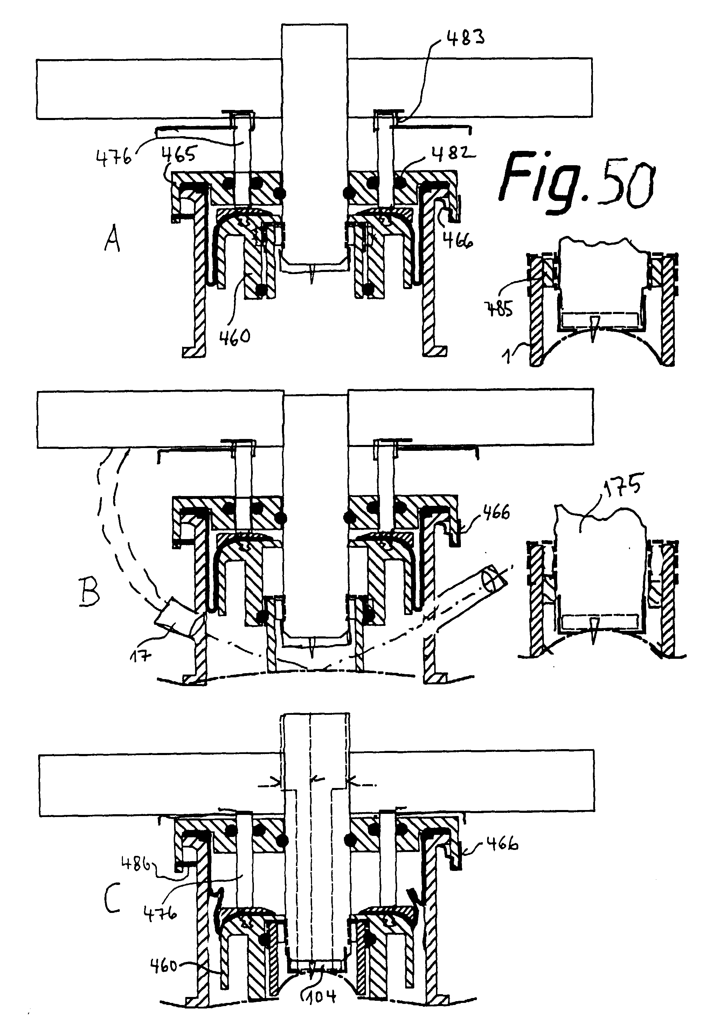

Die Figur 50 zeigt auf drei Längsschnitten durch die Punktionseinrichtung längs der Schnittlinie C - D des Querschnittes in Fig.49 unten drei Funktionsstadien A - C . Das Stadium A entspricht demjenigen im oberen Längsschnitt der Fig.49, das Stadium C demjenigen im unteren Längsschnitt in Fig.49 unter Hautansaugung.

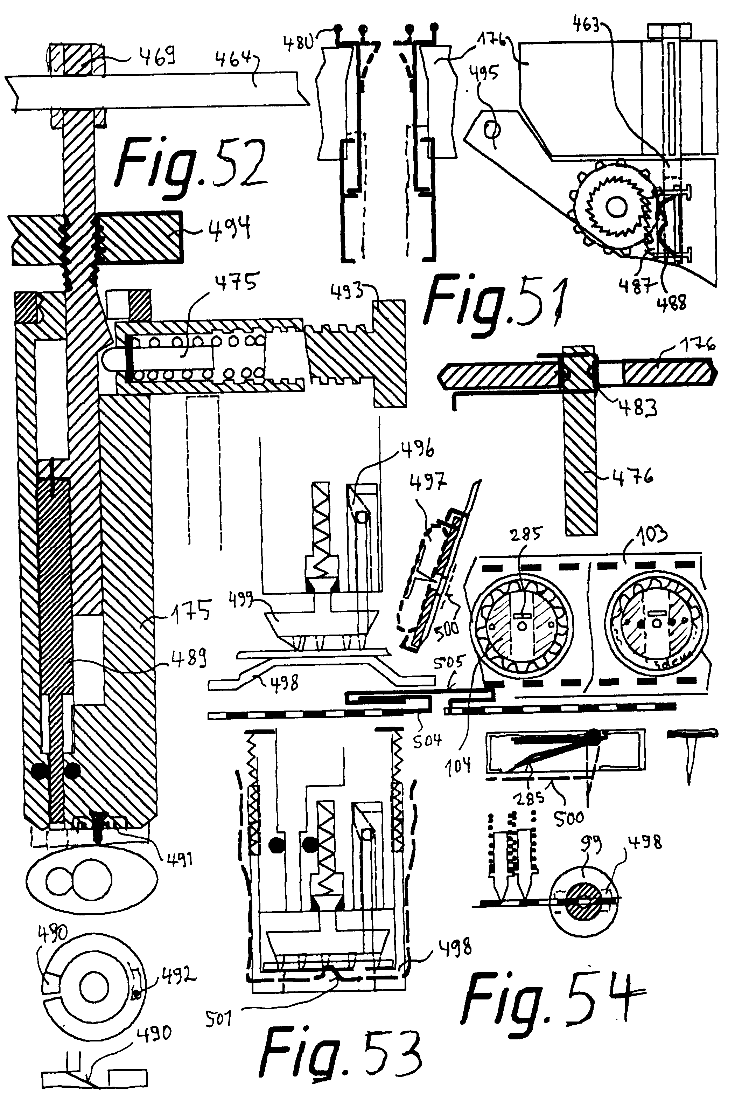

Die Figur 51 zeigt im Maßstab 2 : 1 rechts einen Längsschnitt durch die Kappe (495) mit einem der Zahnradpaare für den Teststreifentransport. Links davon ein Vertikalschnittdetail durch den Schacht für die Mechanik des Teststreifentransportes.

Die Figur 52 zeigt im Maßstab 2 : 1 einen Punktionsgriffel (175) mit spezieller Ausstattung für die Rotationsbewegung einer Schneidespitze in einem um einen kleinen Sektorwinkel drehbaren Testfeld innerhalb eines Teststreifenbandes im Längsschnitt.
Darunter ein Querschnitt etwa in Griffelmitte und tiefer ein Querschnitt am Ende des Griffels zur Demonstration des Mechanismus für die Testfelddrehung mit der Punktionsspitze (ganz unten in einer Vertikalsichtaufrollung).

Die Figur 53 gibt im Maßstab 2 : 1 Details über eine Ankoppelung eines Testfeldes an das Punktionsgriffelende oben in einem Vertikal- und unten in einem Längsschnitt (bezogen auf den Längsschnitt der Fig.52).

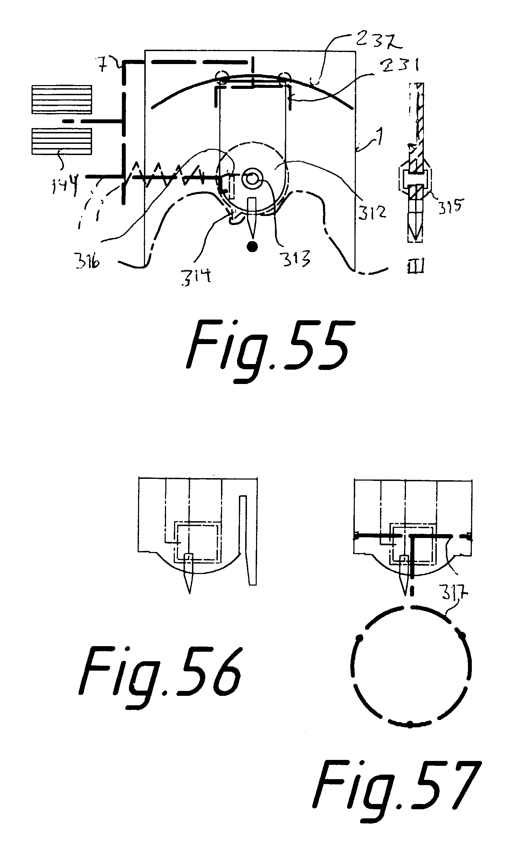

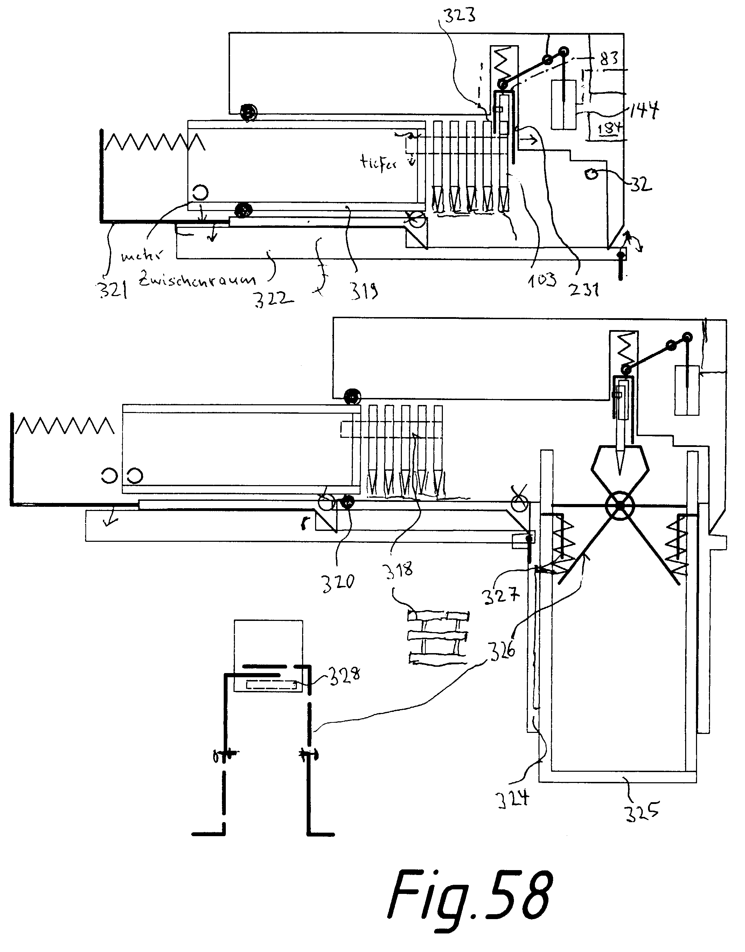

Die Figur 54 zeigt im Maßstab 2 : 1 oben im Längsschnitt nochmals eine Darstellung der Verriegelung eines Stiftes (476,vgl.Pig.50) mit der Bodenwand des Glukometers (176). Darunter eine Draufsicht auf ein Stück Teststreifen im Maßstab 2 : 1. Man erkennt die seitliche Randperforationen des Teststreifens (103).
Zwischen der mittleren Draufsicht der Fig.54 bis zwischen die Abbildungen der Fig.53 sind zwei Teststreifenenden dargestellt , die mittels der Haken (504, 505) miteinander vereinigt sind. Ein Längsschnitt darunter zeigt eine Schneidespitze, welche unter Spannung gegen einen der oberen Wandumkleidung eines Testfeldes anliegenden Schenkel nach links abgebogen ist.
Ganz unten im Längsschnitt ein Stück Teststreifen im Maßstab 2 : 1 zur Darstellung einer alternativen Schnitterzeugung. Links werden zwei parallele Keilstößel bei (nicht gezeigtem) Druck auf ihre Druckfedern zum Teststreifentransport gegen den Teststreifen gedrückt. Rechts davon eine Magnetspule für die Schneidebewegung der Schneidespitze quer zum Teststreifentransport.

Die Figur 55 zeigt linksseitig im schematischen Längsschnitt durch eine Saugglocke(1) im Maßstab 2 : 1 eine Vorrichtung ähnlich derjenigen der Fig.40 mit der gebogenen Schiene(237) für die Führung der Aufnahmebuchse(231).

Die Figur 56 zeigt in Seitenansicht im Maßstab 2 : 1 , unter starker Übertreibung der Breite des Punktionsstiftes, ein Beispiel für einen Teststreifen mit Punktionsspitze, wie er sowohl für eine Bewegung der Punktionsspitze nach dem Schwing- als auch nach dem Rotationsprinzip angewandt werden kann.

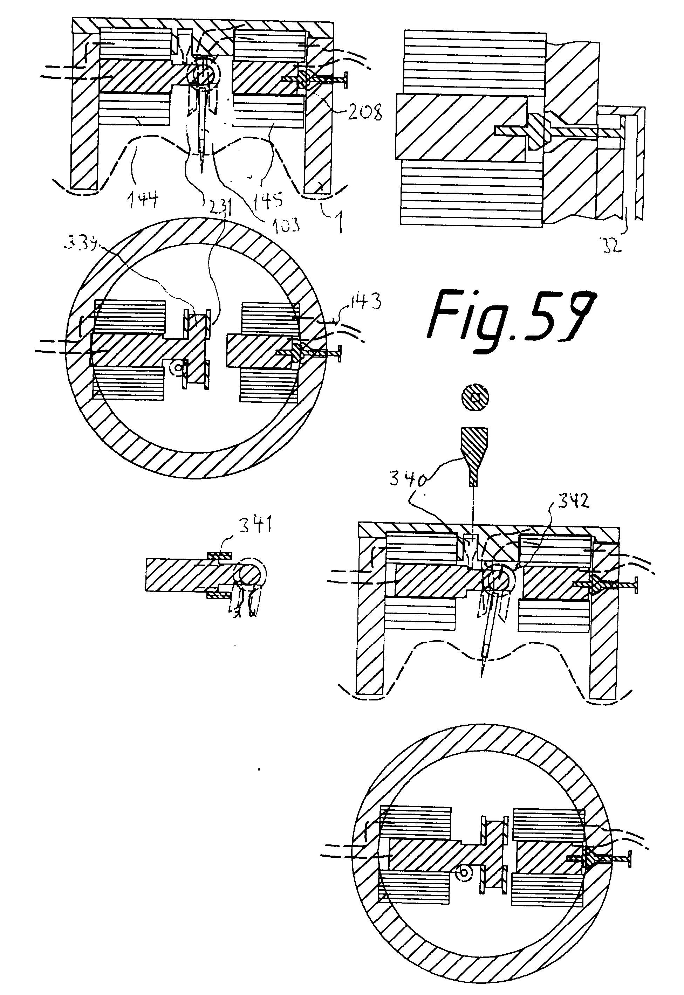

Die Figur 57 zeigt oben die Seitenansicht auf einen Teststreifen, welcher seitlich Schlitze aufweist, in welchen der Drahtring(317) Eingeführt ist, der mit seinen drei Stützen auf die Haut herabreicht.

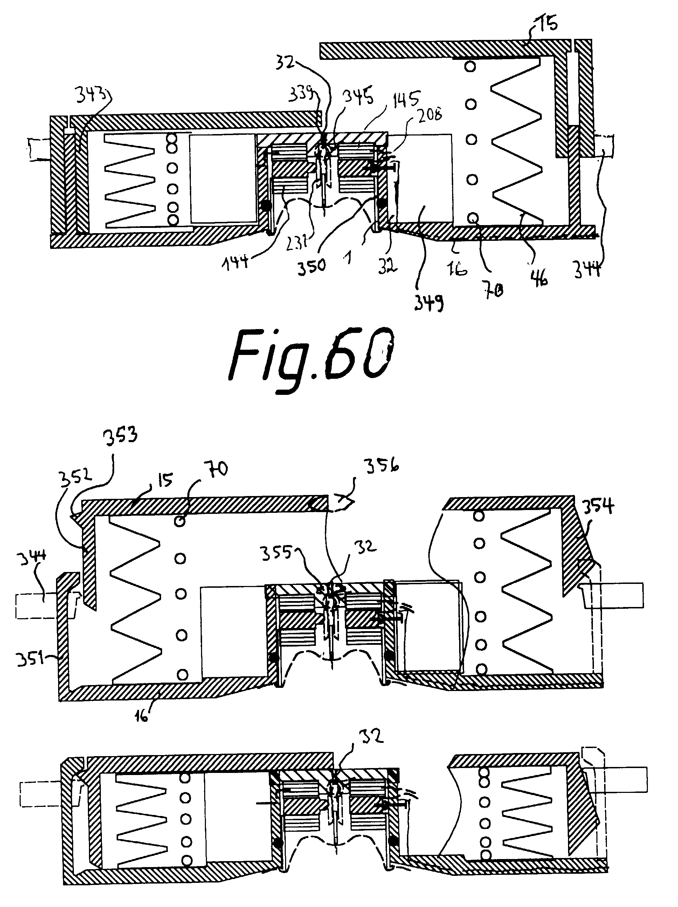

Die Figur 58 zeigt etwa im Maßstab 1,5 : 1 schematisch in Längsschnitten eine Vorrichtung zum halbautomatischen Teststreifenwechsel als Vorstufe eines Vollautomaten.
In der unteren Abbildung ist der Schieberkasten(319) mit dem Schiebedeckel zurückgezogen und die Rohrtülle(324) von unten in die Saugglocke bis zum Anschlag eines Kragens eingeschoben.

Die Figur 59 zeigt links oben einen Längsschnitt darunter einen Querschnitt durch das Detail einer Saugglocke (1) in Höhe der Tauchmagnete im Maßstab 2 : 1. Rechts ist das Detail des rechten Tauchmagneten für die Ventilbetätigung im Maßstab 4 : 1 im Längsschnitt herausgezeichnet.

Die Figur 60 bringt Längsschnitte durch eine Punktionsvorrichtung in natürlicher Größe, wobei der als Saugpumpe dienende Faltenbalg die Saugglocke (1) völlig und konzentrisch umschließt.
In der oberen Abbildung entspricht die linke Hälfte dem Stadium der Anspannung der starken Druckfeder (70) unter Verkleinerung des Faltenbalges (46), während die linke Bildhälfte den Zustand nach Entspannung der Druckfeder unter Ausdehnung des Faltenbalges zeigt. Von den unteren Längsschnitten, rechts der Mitte aufgebrochen, entspricht der oberen einer Sogstellung, der untere dem Zustand der Faltenbalgkompression.

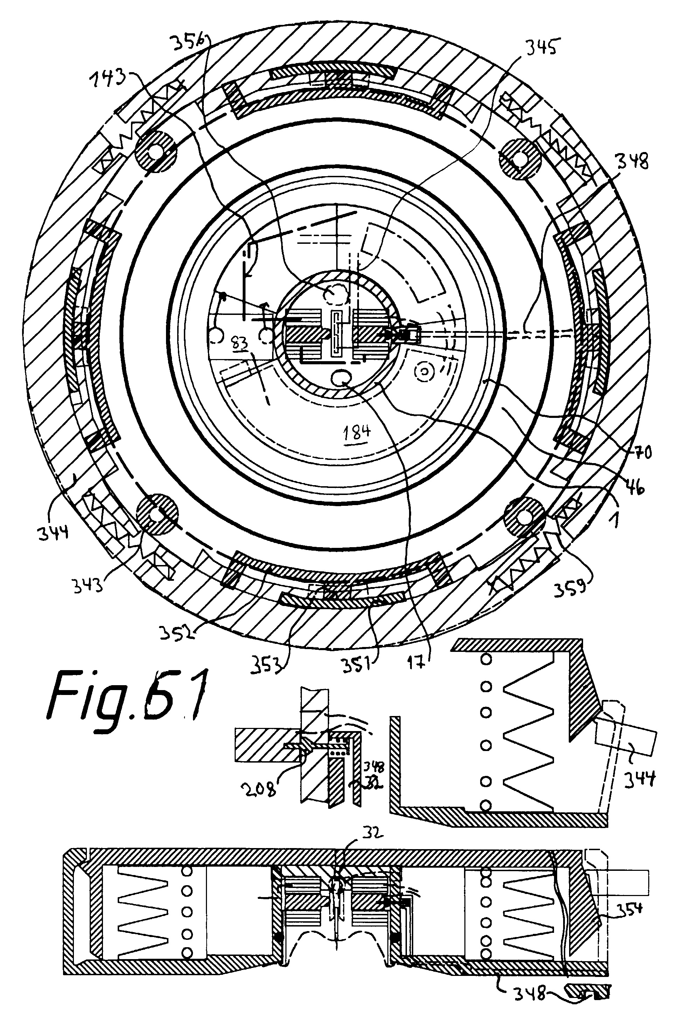

Die Figur 61 zeigt oben in natürlicher Größe einen Querschnitt durch eine Punktionseinrichtung nach Fig.60 in Höhe der Elektromagnete im Querschnitt. Darunter im Maßstab 2 : 1 links ein Längsschnitt durch das Belüftungsventil zur Saugglocke, rechts ein Längsschnittdetail der entspannten rechten Faltenbalghälfte. Unten ein Längsschnitt durch die Einrichtung unter Sektordrehung im rechten äußeren Abschnitt, um den Sperrmechanismus für die starke Druckfeder im Faltenbalg darzustellen. recht unten das kleine Vertikalschnittdetail durch die radiäre Rinne zum Sogkanal der Saugglocke.

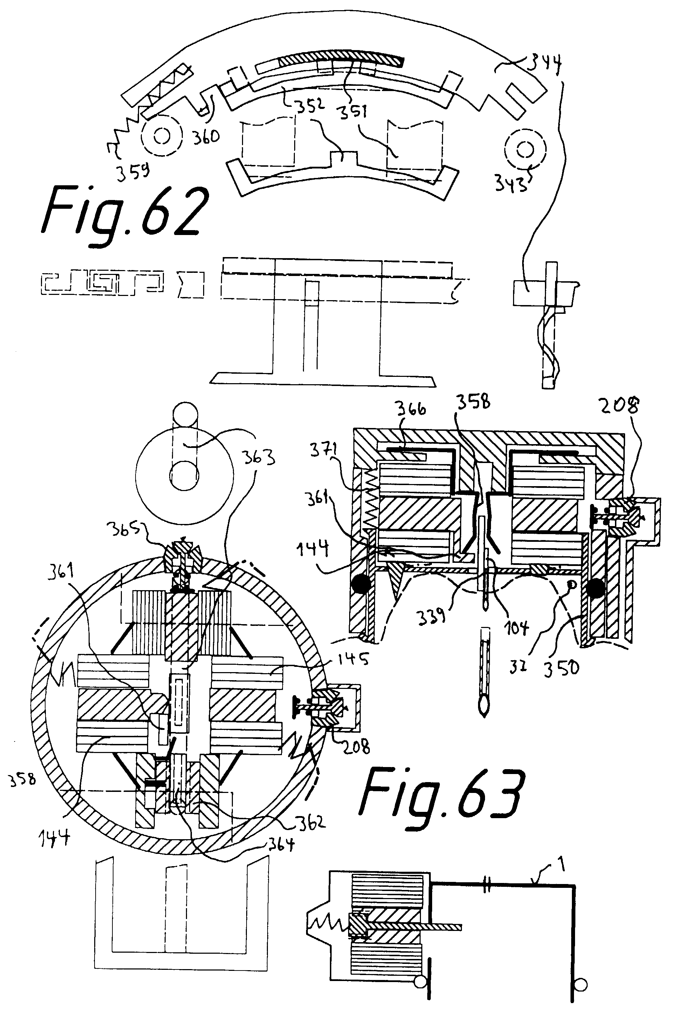

Die Figur 62 zeigt in natürlicher Größe eines der vier Segmente des Auslöseringes (344) entsprechend der Fig.61 oben im Querschnitt. Links darunter im Längsschnitt das Detail einer Verkettung der Segmente. Rechts davon in der Mitte die Seitenansicht in Aufrollung einer Rückfeder für den Auslösering (darüber im Querschnitt, bei Kippung der, rechts das Längsschnittdetail einer Federzunge.

Die Figur 63 zeigt eine Variante der Ausstattung einer Saugglocke für eine Punktionseinrichtung links im Querschnitt und rechts im Längsschnitt im Maßstab 2 : 1. Auf dem Längsschnitt wird gezeigt, wie links eine Kegelspitze durch ein Loch im Deckel des Blechringes (350) hindurch die angehobene Haut erreicht; er dient als ein elektrischer Berührungsschalter.
Links unten ist ein Fragment einer Kralle dargestellt, welche bei der Einführung und Befestigung der Magnetausrüstung in die Saugglocke dienlich ist. Rechts unten ist wird skizzenhaft die Möglichkeit demonstriert, zur Herabsetzung der Gerätehöhe Magnete (hier einen) innerhalb des Sograumes außerhalb der Saugglocke(1) zu plazieren.

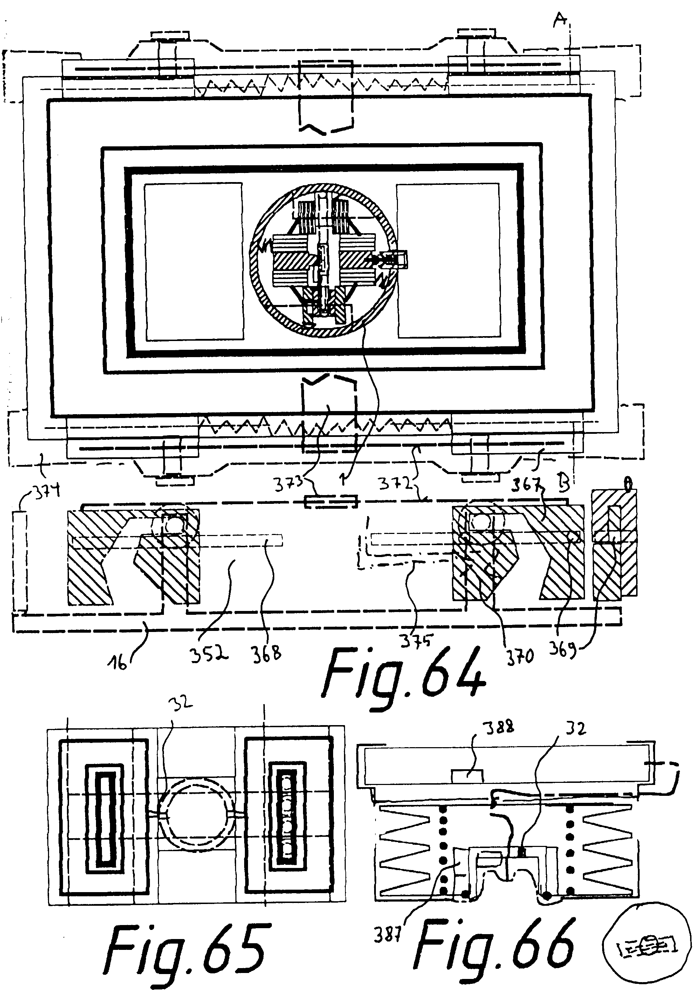

Die Figur 64 zeigt in natürlicher Größe einen Querschnitt durch eine Punktionseinrichtung ähnlich wie in Fig.60 bis 63, bei welcher aber die taschengängigere Rechteckform gewählt wurde. Die Saugglocke mit der Magnet- und Punktionsausstattung liegt zentral innerhalb des kastenförmigen Faltenbalges (46), der die Druckfeder (70) umgibt.

Die Figur 65 zeigt in einem Querschnitt im Maßstab 1 : 2 eine Einrichtung wie in Fig.64, jedoch ist die Saugglocke von zwei quadratischen Faltenbälgen mit jeweiliger Druckfeder flankiert. (Rechts ist als Alternative durch Kreise innerhalb des Falten -balges die Verwendung mehrerer runder Druckfedern in Reihe angedeutet).

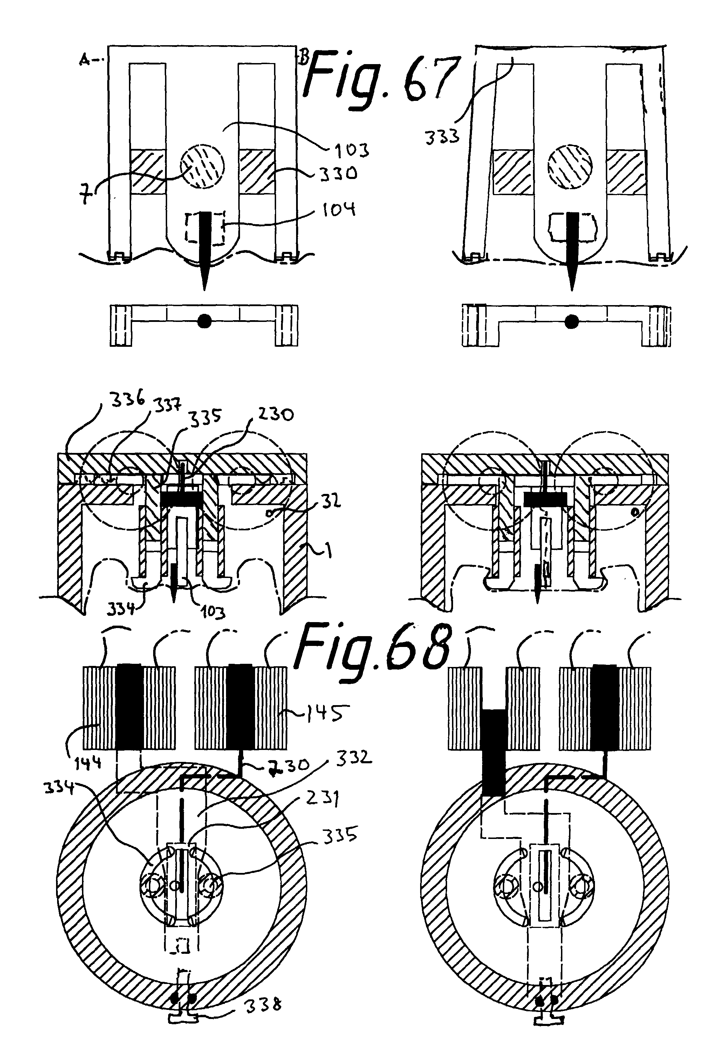

Die Figur 66 zeigt in einem Längsschnitt skizzenhaft im Maßstab 1 : 2 die Kombination einer Punktionseinrichtung mit innerhalb des Faltenbalges liegender Saugglocke, einem Elektromagnet und Batterie und Versorgungseinrichtung im Ringgehäuse mit einem handelsüblichen Meßgerät (etwa einem Glukometer).
Das Detail rechts unten skizziert in einem Querschnitt einen Elektromagneten, der als Rotor im Zentrum einer Saugglocke montiert werden kann und dessen zentrale Achse die Aufnahmebuchse mit dem Teststreifen (gestrichelt gezeichnet) trägt.

Die Figur 67 zeigt oben in einer Aufsicht oben in einem Querschnitt in Höhe der Schnittlinie A - B im Maßstab 3 : 1 - bei starker Übertreibung der Punktionsspitze - die Variante eines Teststreifens nach Fig.11 mit seitlichen Lappen, die im links gezeichneten Stadium durch Keile auseinander gedrängt werden, um den Wundspalt zu erweitern.

Die Figur 68 zeigt oben in einem Längs- und unten in einem Querschnitt das Detail einer Saugglocke mit einer besonderen Vorrichtung zur Wundspaltspreizung (bei starker Übertreibung der Punktionsspitze) im Maßstab von etwa 1,5 : 1 links im Stadium vor und rechts im Stadium nach der Wundspaltspreizung.

Figur 69 gibt in einem Längsschnitt in natürlicher Größe eine Punktionseinrichtung für den Mehrfachgebrauch auch bei verschiedenen Personen wieder. Unter der Saugglocke ein Querschnitt durch diesselbe.

Die Figur 70 zeigt im Längsschnitt schematisch eine Saugglocke(1) zum Einmalgebrauch mit umgebendem Vakuumspeicher (383), der industriell evakuiert ist. Die Spannung des gegen den Träger der Kapillare gerichteten Federbogens wird durch die Elektrodrähte (384) gehalten.

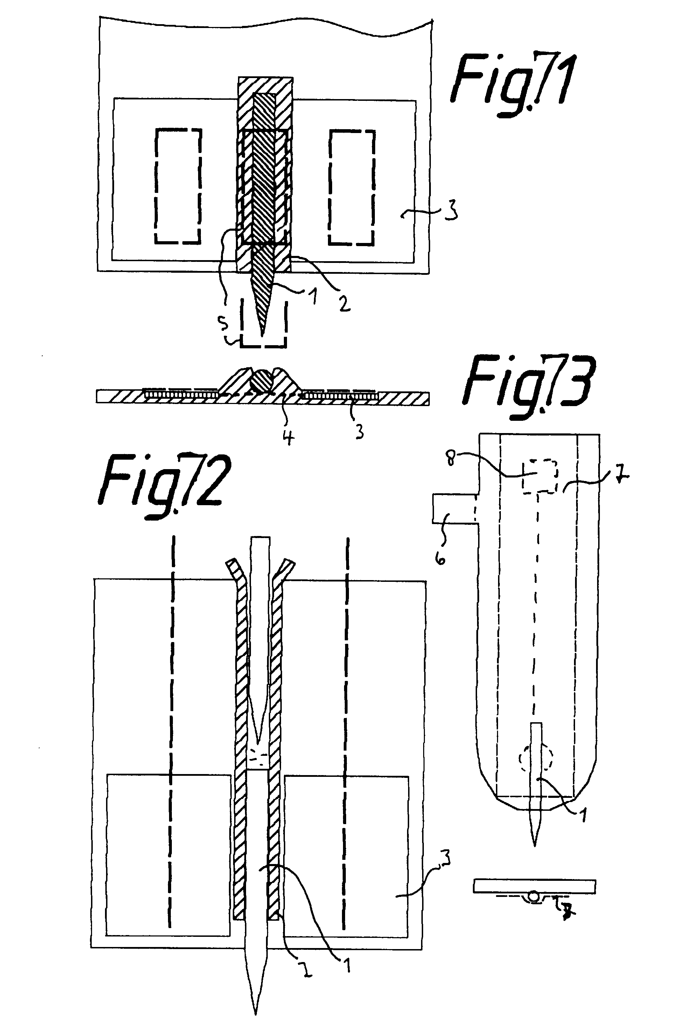

Die Figur 71 zeigt im einem Längsschnitt durch den unteren Teil eines Teststreifens etwa im Maßstab von 1 : 15 den bereits angespitzten Stahlstift(1), der in eine Art Röhre(2) aus Kunststoff gesteckt oder mit in die Kunststofform bei der Einbettung der Reaktionsfelder in eine solche Röhre geriet. Der Querschnitt zeigt die Variante einer Hohlrinne mit vorspringenden Kanten, hinter welchen der Stift festgehalten wird.

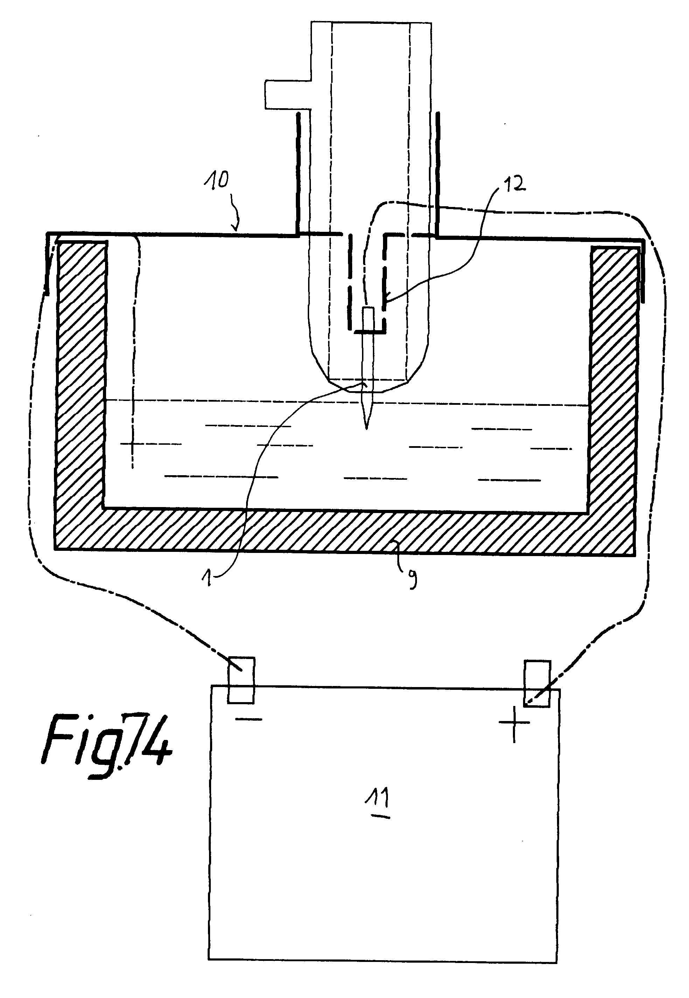

Die Figur 72 entspricht in ihrer Dsrstellungsweise der Fig.2 und wird ebenfalls im Längsschnitt gezeigt. Die Kunststoffröhre(2) setzt sich in diesem Falle fort. Es wurde von oben ein zweiter kleinerer Stift als Kontaktstift eingeführt. Zur Stromleitung kann sich zwischen Stiftende(1) und oberem Kontaktstift eine Elektrolytflüssigkeit befinden.

Die Figur 73 zeigt einen handelsüblichen Teststreifen in der Ansicht von der Rückseite im Maßstab von etwa 4 : 1, wobei die Stärke(Breite) des Stahlstiftes(1) noch weit übertrieben wurde. Dieser ist von der Rückseite dem Teststreifen so aufgeklebt, daß der Spitzenbereich den Teststreifen überragt. Zur Befestigung dient noch verstärkend die Folie(7), wie auf dem Querschnitt unten erkennbar ist. Die Stromzufuhr für das Elektrolytbad erfolgt über eine Leitung zum Metallfolienfleck(8).

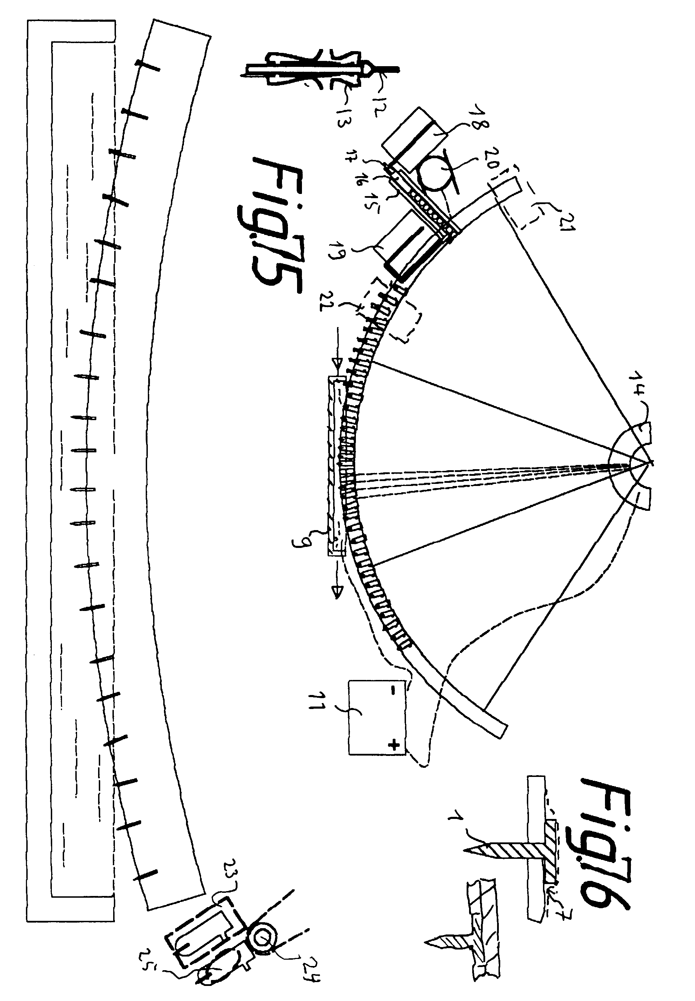

Die Figur 74 zeigt eine Grundanordnung zur Spitzenherstellung an einem Stahlstift(1). Die Wanne(9) ist bis in vorbestimmter Höhe mit 20%iger Schwefelsäure gefüllt.

Die Figur 75 zeigt oben in einem Maßstab von etwa 1 : 4 , unten in etwa natürlicher Größe - in Wirklichkeit natürlich (abgesehen von den noch kleineren Stahlstiften) noch großzügiger dimensioniert. schematisch in einer Draufsicht und teilweisem Längsschnitt (durch die Wanne) ein Fertigungsverfahren unter Anspitzung der Stahlspitze im Säurebad, das unten etwa in natürlicher als Ausschnitt gezeigt wird. Links das Detail eines Teststreifens mit noch nicht geschlossenen Verpackungsfolien im Vertikalschnitt. Rechts unten die Stanze(23) für die Gewinnung des Teststreifens aus dem Band und rechts davon die Rolle(249 mit der Verpackungsfolie, die um 90 Grad gekippt dargestellt ist, mit dem Stempel(25) für die Ausformung der Einzelverpackung.

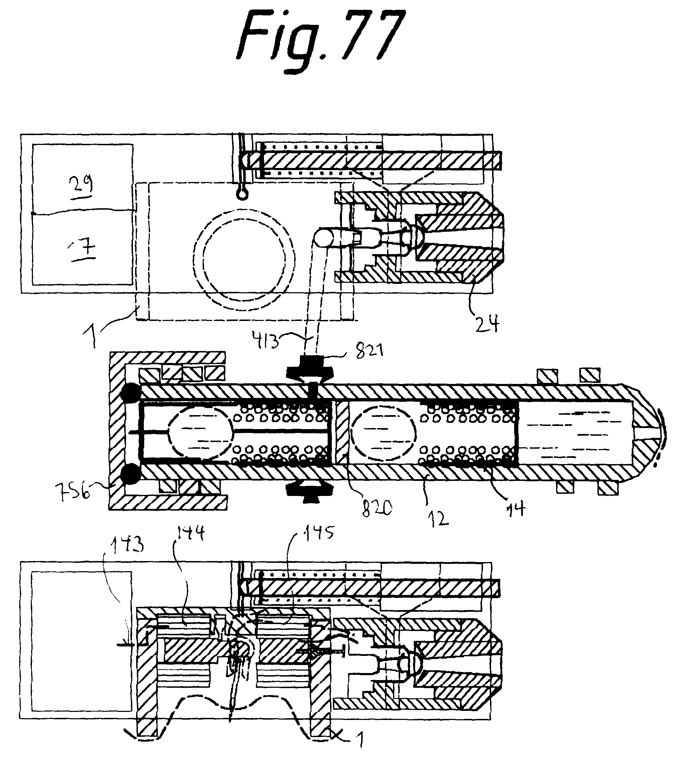

Die Figur 76 zeigt in einem Längsschnitt durch das Ende eines Teststreifens im Maßstab von etwa 4 : 1 einen angespitzten Stahlstift(1), der quer zum Teststreifenverlauf dort durch ein Loch gesteckt ist (das umgebende Testfeld wurde weggelassen).
Die Figur 77 zeigt im Vertikalschnitt oben im Maßstab 2 : 1 einen Arzneiinjektor mit Saugglocke(7), Gasstrahlpumpe(24), Batterie(29) und Steuerzentrale (17) in natürlicher Größe. Die Punktionsmittelausstattung zu diagnostischen Zwecken entspricht derjenigen der Fig.59. Im oberen Längsschnitt ist die Lage des Schraubgewindes für die Treibsatzpatrone angegeben, im unteren die Ausstattung mit den Elektromagneten.

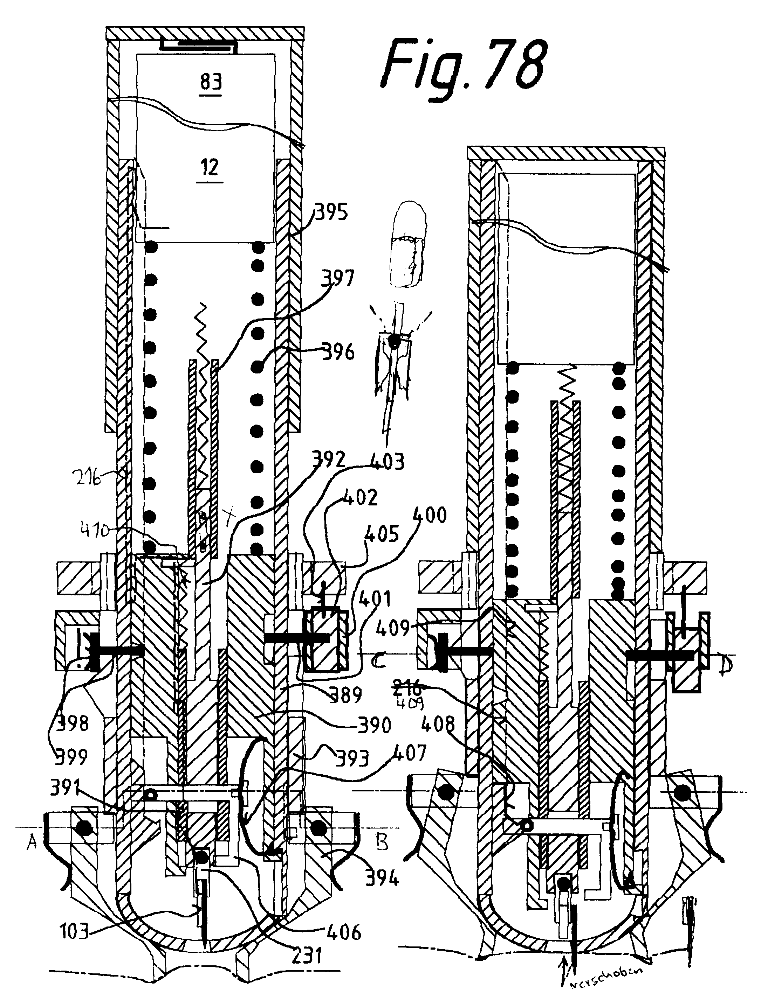

Die Figur 78 zeigt im Längsschnitt im Maßstab 1 : 1 eine Punktionseinrichtung mit einer Punktionsspitze an einem Teststreifen ohne Anwendung einer Saugglocke. Links wird die Phase vor und rechts die Phase während der Punktion dargestellt.

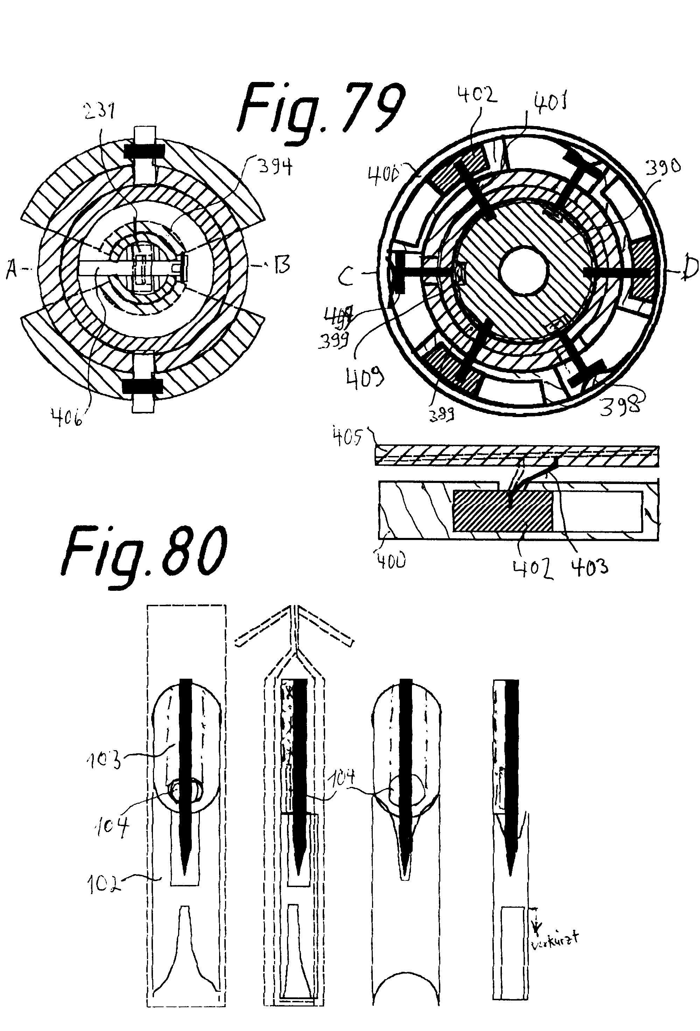

Die Figur 79 zeigt Querschnitte längs der Schnittlinien A - B und A - C des Längsschnittes der Fig.78 , rechts unten eine seitliche Aufrollung als Detail eines Verstellmechanismus für die Länge der Stiftquerbewegung.
Die Figur 80 zeigt einen Teststreifen links in Verpackung, rechts nach Benutzung in Längs- und Vertikalschnitt, Maßstab 2 : 1.

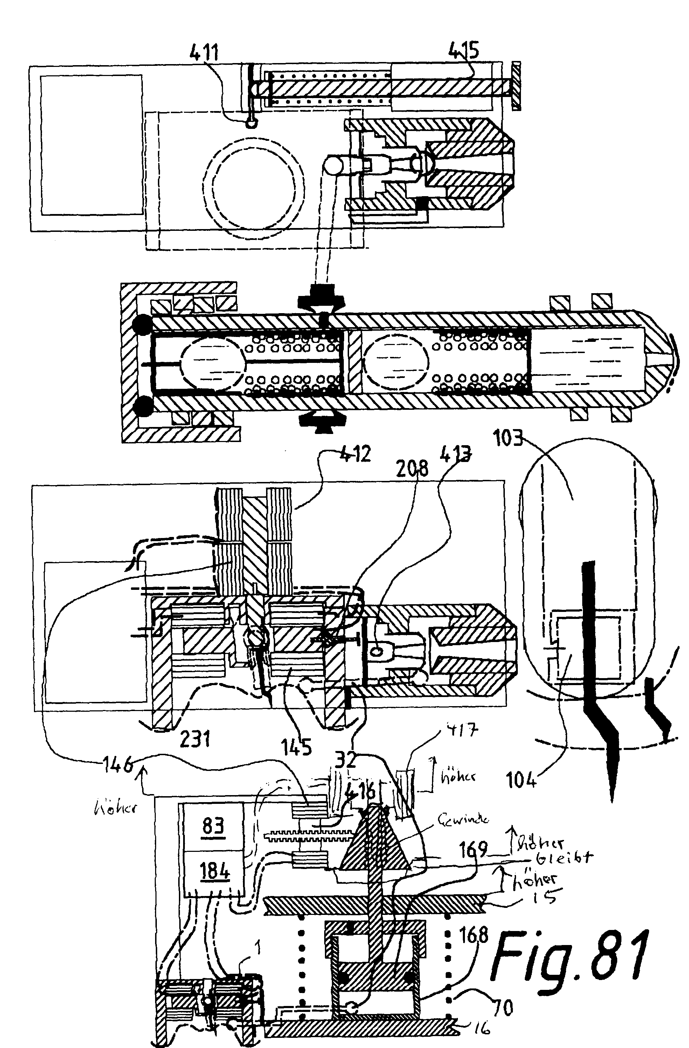

Die Figur 81 wiederholt im Längsschnitt die Fig.77 und ergänzt deren Beschreibung. Der untere Längsschnitt stellt eine Variante dar, die durch Magnete für eine zusätzliche Rotation und eine Absenkung des Teststreifens außer dessen Abschwenkung nach dem Schwingprinzip ausgestattet ist. Rechts davon ein Teststreifen im Maßstab 4 : 1 mit Bajonettkrümmung des Punktionsstiftes.

### Ausführliche Beschreibung der Ausführungsbeispiele

Die Figur 1 zeigt in natürlicher Größe in der Mitte rechts einen Horizontal- oder Querschnitt durch eine Punktionseinrichtung. Rechts oben leicht vergrößert das Detail eines Druckgeberauslösers im Längsschnitt in drei Funktionsstadien. Unten rechts ein schematisierter Längsschnitt links durch die Saugglocke, rechts durch den Faltenbalg der Saugpumpe. Links oben zwei schematisierte Darstellungen der Kraftübertragungs- und Steuerungsschieber in zwei Funktionsstadien. Darunter, links von der Saugglocke im Querschnitt, ein Längsschnitt durch die Saugglocke, darunter ein solcher durch den Faltenbalg in ausgedehntem Zustand. Beginnend mit dem Querschnitt ist zu erkennen, daß der Stößel(7) durch den Dichtungsring(5) in der Saugglockenwandung hindurchgeschoben ist und mit seiner gerundeten Kuppe dem Teststreifen(103) anliegt. Auf seinem anderen Ende ist längs eines Gewindes die Einstellschraube(54) für die Begrenzung der Stößelbewegung in Nähe seines Hindernisses, der Klappe(18), die auf den Stößel vor einem Teil der Einstellschraube herabreicht.
Die Klappe - sie ist gestrichelt dargestellt - ist um den paarigen Achsstift(19) drehbar, welcher symmetrisch am Gehäuseblock der Grundplatte(16) befestigt ist. Längs der Seitenwände des Gehäuseblockes ist der Schieberahmen(20) montiert, dessen Ende die beiden Druckfedern(30) jeweils anliegen, die sich auf der Gegenseite gegen die Stützplatten(21) am Gehäuseblock abstützen.
Vom Schieberahmen ausgehend richtet sich eine Rohrmuffe gegen die auf dem Stößel verschraubbare Justierschraube(22). Die auf dem Stößel nachfolgende Rändelschraube(23) findet ihr Widerlager im Puffer(26), dessen dem Stößelende zugewandte Keilflanke und Rückfederung mittels Druckfeder links auf dem Längsschnitt verdeutlicht wird, und der in diesem Falle paarig symmetrisch angelegt ist. An der Drucktaste(222) für einen Nachschnitt nach erfolglosem Einsatz über Einstellschraube(54) entspringt der Haltearm für den Sperrknopf(28,vgl.Fig.7 unten), der in eine Bohrung der Auslöserplatte(53) eingreift. Der paarig angelegte Führungsstift(31) ist am Gehäuseblock befestigt und verläuft in einem senkrechten Führungsschlitz der Drucktaste. (Dies ist links oben in den schematischen Längsschnitten zu erkennen.)
Im Schrägschlitz der Platte der Drucktaste wird der Stift(33) geführt, der vom Schieberahmen(20) ausgeht, in welchem für seiner Schiebebewegung entsprechende Fensterausschnitte für den Führungsstift(31) und den Achsstift(19) vorhanden sind. Der horizontale Schlitz, in welchen der Schrägschlitz mündet, erlaubt bei Tiefstand der Drucktaste die Rückführung des Schieberahmens unter Wirkung der Druckfeder(30)unter Nachschnittauslösung.
Auf dem oberen Längsschnitt ist das Stadium wiedergegeben, in welchem durch Absenkung der Drucktaste mittels des Schrägschlitzes der Schieberahmen nach links bewegt wurde. (Über eine Brücke sind die beiden symmetrisch angelegten Drucktasten miteinander zu einer verbunden.) Die Hilfsfeder(34) unter Drucktaste und gegen die Boden- oder Gehäuseplatte gestützt wurde dabei gespannt. Die Klappe(18) war schon zuvor mit der Deckelhebung um den Achsstift mittels der Zugsaite((36,Fig.4 Mitte) nach oben geklappt worden. Die funktionellen Veränderungen zum Zustand der Drucktastenhebung gehen aus dem Vergleich mit dem Längsschnitt darunter hervor. Auf dem Längsschnittsdetail durch die Saugglocke unten geht die Lage des Stößels(7) mit Dichtungsring(5) und der Druckfedern(30) hervor. Es wurde auch noch die Einmündung des Sogkanals (32, vgl.Fig.7) skizziert.

Auf dem Längsschnitt links ist der Klappdeckel(206) eingezeichnet, der gegen den Dichtring(38) das Saugglockeninnere abdichtet. Er ist um das paarig angelegte Scharniergelenk(39) zur Saugglocke hin gefedert(vgl.Fig.5,Längsschnittsdetail rechts oben).Der Teststreifen ist symbolisch durch einen Strich ersetzt, um zu demonstrieren, daß dieser beim Deckelschluß auf den Stößel(7) zurückschiebend wirkt. Auf das Ende des Stößels, das zweckmäßigerweise keinen quadratischen Durchmesser hat - etwa ovalär ist - ist die Hülse (41) aufgeschoben, deren exzentrische Endplatte eine gegen den Teststreifen(103) gerichtete Noppe aufweist, den Anschlagspunkt für den Druckgeber nach unten verlagernd.
Der Schacht(62) unten im Deckelzentrum zur Befestigung des Teststreifens läßt die Ableitungskontakte und Leitungsenden für die Signalübermittlung erkennen.
Rechts wird das Ende des Hebels(42,vgl.Fig.5 Mitte) zur Öffnung des Saugglockendeckels über einer Randleiste der Saugglocke gezeigt, links (gestrichelt) eine Rückstellfeder(43,vgl.Fig.4 Mitte links) für den Stößel als Lösungsvariante.
Die Spannung der paarigen Druckfeder(30)erfolgt über den paarigen Keilschieber(37), der von einem von der Deckelplatte(15,Längsschnittsdetail oben rechts) herabreichenden Schieberhaken(44) abgedrängt wird.
Der Längsschnitt rechts oben zeigt links das Stadium vor dieser Abdrängung, rechts daneben dasjenige der vollständigen Abdrängung unter Sperrung der Keilschieber- und damit der Srößelrückbewegung. Bei der Schieberhakenabsenkung wird von dessen Ende die an der Deckelplatte befestigte Zugfeder(218) gespannt und der Schieberhaken an der elastischen Zunge am Ende des Keilschiebers(37) festgehalten. Da der Schieberhaken - an einer nicht dargestellten Stift- in Rillenführung - dort die ihn umfassende von der Deckelplatte herabreichende Schiebemuffe nicht verlassen kann, kommt es während der Hebung der Deckelplatte durch die starke Druckfeder(70) um den Faltenbalg(46) herum zur Hebung der elastischen Zunge durch den Haken des Schieberhakens, wonach dieser unter Wirkung der Zugfeder nach oben schnellt und die Bewegung des Keilschiebers freigibt(ganz rechts im Detail). Unten links und rechts ist im Längsschnitt das Detail des Faltenbalges(46) als Saugpumpe wiedergegeben, links im Zustand der Balgausdehnung durch die Druckfeder(70), rechts im zusammengepreßten Zustand. Das Hülsensystem ist auf Strichstärke vereinfacht. Der Topf(41) deckelt oben den Faltenbalg(46) dicht ab und trägt unten eine zentrale Tülle mit Außengewinde. Tn letztere ist die Schraubkappe(45) mit Innengewinde eingeschraubt, deren Bewegung innerhalb der zentral durchbohrten Wanne der Deckelplatte (15) nach einer Schiebestrecke oben von einem Stift am Wannenrand gehemmt wird. Man erkennt deutlich, daß bei herabgeschraubter Schraubkappe die Topfanhebung mit Faltenbalgentfaltung im rechts dargestellten Falle sofort beginnt, während beim Schraubkappenstand links bis zur Topfanhebung eine Freistrecke gegeben ist, also eine geringere Sogentwicklung.
Über den Rand der Gehäuseplatte(16) gleichmäßig verteilte Nischen oder Schächte dienen dem Einrasten und der Bewegungsbegrenzung von Federzungen(55.vgl.Fig.7), welche dem Federblechkastenrahmen(48, vgl.Fig.7) entspringen und eine Abfederung beim Aufsetzen der Saugglocke auf die Haut ermöglichen. Die drei Stützhülsen(64) nehmen die drei Deckelstifte(213) auf, welche der Deckelplatte entspringen(Fig.4,15 rechts oben).
Die beiden Auslöser(52) entsprechen in einer Variante demjenigen in Fig.16 rechts in Kombination mir den zwei Teleskopgestänge(69) jedoch der Auslöservariante nach Fig.8 .
Die Verbindung mittels der Befestigungsangeln(56) Zum Meßgerät hin wird in den Fig.8 (oben) und 6 (unten) näher angegeben.
Die Verhältnisse im Klappdeckel und Deckelplattenbereich werden in und zu Fig.4 und 5 näher erläutert, die Verhältnisse im Bereich der Auslöserplatte in Fig.4 und 7, eine bevorzugte Sogauslöserkonstruktion in den Fig.8 und 9.
Der Keilschieber(37) kann für ein Stiftende mit (halb-)kugeligem oder kegeligem Querschnitt eine Gabel, für einen Leistenhaken eine (Keil-)Schräge aufweisen (Quer- und Längsschnittdetails ganz oben rechts)

Die Figur 2 bezieht sich auf eine Einrichtung wie in Fig.1 . wobei als Variante für eine wundspaltsperrende Weiterbewegung der Punktionsspitze über eine gebremste Stößelbewegung eine besondere Vorrichtung vorgesehen ist. Im Maßstab 2 : 1 ist oben ein Vertikalschnitt- und darunter ein Querschnittsdetail längs der Schnittlinie A - B des unteren Querschnittes durch den Hauptteil der Einrichtung dargestellt. Der Hebelarm(58) wird mit seinem Querbalken am Ende durch eine Druckfeder mit Gegenlager an Stiften aus dem Gehäuseblock der Grundplatte(16) in Gegenrichtung zur Stößelbewegung gehalten und abgefedert. Der längere Winkelarm am anderen Ende dient der Rändelschraube für den Nachschnitt zum Anschlag. Die um den Hebelarm frei drehbare Schaltrolle(59) ist in einer Mulde des Geräteblockes gelagert und weist einen Segmentschlitz auf. Letzterer läßt eine kurze Querstange am Hebelarm erst durch, wenn die Schaltrolle durch den von der Drucktaste(222) ausgehenden in eine Randkerbe der Schaltrolle eingreifenden Stift während der Abwärtsbewegung der Drucktaste gedreht wird. Die Rändelschraube, die sich nach der plötzlichen Nachschnittbewegung gegen den Winkelarm stützte kann sich nun gedämpft durch die geschilderte Druckfeder für den Hebelarm noch ein Stück weiter saugglockenwärts bewegen. Auf dem Längsschnittdetail ganz unten kommt die leichte Exzentrizität zwischen Schaltrolle(58) und Druckfeder zur Darstellung. Der gestrichelte Ring um die Saugglocke entspricht der Möglichkeit einer Blattfeder, die dann zweckmäßigerweise exzentrisch der Rändelschraube entgegen gelagert wird und deren Endbewegung alternativ dämpfen kann.

Die Figur 3 zeigt im Maßstab von etwa 10 : 1 im schematischen Längsschnitt eine Sonderform eines Teststreifenendes mit zwei Punktionsnadeln am Ende. Diese Punktionsnadeln setzen sich in zwei Lamellen dieses Teststreifens fort, in welche sie in den Spitzen konvergierend eingeschweißt sind. Ein zentraler Keil wird nach dem Einstich der Doppelspitze in die Haut bei Höhenfixation der Teststreifenlamellen (durch die beiden Rechtecke symbolisiert) zwischen die Punktionsnadeln abgesenkt und dabei eine (gestrichelt dargestellt) Brückenverbindung zwischen den Lamellen gesprengt. Der Querschnitt unten längs der Schnittlinie A - B des Längsschnittes zeigt die Hautspaltbildung für den Blutaustritt auf die oder das Testfeld(104), das hier im Keil gelagert ist (aber auch in den Lamellen plaziert sein kann).

Die Figur 4 zeigt in natürlicher Größe über dem Querschnitt der Vorrichtung, der demjenigen in Fig.1 entspricht und der Orientierung dienen soll, oben in zwei Funktionsstadien Längsschnitte durch den Faltenbalg. Dieser wird durch einen (nicht dargestellten) Drahtbügel von einer Torsionfeder angehoben(vgl.Fig.15 unten).
Die Sogstärkenregelung erfolgt über die Drehung des Schraubbolzens(63, der mit einem Sechskant in einen ebensolchen in einer Schraubmutter eingreift, deren Außengewinde in das Innengewinde der Sockeltülle Innengewinde eingreift, die fest mit dem Topf als Deckel des Faltenbalges verbunden ist. Abgerundete Kanten lassen den Sechskant des Schraubbolzens sicher in den Sechskant der Schraubmutter eindringen. Das freie Bewegungspiel für die Anhebung der Deckelplatte ist in der oberen Darstellung größer eingestellt als in der unteren.

Rechtsseitig ist noch aus einer hinteren Ebene in die Mittellinie vorgezogen und etwas verkleinert einer der drei Deckelstifte(213) innerhalb der an der Grundplatte befestigten Stützhülse(64). Letztere weist einen Schlitz für den Durchtritt der Auslöserplatte(53,vgl.Fig.7) auf, welche nach links bewegt, die Bewegung des Deckelstiftes freigibt.
Linksseitig wird die Deckelplatte(15)durch die Scharnierklappe(65) verlängert, deren zugespitzter Endbalken auf den Keilschieber(37, vgl.Fig.1)- parallel zum Endbalken direkt am Ende der Deckelplatte für den parallelen Schiebekeil - herabreicht.
Die untere Abbildung zeigt die Scharnierklappe nach Rückzug des Schiebers(66) von der Noppe der Scharnierklappe um ihr Scharnier hochgeklappt, um den Klappdeckel der Saugglocke vorbeizulassen (vgl.Fig.5).
In der Mitte links ist über einem Längsschnittsdetail der Saugglocke ein der saugglockenabgewandte Teil des Querschnittes (unten) wiederholt und zugleich abgewandelt.
Die Tülle des Schieberrahmens(20) wird hier von einer paarigen Blattfeder als Rückstellfeder(43) mit Verbindungssteg von der Justierschraube(22) in Distanz gehalten und damit auch die Einstellschraube(54)von der Klappe(18). Dies geschieht jedoch nur während der Passage der Rückstellfeder, die an den Verbindungslaschen des Klappdeckels(206) befestigt ist. Die Permanentmagnete(67) oder andere Raster sichern die Stellung und damit einen Vorlauf für einen Rammstoß des Stößels(7).
Gleitkugeln(68) zwischen Schieberrahmen und Gehäusblock können einem Verklemmen entgegenwirken. Wie das Längsschnittsdetail darunter zeigt, wurde die Stößelachse höher angeordnet und die Druckfeder(30) - hier nur noch eine einzige - darunter angeordnet.

Sie wurde um eine Kolbenachse gelegt, die am Schieberrahmen(20) befestigt ist und gegen einen Kolben gerichtet, welcher die Führung erhöht. (Wegen Wegfalles der seitlichen Druckfedern kann der Gehäuseblock schmaler gestaltet werden). Rechts (und mittig) wird ein Vertikalschnittsdetail durch den Bereich von Stößel und Druckfeder(30) gezeigt.
Darüber befindet sich noch in einem Längsschnittdetail die Darstellung der Zugsaite(36), die hier über eine Rolle der Klappe (18,vgl.Querschnitt unten) entgegengeführt wird und sich unter dem Deckel in eine Zugfeder fortsetzt.
Darunter bis ganz nach rechts unten reichend das Längsschnittsdetail durch einen Startmechanismus für die Sogauslösung.
Der Auslöser(52) liegt mit seiner Keilschulter einer Winkelkante der Auslöserplatte(an) und wird von einerblattfeder auf der Deckelplatte nach unten gehalten. Geführt wird der Auslöser zentrisch auf einem Stift der Gehäuseplatte(16). Durch eine Bohrug in letzterer ist ein Teleskopgestänge(69) hindurch gesteckt, das am Ende einen Blattfederbogen aufweist, der gegen eine Nase am Auslöser(52 gerichtet ist, wobei der Auslöser drehgesichert ist. Der Verbindungssteg(71) verbindet die Stangenenden zweier solcher Teleskopstangen unterhalb des Faltenbalges(721,vgl.Querschnitt unten). Wird das Teleskopgestänge durch Druck vom Verbindungssteg her maximal gehoben, so wird der paarige Auslöser angehoben und die Auslöseplatte nach rechtsverschoben, wodurch die Deckelstiftbewegung freigegeben wird (Längsschnittsdetail rechts oben). Bei Zug an dem Teleskopgestänge überholt dessen Blattfeder die Nase am Auslöser; es kann jetzt analog zu Fig.12 die visuelle Hautkontrolle vorgenommen werden. Unter dem Längschnittdetail die Quersschnitte durch Auslöser und zusammengeschobenem Teleskopgestänge.

Die Figur 5 zeigt links oben in natürlicher Größe den Klappdeckel zu einer Einrichtung nach Fig.1 in einer Draufsicht in Projektion auf den Grundriß der Saugglocke in der Mitte links.
Der quere paarige Achsstift(71) mit den Deckellaschen - hier etwas nach innen verschoben der Einrichtungsvariante Fig.4 Mitte links entsprechend - umrahmt das Band der Signalleitungen(72) - hier drei - vom in der Breite überzeichneten Schacht(62) mit seinen Klemmfedern, um die Leitungen auf dem Teststreifende mit den Signalleitungen in Kontakt zu halten. Rechts des Querschnittsdetails eine Seitenansicht mit Aufbruch, um die Lage des Teststreifens(103) und die Torsionsfeder(74) für den Deckel-schluß zu zeigen.
Der Keil aus einer Federblechumbiegung an der Auslöserplatte(53) im Längsschnittdetail in der Mitte oben verdeutlicht eine Variante der Sogauslösung durch Verdrängung dieses Keiles nch rechts von einem (als Strich symbolisierten) Starttaste aus.
Das Querschnittsdetail (obere Mitte) entspricht einer Aufsicht auf die Deckelplatte(15). Die Scharnierklappe(65) ist durch Linksverschiebung des Schiebers(66) bis zum Anstoß seines Randes an deren Noppe verriegelt. Der Linksschub erfolgt durch die entsprechend gelagerte Blattfeder(76), die aber zweckmäßiger unter der Deckelplatte angeordnet ist und dort gegen einen Stift am Ende des Schiebers innerhalb einer Schlitzführung in der Deckelplatte erfolgt, wobei die beschriebene Noppe auf der Schar-nierklappe entfallen kann. Im dargestellten Beispiel wird der Schieber in einem Binnenschlitz geführt. Er muß nach rechts gegen die Blattfeder zurückgeschoben werden, was durch die Öffnung des Klappdeckels über der Saugglocke erfolgt. Die Lage dreier Auslöser(52) in der Grundplatte unter der Deckelplatte um den Faltenbalg(46) herum ist eingezeichnet.
Der mittels einer Schwenkachse auf dem Geräteblock der Grundplatte befestigte
Hebel(42) zur Saugglockenöffnung für die Wiederbelüftung derselben ist in einer Seitenansicht unten mit seiner nach unten ausschwenken Feder zur Aufrichtung des längeren Hebelarmes herausgezeichnet.

Die Figur 6 zeigt Varianten der Befestigung eines Meßgerätes an der Punktionseinrichtung nach Fig.1.
Oben ist in einer Aufsicht ein Befestigungsrahmen, in welchen ein Meßgerät mit Schacht(262) für das Einschieben einer Trägerlamelle mit den Signalkontakten eingezeichnet, welcher mit der paarigen Befestigungsangel in eine Leiste an der Grundplatte(16) eingelassen ist und unter dieses abgeschwenkt werden kann.
Darunter zwei Detailvarianten lösbarer Rahmenverbindungen mit der Grundplatte(16). An dem Meßgerät oder seinem Befestigungsrahmen befestigte Federn rasten im oberen Beispiel in Nischen mit Randhinterschniedungen ein.
In der untersten Querschnittdarstellung werden die Befestigungsangeln des Meßgerätes mittels einer Blattfeder von der Grundplatte aus je in eine seitliche Hinterschneidung der Gelenknische gedrückt. Das Meßgerät wird zur Lösung der Verbindung zuerst nach links gegen die Blattfeder verschoben. Das Detail in der Mitte zeigt, wie die Hinterschneidung durch eine Federblech-abdeckung ersetzt werden kann, die zugleich die federnde Schibezunge für die Befestigungsangeln in die Nische hineinragen läßt.(Die Befestigungsangeln wurden hier nicht eingezeichnet).

Die Figur 7 zeigt oben ein Querschnittsdetail, unten ein Längsschnittdetail durch eine Einrichtung nach Fig.1 in natürlicher Größe. Es soll Lage und Form der Auslöserplatte(53) verdeutlicht werden. Diese ist in mehrere Segmente geteilt,
um Bewegungssspielraum vor den ihren Verlauf kreuzenden Funktionsgliedern zu schaffen. An Stellen besonderer Platzknappheit in der Horizontalen ist die Platte durch Aufbiegungen in die Höhe verstärkt. (Ein solches Beispiel bietet die Verbindung zwischen dem Rastersegment im Eingriff innerhalb des rechten Deckelstiftes und einer Stützhülse(64)hinter dem Telskopgestänge(69) zum Auslöser (52,vgl.Fig.4 Mitte rechts) hin, welcher die Auslöser-platte bei seiner Anhebung nach links verschiebt.
Die paarige beiderseits in Wandnischen der Grundplatte verlaufende Federzunge(55) sorgt für Rückfederung der Auslöserplatte nach rechts in die Verriegelungsstellung.
Der Dichtungsring(77) - es kann ein in die Länge gezogener O-Ring sein - umrahmt den Sogkanal(32) der teilweise aus einer Rinne an der Unterseite der Grundplatte auf der Strecke zwischen Faltenbalgzentrum und Saugglockenwandung gebildet wird. Er wird vom Bodenblech(78) gegen die Grundplatte gedrückt. (Die Befestigungsschrauben für das Bodenblech wurden weggelassen.)
Auf dem Längschnittsdetail ist zu erkennen, daß eine Brücke der Auslöserplatte(53) unterhalb des Bodenblechs hindurchgeführt wird. Auch wird die Lage der von der Auslöserplatte ausgehenden Federzunge(55) innerhalb der Nische in der Grundplatte (gestricheltes Rechteck) noch einmal verdeutlicht.
Ganz unten die Darstellung wie der von der Drucktaste(222) - nach einer Auslöservariante - ausgehende Sperrknopf(28) sperrend in eine Loch der Auslöserplatte(53) hineinragt. Die Drucktaste, deren Anhebung in diesem Falle nicht durch die Hilfsfeder(34,vgl.Fig.1) unterstützt wird, muß in diesem Falle von Hand unmittelbar vor dem Aufsetzen des Verbindungssteges(71,vgl.Fig.4) für die Auslöser hochgezogen werden, um die Bewegung der Auslöserpatte freizugeben.

Die Figur 8 zeigt in natürlicher Größe oben einen Querschnitt durch eine Einrichtung nach Fig.1 bei Ergänzung durch eine Vorrichtung für die visuelle Hautkontrolle und einer Variation des Auslösers für die Sogpumpe; es wird auch noch die Verbindung zum Meßgerät dargestellt, welche mechanisch mittels der paarigen Befestigungsangel(56) erfolgt und elektrisch durch die Signalleitungen(72), die aus dem Schacht unter dem Klappdeckel in die Trägerlamelle(209) führt. Letztere ist anstelle eines Teststreifens in den Schacht(262) des Meßgerätes eingesteckt. Darunter in der Mitte im Maßstab 2 : 1 die Verbindung zwischen Auslöser(52) und Teleskopschienen(269).
Dicht darüber ein Längschnittsdetail der Schlusselöchähnlichen Verriegelung für den in die Senkrecht hochschwenkbaren Haltebügel (79), der dem Andruck der Vorrichtung gegen die Haut dient. Nach Drehung in der Rundung gerät die zur Grundplatte hin abgewinkelte Rechteckachse des Bügels in die engere Längsschlitzführung des Federblechkastenrahmens und kann nun nicht mehr seitlich wegkippen.
Rechts wird nochmals die Lage der Federzunge(55), die vom Federblechkastenrahmen entspringt(vgl.Fig.1) wiedergegeben, deren vorspannung durch die Stellschraube(81)reguliert werden kann. Dem Querschnittsdetail durch einen Auslöserkomplex in natürlicher Größe unterhalb entsprechend werden auf der unteren Bildhälfte Längsschnittdetail zur Erläuterung der Funktionsstadien des Auslösemechanismus wiedergegeben, welche durch die schematische Darstellung der Fig.9 oben ergänzt wird.
Nur beispielsweise werden die zwei Teleskopschienen dudrch das elliptische Mantelhüllsegment(69) zusammengehalten.
(Die Führungsrillen für die Stifte zur Begrenzung der Auszugslänge in den Längschlitzen müssen deshalb um 90 Winkelgrade gedreht vorgestellt werden.) Vorteilhaft wäre auch eine gegenseitige Schwalbenschwanz- oder Mäanderverfugung der Teleskopschienen(269). Die Hülse des Auslösers(52) trägt einander gegenüberliegend zwei Querstifte, einen unteren(88), der von der vorderen oder rechten Keilschieberflanke(85) angehoben wird und einen oberen oder linken(84), der von der hinteren oder linken Keilschieberflanke (86) abgesenkt wird. Der Keil- oder Federschieber(87),der in einer Mulde der Grundplatte(16) liegt, wird an seinem keilförmigen linken Ende von einem Deckelplattenstift (oder hier von einer Deckelkante) nach links verdrängt, wobei die Federkraft in einem Bogen gespeichert wird (vgl.Fig.9, rechts oben). Dies ist jedoch nur der Fall, insoweit die rechte Keilschieberfläche durch Anhebung mit dem Auslöser in der Endphase der Hebung des Teleskopgestänges(69) mit ihrem unteren Querstift((88) die rechte Keilschieberflanke hochschiebt.
Verläßt der untere Stift die rechte Keilschieberflanke, sie in einem Schlitz oben (oder an ihrer Oberkante) überholend, dann kann die im Federbogen gespeicherte Kraft wirksam werden und den Auslöser(52) nach unten mittels des linken Keilschiebers abdrängen. Dies ist bei Deckelplatten- und Teleskopgestängeannäherung im Stadium oben rechts der Fall. Die elliptische Rasterkörper wird aus seiner Rastmulde im Auslöser verdrängt und in die Rastmulde an der unteren Teleskopschiene verschoben. Wegen einer Randleiste, bei Sicherung durch in die Rastkörperbohrung eingebrachte Haltescheiben, kann der Rasterkörper nicht aus seiner Bohrung herausfallen. Im Stadium unten rechts ist das Teleskopgestänge beginnend gesenkt, im Stadium links angehoben bei noch gehobener Deckelplatte, im Stadium links unten vor der Anhebung.
Im Querschnitt links wird gezeigt, daß drei derartige Auslöser um den Faltenbalg(46) herum angeordnet sind, und daß die Stiftenden der Telskopgestänge des hier sternförmig in einem Mittepunkt zusammenlaufenden Verbindungssteges(71) miteinander verbunden sind.

Die schematische Darstellung des Auslösemechanismus nach Fig.8 in Figur 9 oben macht die Funktionsweise und -stadien neben einem Längsschnitt durch den Auslöser(52)und eine Aufsicht auf den Keil- oder Federschieber im Längsschnittdetail noch deutlicher. Im Stadium links oben hat der Deckelplattenstift (die Deckelplatte wurde weggelassen) den Keil des Keil-oder Federschiebers(87) noch nicht verdrängt. Der Auslöser steht gesenkt. der vordere untere Querstift(88) vor der Keilflanke des Keil-oder Federschiebers.

Der Rasterkörper(89) steht gegenüber der (nicht dargestellten) unteren Teleskopschiene in Verriegelungstellung.
Im Stadium rechts oben ist der Deckelplattenstift abgesenkt und hat am linken Keilende den Keil- oder Federschieber nach rechts verdrängt. Die Federverkürzung bewirkt einen Kraftstau im verformten Federbogen. da gleichzeitig auch der Auslöser(52) soweit angehoben wurde, daß sein rechter unterer Querstift die vordere Keilflanke des Keil- ode Federschiebers nach linksverdrängte durch Anhebung des (nicht dargestellten) Teleskopgestänges von unten her.
Im Stadium links unten neben dem Querschnitt hat bei Ausweichung des unteren vordern Querstiftes in die Schrägrinne der vorderen Keilschieberflanke die linke Keilschieberflanke den oberen Querstift und damit den Auslöser nach unten verdrängt Und damit auch den Rasterkörper aus seiner Rastmulde im Auslöser unter Verriegelung der Teleskopschienenbewegung. Eine Linksverschiebung des Keil-oder Federschiebers war wegen Senkung des Deckelplattenstiftes nicht möglich. Bei Zug am Teleskopgestänge passiert der vordere untere Querstift(88) zuerst in einer senkrechten Rinne der Rückfläche der vordern unteren Keilschieberflanke, dann durch dessen nach vorn Verdrängung denselben.

Die in der Mitte in Längsschnittdetails wiedergegebene Auslöservariante bedient sich zur Verriegelung des Auslösers(52) dessen Anhebung unter Sektorrotation. Die Rastermulde im Auslöser ist hier keine Ringnut sondern eine gerichtete Bohrung(91). Auf deren Gegenseite ist eine Schrägschlitzführung für einen in einer Querbohrung in der Grundplatte(16) gefederten Führungsstift(92) eingelassen, wie links in einem Vertikalschnittsdetail mit der Aufrollung rechts dazu gezeigt wird. Der Keil- oder Federschieber (87) ist dieses mal tiefer in der Grundplatte(16) gelagert und bewirkt bei Rechtsverschiebung mit der paarig auf den Unterrand der Hülse des Auslösers einwirkenden Keilflanke dessen Anhebung (Stadium rechts). Die Rechtsverschiebung erfolgt wiederum über die Einwirkung eines Deckelplattenstiftes senkrecht auf das Keilenende des Keil- oder Federschiebers. Bei der Rechtsverschiebung des Keil- oder Federschiebers wird die Spannung des Bogens in der Feder dadurch bis zuletzt, d.h. zur völligen Deckelplattenabsenkung aufrechterhalten, daß der Galgen(94), der am rechten Bogenende von dem Keil- oder Federschieber entspringt gegen die Führungsschiene(95) stößt, die sich mit der Deckelplatte abwärts senkt und eine der Annäherung des Galgenendes entsprechende Keilkonfiguration aufweist. Eine tiefe Lücke -die auch ein Schlitz sein könnte, läßt dann bei völliger Deckelplattenabsenkung den Galgen nach rechts durch und löst damit die Anhebung des Auslösers unter Verdrängung des Rasterkörpers und Verriegelung der (wiederum weggelassnen) Teleskopschiene aus. Dabei die Bohrung des Rasters (91)von der Achse des Rasterkörpers wegedreht wird, kann bei Zug an dem Teleskopgestänge die Bohrung für den Raster in abweichender Radiärstellung den Rasterkörper passieren, so daß dessen Auslösung unterbleibt. Bei der Anhebung des Teleskopgestänges wird die Bohrung wieder in die Stellung egenüber dem Rasterkörper zurückgedreht, so daß schließlich bei maximaler
Hebung des Teleskopgestänges, die unterhalb der Anhebungsmöglichkeit des Auslösers liegt, der Rasterkörper aus der Teleskopschien ausgelöst. Im Fußteil zum Verbindungsteg(71) ist die Endstange des Teleskopgestänges drehbar.

Die Figur 10 zeigt die Möglichkeit auf, die Punktionsspitzenbewegung quer zur Haut nach Einstich in dieselbe durch das Aufprallen des Stößels außerhalb der Saugglocke auf den Test-streifen zu übertragen und dies sowohl bei einer Spitzenbewegung nach dem Rotations- (linksseitig abgebildet) wie nach dem Schwingverfahren (rechtsseitig abgebildet).
In natürlicher Größe wird oben links ein Längs, und unten links ein Querschnitt durch das Detail einer Saugglocke gegeben.
Beim Rotationsverfahren links dreht sich der rundliche Aufnahmestift mit dem Schacht(62) für die Einführung des Teststreifens (103) im Dachbereich der Saugglocke(1) innerhalb des Dichtungsringes(5). Der Stößel(7) - hier ist auch ein Gegenstößel für eine Nachschnittbewegung eingezeichnet - stößt gegen ein Seg-ment mit Innengewinde, das mit Aufnahmestift über eine Brücke verbunden ist, so daß während der Sektorbewegung durch den Stößel zugleich eine Drehung mit Höhenänderung der Punktionsspitze erfolgt, wobei der Stahlstift die nicht angespitzte Strecke weit parallel zum Teststreifen(103) verläuft und mit diesem verklebt ist. Man erkennt die in der Saugglocke hochgezogene Haut, wobei die angeschlossene Sogpumpe weggelassen wurde. die weitere Ausgestaltung, wie Begrenzung des Rotationswinkels oder Nachbewegung zur Hautspalterweiterung ergibt sich aus den übrigen Schilderungen. Unten ist strichpunktiert die Hautkuppe eingezeichnet, die zentral vom Teststreifen eingedellt wird.
Die Details rechtsseitig sind Längschnitte bei Abweichung der Schnittrichtung um 90 Winkelgraden. Der Schacht, in welchen der Teststreifen eingeführt ist, ist innerhalb der Saugglocke in der Saugglockenwandung durch den paarige Dichtungsring(5) abgedichtet mittels seitlicher Hebel außerhalb der Saugglocke durch Stößel(7) bzw.Gegenstößel schwenkbar. Beim unteren Schnitt ist wiederum strichpunktiert die Hautkuppe eingezeichnet.

Die Figur 11 zeigt im Maßstab 2 : 1 (bei weit überzeichneter Stahlstiftbreite) oben zwei Draufsichten auf einen Teststreifen(103) mit Testfeld(104) und Leitungsbahnen bei in die Haut (strichpunktiert) eingestochener Punktionsspitze.
Rechts davon eine Seitenansicht. Die linke Draufsicht entspricht dme Stadium nach, die rechte demjenigen vor dem Aufprall der Stößelkuppe.
Auf der Seitenansicht erkennt man, der Stahlstift an einer vorderen mittleren Lamelle des Testsstreifens befestigt ist, größtenteils hinter dem Testfeld liegen und mittels des Stößels(7) nach rechts bewegt. Die hintere Teststreifenlamelle die vordere seitlich umrahmende ist starrer gestaltet oder wenigstens von der Schwenkbewegung der Lamelle mit der Punktionsspitze weitgehend bewegungsunabhängig. Ihre, hier rechteckig gestalteten, Enden greifen auf der Hautoberfläche an, diese leicht herabdrückend und fixieren so die Haut in der Nähe der von der Stahlspitze weggeschobenen Hautpartie und begünstigen eine Hautspalteröffnung. Die untere Abbildung zeigt die Draufsicht auf eine Löung mit nur einer einzigen Teststreifenlamelle, welche durch tiefe Einschnitte längs des Mittelstreifens mit Punktionsspitze und Testfeld gespalten ist, wobei der Mittelstreifen beweglich bleibt. Die der Haut aufgestellten Enden der seitlichen Lamellenteile sind mer dreieckig zugespitzt.
Rechts oben ist die Draufsicht auf einen Teststreifen mit beigefügter Punktionsspitze für den Rotationsgebrauch dargestellt.

Die Punktionsspitze ist exzentrisch montiert. im Zentrum reicht eine Zuspitzung der vergleichsweise starren Teststreifenlamelle neben dem Stahlstift bis auf die Haut (strichpunktiert dargestellt).

Die Figur 12 gibt stärker schematisiert eine Längschnittsübersichtsdarstellung zur Demonstration einer visuellen Hautkontrolle im Maßstab 1 : 1 .
Das Teleskopgestänge(69) unterhalb und um den gegen seine Druckfeder zusammengedrückten Faltenbalg(46)ist nach unten ganz herausgezogen und wurde auf die Haut aufgesetzt. Der Haltebügel am Federkastenrahmen steht mittels der Federzungen(55) reguliert an den Einstellschrauben(81) mit seiner Kontur über derjenigen der Grundplatte(16) des Gehäuses. Da die Stromschleife (strichpunktierte Linien rechts) durch Kontaktschluß über leitende Teile des Teleskopgestänges (schematisch als Haken dargestellt) in dieser Endstellung geschlosssen wird, kann Strom von der Batterie(83) die drei Lichtquellen(17)elektrisch versorgen, von denen nur zwei als auf eine zentrale Hautstelle gerichtet, gezeigt werden. Die zentrale Hautstelle entspricht der späteren Punktionsstelle. Am Haltebügel(79) wird nun der Federbleichkastenramen gegen die Haut abgesenkt und darnach auch die Grundplatte und die Saugglocke und dabei die Sogpumpe ausgelöst.

Die Figur 13 gibt links oben in natürlicher Größe, oben in einem Längschnittsdetails eine Saugglocke mit einer Vorrichtung zur Punktionsspitzenrotation wieder; darunter in eine Draufsicht auf eine perforierte Lochplatte(98), wie sie von unten in die Saugglocke eingeschoben werden kann. Die Schnittlinie für den Querschnitt verläuft längs der Linie A - B des Längsschnittes. Die Einführung des Rotoreinschubes(97) mit gegenüberliegendem paarigen Führungstift(99) erfolgt über eine senkrechte Nut(98) in der Sauggglockenwand mit über dem Saugglockenrand vorspringender Innenstufe (Siehe Detail rechts). Der Führungsstift verläuft dann in einer horizontalen Ringnut, welche eine stufige Anschrägung zeigt, längs derer der Rotoreinschub mit dem Schacht für den Teststreifen während der Sektorbewegung durch den (hier weggelassenen Stößel analog zu Fig.15) abgesenkt werden kann. Signalleitungen(72) für die Stoffwechselmessung liegen gewunden für ein Bewegungsspiel in einer Mulde unter dem Klappdeckel. Stromzufuhrleitungen für eine Lichtquelle(17) auf der Lochblende und die Signalleitungen(100) für den zugehörigen Sensor liegen gerollt seitlich auf der Lochplatte und sind mit einem (nicht gezeigten) Photometer in Saugglockennähe verbunden. Ein (gestrichelt dargestellter) horizontaler Lichtstrahl durchdringt eine hochgehobene Hautkuppe und kontrolliert die dort in das zentrale Loch eingedrungene und gegen die Punktionsspitze fixierte Haut auf Eignung. Nach einem Signal kann die Saugglocke(1) mit elastischem Rand weiter angedrückt und die Lochplatte dabei bis zum Eindringen der Haut in die Punktionsspitze in die Saugglocke hochgeschoben werden. Die Hautanhebung erfolgt natürlich nur bei geschlossenem Klappdeckel(206). Hier ist er geöffnet dargestellt um das Schließmoment unter Einfluß einer Zugfeder rechts am Saugglocken-rand zu demonstrieren.
Links ist im Maßstab 2 : 1 ein Längsschnittsdetail der linken Hälfte einer Saugglocke dargestellt, um eine Blattfeder zu zeigen, über die ein Keilvorsprung bei Deckelöffnung und -schluß hinwegzieht und das Kippmoment in den Endstellungen erhält.

Darstellungen von Teststreifen(103) in Aufsicht im Maßstab 4 : 1 mit wiederum in der Breite stark überzeichnetem Stahlstift mit Punktionsspitze zeigen zugleich deren Verpackung an.
Die obere Abbildung rechts zeigt einen solchen Teststreifen mit linksseitigem "Schüsselbart", um eine Polvertauschung bei Einschub in den Schacht für die Befestigung und Signalabnahme zu verhindern. (Der Teststreifen entspricht einem solchen der Firma BAYER für deren Glukometer bei deutlicher Teststreifenverkürzung.) Eine Folienscheide(102) ist über das Ende des Teststreifens(103) geschoben und überdacht dabei die Punktionsspitze, wobei die Querleiste(106) unter einer der Lamellen gegen die Noppe(107) stößt, die sich der Querleiste entgegenwölbt, wie unten in Querschnitt (links) und Längsschnitt (rechts) gezeigt wird. (Das andere Blatt der Folienscheide und die andere Teststreifenfläche können analog gestaltet sein.)
Teststreifen und Folienscheide können aus ihrer gemeinsamen Hülle entfernt werden. Es wird dann das Teststreifenende in den Schacht im Klappdeckel eingeschoben, wobei die Querleiste den Teststreifen an dessen Noppe vor sich herschiebt(Pfeilrichtung nach links). Die Folienscheide kann dann abgezogen werden.
Zur Entfernung des verbrauchten Teststreifens wird die Folienscheide, deren beide Blätter oder Lamellen an den Enden zweckmäßigerweise leicht angeschrägt oder maulartig aufgebogen sind, über die Punktionsspitze entlang dem Teststreifenende hochgeschoben und durch verstärkten Druck bewirkt, daß die Querleiste der Folienscheide über die Noppe des Teststreifenstritt. Beim Rückzug der Folienscheide wird der Teststreifen aus dem Schacht gezogen und die kann unter Deckung der Punktionsspitze durch die Folienscheide weggeworfen werden. Seitenränder an der Folienscheide, die ein seitliches Herausfallen des Teststreifens verhindern, können vorhanden sein.
Darunter linksseitig wird eine ähnliche Verpackungslösung dargestellt, bei der aber die Noppe(107) des Teststreifens an dessen Rand unter halb des "Schlüsselbartes" angebracht ist.
Die Folienscheide hat hier eine Lamelle mit einen kürzeren Rand und eine mit längerer beweglicher Randzunge mit Haken(108). Die Folienscheide wird, wie unten gezeigt, in Pfeilrichtung in den Schacht eingeschoben, wobei das fest kurze Ende der Folienscheide gegen die Randnoppe des Teststreifens stößt und diesen mitbewegt. Zur Entfernung des Teststreifens muß die Folienscheide um 180 Winkelgrade umgedreht auf den Teststreifen aufgeschoben werden. Der Haken überholt dabei die Noppe und bei Rückzug (in Pfeilrichtung nach unten) wird der verbrauchte Teststreifen in der Folienscheide herausgezogen.
Das Sägezahnprofil für die Noppe, wie es links herausgezeichnet ist, sichert die Unlösbarkeit der Verbindung für die Punktionsspitzen- und Testfeldentsorgung.
Bei der Verpackungslösung in der Mitte rechts richtet sich eine Folienscheide, welche für die Aufnahme des Punktionsstifte noch eine Rinne oder einen Schlitz aufweist (siehe Querschnitte in Höhe der Schnittlinien links, darunter ein Längsschnitt), gegen zwei Noppen auf dem Teststreifen - es können analog zur zuvor beschriebenen Lösung auch Seitenränder gegen seitliche Konturensprünge des Teststreifens anstoßen - wodurch die Schachtein-führung ermöglicht wird.
Zum Herausziehen des Teststreifens aus dem Schacht wird, wie unten dargestellt, die Folienscheide noch stärker über den Teststreifen und auch über die Noppen geschoben, wobei die beiden
Blätter oder Lamellen der Folienscheide unter stärkerer Klemmwirkung gespreizt werden, so daß ein Herausziehen des Teststreifens jetzt möglich ist. Der Endabschnitt in der Folienscheide kann auch mit einer Klebemasse beschichtet sein, welche die dauernde Verbindung zwischen Teststreifen und Folienscheide garantiert. Statt der Niveauunterschiede zwischen Querleiste und Noppen, deren Sitz zwischen den Schiebeteilen auch vertauscht sein kann, können auch Vorsprung und Loch- oder Schlitzbildung für die Aufnahme des Vorsprungs dem selben Zwecke der Verschiebung des Teststreifens dienen.
Dies wurde unten in Draufsicht und Längsschnitt als viertes Beispiel im Maßstab 2 : 1 demonstriert. Dabei wurde als weitere Variante das eine Folienblatt kürzer gehalten, so daß es mit der Unterkante des Teststreifens(104) zusammenstößt und dem Einschub desselben in den Schacht dienen kann. Die Noppe(107) wird zur Teststreifenentfernung bis in die Höhe des Loches(109) im Test-streifen vorgeschoben. Derart verhakt kann der Teststreifen innerhalb der Folienscheide an letzterer aus dem Schacht herausgezogen werden.

Die Figur 14 gibt im Maßstab von etwa 10 : 1 das Ende eines Teststreifens(103) mit einem Stahlstift wieder, der auf Stoß mit dem Teststreifen verklebt ist und zunächst die gestrichelte Kontur aufweist. Linksseitig ist eine Seitenansicht, rechte seine Draufsicht gegeben. Der stählerne Rohling kann nach der Extrusion aus der Düse und Abtrennung beispielsweise in einer Form gepreßt werden. Er zeigt um 90 Winkelgrade gegenseitig versetzt zwei plattenförmige Erweiterungen der Stiftenden: die untere kleinere zur Erzeugung einer etwa bis lmm langen Schneide hinter der Spitze im Elektrolytbad und die oberer größere für eine Befestigung auf dem Teststreifen, welche der Schnittrichtung der erzeugten Schneide Rechnung trägt.

Die Figur 15 zeigt in der Mitte in natürlicher Größe und leicht nach unten verschoben den Querschnitt durch eine Punktionseinrichtung nach dem Rotationsprinzip. Darunter ist ein Längsschnitt wiedergegeben (bei Aufprojektion des Drahtbügels für die Anhebung des Paltenbalges mit der Deckelplatte), oben zwei Faltenbälge mit Teilen der Deckelplatte als Längsschnittdetail.
Die Saugglocke(1) weist, wie der Querschnitt zeigt, zwei Bohrungen auf, welche mit Dichtungen versehen sind, durch welche Stößel(7) und Gegenstößel des Schiebebalkens(220) für die Bewegung der Leisten des Rotoreinschubes(97) ins Saugglockeninnere eintreten. Die paarige Druckfeder(30) lehnt gegen die Grundplatte(16) und richtet sich gegen Einstellschraube(54) für den Stößel(7) bzw. die andere für den Gegenstößel. Das dargestellte Stadium ist das der Federspannung, welche für den Stöβel(7) vom Keilschieber(37,vgl. Fig.1 oben rechts) durch die Absenkung des Keiles(112, siehe Längsschnittdetail oben) bewirkt wird. (Dies erfolgt von einem von einem Ausläufer der Deckelplatte ausgehenden Stift aus am bezeichneten Endpunkt, was mittels einer dicken waagerechten Linie ausgedrückt wurde.) Für die Arretierung des Stößels (7) ist in diesem Falle die Rasterhülse(110) vorgesehen, welche eine Sperrkugel in einer Rastnische des Stößels nahe seinem u-förmigen Knick mit Schenkel zum Keilschieber hin verriegelt. Die Auslösung erfolgt durch Anhebung der Rasterhülse durch den in einem Schlitz des letzten geführten Endglied des Schiebegestänges (111,vgl.Fig.16 oben) mit der Anhebung der Deckelplatte(15).
Die Anhebung der Deckelplatte und des mit ihr verbundenen Faltenbalges(46) erfolgt über den Drahtbügel(207), der mit einem Nischenbogen eine Rolle umfaßt, deren Achse quer an der Deckelplatte befestigt ist (siehe oberstes Längsschnittdetail) durch die Wirkung der paarigen Torsionsfeder(149).

Im unteren längsschnitt entsprechen Stellung des Drahtbügels und der Deckelplatte(15) einem entfalteten Faltenbalg (Längsschnittdetail ganz oben); unten ist er aber komprimiert dargestellt.
Die Einstellschrauben am Feingewinde des Stößels(9) und des Gegenstößels oder Schiebebalkens(220) sind im unteren Längsschnitt eingezeichnet und so plaziert, daß sie an der Oberkante der Grundplatte(16) vom Finger gerade noch erreicht werden können.
Die Ausziehschiene(113) längs des Drahtbügels verlängert den Federarm zur Anspannung der Torsionsfeder(149); sie wird nur in einem Bruchstück gezeigt.
Die Keilführung(221) hat das U-Bogenende des Schiebebalkens(220) verdrängt. Sie ist Teil der Drucktaste(240). deren Aufbau und Funktion als "Nachschnittaste" in den Figuren 16,18 und 19 näher erläutert wird. Der paarige Auslöser(24) entspricht in Aufbau und Funktion demjenigen, der im längsschnittdetail rechts der Fig.16, dem zweiten von oben, beschrieben wird. Wie der hintere Keilschieberstift(114), so ist auch die der Sperrknopf(28), der in die Auslöserplatte eindringt(vgl.Fig.7 unten) über den Brückenarm(115) mit der Drucktaste(240) verbunden. Der Sperrknopf bewirkt, daß auch bei Betätigung des paarigen Auslösers(24) durch Druck auf den Deckel bei Auflage der Einrichtung etwa auf den Tisch oder gegen den Körper zur Spannung der Torsionsfeder, die Auslöserplatte sich unter Einfluß ihrer Federzungen(55,siehe Fig.7) nicht verschieben kann, um die Deckelstifte(213) für die Faltenbalgerweiterung durch Deckelplattenanhebung freizugeben(216, siehe Fig.4, Längsschnittdetail rechts oben).
Das Längsschnittdetail oben mit dem komprimierten Faltenbalg zeigt nochmals den Mechanismus zur Sogregulierung, wie in den Längsschnittdetails oben in Fig.4 bereits erläutert wurde.
Die Druckfeder(117) im Faltenbalg (oberstes Längsschnittdetail) dient der Unterstützung der Sogerzeugung bzw. der Entlastung der Torsionsfeder(149).

Die Figur 16 wiederholt zur Lageübersicht den Querschnitt der
Fig.15 und erläutert einige Funktionselemente für die Auslösefunktionen derselben.
Zur Lageorientierung wird der Querschnitt der Figur 15 wiederholt. Ganz oben ist ein Längsschnittdetail durch den Auslöser nach Fig.15 für die seitlichen Schnittbewegung mit der feststehenden Rasterhülse(110) wiedergegeben, deren verschieblicher Stift im Zentrum, mit seiner Rastmulde nach unten verschoben ist, so daß die Sperrkugel durch die Lücke der Rasterhülse nach links in die Rastnut des Stößels(7, Querschnitt unten verdrängt ist und dessen Bewegung sperrt. Diese Sperrung durch Bewegung nach unten geschieht unter Einfluß der Blattfeder(119). Die Anhebung des sperrenden Stiftes zur Entriegelung erfolgt durch Anhebung der Teleskopglieder rechts von der Rasterhülse, deren letztes an der Deckelplatte(15) befestigt ist über die Vermittlung von Querstiften, welche innerhalb von senkrechten Führungsschlitzen der Hülsen ihr Bewegungsmoment übertragen. Mit dem linken Längschnittsdetail wird das Stadium der Verriegelung demonstriert, mit dem rechten das der Entriegelung.
Das Querschnittsdetail über dem linken Längsschnittdetail entspricht der Darstellung im Gesamtquerschnitt unten.
Rechts unter dem linken beschriebenen Längschnittdetail wird in einem Längsschnittdetail einer der beiden Auslöser(52) unter einem Querschnitt durch denselben gezeigt, welche der Auslösung der Saugpumpe dienen. Der Auslöser wird mit seiner Keilflanke durch die Blattfeder(120) unterhalb dessen Hakenausläufers der Auslöser-platte(53) gehalten, welche auf der Deckelplatte(15) befestigt ist. Der Auslösebolzen(121) ist mittels Feingewinde in einem ebensolchen der Einstellmutter(121) verschraubt. Seine Höhenverstellung erfolgt über Rotation der Einstellmutter(122) in ihrem sechskantigen Sitz in einer Mulde der Grundplatte. Mit der Anhebung der Auslösebolzen, welche durch den Verbindungssteg(71, vgl.Fig.4 Querschnitt unten) miteinander verbunden sind, hebt die Einstellmutter jeweils den Auslösebolzen, dessen Keilflanke den Zungenoder Hakenausläufer der Auslöserplatte gegen deren Federzungen (55,vgl.Fig. )nach links zur Sogauslösung verdrängt.
Links oberhalb des Gesamtquersehnittes ist ein Vertikalschnitt durch die Mitte der Saugglocke(1) wiedergegeben. Rechts davon ein Längsschnitt durch die Drucktaste(222) mit dem an ihm befestigten Brückenarm(115), der im hinteren Keilschiebersstift(114) endet. Der Keilschieber(37) unter ihm wurde zur Funktionsverdeutlichung um 90 Winkelgrade in der Ebene gedreht, so daß die - direkt hinter ihm liegende - Projektion des Deckelplattenstiftes(123), dessen Absenkung mit der Deckelplatte ebenfalls eine Stößelverschiebung gegen die Druckfeder(30) bewirkt.
Die Schiebewirkung des Keilschieberstiftes(114) mit der Absenkung der Drucktaste erfolgt in einer Absenkphase. welche der Verdrängung des Schieberbalkens(220) gegen dessen Druckfeder durch die Keilschräge an der Drucktaste(unterer Vertikalschnitt links vom Gesamtquerschnitt) vorausgeht. Es wird so die Blockade des Rotoreinschubes(97) durch den gegen ihn vorgeschobenen Stößel(7) aufgehoben, bevor der Gegenstößel des Schiebebalkens für die Punkionsspitzenbewegung quer zur Haut in Funktion tritt. Die Funktion der Drucktaste wird in Fig.19 in ihren Stadien erläutert.
Der Längsschnitt durch die Mitte des Faltenbalges(46) mit der äußeren Druckfeder((70) für dessen Entfaltung durch die Anhebung der Deckelplatte, zeigt auch die Lage der Drucktaste(222).

Die Figur 17 zeigt über dem Gesamtgrundriß der Fig.15 (mit dem Längsschnitt durch die Saugglocke in natürlicher Größe darunter) oben links ein Querschnittsdetail durch den Rotoreinschub und Endteil eines Schieberbalkens im Maßstab 2 : 1 Saugglocke(1) und darunter zwei weitere Längsschnittdetails durch eine Saugglocke mit Klappdeckel in natürlicher Größe(vgl.Fig.13, Längsschnittdetail links oben).
Im Zentrum des Rotoreinschubes(97) auf dem oberen Längsschnitt liegt der Schacht(62) mit dem eingeschobenen Teststreifen. Die paarige Signalleitung(72) darüber liegen als Schleifen in einer Mulde des Rotoreinschubes oben auf, um die Drehung des letzteren zuzulassen. Der paarige Führungsstift(124), der den Rand des Rotoreinschubes überragt, liegt innerhalb einer Querrinne oder -nut der Saugglocke. Seitliche Blattfeder halten von der Grundplatte aus die Saugglocke nach unten, wobei ein Außenstift in einer Führungsrinne der Grundplatte nach unten ein Gegenlager bildet.
Die Einzelheit einer linken Saugglocken- und Rotorhälfte links entspricht einer leichten Sektordrehung derselben, um die senkrechte Einführungsnut(125) zur Querrinne aufzuzeigen, welche zur Montage oder Herausnahme des Rotoreinschubes (am besten doppelseitig) zweckmäßig ist. Eine solche Senkrechtnut kann auch dazu dienen einen Rotor zur Teststreifeneinführung in den Schacht jeweils unter Einfluß des dann besonders sinnvollen Blattfederdruckes von oben in der Saugglocke tiefer treten zu lassen.
Das Längsschnittdetail rechts entspricht jeweils einer Achsendrehung der Saugglocke um 90 Winkelgrade und zeigt die Breitseite des Teststreifens(103) mit exzentrischer Punktions-spitze und zentrischem Haltezahn für die Hautfixierung. Auch die Lage von Stößel(7) und Gegenstößel sind angegeben.

Die beiden Längsschnitte der unteren Reihe entsprechen bei entsprechender Schnittrichtung einer Variante, bei welcher der Test-streifen nach Anheben des nach unten gegen einen Dichtungsring Endkante des Schieberbalkens bei der Drucktastenabsenkung ein. Der Sperrstift wird dann unter Spannung der Blattfeder in der letzten Phase der Drucktastensenkung plötzlich unten geschnellt und gibt die Gegenstößelbewegung des Schieberbalkens frei.
In der unteren Reihe entspricht die Gestaltung des Endes des Schieberbalkens der im Querschnitt in der Mitte darüber dargestellten. Es sind nur zwei Funktionsstadien dargestellt, während die auf den Maßstab 2 : 1 vergrößerte Detaildarstellung links das über der Saugglocke gefederten Klappdeckels(206) in den Schacht mit den Federkontakten für die Signalleitungen(72) von oben eingeführt wird. Der Klappdeckel ist hier leicht angehoben.
Im Querschnitt links oben eines Rotoreinschubes(97) und des Endes eines Gegenstößels oder eines Schieberbalkens(220) ist eine Saite als Zugverbindung(129) an der Kuppe des Schieberbalkens befestigt durch die Plattenausladung des Rotoreinschubes in einer Bohrung hindurchgeführt und durch Endplatte fixiert. Die Zugverbindung, die analog auch mittels Stange durchgeführt werden kann, bewirkt mit dem Rückzug des Schieberbalkens die Rückdrehung des Rotoreinschubes in die Ausgangslage vor dessen stoßartiger Sektordrehung.

Die Figur 18 zeigt schematisch unterhalb eines Querschnittdetails links(vgl. Gesamtquerschnitt der Fig.15) schematisiert drei Funktionsstadien der Drucktastenbetätigung in Längsschnitten. Die Drucktaste(222)weist den Rahmen(126) auf, der nach ihrer Anhebung und in der ersten Phase ihrer Wiederabsenkung durch Klemmwirkung hinter der Abwärtsbewegung zurückbleibt, so daß der Rahmen vor dem Ende des Schiebebalkens(220) dessen Bewegung (durch die Druckfeder) sperrt. Erst in einer letzten Bewegungsphase wird auch der Rahmen gesenkt. Eine Abbremsung der seitlichen Punktionsspitzenbewegung durch die Steilflanke der Drucktaste wird so vermieden.
Im Beispiel des unteren Längsschnitttriplets, zu der auch Mitte unten der Querschnitt gehört, ist der Rahmen zum Schiebebalken hin offen und letzterer vorn verbreitert und mit zwei zusätzlichen seitlichen Rastkuppen versehen. In diese greift der paarige, von je einer Blattfeder an der Unterseite der Drucktaste angehobene, Sperrstift(127) mit leichtem Nasenvorsprung in die jeweilige Schieberbalkens gezogen wird und dessen Horizontalbewegung nach rechts unter Federwirkung(vgl.Fig.15) freigibt.
Der Querschnitt in der Mitte rechts zeigt den U-Bogen des Schieberbalkens und dessen Zuordnung zur Drucktaste, während der Längsschnitt links unten für eine Lösungsvariante das Endprofil des Kopfes des Schieberbalkens wiedergibt. Die paarige Blattfeder(128) mit Endbogen ist am Rahmen(126) der Drucktaste befestigt und um befestigt und rastet mit dem Endbogen auf der seitlichen Kuppe des Schieberbalkens, diese umfassend, ein. Während die Drucktaste weiter gesenkt werden kann bleibt der Rahmen bis zur Überwindung des Kraftstaues in der Blattfeder unter deren Zurückweichen höhenfixiert(Stadium links). Darnach kann der Schieberbalken unter Einfluß seiner (hier nicht dargestellten) Druckfeder am Rahmen vorbei und der ihm parallel zugeordnete Stößel trifft auf den Teststreifen.

Die Figur 19 stellt eine bevorzugte Lösung der bereits in Fig.18 behandelten Lösung für eine Nachschnittaste dar, welche in ihrer ersten Absenkungsphase die Druckfeder(30) für den Stößel(7,Fig.15) spannt, um in einer überlappenden zweiten die Druckfeder für den Schieberbalken(220) über ihre Keilflanke zu komprimieren und dann ruckartig loszulassen. Es wird jeweils rechts neben einem Längsschnitt ein Querschnitt gezeigt.

Von der Drucktaste(222) wurde der Rahmen(126) hochgehoben, dessen keilförmiger Rand dabei die Rundkuppe des Schiebebalkens im Anschluß an die Keilflanke überholt hat und durch Querstiftführung in Nuten der Drucktaste (siehe untere Reihe ohne Schraffur unter Tastendrehung um 90 Winkelgrade) Unter Einhaltung dieses oberen Höhenniveaus zwischen Keilführung und angeschrägter Rahmenkante weiter hochgezogen wurde(Stadium rechts oben). Während der Absenkung der Drucktaste (Stadium links unten) bleibt die Kuppe des Schieberbalkens in einer Delle am Rahmen(126) hängen und seine Horizontalbewegung dadurch gesperrt. In der letzten Absenkungsphase der Drucktaste(Stadium recht unten) bewirkt die Stiftführung (untere Reihe ohne Schraffur) zwischen Drucktaste und Rahmen, daß letzterer mit seiner Delle aus der Kuppe des Stößels gezogen wird, sodaß letzterer an der Schräge des Rahmenrandes vorbeistößt.

Eine Einrichtung nach Figur 15 wird nach Hochheben des Klappdeckels und Entnahme des Teststreifens aus dessen Schutzhülle in den Schacht eingesteckt und der Klappdeckel losgelassen. (In der Variante der Einführung des Teststreifens von unten mit Hilfe einer Folienscheide entfällt die Klappdeckelanhebung). Die Einrichtung wird gegen eine feste Unterlage gedrückt und dabei die starke Druckfeder (oder Torsionsfeder in der Variante) neben dem Faltenbalg der Saugpumpe bis zur Verrasterung gespannt. Nach Sichtkontrolle der gewählten Punktionsstelle auf der Haut - am einfachsten durch kurzen Andruck des Saugglockenringes gegen die Haut und späteres Aufstellen der Einrichtung auf entsprechend markierte Projektionspunkte - werden die Auslöser für die Saugpumpe nach Anhebung der Drucktaste (unter Anhebung des Sperrknopfes aus der Auslöserplatte) entriegelt und durch Andruck gegen die Haut ausgelöst. Der verbrauchte Teststreifen wird nach Aufschieben der Folienscheide auf den Teststreifen herausgezogen und entsorgt.

Die Bremsung für eine Nachführung der Punktionsspitze zur Hautspalterzeugung kann analog zur Methodik zur Schwingungsdämpfung für Varianten nach Fig.1 durchgeführt werden, etwa durch Federeinführung oder Bewegung beim Klappdeckelschluß oder durch Vorlagerung eines gefederten Keils vor die Stößelspitze oder die Anbringung einer Rotationsfeder, welche der Drehung des Rotoreinschubes entgegen wirkt.

Die Figur 20 zeigt unten im Maßstab von etwa 2: 1 bei Übertreibung der Stahlstiftbreite einen schematisierten Ausschnitt aus einer Herstellungskette für die elektrolytische Anspitzung und Montage von Punktionsspitzen auf Teststreifen in Seitenansicht bzw. (das Elektrolytbad betreffend) im Längsschnitt. Darüber einige Kettenglieder und die Nahtstelle zur Teststreifenzuführung im Maßstab 10 : 1 oben in einer Seitenansicht, unten in einem Querschnitt. Je zwei Kettenglieder(130) aus stark wärmeausdehnbarem Material werden von einer Klammer(171) zusammengehalten, welche von einem Transportband(132) bzw. über (nicht dargestellt Transporträder, dessen Stifte oder Zähne etwa in den Raum zwischen je zwei Kettenglieder unter dem Führungsstift(133) eingreifen, langsam nach links bewegt. Die Profile zwischen Kettenglieder und Klammern sind so gewählt, daß eine Aufbiegung der Kette nach oben für eine Endlosführung möglich ist. Den Halbrinnen an der Außenseite der Kettenglieder werden über ein Schiebemagazin(135,unten in vertikaler Zufuhrrichtung) je ein Stahlstift(141) zugeführt. Über den Heizdraht(136) werden die Kettenglieder nach Stiftbestückung beheizt und halten durch Wärmeausdehnung die Stifte in den Hohlrinnen fest. Angeschrägte Höhenänderungen einer Führungsschiene, auf denen die Führungsstifte aufliegen, bewirken, daß die Kettenglieder über den Rand des Elektrolytbades gehoben und mit den vor Beheizung der jeweiligen Glieder an der Schienenkante längenausgerichteten Stahlstifte in das Elektrolytbad eintauchen, dessen Zu- und Abfluß durch Kreise symbolisiert wurden. Die Kathode(139) wurde als Strich eingezeichnet. (Die Stufe des Wannenrandes am Elektrolytbad kann durch bloße Niveausenkung vermieden werden, so daß nur eine Absenkbewegung notwendig wird). Das Rechteck rechts bezeichnet die Berührungszone der inzwischen angespitzten Stahlstifte in den beheizten Kettengliedern mit der Trommel(142) bzw. dem dort ausgespannten Folienband, welche zugleich mit den Kettengliedern weiterbewegt wird, wobei jeweils ein angespitzter Stahl stift an einen Bandabschnitt (dessen Breite durch die Transportgeschwindigkeit bestimmt wird) angeschmolzen wird.
Es können auch über ein Magazin (analog zum Schiebemagazin) fertige Teststreifen jeweils einer Halbrinne am Kettenglied zugeführt werden. Die Leitung(143) bezeichnet die Stromzufuhr zu den Stahlstiften mit Anodenwirkung für den elektrolytischen Prozeß.

Die Figur 21 gibt oben in einem Quer- und unten im Längsschnitt im Maßstab 2 : 1 eine Lösung für die Punktionsspitzenbewegung quer zur Haut an, bei welcher Elektromagnete innerhalb der Saugglocke eingesetzt werden und zumindest bewegte Teile nicht durch Dichtungsringe in der Saugglocke hindurchgeführt werden müssen. Links wir ein Beispiel für die Schwingmethode gegeben, rechts eine für Rotation.
Die Elektrotauchmagnete(144) und(145)sind unterhalb des Daches der Saugglocke(1) so montiert, daß die freien Enden ihrer Magnetkerne oder -anker über (am besten analog (47)Fig.1 aufsteckbare, hier festverbundene) Endplatten mit einer Noppe zum Aufschlag gegen den Teststreifen(103) mit den beiden Seitenlappen zur Hautfixierung (vgl. Fig.11 oben) nach unten ragen. Die strichpunktierte paarige Leitung(143) dient der Stromversorgung. (Batterie und Steuerzentrale wurden weggelassen und liegen in der Regel außerhalb der Saugglocke). Die Magneterregung erfolgt zeitversetzt, wozu das obere Profillinienpaar unten eine Rechteckimpulsreihe für den Magneten(144, obere Linie) für den Magneten(145, untere Linie) anfzeigt: es sind jedoch auch verschiedene Stromstärkenanwendungen oder das An- und Abschalten von Magnetwicklungen lösungsgemäß.
Dem zeitverschobenen Magnetschlag, dessen Zeitverschiebung das zurücklegbare Streckenmaß des Magnetankers(144) wegen seines anfänglichen Übergewichtes wiedergibt, folgt eine unterbrochene Impulsreihe, die einem langsameren Vorrücken des linken Magnetankers mit Verschiebung der Punktionsspitze nach rechts entspricht und bis zur Meldung der Blutsättigung durch das (hier weggelassene Meßgerät) an die Steuerzentrale, welche dann beide Magnete abschaltet. Die Signalleitungen (73) wurden in Teststreifennähe eingezeichnet.
Unten ist - unter Weglassung der Saugglocke - ein Querschnittsdetail mit einer Variante der Magnetfunktionsausgestaltung angegeben, und zwar im Erregungszustand beider Magnete. Die Endplatten auf den Magnetankern sind so gestaltet, daß der Elektrotauchmagnet(144) den zentralen Teil des Teststreifens mit der Punktionsspitze bis zur Berührung mit der Endplatte des Elektrotauchmagnetes(145) nach rechts verschoben hat.(Er befindet sich nach Darstellung des unteren Profillinienpaare für den Erregungsablauf in einer Dauererregung, was ebenfalls variiert werden kann). Der rechte Elektrotauchmagnet(145) trifft mit den den Anker des linken Magneten umfassenden Enden seines Ankers auf den äußeren Laschen des Teststreifens und hat diese mit der Haut nach links verschoben.
Diese Funktion entspricht der untersten Profillinie mit gehäuften, also unterbrochenem - der gewünschten Rechtsverschiebung des Magnetankers des linken Magneten angepaßtem-Impulsempfang. Es kann auch ein einziger Magnet für die Teststreifenverlagerung vorgesehen werden und zwar mit oder ohne (auch eventuell rückfedernd) nachstellbarem mechanischem Gegenlager (wie etwa einem verschieblichen Keil).
Der Querschnitt oben rechts und Längsschnitt darunter zeigen unterhalb des Saugglockendaches den Rotationsmagneten(146) - einen sogenannten Schrittmotor - mit paariger Leitung(143) zur Stromzufuhr, dessen Zentrum als Magnetanker ausgestaltet ist(feine Schraffur) und dem Schacht mit Kontaktfeder und Signalleitungen für die Aufnahme des Teststreifens(103) im Zentrum.
Das Querschnittsdetail unten in der Mitte erläutert, daß ein Sektorschlitz(150) als Führung für den mit dem Rotor verbundenen Anschlagstift(147) diesem, weil mit dem stehenden Teil verbunden, ein Widerlager für die Rotationsbewegung bietet. Es kann so über Rücklauf des Motors die Größe des Drehwinkels vorher eingestellt werden (nach Steuerung über die nicht eingezeichnete Steuereinheit in Verbindung mit der Batterie).
Links und rechts des mittleren Querschnittsdetail wird als Ausgestaltung der Erfindung eine zusätzlich Scheibe mit Sektorschlitz(148) angegeben, welche über der Scheibe in der Mitte drehbar um eine Achse im Saugglockendeckel angeordnet ist, sie wird je nach Eindringtiefe der im Saugglockendach gedichteten Stellschraube(149) in ein Loch in dieser Scheibe(148)auf Bruchteile eines Millimeters genau relativ zum Sektorschlitz(150) verschoben und verengt den Sektorwinkel für den Anschlagstift (147). Für eine derartige Zusatzausstattung muß das Saugglockendach etwa in dem gestrichelt angedeuteten Ausmaße angehoben werden.
Das Querschnittsdetail ganz unten rechts zeigt die Möglichkeit, mittels der Madenschraube(151) den Korb(152 mit den Klemm- und Kontaktfedern für den Teststreifen(103) mit Signalleitungsansatz exzentrisch innerhalb eines hier quadratischen Schachtes im Zentrum des Rotationseinschubes gegen die Blattfeder(153) zu verschieben und so die Exzentrizität der Punktionsspitze und damit die Schnittlänge zu verändern.
Die Figur 21 variiert und präzisiert nochmals den Rastermechanismus für den Impulsgeber auf Fig.20.
Es handelt sich um einen Längsschnitt unter Weglassung einiger Teile, die unschwer aus Fig.20 und den anderen ergänzt werden können. Der Maßstab ist 2 : 1. Unten sind die Rasterteile am Endteil der Deckelklappe oder -platte (15) nochmals herausgezeichnet; die elastische Zunge in Fortsetzung der Keilführung am Schiebebalken(256) zeigt ein zentralen Loch für die Aufnahme des keuligen Endes des Rasterstiftes. Das Funktionsstadium oben entspricht einem solchen vor, das Detail unten einem solchen nach der Spannung der Feder(30). Rechts im Maßstab 4 : 1 nochmals die Einzelheit der neben dem Schiebebalken nach unten reichenden Keilführung mit elastischer Zunge und Rasterloch (das zweckmäßig mit steilen Kanten bei gießender Herstellung ausgeformt wird).
Daneben ganz, rechts unten, eine Vertikalansicht des Details des Schiebers mit Zunge.(Der Körper des Schiebebalkens wurde der Deutlichkeit wegen schraffiert) In einer Mulde der Deckelplatte ist mittels Stiftes das Ende einer Zugfeder befestigt, die über eine Rundung in das vom Deckel ausgeformte Rohr für die Aufnahme des Deckelstifte führt und an letzterem mit dem anderen Ende befestigt ist. Innerhalb der Feder läuft ein Seilzug (strichpunktiert), welcher die Absenkbewegung des Stiftes nach unten exakt begrenzt. Bei Deckelplattenabsenkung gerät das keulige Ende des Stiftes über die Keilschräge des Schiebebalkens, diesen unter Spannung der Feder(30) nach rechts verschiebend, auf die elastische Zunge. Er drückt diese etwa nieder und gerät mit dem keuligen Ende in die Bohrung der elastischen Zunge, die als Raster wirkt. Bei Anhebung der Deckelplatte bleibt der Kopf des Stiftes in der Bohrung hängen und die Zugfeder wird ausgezogen, solange der Seilzug dies gestattet. In der Endphase der Hebung wird der Kopf des Stiftes herausgezogen und schnellt unter Zugfederwirkung nach oben, so daß die Keilfläche nicht mehr bremsend auf den jetzt von der Feder(30) bewegten Schiebebalken wirkt.

Die Figur 22 bringt im Maßstab 2 : 1 im Längsschnitt zwei Beispiele für eine Sogstärkenregelung mittels Ventiles im Topf des Faltenbalges, das zugleich der Wiederbelüftung der Saugglocke dienen kann. Das erste, obere Beispiel zeigt den Faltenbalg in zwei Ausdehnungsstadien.
Der Topf(41) bildet zugleich das Dach des Faltenbalges(46). Im Topf gleitet der Ventilstößel(154), an dessen Kopfplatte durch Drehung der Stützring(155) für die kleine Druckfeder(156) zum Ventilring(157) innerhalb eines Gewindes zwischen unterem Rand des Ventilstößels und Ventilring den Spannungszustand der kleinen Feder verändern kann. Vom Topfgrund bis zur Oberfläche des Ventilringes spannt sich die Membran(158). Es ist erkennbar, daß in dem Maße wie bei hochgeschraubtem Stützring die kleine Feder stärker zusammengepreßt wird, der Unterdruck, der vom Faltenbalg aus sich durch eine zentrale Öffnung des Topfes unter der Membran(158) auswirkt, stärker sein muß, um den Ventilring vom unten auf dem Topf aufstehenden Ventilstößel abzuziehen, so daß Luft durch den entstehenden Spalt zwischen Ventilring und Ventilstößel in den Raum unter der Membran und damit auch in die angeschlossene(hier nicht gezeigte) Saugglocke zur Druckentlastung eindringen kann. Der um einen Achsstift auf dem Topf schwenkbare Hebel(42) greift mit seinem kurzen Ende unter den Kopfteller des Ventilstößels, der durch Druck auf den langen Hebelschenkel vom Ventilring zur Wiederbelüftung des Faltenbalges und damit auch der Saugglocke abgehoben werden kann.
Zur Hebelbetätigung wird in der Deckelplatte(15,im Längsschnitt darunter) eine Lücke zum Durchgriff gelassen.
Durch den Klemmring(159) gegen die Deckelplatte höhenfixiert, reicht der Gewindestift(160) in die Gewindebohrung des unteren Schenkels der vom Topfrand vertikal herabreichenden U-Lasche(161). Diese paarig-symmetrische Einrichtung ist eine - das Ventil ersetzende - Alternative für eine Unterdruckregelung durch einstellbare Vorlaufstrecke für die (nicht dargestellte) starke Druckfeder (70,Vg.Fig.15) außerhalb des Faltenhalges unter Begrenzung von dessen Endausdehnung.
Unten ist bei zusammengepreßtem Faltenbalg in dessen Topf ein durch Querstift in diesem abgestützter Ring(162) mit Innengewinde als alternative Ventillösung zu erkennen, in welchem der röhrenförmige Ventilstift mit der seitlichen Wandöffnung(163) am Kopfteller höhenverstellbar eingeschraubt. Der Ventilstift wird vom Dichtungsrand des kleinen Faltenbalges(164) umfaßt, der mit zentraler Öffnung in den Sogkanal(32) um diese gedichtet der Abdeckplatte(165) aufsitzt. Der kleinen Faltenbalg hat relativ starre und elastische federnd wirkende Wände. Bei Unterdruck innerhalb desselben, senkt sich durch seine Verkleinerung gegen seine innere Elastizität sein Dichtungsrand entlang dem Ventilstift nach unten und läßt die Wandöffnung(163) bei entsprechender Sogstärke bzw. Höheneinstellung des Ventilstiftes über den Dichtungsrand des kleinen Faltenbalges geraten wobei Luft in diesen eintritt. Auch hier kann mittels Absenkung des (hier rückgefederten) Hebels(42) an seinem langen Schenkels der Ring(162) durch das unter den Rand des Topftellers des Ventilstiftes greifenden kurzen Hebelendes hochgehoben und der kleine Faltenbalg und die angeschlossene Saugglocke wiederbelüftet werden. Die skizzierte Taste Mit Bowdenzug(170), die auf der Deckelplatte montiert ist, wobei das Ende des Bowdenzuges auf den langen Schenkel des Hebels(42) einwirkt, gleicht die Niveauunterschiede zwischen Deckelklappe und oberem Topfrand durch Sogstärkenverstellung aus.
Der Sogkanal wird hier vorteilhafterweise aus einer Rinne von ooben ind er Grundplatte gebildet welche mittels der Abdeckplatte (165) eventuell unter Verwendung einer Dichtung der von einem Satz der Befestigungsschraube(166) abgedichtet ist. Diese Anordnung kann die Montage der Auslöserplatte(53,vgl.Fig.7,Längsschnitt) erleichtern und deren Formgebung vereinfachen.

Die Figur 23 ist ein Nachtrag zur Einrichtungsvariante nach Fig.1 unter dem Schwingprinzip für die Punktionsspitze.
Sie verdeutlicht, wie nach dem Aufprall des Stößels(7) auf den Teststreifen innerhalb der Saugglocke(beides nicht dargestellt) ein langsameres Weiterrücken des Stößels zur Hautspalterweiterung erzielt werden kann.
Auf dem schematischen Längschnittsdetail oben in natürlicher Größe erkennt man die Einstellschraube(54), die auf dem Gewinde des Stößels(7)verstellt werden kann und nach einer (noch nicht erfolgten) Auslösung der Feder(30) im Bereich des Keilschiebers(37, Fig.1, Längschnittsdetail oben rechts) gegen die Vorderkante der Klappe(18) prallt. Im Stadium der weiteren Hebung der Deckelplatte(15) wird mittels der eine Strecke lang gefederten Zugverbindung(36,vgl.Fig.4 mittleres rechtes Längsschnittsdetail) die Klappe(18) angehoben, wobei deren zur Einstellschraube zunächst herabreichende bogige paarige Federzunge(167) die weitere Schubbbewegung des Stößels abfedert. Die Einstellschraube trifft, nachdem sich die Klappe ganz gehoben hat, gegen das Hindernis des Schieberahmens(20).
Das Querschnittsdetail unten zeigt die Befestigung der Drehachse(19) für die Klappe in der Grundplatte(16) und das die Fensterlücke für die Verschiebestrecke des Schiebrahmens.

Die Figur 24 zeigt schematisch einen Längsschnitt durch eine Zylinder-Kolbenpumpe mit Vorlaufmöglichkeit der Deckplatte zur Sogregulierung. Der Dichtkolben(169) wird durch seine Stange im Zylinder(168) gehoben, wobei der Unterdruck über den Sogkanal(32) sich zur Saugglocke hin fortpflanzt. An der Stellschraube(171), die auf dem Ende der Kolbenstange verschraubt ist und die Mitnahme der durch ein Loch in der Deckelplatte hindurchtretenden Kolbenstange bewirkt, kann die Vorlaufstrecke der durch die starke Druckfeder(70) angehobenen Deckelplatte vor dem Dichtkolben reguliert werden.

Die Figuar 25 zeigt im Maßstab von etwa 10 : 1 links eine Draufsicht und rechts einen Längsschnitt durch die Mitte der Draufsicht auf einen Teststreifen mit beigehefteter Punktionsspitze(schwarz). Neben der Stelle des Heraustretens der Punktionsspitze aus Niveau des unteren Teststreifenrandes (der ähnlich wie bei Fig.11 oben auch abweichend gestaltet sein kann) mündet ein mäanderähnlich innerhalb des Teststreifens(103) verteiltes Kapillarsystem zur Aufnahme und Abgabe von Blut, so daß eine Meßausstattung in der Einrichtung selbst auch entfallen kann. Die Folienscheide(102,vgl.Fig.13 Mitte) schiebt mit leicht keilförmig angeschrägten seitlichen Endkanten eine verkürzten Folienblattes oder, wie hier gezeigt, mit keilförmigen Noppen(107) den Teststreifen zur Einführung in die Klemmung eines Schachtes vor sich her. Bei stärkerem Vorschub zur Teststreifenentfernung tritt die Keilkante über den Teststreifenrand und der Querriegel (172), der von einem oder höhengestaffelt von beiden Folienblättern der Folienscheide entspringt, schiebt die lösbar angeheftete Punktionspitze vor sich her, so daß bei der Kapillarblutentnahme die Punktionsspitze kein Hindernis mehr bildet. Die Punktionsspitze kann hierzu (oberes Querschnittsdetail längs der Schnttlinie A - B der Draufsicht rechts mit dem Stadium der zurückgeschobenen Punktionsspitze) in einer Bohrung(173) des Teststreifens gelagert sein.
Der Vertikalschnitt ganz links zeigt eine über den Punktionsspitzenbereich zur Entfernung des Teststreifens mit der Kapillare(174) vorgeschobene Folienscheide mit der Noppe(107), die nun hinter das stumpfe Ende der Punktionsspitze oder des Stahlstiftes eingreift und ein Widerlager zum Herausziehen des Teststreifens aus dem (nicht dargestellten) Schacht der Einrichtung bildet.

Die Figur 26 lehnt sich an Fig.7 an und bringt eine weitere Lösung für die Sogauslösung im Zusammenhang mit dem Problem der Spannung der Starken Druckfeder (nicht dargestellt) für den Faltenbalg. Nur beispielsweise wurde der Bereich um die Saugglocke entsprechend dem Beispiel der Fig.1 gestaltet, wobei durch die Verlagerung der Druckfeder für den Stößel unter denselben gemäß Fig.4 zum Querschnittsdetail Mitte links der Gehäuseblock enger gestaltet werden konnte. Oben wird ein Querschnitt wiedergegeben, darunter ein Längsschnitt und rechts Längschnittsdetail aus der Randzone des Querschntts mit schematischer Darstellung des Folienblattes oder, wie hier gezeigt, mit keilförmigen Noppen(107) den Teststreifen zur Einführung in die Klemmung eines Schachtes vor sich her. Bei stärkerem Vorschub zur Teststreifenentfernung tritt die Keilkante über den Teststreifenrand und der Querriegel (172), der von einem oder höhengestaffelt von beiden Folienblättern der Folienscheide entspringt, schiebt die lösbar angeheftete Punktionspitze vor sich her, so daß bei der Kapillarblutentnahme die Punktionsspitze kein Hindernis mehr bildet. Die Punktionsspitze kann hierzu (oberes Querschnittsdetail längs der Schnittlinie A - B der Draufsicht rechts mit dem Stadium der zurückgeschobenen Punktionsspitze) in einer Bohrung(173) des Teststreifens gelagert sein.
Der Vertikalschnitt ganz links zeigt eine über den Punktionsspitzenbereich zur Entfernung des Teststreifens mit der Kapillare(174) vorgeschobene Folienscheide mit der Noppe(107), die nun hinter das stumpfe Ende der Punktionsspitze oder des Stahlstiftes eingreift und ein Widerlager zum Herausziehen des Teststreifens aus dem (nicht dargestellten) Schacht der Einrichtung bildet.

Die Figur 26 lehnt sich an Fig.7 an und bringt eine weitere Lösung für die Sogauslösung im Zusammenhang mit dem Problem der Spannung der Straken Druckfeder (nicht dargestellt) für den Faltenbalg. Nur beispielsweise wurde der Bereich um die Saugglocke entsprechend dem Beispiel der Fig.1 gestaltet, wobei durch die Verlagerung der Druckfeder für den Stößel unter denselben gemäß Fig.4 zum Querschnittsdetail Mitte links der Gehäuseblock enger gestaltet werden konnte. Oben wird ein Querschnitt wiedergegeben, darunter ein Längsschnitt und rechts Längschnittsdetail aus der Randzone des Querschnitts mit schematischer Darstellung des Auslösemechanismus mittels des Federblechkastenrahmens. Ganz unten noch das Längsschnittdetail durch den Sperrknopf für die Auslöserplattenbewegung.
Die Auslöserplatte(53) ist, wie auf dem Längsschnitt unten zu erkennen ist, dort wo sie an der weitesten Ausladung des Faltenbalges(46) vorbeigeführt wird, in die Höhe hin aufgebogen und so verstärkt. Sie wird durch die paarig angelegte Federzunge(55), wie sie auf dem Längsschnitt in ihrer Funktion verständlich wird, nach Verschiebung nach links wieder nach rechts in die Ausgangslage zurückgebracht. Die Linksverschieberung erfolgt am Keil(75,Detail Mitte rechts,vgl.Fig.6 oben Mitte) bei Absenkung des des Querstiftes(175) am Federblechkastenrahmen - hier als Stift besser sichtbar gemacht - gegen diesen, aber gleichzeitig auch bei der Absenkung des Querstiftes(177) auf die paarige Keillasche (176), wobei die beschriebenen Keile aus der Auslöserplatte samt ihrer Stützverstärkung hochgebogen sind. Die entsprechenden Lücken in der Federplatte sowohl wie der Einlauftrichter für den Querstift(175)im Schlitz der Grundplatte(16) wurden nicht dargestellt.
Das untere der Längschnittdetails rechts gibt des Zustand bei Anspannung der großen Druckfeder nach Senkung der Deckelplatte(15) wieder. Im Querschnittsdetail unten wird die Breitenausladung des Querstiftes(177) in der Hülsenwandung unter den Keilflanken(275) der Auslöserplatte deutlich, der nach Kalibersprung nach innen zugleich der Führung des Zentralstiftes(178) in dessen Schlitz (gestrichelt) dient. Als Sperrklinke für einen Querstift am Ende des Deckelplattenstiftes ist um die Drehachse ein Schwenkhaken mit dem Keil(75) drehbar. Im Detail ganz oben entsteht durch Einzeichnung zweier Stellungen eine Art Hakenkreuz, das verdeutlicht, wie der Schwenkhaken, der bei der Absenkung durch Schub des Querstiftes gegen die Keilschulter nicht weiter im Uhrzeigersinne gedreht werden kann, weil der Haken oben links gegen die Haltelasche der Auslöserplatte stößt. (Eine Spiralfeder dreht in diese Stellung zurück und ist nur hier eingezeichnet). Der Keil (75) ist als bereits nach links zurückgeschoben eingezeichnet unter Weglassung der abgesenkten Querstifte(75,177). Nach der Auslösung des unter der paarigen Keilflanke(176) verhakten Querstiftes(177) durch Druckeinwirkung des Querstiftes(175) auf den Keil(75) kann der Querstift(175) den Schwenkhaken im Gegenuhrzeigersinne wegdrehen und ihn passieren. Bei noch durch Kugeln(180), die in seitlichen Mulden der Grundplatte und in Dellen des Federblechkastenrahmens gelagert sind, gegen eine Verklemmung gesicherter Absenkung desselben wird also jeweils die Teleskophülse für die Deckelplatte und damit die Sogpumpe ausgelöst. Der Federblechkastenrahmen kann aber unter Wirkung seiner Federzunge(181), von denen rechts neben dem unteren Längsschnittsdetail eine herausgezeichnet ist, aber mehrere symmetrisch angeordnet vorhanden sind, kann losgelassen wieder nach oben rücken.
Der Sperrknopf(28,unteres Längsschnittdetail) der von einer Druckfeder in Anlehnung an die Grundplatte(16) in einem Loch der Auslöserplatte(53) festhalten wird, kann an der Schlaufe(182) vor Auslösung der Sogpumpe durch Absenken des Federblechkastenrahmens gegen die Haut durch Zug entriegelt werden. (Ein schwaches Bewegungsspiel zwischen diesem Sperrloch und dem Keil(175) verhindert eine vorzeitige Verriegelung bei ungleichzeitiger Betätigung dieser Auslösemechanismen.
Im Querschnitt oben wurden zwei gegenüberliegende Sperrvorrichtungen der eben beschriebenen Art dem Faltenbalg(46) mit seiner starken Druckfeder(70) zugeordnet.
Die Abdeckelung der Rinne für den Sogkanal(32) oben in der Grundplatte kann durch einen Lappen(185) geschehen, der vom Rand des Faltenbalges(46) ausgeht und geklebt und am Ende in einen Schlitz der Saugglocke eingepaßt werden kann. Ein Dichtungsring entfällt.

Der Sperrknopf(28) kann, wie oben auf dem Querschnitt zu erkennen ist, in einer bevorzugten Variante auch nahe der Drehachse des Haltebügels(79,vgl.Fig.8) - als Sperrknopf(228) montiert sein und kann anstelle der Schlaufe eine Zugsaite zu einer Querstange des Haltebügels aufweisen, so daß dieser mit Aufklappen des Haltebügels vor dessen Absenkung in der Senkrechten ausgelöst wird. (Die beiden die Grundplatte umrahmenden Schenkel des Haltebügels werden zum Durchfassen von Fingern durch einen Bogen vereinigt. Die Saitenverbindung kann entfallen, wenn das obere Ende des Sperrknopfes(280) als Klemmfeder ausgebildet ist, in welche die Querstange des Haltbügels lösbar einrastet.
Die Auslöserplatte(53) kann auch auf einen nach rechts offenen U-Bogen reduziert werden und kann auch gußtechnisch aus Kunststoff gefertigt, ihre seitlichen Federzungen können durch Noppen ersetzt werden die sich in den Rastnischen oder Mulden der Grundplatte gegen je eine Druckfeder anlehnen.
Der Querstift(175) kann auch längs einer senkrechten Rille im Federblechkastenrahmen - der ebenfalls durch Kunststoff ersetzbar ist - durch Verschraubung gegen einen Klemmring höhenvariabel aber -feststellbar gestaltet sein.
Der Längsschnitt unten wurde links durch die Mitte der Saugglocke geführt. Es wurde eine Variante zur visuellen Hautkontrolle skizziert. Von der Lichtquelle(17) mit ihrer Stromleitung zur (nicht dargestellten Batterie), wobei der Stromkreis auch bei Körperberührung des Saugglockenrandes durch Erdschlußkontakt selbsttätig erfolgen kann (nicht dargestellt), geht die Strahlenführung schräg auf die noch nicht angehobene Haut und über das Prisma(183) durch ein linsenbestücktes Sichtfenster im Saugglockendeckel.

Die Figur 27 entspricht weitgehend in der oberen Querschnittdarstellung in natürlicher Größe der Fig.26.
Bei der Einrichtung nach Fig.26 mußte der Keil (765) für den herabtretenden Querstift(177) entweder elastisch nachgiebig sein oder es muß der Sperrknopf(280) während der Absenkung der Deckelplatte zur Spannung der Druckfeder(70) kurz angehoben werden. Der Aufgabe eines möglichst selbsttätigen Funktionsablaufes entsprechend wurde der Schiebekeil(186) hinzugefügt, welcher am Ende der Auslöserplatte(53) vom Querbolzen(187) eines von der Deckelplatte(15) herabreichenden Stiftes bei dessen Absenkung zuletzt nach links verschoben wird. An seinem rechten Ende in einem Schlitz der Auslöserplatte hinaufreichend weist dieser Schiebekeil eine zweite Keilführung(188) auf, welche den Sperrknopf gegen seine Druckfeder aus dem Loch der Auslöserplatte hebt und seine Sperrfunktion dieser gegenüber damit aufhebt. Nach der etwas höher angesetzten linken Keilfläche geschieht dies knapp vor der Linksverschiebung der Auslöseplatte, wobei der Keil(75) unter dem Querstift(177) weggezogen wird(Längsschnittdetail unten). Das kleinere Längschnittdetail darüber in der Blattmitte rechts zeigt die Teleskophülse in zusammengeschobenem, abgesenktem Zustand und den Querschnitt in Verrieglung unter halb des Keiles(75). Dessen Schulter ist leicht abwärts geneigt, um die Verschiebung der Auslöserplatte gegen die Hemmwirkung der Starken Druckfeder(70, Querschnitt) zu unterstützen. Links vom Längsschnittdetail ein Querschnittsdetail mit Schnittführung durch den Querstift(177), der auch durch zwei Nieten oder Noppen am äußeren Rohr ersetzt werden kann unter Vermeidung auch der Schwächung eines Innenstabes oder - rohres durch einen Schlitz unter üblicher Teleskophülsenhemmung zur Auszugsbegrenzung. Es sind zwei den Faltenbalg(46) einfassend einander gegenüber liegende Teleskopvorrichtungen vorhanden, die während der Absenkung des Schaltstiftes(189) auf der Keilführung(190) zur Auslösung der beiden Teleskopvorrichtungen unter Linksverschiebung des Keiles(75) die Saugpumpe betätigen. Der Schaltstift und die zugehörige Keilführung, welche auf einer Hochbiegung der Bodenplatte sitzt, sind paarig angelegt (Längsschnittdetail in der Mitte längs der Schnittlinie A - B des Querschnittes). Der Schaltstift(189)am Federblechkastenrahmen(48) kann längs eines senkrechten Schlitzes zur Einstellungs justierung verschoben und verschraubt werden.
Die Absenkung des Federblechkastenrahmens geschieht gegen die Druckfeder in Nischen der Grundplatte(16) mittels des Haltebügels(79) nach dessen Aufrichtung aus der Waagerechten.
Während der letzten Phase der Bügelaufrichtunng übt die Schlaufe (182) oder Saite vom Ende des Sperrknopfes(28) zu einer Befestigungsöse im Haltebügel oberhalb der Drehachse Zug auf den Sperrknopf aus und entriegelt diesen.
Der Schiebekeil(186) samt Sperrknopf können auch entfallen, wenn die unbeabsichtigte Absenkung des Federblechkastenrahmens dadurch verhindert wird, daß der Haltebügel in umgeschlagenem Zustand gegen den paarigen Stift(191) seitlich an der Grund- oder Gehäuseplatte lehnt und dann in der paarigen Halteklammer(192) des Federblechkastenrahmens einrastet.
Die Stößel(7)-Betätigung erfolgt nach der Variante von Fig.4 (Längsschnittdetail Mitte) durch einen paarigen, die Führung verstärkenden, Kolben, der von der Druckfeder(30) angetrieben wird (Querschnitt oben).
Letztere wird aber hier oberhalb des Stößels plaziert (Längsschnittdetail durch die Saugglocke(1) links unten).
Die Saugglockenränder sind nach außen für den Hauteinzug gerundet, weisen jedoch einen schärferen Randring auf, der sich bei Druck auf die Haut zwecks visueller Vorkontrolle gut abzeichnet, ein Effekt, der mit Farbpigmenten oder Kreide verstärkt werden kann. Eine Einkerbung oben auf der Justierschraube(22) dient der Einstellung der Nachschnittslänge , welche nur einer kleinen Sektordrehung aus der Mittelstellung bedarf. Ähnliches gilt für die Einstellschraube(54) Querschnitt oben), welche vor der Anhebung der Klappe(18) mit der Deckelklappenhebung die eigentliche Schnittlänge bestimmt.
Rechts daneben die schematische Variante eines Auslösers für diese Stößel- oder Schnittbewegung unter Ersetzen des Mechanismus nach Fig.1 (Längsschnitt links oben) durch eine Variante des Mechanismus für den Nachschnitt nach Fig.19. Der Rahmen(126) oder Schieber wird durch den Deckelstift(213) bei dessen Anhebung wie eine Wand hochgezogen bis zu dessen Bewegungshemmung. Es wird so bei der Stößelauslösung die bremsende Keilfläche des Schieberahmens(20) überbrückt.
Ganz rechts unten wird die Variante einer Rückfederung für die Saugglocke mit gesamter Grundplatte gezeigt, wobei an Stelle des Federblechkastenrahmens(48) ein Rahmen Stifte oder Zungen gegen je eine Druckfeder in einer Nische der Grundplatte(16) richtet, eine Variante, die auch für die Rückfederung der Auslöserplatte(53) gilt. (Im Querschnitt oben erkennt man die Spalten für das Bewegungsspiel zur Entriegelung aus den beiden Teleskopvorrichtungen).
In der Mitte links oben ist ein Längsschnittdetail durch eine Nachschnitts- oder Drucktaste(222) mit nach außen abstehender Oberkante für die Betätigung. Der Führungsstift(31) ist in der Grundplatte befestigt und verläuft in einem Senkrechtschlitz der Drucktaste. Der Stift(33) ist an der Drucktaste befestigt und läuft in einem Schrägschlitz mit horizontalem Schenkel im Schieberrahmen. Der Federblechkastenrahmen ist mit einem Fenster heruntergezogen, um die Absenkung der Drucktaste zu ermöglichen. Unter dem Längsschnitt eine Querschnittsdetail durch eine Saugglocke mit einer Anschlagschraube(193), an welcher als Variante die Ausschlaglänge des Teststreifenendes oder der Stößelbewegung eingestellt werden kann. Lagerkugeln(194, Querschnitt) zwischen Grundplatte und Federblechkastenrahmen - mit nicht dargestellten halbkugeligen Ausbuchtungen dafür - aber auch zum Schieberrahmen können ein Verklemmen bei einseitiger Druckausübung verhindern.
Auf dem Längsschnitt unten wird noch eine Variante zur Befestigung des Glukometers(12) oder eines anderen Meßgerätes vorgestellt. Sie erfolgt auf der Deckelplatte(15) mittels eines elastischen Bandes(195) vom einen Randbezirk zum anderen unter Klemmung. In den Schacht(216) wird die Trägerlamelle(216) mit den Meßdrähten zur Signalableitung über Federklemmen dort eingesteckt. Vor Herausziehen des Meßgerätes wird die Trägerlamellen in den Schacht (62) der Punktionseinrichtung zurückgesteckt, wobei eine Feder (als Zugfeder(!96) symbolisiert) das freie bandförmige Verbindungskabel ins Geräteinnere zieht.

Die Figur 28 lehnt sich konstruktiv eng an die Fig.22 an. Es handelt sich wiederum um zwei Längsschnitte verschiedener Funktionsphasen eines Druckregelungsventiles innerhalb des Topfes eines Faltenbalges(64) als Saugpumpe im Maßstab 2 : 1.
Der Hebel(42) für die Wiederbelüftung ist auf der Deckelplatte(15) montiert, was auch für das Beispiel der Fig.22 möglich ist.

Die Druckfeder(156) hält den Ventilring(1557) im geschlossenen Sitz. Die Spannung der Druckfeder kann durch Drehen des Ventilstößels in seinem Gewinde mit dem Stützring für die Feder reguliert werden. Wesentlich ist in diesem Beispiel der verbreiterte Knopf- oder Kopfring des Ventilstößels eine Bohrung der Deckelplatte überragt, durch die er hindurchgezogen ist und daß zwischen der (in der Phase unten) hochgehobenen Deckelplatte und dem Topf(41), der zum oberen Rand des Faltenbalges gedichtet ist, ein Schaltzwischenraum frei bleibt.
Auch deutet rechts unten die breite Klammer symbolisch an, daß die Hubbewegung der Deckelplatte nicht von der Elastizität des Faltenbalges bestimmt wird, sondern von außen durch einen Anschlag an der Grundplatte festgelegt wird. Während der Anhebung des Ventilstößels unter dem Einfluß der (hier nicht dargestellten Druck- oder Rotationsfeder, die an der Deckelplatte angreift,
wird - bei Sogwirkung von unten in den Topf unter die Membran(158) letztere mit dem Ventilring gegen die Druckfeder gesenkt. Die gleichzeitige und plötzliche Anhebung des Ventilstößels bewirkt num daß der Ventilring aus der im oberen Konus des Ventilstiftes geschlossenen Stellung in eine solche im unteren Konusring mit gegenläufiger Schräge gerät und also wiederum schließt (untere Abbildung). Die unten dargestellte Phase des Ventilschlusses ist jedoch nicht stabil, da die Druckfeder den Ventilring wieder anhebt, sobald der Stößel nicht mehr von oben angehoben, d.h.gezogen wird. Letzterer rückt dann bei starker Federspannung unter Ventilöffnung nach oben.
Es wird so nahezu die gesamte Saugkraft im Stadium der Hautanhebung (bis zur Blutleere etwa) in der Saugglocke wirksam. Darnach kann die Sogstärke nach den Bedürfnissen wegen der unterschiedlichen Durchlässigkeit auch unverletzter Gefäße für Blut unter Sog herabgeregelt werden. Im Stadium der Blutfülle kann so ein geringerer Unterdruck hautschonend angewandt werden.

Die Figur 29 zeigt ebenfalls in zwei Funktionsstadien im Längsschnitt, aber in natürlicher Größe eine Vorrichtung für die Lösung der gleichen Aufgabe. Der Elektrotauchmagnet(144) übernimmt dabei als Beispiel die Rolle einer Zuhaltung des Ventilverschlusses zwischen Ventilstößel(154) und Ventilring(157) während der Entfaltung des Faltenbalges durch Heraufziehen seines als Deckel dienenden Topfes(41). Der Ventilring enthält Eisen, welches vom aktivierten Elektrotauchmagnet angezogen wird (oder abgestoßen werden kann, wenn die Funktionsumkehr gewählt wird). Wiederum ist es der durch Drehung des Vetilstößels veränderliche Druck der Druckfeder(156), von dem es abhängt, bei welcher Sogstärke von unten aus dem Faltenbalg der Ventilring von der Membran(158) ins Öffnungsstadium herabgezogen wird.
Über die (nicht dargestellte Batterie) wird die elektronische Steuerzentrale(184) mit Energie versorgt, die ihrerseits den Elektrotauchmagneten aktiviert. Dies geschieht im dargestellten Beispielsfall bis zu dem Augenblick, in welchem zwei Berührungskontakte zwischen den Hülsen der Teleskopvorrichtung an der Deckelplatte durch Auszug durch die (nicht dargestellt starke Druckfeder) die Stromschleife(197) schließt. Die von der Steuerzentrale veranlaßte Stromunterbrechung beim Stand der höchsten Faltenbalgausdehnung läßt in dem Maße Luft zwischen Ventilstößel und Ventilring in den Unterdruckspeicherraum eindringen, wie die Druckfederspannung zuläßt. Es kann also auch durch Impulssteuerung für den Elektrotauchmagneten das Ausmaß eines Unterdruckabfalles bestimmt werden. Einem Ventilflattern kann vorgebeugt durch den Einsatz des Elektrotauchmagneten werden.
Rechts oben ist das Detail eines Elektrotauchmagneten(145) im Ventilzusammenhang herausgezeichnet. Bei sonst gleicher Anordnung im Faltenbalg wurde der Stützring und das Gewinde mit dem Ventilstößel weggelassen (es hätte auch zusätzlich bleiben können). Zwischen Ventilstößel und Ventilring ist nun die Zugfeder(198) ausgespannt, welche den Ventilring solange herabzieht, wie der Elektrotauchmagnet nicht aktiviert ist. Die Sogregulierung erfolgt über die Erdschluß-Meldung des Hautkontaktes mit der Kanülenspitze und verschiedenen
Berührungspunkten innerhalb der Saugglocke, so daß das Sitzventil zur Wiederbelüftung -vorzugsweise in getrennten, wiederholten Impulsen - bis zum erforderlichen Unterdruck geöffnet wird.

Die Figur 30 behandelt das Verpackungsproblem eines Teststreifens mit Punktionsspitze ähnlich wie die Fig.11 und 13.
Der Maßstab ist 4 : 1 . Die Punktionsspitze ist wiederum stark überzeichnet. Links ist eine Draufsicht gegeben, wobei strichpunktiert die Konturen einer noch geschlossenen Verpackungshülle (199) eingezeichnet ist, deren beide Blätter an den sich berührenden Rändern durch eine Klebeschicht hermetisch abgeschlossen sind. Oben ist eine (als Quadrat gezeichnete) längere überstehende Lasche des einen Blattes zu erkennen, die zur Öffnung herabgezogen wird. Dieses Herabziehen ist aber nur etwa zur Hälfte der Gesamtlänge möglich bis zur Stellung der beiden Blätter der Verpackungshülle wie die rechts im Längsschnitt erkennbar sind. Im übrigen liegt eine festere Verbindung mit dem Folienblatt (200) über Verschweißung oder stärkerer Verklebung vor.
Auf der Draufsicht erkennt man zwei tiefe Einschnitte des Teststreifens(103) seitlich vom Testfeld(104) mit seinen Leitungsbahnen. Es werden so zwei seitliche Laschen abgeteilt (vgl.Fig.11, die unabhängig in ihrer Stellung vom Zentrum des Teststreifens mit der Punktionsspitze gehalten und bewegt werden können. Das Folienblatt(200) ist - ebenfalls am Fuße zusammengehalten, also ohne durchgehende Trennlinie - in ein linkes(201) und ein rechtes Blatt(202) aufgespalten. Die eine Hälfte - bei dieser Lage der Punktionsspitze fast im Zentrum - liegt diese auf dem linken Blatt(201) und dort in einer sich zur Vereinigungsstelle der Blätter hin verjüngenden Rinne geschützt.
Beide Blätter des Folienblattes umklammern also den Teststreifen von verschiedenen Seiten. Das rechte Blatt weist, wie im Längsschnitt erkennbar, eine Noppe(107) auf, welche gegen das Ende des Teststreifens stößt und die Einführung des letzteren in den Schacht einer Punktionseinrichtung begünstigt.
Die anschließenden Abbildungen rechts zeigen das Stadium vor dem Herausziehen des Test streifens aus dem Schacht (nicht dargestellt, oben liegend) nach Verbrauch des Testfeldes, nachdem die Verpakkungshülle mit der Scheide des Folienblattes (die entspre-chende nach außen aufgebogene Enden aufweisen können) über Punktions-spitze und Teststreifen geschoben wurden.
Die Noppe auf dem linken Blatt des Folienblattes passierte die Unterkante des Teststreifens durch größere Schubanwendung von unten, die Punktionsspitze trat in ihrer Halbrinne tiefer und klemmt nun in deren Verengung. Die Punktionsspitze kann auch einen Quersteg(203) des Folienblattes durchdringen (Detail ganz rechts). Bei Zug an der Verpackungshülle wird nun der Teststreifen aus dem Schacht gezogen, wobei die Punktionsspitze innerhalb der Verpackungshülle entsorgt wird. Eine Mittelleitung(311) führt von der Punktionsspitze bis zum anderen Teststreifenende für die Erdschlußmeldung in der elektronischen Steuereinheit.

Die Figur 31 zeigt ein Beispiel für eine Punktionseinrichtung, bei welcher Heizdrähte als Saugpumpe Verwendung finden. Die Darstellung erfolgt im Längsschnitt im Maßstab 2 : 1. Es wird eine flache an den Kanten abgeflachte Rechteckform mit leichter Wölbung auf der Unterfläche mit der Saugglocke(1)gewählt, schon der besseren Auflage auf dem Körper wegen.
Der schematisch unten dargestellte Kasten kann - wie mit strichpunktiertem Pfeil angezeigt - der Länge des Punktionsgerätes noch Der schematisch unten dargestellte Kasten kann - wie mit strichpunktiertem Pfeil angezeigt - der Länge des Punktionsgerätes noch bei übergreifendem Steckrahmen(204) aufgeschoben werden und kann das Glukometer oder ein sonstiges Meßgerät enthalten.
In einem Einschub aus Kunststoff mit dem Schiebedeckel(205) befinden sich Batterie(83) und elektronische Steuerzentrale(184). Am Drehknopf(206) - oben in einem Querschnittsdetail - kann der Stromkreislauf entweder zur Versorgung des Heizdrahtes(136) , der in Spiralen innerhalb des Gehäuses mit der gedichtet aufgeschraubten Deckelplatte(15) ausgespannt ist, oder zur Betätigung der Punktion umgeschaltet werden. Die Abschaltung nach Entweichen der erhitzten Luft durch die mittels der paarigen Druckfeder Druckfeder(207) geschlossenen Sitzventile(208,209) kann in technisch bekannter Weise z.B. über einen Bimetallstreifen(nicht dargestellt) erfolgen. Das Sitzventil(208) zur Saugglocke hin wird durch Stoß des Elektrotauchmagneten(210) erst geöffnet, wenn durch die Berührung dreier regelmäßig auf dem Saugglockenrand verteilte Erd- oder Berührungskontakte(211) der Steuerzentrale der sichere Sitz der Saugglocke gemeldet wurde. Die Leitungsverbindung sind strichpunktiert oft außerhalb des Gehäuses angedeutet, in Wirklichkeit aber in die Gehäusewandung integriert. Beim Verbinden des Einschubes mit Batterie und Steuerzentrale, die mit einer Art Stecker erfolgt, werden verschiedene Steckerstifte(213) aber auch Klemmfedern(212) eingesetzt, um Strom und Signale weiterzuleiten. Nach Öffnen des Sitzventiles(208) wird die Luft aus der Saugglocke zu einem wesentlichen Teil abgesaugt und die Haut hochgezogen. Wiederum über Erdschluß bei Berührung der Haut mit den (aufsteckbaren,vgl.Fig.1) Magnetankerhülsen (214,215) - oder derem Einschieben in eine Schlitzverbindung - über die Signalleitung(216) zur Steuerzentrale die Aktivierung der Elektrotauchmagnete (144, 145)nacheinander, wie zu Fig.21 beschrieben ausgelöst. Das auf das Meßfeld des Teststreifens (113) - sofern nicht Sammelkapillaren (vgl.Fig.25) für externe Messungen gefüllt werden - bewirkt einen Signalfluß über die Signalleitungen (72) zum Meßinstrument. Dieses kann über ein Verbindungskabel ähnlich wie in Fig.27 zu einem aufgeschobenen Instrument erfolgen.
(Es können Steckerverbindungen zu den verschiedensten Gerätetypen geschaffen werden, in dem die Trägerlamelle entsprechend dem jeweiligen Teststreifen gestaltet und in den Schacht des jewei-igen Gerätes eingeführt wird.)
Das Meßgerät kann auch im Punktionsgerät integriert sein, etwa in dem Gehäuse mit dem Steckrahmen(204).
Zur Wiederbelüftung aber auch zur Unterdruckregulierung (vgl.Fig. 28,29) wird der Elektrotauchmagnet(210) mit umgekehrter Polung erreicht und stößt das Sitzventil(209) auf. (Die Enden der Sitzventile umgreifen den Magnetanker hülsenartig).
Das Gehäuse mit dem Heizdraht kann mit der Saugglocke aus Metall gefertigt werden, um den keimtötenden Hitzeeffekt und die oligodynamische Wirkung auszunützen. Es muß ein Thermofarb-indikator oder Thermometer auf dem Gehäuse angebracht sein, um vor noch zu großer Hitze zu warnen. Eine hitzeisolierende Gerätehülle zum Einstecken des Gerätes erscheint zweckmäßig. Es kann aber auch wärmeisolierender Kunststoff als Gehäusematerial dienen und für eine allmähliche Abkühlung der erhitzten Luft gesorgt werden. Links unten ist die Variante eines Hebelmechanismus im Querschnitt unter den Elektrotauchmagneten(144,145) im Maßstab 4 : 1 dargestellt, um die Möglichkeit aufzuzeigen, die Schiebestrecke auf die beiden seitlichen Lappen des Teststreifens über die Schubstrecke des Magneten hinans zu verlängern. Eventuell kann auf diese Weise eine Hautnachführung zur Spalterweiterung auch mittels eines einzigen Elektrotauchmagneten für die quere Punktionsspitzenbewegung in der Saugglocke erreicht erreicht werden.
Die Magnetankerhülse(215) wirkt über eine Seitenplatte verschiebend auf das Gelenk(217), so daß der sichelfömige Hebelarm um das Fixgelenk(218) derart schwenkt, daß der seitliche Lappen des Teststreifens(103) in Gegenrichtung zur Verlagerung der Magnetankerhülse(gestrichelte Stellung) verdrängt wird. Die Vorrichtung kann symmetrisch verdoppelt werden, um auch den anderen Lappen oder die andere, seitliche Klappe des Teststreifens mit der Haut, auf der ihr Ende eingedrückt steht, zu bewegen.
Auch die Leitung(143) zur Stromzufuhr zu den Elektrotauchmagneten(144,145,210) ist in die Wandung oder Grundplatte(16) der Einrichtung integriert und wurde nicht genauer bis zur Batterie(83) hin durchgezogen.
Rechts oben noch drei Längsschnittdetail über strukturelle und funktionelle Varianten der Elektrotauchmagnetes für die Punktionsspitzen- und Teststreifenbewegung in natürlicher Größe. Links sind zwei Magnete ineinander teleskopartig verschachtelt. Der Elektrotauchmagnet (144) verlängert seinen Anker im Stößel(7). der zum Teststreifenteil mit der Punktionsspitze im Zentrum führt. Der Stößel wird von einer Hülse umgeben, welche zugleich Anker des Elektrotauchmagneten(145) ist und dessen Magnetwicklungen in scheibenartig horizontal längs des Ankers aufeinander folgender Schichtung nacheinander erregt werden, damit die Bewegung der seitlichen Lappen des Teststreifens(103) gebremst erfolgt. Rechts daneben - durch die Dichte von Punkten als erregte Wicklungsquerschnitte ausgedrückt - eine Magnetaktivitätsbremsung durch die Verminderung der Zahl der in einem Magneten jeweils erregten Drahtwicklungen.

Ganz rechts wird die Lösung einer Magnetverlangsamung durch schwächere Stromzufuhr aus der Batterie(83) über die Leitung (143)durch die Einzeichnung einer zweiten dünneren paarigen Zuleitung zum Elektrotauchmagneten symbolisiert.
Eine Bremsung der Magnet- und Stößelgeschwindigkeit, insbesondere ihrer Endgeschwindigkeit, kann - wie früher beschrieben - auch durch die Stößelbewegung gegen eine Feder erfolgen und auch in einer Kombination mit einer Hebelkonstruktion wie links unten.

Die Figur 32 zeigt oben in einem Quer- oder Horizontalschnitt darunter in einem Längsschnitt im Maßstab 2 : 1 eine Einrichtung unter Verwendung einer ovalären Kolben-Zylinder-Pumpe. Unten ist im Maßstab 4 : 1 der Querschnitt durch eine Nachschnittvorrichtung verdeutlicht. Rechts davon ein Vertikalschnitt mit aufgeschobenem Meßgerät und ganz rechts die Draufsicht auf ein Verbindungskabel, jeweils im Maßstab 2 : 1 .
Der doppelläufige Elektromagnet(210) betreibt das Sitzventi81(208) für die Belüftung der Saugglocke(1), welche mit dem Gehäuseteil(228) gedichtet, aber durch Verschraubung lösbar verbunden ist. Der Gehäusteil(228) enthält hinter der gedichteten Klappe (229) die Batterie(83) und die elektronische Steuerzentrale (184). In der Gegenrichtung wirkt der Elektromagnet über das Gestänge (230) auf die Aufnahmebuchse(231) ober halb deren Drehpunkt ein und bewirkt die Abkippung des Teststreifens(103, gestrichelt auf dem Längsschnitt unten). Für das Bewegungspiel des Gestänges ist Spaltraum im Gehäuseteil gelassen, worin ansonsten Hohlräume vermieden wurden, um den Sograum nicht zu vergrößern.
Der Drehknopf(236) zur Geräteeinschaltung wird ebenfalls durch einen Dichtring in den Gehäuseteil eingeführt.
Das kleine Querschnittsdetail unter dem großen etwas tiefer als die oben dargestellte Schnittebene zeigt den Teststreifen(103) zwischen zwei Klemmfedern, welche zugleich der Ableitung der Meßströme dienen. Eine (nicht dargestellte) Kontaktfeder vom Dach der Aufnahmebuchse nach unten leitet im Zentrum der Buchse die Ströme von der Leitung (als Punkt) zwischen Punktionsspitze und Teststreifenende zur Steuerzentrale für die Erdschlußmeldung. (Die Leitungsanfänge sind strichpunktiert eingezeichnet).
Der rechte Arm des Gestänges ist gegen den kleinen Konus an der Drucktaste(222) gerichtet, welcher ihre Bewegung nach rechts begrenzt.
Wie auf dem Längsschnitt erkennbar ist wird die Aufnahmebuchse(231) von einer gebogenen Platte überdacht und ist an ihr befestigt, welche um die gekrümmte Schiene(237) seitlich verschieblich und dabei schwenkbar ist. Für den Durchtritt des Verbindungsarmes zwischen gebogener Platte und Aufnahmebuchse mit ihren Klemmfedern und das Gestänge(230) sind entsprechende Schlitze in der Schiene angebracht. (Strichpunktiert ist eine Schleife für den Bewegungsraum der Ableitungsdrähte lediglich angedeutet (und liegt natürlich unterhalb des Saugglockendaches). Die funktionelle Bedeutung dieser Vorrichtung wird zu Fig.40 näher erläutert.

Die Grundplatte(16) oder Gehäusewandung rechts der Saugglocke ist als ovalärer Pumpenzylinder ausgebildet, in welchem der Hohlkolben(232) verschieblich ist. Letzterer weist innen das Rohrsegment(233) auf, in welchem in Querbohrung der Schaltkörper (234) gelagert ist und der vom Schaltsegment(235) umgeben ist. Letzteres ist gegen das Rohrsegment(233) mittels einer Blattfeder so rückgefedert, daß ihre Ringnut vom Schaltkörper wegbewegt und dieser in der Ringnut des Zentralstempels(238) verriegelt wird.
Für die Entriegelung durch Bewegung nach rechts ist das sich selbst einrollende Zugseil(239) zwischen Schaltsegment und Enden der Auslösegabel(241) paarig angelegt. Die Enden der Auslösegabel werden längs zweier Schlitze(242) im Zentralstempel geführt. Die starke Zugfeder(270) für die Kolbenbewegung ist zwischen dem Stift(243) (und Kolbenringsegmenten) und der Halterung(244) am Ende des Pumpenzylinders eingespannt. Der Federblechkastenrahmen (175) - der hier in Kombination mit getrennten Druckfedern auch aus Kunststoff hergestellt sein kann - zeigt, wie auf dem Längs-schnitt unten ersichtlich die paarig angelegte Keilnase(245), welche auf die Querholme der Auslösegabel(242) einwirken und diese nach rechts ziehen, wenn der Federblechkastenrahmen gegen seine vier symmetrisch verteilten Druckfedern(247) abgesenkt wird. Dabei wird das Zugseil gespannt und durch Bewegung des Schaltsegments nach rechts die Verriegelung zwischen Zentralstempel und Rohrsegment gelöst und die Zugfeder auf den Kolben wirksam.
Für den Stift(243), der den Spalt zwischen Rohrsegment und Kolben überbrückt ist ein Schlitz im Schaltsegment vorgesehen.
Der gestrichelt-ovaläre Ring im Zentrum des Querschnitts soll lediglich die Ovalform des Pumpenzylinders deutlich machen. Der Nasenvorsprung(250) im Zentrum der Querverbindung des Blechkastenramens wirkt gegen eine Keilschulter des Zentralstempelendes diesen festhaltend und bei Absenkung des Rahmens dessen Bewegung freigebend.(In einer auch hier anwendbaren Variante wird der Vorgang zu Fig.36 nochmals erläutert).
Die Batterie(83) wird von oben eingelegt und gedichtet abgeschlossen.
Das Querschnittsdetail links unten im Maßstab 4 : 1 zeigt die Drucktaste(222)die unter ihrer Tellerplatte mit der Kappe(251) verschraubt und durch einen Dichtungsring gegen die Grundplatte (16) des Gehäuses gedichtet ist. Die Kappe ist gegen eine kleine Druckfeder in eine Ringmulde der Grundplatte hinein absenkbar. Das Ende der Drucktaste ist konisch und in eine Bohrung am Ende ist der kleinere Konus(252) eingeschraubt.
Im dargestellten Funktionsstadium ist das Ende des Gestänges(230) gegen diesen kleineren Konus gerichtet. Die Tiefeneinstellung des großen Konus an der Drucktaste für den Nachschnitt kann durch Drehung am Teller der Drucktaste verändert werden. Dieser Teller und die Kappe können auch gemeinsam gedreht und dabei über den Drehknopf(236) der Elektromagnet aktiviert werden, so daß der kleinere Konus fixiert wird während der Umdrehung der Drucktaste zur Höheneinstellung, welche dann den Freiraum für die Bewegung des Gestänges bestimmt. Wird die Drucktaste gedrückt, so gerät deren größerer Konus in die Nachbarschaft des Gestänges und begrenzt deren Rechtsverschiebung während der Punktion.(Der Zwischenraum zwischen Kappe und Grundplatte ist zu knapp gezeichnet). Das Vertikalschnittsdetail im Maßstab 2 : 1 rechts der Mitte läßt erkennen, daß die Randkontur des Federblechkastenrahmens(175) an ihrem oberen Rand vor dem Nasenvorsprung(250) des Zentralstempels zurückweicht. Die anschließende Schulter liegt der Keilschrägen des Endes des Zentralstempels auf. U-förmige Abwinkelung an Teilen des oberen Randes des Federblechkastenrahmens bieten der Schiene (253)
am Meßgerät Halt, das aufgeschoben wurde. Die Enden der Druckfedern stehen in Verbindung mir dem Zylinder der Saugpumpe und des Gehäuseteiles und bewirken deren Absenkung unter Abstützung gegen den Federblechkastenrahmen bei druck von oben auf das Meßgerät. In den Schacht(62) des Punktionsgerätes kann die Trägerlamelle(209) eingesteckt werden, während die Trägerlamelle des anderen Endes des Verbindungskabels(254) in den Schacht(262) des Meßgerätes eingeführt wird. Das Verbindungskabel ist in einer Draufsicht rechts - bei verkürztem Kabel - dargestellt, wobei die Trägerlamellen in Form und Farbe entsprechend gekennzeichnet werden. Es können verschiedene Kabel für verschiedene Trägerlamellen für die Schachteinführung in Meßgeräte verschiedener Hersteller produziert werden, wobei die Anschlüsse variieren können.

Die Figur 33 gibt in Querschnittsdetail im Maßstab 2 : 1 vier Funktionsstadien A - D einer Einrichtung nach Fig.32 wieder. Oben (in A) ist der Zentralstempel mit dem Rohrsegment des Kolbens verriegelt und die Zugfeder ausgespannt nach Bewegung des Zentralstempels nach links bis zu dessen Stopp und Rückkehr-blockierung, die durch ein Festhalten des Keilendes des Zentralstempels durch den Federblechkastenrahmen bewirkt wird. Das Zugseil zwischen Auslösegabel und Schalthülse ist gestrafft.
Im Funktionsstadium B wurde durch Absenkung des Federblechkastenrahmens das Gestänge am Zentralstempel gezogen und damit die Schalthülse nach rechts gezogen und der Kolben damit entriegelt; der Kolben befindet sich also unter Entspannung der Feder auf dem Weg nach rechts.
Der Zustand nach der Kolbenbewegung wird im Stadium C demonstriert, wobei die Blattfeder im Schaltsegment eine Entriegelung des Kolbens noch nicht vornehmen kann, weil die Ringnut des Zentralstempels erst im Stadium D dem Schaltkörper die Möglichkeit gibt, in den Zentralstempel auszuweichen. Dies erfolgt unter Einfluß der Keilschrägen bei Rückfederung des Schaltsegmentes. (Die Auslösegabel ist ja losgelassen, der Zentralstempel herausgezogen.) Erst nach Eindrücken des Zentralstempels - etwa gegen eine Tischplatte - wird die Zugfeder gespannt, weil der Kolben ja gegenüber dem Zentralstempel verriegelt ist. Der Federblechrahmen kann sich nun wieder bis vor die Keilschräge des Endes des Zentralstempels heben und dieses feststellen.
Der Tauchmagnet(210) im Gehäuseteil kann über Rolle(318) am Zylinderende und Seilzug(319) zum Schaltsegment(235) die Pumpenauslösung bewirken.

Die Figur 34 gibt oben die schematische Seitenansicht auf und darunter einen Längsschnitt durch eine Einrichtung ähnlich wie die in Fig.32 und 33 beschriebene. Der Zylinder oder die Grundplatte(16) wurde aber wesentlich verkürzt und für den Auszug der starken Zugfeder(270) wurde diese am Ende eines Rahmenschenkels(255) befestigt, welcher überlappend mit dem verkürzten Federblechkastenrahmen(175) diesen verlängernd nach rechts herausgezogen wird. Der in der Seitenansicht angedeutete paarige und gegenseitg rückgefederte henkelartige Ausziehbügel(276), dessen Hakenenden(schwarz) in Schienen des Federblechkastenrahmens geführt werden, ist durch entsprechende Formgebung für Griffigkeit an den Enden des Rahmensschenkels entbehrlich.
Unten links wird je in einem Vertikalschnitt- und Längsschnittdetail eine Möglichkeit zur Verriegelung der beiden gegen die Zugfeder auseinander gezogenen Rahmenteile in gelöstem (links) und verriegeltem Zustand (rechts) vorgestellt. Die Darstellung wird durch diejenige zu Fig.35 ergänzt.
Der doppelplattige Rasterbolzen(259) ist durch einen Vertikalschlitz(260) im Rahmenschenkel(255) durchgesteckt und verläuft im Längsschlitz(261) des Federblechkastenrahmens(175). im Verlauf der Auflage auf letzterem liegt der Achse des Rasterbolzens eine dort mit einem Schenkel befestigte Blattfeder auf, deren aufgebogene Seitenlaschen(263) den Federblechkastenrahmen umfassen. Gerät bei vollständigem auseinanderziehen der beiden Rahmenteile der Rasterbolzen in die Randaussackung des Längschlitzes, so treibt die Blattfeder mit den Seitenlaschen den Bolzen sperrend nach unten (rechte Abbildung und Seitenansicht oben).
Auf einem Vertikalschnitt rechts wird unter Weglassen der Zugfeder und des Kolbens der Zylinder als Grundplatte(16) gezeigt, welcher durch eine Art Federblechkastenrahmen (175, ausgezogen in Linie) über die Vermittlung paariger Druckfedern nach unten gedrückt wird. Die Druckfedern können zweckmäßig auch durch paarige Torsionsfedern (254)ersetzt werden, welche über Hebel wirken, die ihre Achslager an Endlaschen des Zylinders bzw. des gegenseitig an der Schmalseite des Gerätes liegenden Geräteteiles haben. (Der gestrichtelte Ellipsenring entspricht einer gehobenen Ausgangslage.) Federblechkastenrahmen(175) und Rahmenschenkel(255) liegen gebogen dem Pumpenzylinder parallel.

Die Figur 35 zeigt wiederum im Maßstab 2 : 1 die Variante der Einrichtung nach Fig.32,33 in der Art, wie in Fig.34 ausgeführt. Es handelt sich um Querschnittsdetails in zwei verschiedenen Funktionsstadien und in verschiedener Ausstattung des Saugglockenbereiches; auch wurden der Kolben, Schalthülse und Zentralstempel weggeslassen(vgl.Fig.32). Links oben ist ein Längsschnittdetail eingefügt.
Die Lage einer der beiden Ausziehbügel(256,vgl.Fig.34) ist eingezeichnet. Es kann aber auch die Querstrebe(265)zwischen dem paarigen Rahmenschenkel(255), die wegen des Kolbens und des Zentralstempels bogig geführt werden muß, als Auszugsmittel benutzt werden. In der Abbildung oben ist nach Ausziehung und Verriegelung der Rahmenteile mittels des paarigen Rasterbolzens(259) die Zugfeder durch die Kolbenbewegung nach links mittels (des hier weggelassenen) Zentralstempels gespannt worden, unten wurde der Kolben durch die Zugfeder nach rechts verschoben unter Sogentwicklung und -fortpflanzung durch den Sogkanal(32) in die Saugglocke(1). Die Auslösung der Saugkolbenbewegung erfolgt über die Lasche(266)am Kolben(232, in deren Bohrung das angeschrägte Ende des Galgens(267, Längsschnittdetail oben links) einrastet. Dies Einrasten wird durch eine Druckfeder bewirkt, wobei drei solcher Galgen mit über den Saugglockenrand und außerhalb desselben herausragende Galgenenden zum Inneren des Gehäuseteiles(228.vgl.Fig.32) gedichtet sind. die bogige Querplatte(268) verbindet die Auslösestifte oder Galgen untereinander.

Die Figur 36 gibt noch einmal einen Gesamtquerschnitt (oben) und eine Seitenansicht längs der Seitenwand einer Einrichtung nach den Fig.33-35 mit einer weiteren Variation der Verrasterung der Rahmenteile. Der rückgefedert am Federblechkastenrahmen(175) mit seiner Achse befestigte Rasthebel(271) greift mit seiner Keilnase in eine Nut des Rahmenschenkels(255) ein, der hier als äußeres Schiebeglied angeordnet ist. Der Seilzug(272) läuft über Rollen zum Elektromagneten(144), der außerhalb des Dichtraumes im Gehäuseteil(228) liegt und auch den Rasthebel der Gegenseite betätigt. (Anstelle von Seilzügen sind auch Hebelverbingungen einsetzbar). Die Aktivierung des Elektromagneten (210) kann über elektrischen Kontaktschluß (etwa eine Erdschlußleitung) längs der Schmalseite des Rahmenschenkels durch Handkontakt dort erfolgen. (In diesem Falle wirkt die Meldung eines Hautkontaktes an den drei Erdkontakten(211) auf dem Sauggglockenrand über die Signalverarbeitung in der elektronischen Steuerzentrale(184) sperrend auf diese Funktion.) Wird die Drucktaste(222,vgl.Fig.32) für den Nachschnitt nach oben neben den Saugglockendeckel verlegt, so kann der paarige Rasthebel271) günstiger nach oben verlegt werden. Nach Entriegelung der Rahmenteile kann das Gerät auf seine kürzeste Länge zusammengeschoben werden.
Die Schieberahmenteile ersetzen in diesem Falle den Federblechkastenrahmen. Die Druckfedern(247) liegen jeweils in einer Schiene an Gehäuseteil bzw. Zylinderwandung, eingespannt zwischen einem Winkelstück des "Federblechkastenrahmens"(175) und einer mit dem Gehäuseteil bzw. Zylinder verbundenen Bodenlasche(273, siehe Vertikalschnitt unten links). (Nicht dargestellt e Verfugung nach Schwalbenschnwanzart verhindert ein seitliches Herausdrängen der Rahmenteile.) Die Schraube(274) kann durch Druckeinwirkung den Spannungszustand der Druckfeder verändern.
Das dem Zentralstempel zeitweilig mit einem Hakenprofil anliegende Ende des Rahmenschenkels(255) wurde als Fettstrich in einem Längschnittdetail unten herausgezeichnet. Es trifft auf die Keilschulter(275) des Zentralstempels(238). Beidseits seitlich dieses Hakens wirkt die Keilnase(276) auf den Querholm der Auslösegabel(241) ein; dies geschieht sowohl bei deren Anhebung als auch bei deren Absenkung. Da während der Anhebung des Rahmenschenkels unter Wirkung der Druckfedern(247) der Vorsprung (276) unter Druck der manuellen Federspann- oder Schiebebewegung (Tischkante) bleibt, wirkt sich funktionell nur die Absenkungsbewegung entsperrend auf die Pumpenfunktion aus.
In einer Variante können zur weiteren Verschmälerung der Einrichtung die Druckfedern(247) paarig in das Federlager(277) außerhalb des Sograumes im Gehäuseteil bzw. in das Federlager(278) am Querstück des Rahmenschenkels nach innen verschoben werden. Das untere Druckfederende des Federlagers(278) findet dann seinen Gegensitz auf dem nach unten gebogenen Knie der Halterung(244) für die Zugfeder, die in der paarigen Schiene(279) des Rahmenschenkels höhenverschieblich ist. Eine entsprechende Schienung(281) muß dann auch für den "Federblechkastenrahmen" ohne Horizontalverschieblichkeit vorgesehen werden. Damit sind alle wichtigen Auslösungsfunktionen hier vereint: die Auslösung der Saugpumpe durch den manuellen Andruck der Rahmenteile gegen die Haut unter Absenkung. Die Kontrolle des Unterdruckes über die Erdschluß-kontakte am Saugglockenrand aber auch im Saugglockeninneren (vgl.Fig.42) mit der Unterdruckkontrolle über das Sitzventil(208) nebst quere Hautpunktion sowie die Entriegelung der Rahmenteile zum Verkleinern der Außendimension der Einrichtung nach Wiederbelüftung durch Meldung vollzogener Blutsättigung des Testfeldes über das Meßgerät über Schacht(62).
Es ist noch das Staub- oder Quarzfilter(282) nachzutragen, das in eine senkrechte Bohrung im Sogkanal(32) zwischen Zylinder-Kolbenraum und Saugglocke eingeschoben ist. Es besteht aus einer Trommel (vgl.Längsschnittdetail in der Mitte) mit feinen Gitterwänden, die möglichst klebrig überzogen sein sollten, um die feinsten Quarzkörner der Haut aufzufangen.

Die Figur 37 zeigt schematisiert im Maßstab von etwa 3 : 1 im Längsschnitt eine Auslösevorrichtung für den Stößel(7) einer mechanischen Einrichtung etwa nach Fig.27 (u.a., z.B.Fig.1) Von der Deckelplatte wirkt ein Stift gegen den Keilschieber(37) und hat diesen (obere Darstellung) nach links verdrängt.
In Dieser Stellung verharrt der Keilschieber, weil der Schieber (283) unter Rotation des Radsegmentes(284) im Gegenuhrzeigersinne mit seiner kleinen Blattfeder an der Keilnase des Schiebers hängenblieb. Die Torsionsfeder(285) wurde dabei gespannt. Das Ausmaß der Sektordrehung kann durch die Stellschraube(286) beeinflußt werden, deren Querstift in einen Sektorschlitz des Radsegments eingreift und deren kantige Gegenmutter von unten in eine Lücke der Deckelplatte(15) eingelassen ist. Sobald bei weiterer Anhebung (Abbildung unten) die kleine Blattfeder die Keilnase verlassen hat, wird der am Keilstößel befestigte Schieber(283) von der Zugfeder(287) herabgezogen und gibt die Stößelbewegung nach rechts unter Einfluß der(nicht dargestellten) Druckfeder (30.vgl.Fig.1) frei.
Anstelle des senkrecht verschieblichen Schiebers kann auch ein um eine Achse auf dem Stößel schwenkbares Sperrsegment eingesetzt werden, wie das Detail rechts unten zeigt.
Das Detail links unten im Vertikalschnitt zeigt, wie die Stellschrauben(286) über den Querstift(schwarz) nicht nur das Bewegungsmaß im Sektorschlitz des Radsegments(284) beschränkt sondern auch die Höhenbewegung Der Deckelplatte(15) durch einen paarigen Seilzug(288) begrenzt. Es wird also der Auslösepunkt für den Stößel zugleich mit der Ausdehnung des (nicht dargestellten) Faltenbalges durch Beschränkung der Ausdehnung der Druckfeder(70) reguliert. Bei Endanspannung des Seilzuges zieht dieser den Ventilstößel(154) gegen die kleine Zugfeder aus seinem Sitz, so daß Luft durch die Grundplatte in den darüber geschlossenen (hier offen dargestellten) Raum eindringen kann. Zuletzt aber verschließt die Gegenkonizität des Ventilstiftes wiederum die Ventilbohrung.
Das Ausmaß des Luftdruckzunahme durch Ventilöffnung kann durch Verschraubung eines zentralen Stiftes im Gewinde des Ventilstiftes mit der Aufhängung der Zugfeder reguliert werden. Das Prinzip entspricht dem zu Fig.28 beschriebenen. Es kann auch so das Ziel erreicht werden, nach anfänglich kräftiger Zugwirkung auf die Haut den Sog zur Hautschonung zu vermindern.
Im Maßstab 4 : 1 ist das Ventil unten in der Mitte im Längsschnitt nochmals skizziert.

Die Figur 38 zeigt im Maßstab von 2 : 1 schematisch im Längsschnitt die Möglichkeit auf, einen Teststreifen(103) mit Punktionsspitze von seiner Schmalseite her mittels eines Stössels(7)- eventuell gegen eine leichte Rückstellfeder oder gegen die Federwirkung der Haut - nach rechts zu bewegen, indem die Aufnahmebuchse(231) für den Teststreifen um eine Achse geschwenkt wird.

Die Figur 39 zeigt im Maßstab 2 : 1 im Querschnitt die Möglichkeit auf, einen Stößel(7) auf eine exzentrisch unter dem Saugglockendach um eine Achse drehbaren Scheibe(289) - etwa an einer Segmentkerbe - einwirken zu lassen. (Dies geschieht wiederum eventuell gegen eine leichte Rückstellfeder.)
In der exzentrisch in der Scheibe(289) aber in Ausgangsstelle zentrisch zur Saugglocke montierten Aufnahmebuchse(231) wird der Teststreifen mit Punktionsspitze zunächst fast nur quer zur Saugglocke und längs der Stößelachse und dann seitlich bewegt. (Letzere Bewegung fördert, langsam ausgeführt, die Hautspalteneröffnung für den Blutaustritt.)

Die Figur 40 zeigt schematisch im Maßstab 2 : 1 im Längsschnitt die Möglichkeit auf, durch eine Führung der Aufnahmebuchse (231) mit dem Teststreifen längs einer nach oben konvex unter dem Saugglockendach gebogenen Schiene(237) so zu führen, daß die Punktionsspitze unter Einfluß einer Stößelbewegung nach rechts eine geringere Strecke längs der Haut, in die sie eingedrungen sein muß, zurücklegt als die Basis der Punktionsspitze, welche in die oberen Hautschichten eindrang. Links ist der Drehpunkt für die Satelitenbewegung nach unterhalb der Spitze verschoben und die Bewegung von Spitze und Stiftbasis gleichlaufend. Dagegen ist der Drehpunkt rechts in die Stiftmitte verlegt und Spitze und Basis bewegen sich gegenläufig, zur Hautspalteneröffnung **gleichzeitig** mit der Kapillarzerstörung.

Die Figur 41 bringt in einem schematischen Längsschnitt im Maßstab 2 : 1 eine Lösung, bei welcher der Teststreifen das Gelenk(290) aufweist, um welches er knickbar ist. Der Knickwinkel wird beispielsweise dadurch beschränkt und festgelegt, daß das Gelenk von auf, einen Stößel(7) auf eine exzentrisch unter dem Saugglockendach um eine Achse drehbaren Scheibe(289) - etwa an einer Segmentkerbe - einwirken zu lassen. (Dies geschieht wiederum eventuell gegen eine leichte Rückstellfeder.)
In der exzentrisch in der Scheibe(289) aber in Ausgangsstelle zentrisch zur Saugglocke montierten Aufnahmebuchse(231) wird der Teststreifen mit Punktionsspitze zunächst fast nur qsuer zur Saugglocke und längs der Stößelachse und dann seitlich bewegt. (Letzere Bewegung fördert, langsam ausgeführt, die Hautspalteneröffnung für den Blutaustritt.)

Die Figur 40 zeigt schematisch im Maßstab 2 : 1 im Längsschnitt die Möglichkeit auf, durch eine Führung der Aufnahmebuchse (231) mit dem Teststreifen längs einer nach oben konvex unter dem Saugglockendach gebogenen Schiene(237) so zu führen, daß die Punktionsspitze unter Einfluß einer Stößelbewegung nach rechts eine geringere Strecke längs der Haut, in die sie eingedrungen sein muß, zurücklegt als die Basis der Punktionsspitze, welche in die oberen Hautschichten eindrang. Links ist der Drehpunkt für die Satelitenbewegung nach unterhalb der Spitze verschoben und die Bewegung von Spitze und Stiftbasis gleichlaufend. Dagegen ist der Drehpunkt rechts in die Stiftmitte verlegt und Spitze und Basis bewegen sich gegenläufig. zur Hautspalteneröffnung **gleichzeitig** mit der Kapillarzerstörung.

Die Figur 41 bringt in einem schematischen Längsschnitt im Maßstab 2 : 1 eine Lösung, bei welcher der Teststreifen das Gelenk(290) aufweist, um welches er knickbar ist. Der Knickwinkel wird beispielsweise dadurch beschränkt und festgelegt, daß das Gelenk von einer Folienschleife auf der Seite rückwärtig zum Stößel überbrückt wird, die sich weitere Bewegung hemmend strafft.

Der Stoß in das Gelenk bewirkt eine stärkere Bewegung der Punktionsspitzenbasis in der (strichpunktierten) Haut und eine geringere gegenläufige der Spitze, die dann aber streckengleich nachgeführt wird. (Siehe das Detail des von der Seite gesehenen Teststreifens im Maßstab 4 : 1 rechts.)

Die Figur 42 bringt in einem schematischen Längsschnitt durch die Saugglocke(1) im Maßstab von etwa 1 : 1 die bevorzugte Lösung einer Steuerung des Elektromagneten(144) für das Sitzventil(208) über die als drei Ringe eingezeichneten Erdkontakte innerhalb der Saugglocke. Die beiden unteren Kontaktpunkte sind auf Stangen gelegt, welche in die Saugglocke hineinragen (in Wirklichkeit entsprechen sie Hautberührungspunkten, wie sie durch eine die rechtsseitig als Beispiel eingezeichnete Saugglockenkonturierung (etwa in Stufen) sich ergibt. Von den Leitungen zur elektronischen Steuerzentrale(184), zur Batterie(83) und zum Elektromagnet ist jeweils nur eine eingezeichnet. Zur Punktionsspitze hin ist der Teststreifen(103) hier nur beispielsweise abgerundet; von der Spitze führt eine Leitung zu einem Kontaktpunkt in der Aufnahmebuchse(231) und meldet dort die Durchbohrung der Haut.
Der Sogkanal(32) vom Ventilraum zur Saugglocke ist nur schematisch bezeichnet; in Wirklichkeit ist er sehr viel kürzer.

Die Figur 43 zeigt in einem schematischen Längsschnitt im Maßstab 2 : 1 durch eine Saugglocke aus einer Punktionseinrichtung, wie sie bisher dargestellt wurden, daß derselben ein Injektor angeschlossen werden kann. Im dargestellten Beispiel handelt es sich um ein Einmal-Injekt mit Düse, welche durch einen Treibsatz betrieben wird. Die Aufnahmemanschette(291) für das Injekt ist mit die Haken(292) durch Andruck gegen einen Dichtring gedichtet mittels Halteschrauben verklammert. In die Aufnahmemanschette wird die Patrone(293) eingeschraubt, wobei der O-Ring(294) nach unten und die Dichtlippe(265) die Abdichtung des Bajonettverschlusses(296) sichern, in welchen jeweils jede neue Patrone eingeschraubt wird. Unter der Schraubkappe(297) lagert hinter einer Membran Säure, welche mit dem Natriumbikarbonatpulver im Hohlkolben(299) zur Reaktion gebracht wird, sobald durch Herabschrauben der Schraubkappe in einem oberen, äußeren Bajonettverschluß, die eingelagerte Spitze(300) die Membran über dem Hohlkolben zerstört. Der entstehende Kohlendioxiddruck treibt den Hohlkolben vorwärts und die vor diesem gelagerte Arzneiflüssigkeit durch die Düse und die anliegende Haut.
Die Düse wird bei Schräglage der Achse des Injekts in Absenkung zur Saugglocke dort an einer sicheren Berührungsstelle mit der Hautkuppenbasis während der Sogeinwirkung plaziert.

Die Figur 44 zeigt schematisch in einem Querschnitt im Maßstab von etwa 4 : 1 eine Möglichkeit auf, die Punktionsspitze schräg zur Einwirkungsrichtung des Stößels(7) zur Hautspalterweiterung durch denselben zu bewegen. Die Aufnahmebuchse(231) mit den Klemmfedern für den Teststreifen - die in verschiedenen Höhen dem Teststreifen anliegen und damit auch als Kontaktfeder für Leitungen dienen können - wird mittels der nach oben reichenden und hinter der paarigen Schiene(302) hakenförmig abgebogenen Haltelasche längs dieser unter dem Saugglockendach verschieblich. Wie die feingestrichelte Schiene unterhalb zeigt kann der erste Anteil des Schienenschenkels, in dessen Bereich die ruckartige Bewegung sich ereignet, achsenparallele zum Stößel geführt werden.
Besonders vorteilhaft ist diese Konstruktion bei länglichem Punktionsspitzenschaft, der parallel zur Stößelwirkrichtung angeordnet ist. In Kombination etwa mit einer Schienenkrümmung wie in Fig.32 und 40 dargestellt und erläutert - also durch deren Diagonalstellung - läßt sich der Hautspreizeffekt steigern.

Die Figur 45 zeigt oben in einem Querschnitt und unten in einer Draufsicht im Maßstab von etwa 4 : 1 einen Teststreifen(103) mit angeschweißter Stahlfolienplatte und Aushöhlung in einer Hohlrinne in Richtung auf die Punktionsspitze. Dabei kann die Folienplatte entsprechend der gestrichelten Linien verschmälert werden. Die Wölbung der Hohlrinne soll die Stabilität gegen die Stoßbewegung erhöhen und den Blutabfluß begünstigen.

Die Figur 46 bringt in einer Draufsicht und rechts davon in einem Vertikalschnitt im Maßstab 4 : 1 eine Punktionsspitzenvariante, bei welcher zur Stabilisierung gegen die Stoßbewegung der Punktionsspitzenschaft an der Stelle des Austritts aus dem Teststreifenende bogig ausgebildet ist. Der Kontaktstreifen des Stahlbleches mit dem Teststreifen kann bis zu den feingestrichelten Linien verschmälert werden.
Rechts daneben die Variante der Zufügung einer Punktionsspitze von wahlweiser Querschnittsform im Längsschnitt (darüber Querschnitte durch den Schaft der Punktionsspitze in Nähe des Testreifen(103)-Endes für quadratische, sechseckige und dreieckige Gestaltung). Es wurde noch ein Testfeld(104) mit Chemikalien eingezeichnet.

Die Figur 47 zeigt links in einem Längs und rechts in einem Vertikalschnitt im Maßstab von 4 : 1 die Anschweißung oder Anklebung eines Kanülensegmentes an den Teststreifen(10.3) mit dem Testfeld (104). Die Kittmasse(303) zwischen Kanülensegment und Teststreifen wird wahrscheinlich günstiger durch die Schweißung oder Hitzeverklebung mit dem Kunststoff des Teststreifens erspart. In den Fig.44 - 47 und in der nachfolgenden ist die Größe der Punktionsspitze (und auch des Kanülensegments) wieder stark übertrieben eingezeichnet, da der in die Haut eindringende Durchmesser ja unter 0,5 mm und eher bei 0,3 mm liegen soll.

Die Figur 48 zeigt im Maßstab von etwa 4 : 1 ergänzende Einzelheiten zum Herstellungsprozeß des Teststreifens mit Punktions-spitze. Oben der Längsschnitt durch eine sechseckige Trommel deren Kanten mit Halbrinnen für Stahlstifte von innen Elektromagnete anliegen, deren Stromversorgung weggelassen wurde.
Der Stahlstift entstehe in der Phase A durch Austritt der heißen Gußmasse aus der Düse(304), die im Kühlbad abgeschreckt wird, soweit sie nicht warmgewalzt wird. Segmente der beiden Walten sind für die Phase C eingezeichnet deren Oberflächenformgebung in zwei Aufrollungen rechts unten näher erläutert wird.
Als Beispiel ist das Ziel des Walzergebnisses oben in einem Längsschnittdetail(links) und Vertikalschnittdetail(rechts) herausgezeichnet mit Querschnitten darunter längs der Schnittlinie A - B. Der Schaft der Punktionsspitze soll längsgestreckt sein, aber in eine einzige Spitze (ohne Schneide) auslaufen. Dies hat den Vorteil der minimalen Hautverletzung und - narbe.
Man erkennt, daß für eine elektrolytische Spitzenfertigung hierfür das Material der Schmalseite zu einem quadratischen Querschnitt im Bereich der eigentlichen Punktionsspitze aufgefüllt werden muß. Das obere Walzenpaar darunter - für die Herstellung einer ähnlichen Punktionsspitze mit Halteplatte - die Stückelung und eine Formvertiefung an einem Ende, wobei die Blechplatte im Ergebnis die gleiche Wandstärke (Seitenansichtsdetail oben) bei spatelartiger Erweiterung in der anderen Dimension erhält. Im Querschnitt unten die Variante einer Kantendrehung um 90 Grad. Bei dem Walzenpaar unten drücken überstehende Seitenlaschen der oberen Walte in entsprechende Vertiefungen der unteren Walzenfläche mit dem Effekt der Umbiegung des Gußstückes.
In der Fertigungsphase B wird die extrudierte Stahlstange mittels des Lasers(307), der in einer Richtung senkrecht zur Bildebene wirkt, in die Einzelstifte abgeteilt. Diese werden sogleich von der Hohlrinne aufgenommen (die Röhrenführung für die Stange wurde weggelassen) und dort vom Elektromagneten fixiert.
Die Elektromagnete(308) können die Stifte aus der Hohlrinne unter Inaktivierung der Trommelmagnete übernehmen und in senkrechter Richtung verlaufenden Hohlrinnen auf Bändern oder schon einem Kunststoffolienband hinzufügen. Die Transportfunktion gegenüber den Stahlstiften kann auch von Gliedern mit Permanentmagnet(306) im Zusammenwirken mit von dort aus der Hohlrinne(309) abhebenden (weil stärkeren) Elektromagneten übernommen werden.
Das zu Stadium C dargestellte Walzverfahren kann prinzipiell auch für das Aufwalzen (oder Aufstempeln) von Stiften auf das Folienband gelten. Das kleine Detail rechts zeigt einen Stiftquerschnitt zwischen zwei Folienblättern eingeschlossen und von Seiten eines Mikrowellenstrahlers(310) erhitzt.
Die Trommel mit den Halbrinnen für die Stifte wird gedreht und dient der rascheren Stiftverteilung. Vorder Drehung kann der Elektromagnet(308) außen jeweils längs einer Schiene zurückgezogen werden.
Als Saugkolben bei Einrichtungen nach Fig.32 ff. eignen sich in den Zylinder eingeschliffene, solche mit Dichtungsringen, bevorzugt aber solche mit vorspringender Randleiste am Ende, welche die elastische (auswechselbare) Zylinderwandung etwas ausbeult(wie bei Plastikspritzen üblich).

Die Figur 49 zeigt oben und in der Mitte im Längsschnitt und unten im Querschnitt in Höhe der Schnittlinie A - B des mittleren Längsschnittes das Beispiel einer Punktionseinrichtung ohne die Notwendigkeit eines manuellen Teststreifenwechsels.
Die Wiedergabe erfolgt in natürlicher Größe, wobei die obere Abbildung sich auf das Stadium vor und die unter Abbildung auf das Stadium während der Punktion bezieht.

Hinsichtlich der Unterdruckerzeugung für die Saugglocke (1) handelt es sich um eine konzentrische Raumanordnung.
Die kräftige Druckfeder für die Vakuumerzeugung wird hier vermieden; der Unterdruck entsteht hier oberhalb der Saugglocke unter Verwendung der zentral durchbrochenen Topfmanschette oder Rollmembran (458), welche sich zwischen der Oberkante des äußeren Stützzylinders (459) und dem pilzförmigen Kolben (460) ausspannt, mit denen an den nicht abrollenden Teilen der gewebebeschichtete Teil der Rollmembran fest verhaftet ist. Über dem Kolben (460) geschieht dies über eine auf letztere angeschraubte Schale, am Stützzylinder mittels Klebung. Die dargestellte Umschlagfalte stimmt nicht mit dem Stadium der Hautansaugung überein. Die Falte wölbt sich nämlich unter Sogeinfluß nach innen (mittlere Abbildung). Erst wenn der Punktionsgriffel (175), der hier den Teststreifen ersetzt, zur Wiederbelüftung mit der Haut angehoben wird, entsteht der nötige Überdruck für die Hautfaltenbildung. Zur sicheren Überdruckerzeugung empfiehlt sich nach jeder Punktion ein letztmaliger Griffelrückzug nach Verschluß der Saugglocke mit dem Deckel (462), der mit elastischem Rand in die Saugglocke eingreift und zweckmäßigerweise dort mittels des (nicht dargestellten weit über den Saugglockenrand liegenden) Bajonettverschlusses verschraubt wird. An seinem verkürzt und am Ende zu schmal dargestellten Stempel kann der Deckel bei Saugglockenhochstand entfernt werden. Druck gegen den kleinen Faltenbalg im Deckelboden sichert den initialen Unterdruckeffekt.
Die Verwendung einer Rollmembran zur Sogerzeugung wurde erprobt und ist nur deshalb praktikabel, weil der für die Hautansaugung erforderliche Unterdruck verhältnismäßig gering ist. Die Punktionseinrichtung für den automatischen Testfeldwechsel kann auch mittels anderer Sogquellen betrieben werden.
Als Teststreifen (103) dient ein Band, auf dem die Testfelder regelmäßig aufgereiht sind. Die Randperforationen (vgl.Fig.54 Mitte) des Teststreifens dienen den Zahnradpaaren (461) zum eingriff, welche mit der Anhebung des Glukometers (176) über die Laschen (463. vgl.Fig.51) im Gegenuhrzeigersinne gedreht werden. Eine Bandbefördering ist jedoch nur möglich, wenn durch Zug am Handgriff (464) der Deckelzylinder (465) gegen die über den Umfang verteilten Blattfedern (466,Fig.50) so weit abgehoben wird, daß der Teststreifen die Randdichtung zwischen Deckelzylinder und Stützzylinder passieren kann.
Der Handgriff ist zweckmäßig als geschlossener Bügel (nicht dargestellt) innerhalb einer Bohrung der Stange (469) schwenkbar. Die Zahnradpaare (461) liegen in je einer Kappe (495), die um eine Achse der Dachausbuchtung (468) des Deckelzylinders nach oben abschwenkbar und durch einen (vereinfacht symbolisierten) Schieberriegel (467) zum Deckelzylinder feststellbar ist. Innerhalb der Dachausbuchtung liegen zwei weitere Zahnradpaare (470) über welche der Teststreifen mit seinen Randperforationen auf seinem Weg über die Schiene (471) um das Punktionsgriffelende geleitet wird. Jedes der beiden Zahnradpaare ist mittels der Stütze (472) auf der Kappe des Kolbens (460) befestigt. (Um Totraum zu vermeiden und die Rollmembran abzustützen ist der Raum um die Stützen und Zahnradpaare mit Füllmaterial ausgekleidet, das nicht dargestellt wurde).
Die Abdichtung des Pumpenraumes über der Rollmembran erfolgt um den Punktionsgriffel mittels des Dichtungsringes (5) im Deckelzylinder und mittels des Dichtringes (373) zwischen Saugglocke und Kolben (460). Der Teststreifen liegt mit seinen unbenutzten Testfeldern in der rechten Trommel (473) um die Achse (474) mit (nicht dargestelltem) Klemmschlitz für das Einschieben des Bandanfanges. Das Ende des Teststreifens oder Bandes liegt im Klemmmschlitz der Achse der linken Trommel.

Auf dem Querschnitt unten ist die Torsionsfeder (477) eingezeichnet sowie die durch Teleskopauszug verlängerbare Kurbel (478), um die Torsionsfeder zwischen Trommel und Kurbel anzuspannen, so daß sie durch ihr Drehmoment im Gegenuhrzeigersinne den Transport des Teststreifens unterstützt.. (Der Kurbelgriff kann zur Bewegungssperrung - wie nicht dargestellt wird - gegen die Stützzylinderwandung eingeschoben werden).
Das Glukometer stellt - nur gehäusemäßig - eine Sonderkonstruktion dar um Höhe einzusparen. Der zentrale Schacht (479) läßt die Absenkung des Glukometers um die Dachausbuchtung (468) zu.
Weitere Schachtausnehmungen sind für den Rasterbolzen (475) sowie den Rasterwinkel der Laschen (463) und ihrer Verbindungsstangen (480) vorgesehen. Die Lage der beiden Stifte (476) mit ihren Verriegelungslaschen auf dem Gehäuseboden des Glukometers ist ebenfalls eingezeichnet. Ihre Funktion wird mit Fig.50 beschrieben. Die Trommelgehäuse sind fest mit dem Stützzylinder (459) verbunden. Die Einstellschraube (481) erlaubt die Regulierung der Eintauchtiefe des Punktionsgriffels in die Saugglocke. Die Verbindungsstangen (480) dienen der Abkoppelung des Teststreifentransports (vgl.Fig.51 links).

Die Figur 50 zeigt auf drei Längsschnitten durch die Punktionseinrichtung längs der Schnittlinie C - D des Querschnittes in Fig.49 unten drei Funktionsstadien A - C . Das Stadium A entspricht demjenigen im oberen Längsschnitt der Fig.49, das Stadium C demjenigen im unteren Längsschnitt in Fig.49 unter Hautansaugung.
Die Stangen im Punktionsgriffel samt Handgriff wurden weggelassen. Es ging vor allem um die Darstellung der beiden Stifte (476), welche die Absenkbewegung des Glukometers auf den Kolben (460) übertragen. Die Stifte sind durch die Schalen und die Rollmembran hindurch zu dieser gedichtet mit dem Kolben verschraubt und durch die Dichtungen (482) im Deckelzylinder verschieblich.
Mit der Bodenplatte des Glukometers sind die Stifte über die drehbare und in (nicht dargestellter Ringnut) über Verstiftung höhengesicherter Tülle (483) mittels einer Zunge verriegelt (vgl.Fig.54 oben).
Das Stadium B zeigt zunächst die Absenkung der Saugglocke auf die Haut durch die Absenkung des Punktionsgriffels vom Handgriff her. Nur auf der mittleren Abbildung ist eine Vorrichtung zur visuellen Kontrolle der Punktionsstelle skizziert, wie sie im Stadium A angewandt werden kann. Es bestehen (gestrichelt dargestellte Leitungsverbindungen zur Glukometerbatterie über einen - nicht dargestellten - Schaltknopf dort für die Stromeinschaltung).
Die Lichtquelle (17) wirft Licht über eine Linse auf die zur Punktion vorgesehene Hautstelle. Das von dort reflektierte Licht kann über die Linse im Schrägtubus in der Wandung des Stützzylinders beobachtet werden.
Punktionsgriffel (175) und Saugglocke (1) sind über die Gummimembran (484) gedichtet miteinander verbunden. Der Klemmring (485) besorgt die Membranbefestigung zum Punktionsgriffel hin. Die hier übertrieben dargestellte Absenkung des Griffels innerhalb der Saugglocke, in welche die Haut hochgesaugt ist, dient der Demonstration, daß der Andruck der Saugglocke federnd durch die Gummimembran bedingt und durch die Eindringtiefe des Griffels variiert werden kann. Der Patient kann über Druck auf den Stütz-ring das Einschlüpfen der Haut in der Innenzone um die Saugglocke nicht beeinträchtigen oder beeinflussen.
Im Stadium C ist nach Absenkung des Glukometers der Kolben (460) mittels der Stifte (476) abgesenkt worden. Dabei entstand über und mitgenommen, die sich ihrerseits mit dem Endwinkel der Oberkante des Glukometers (176) an dessen Schachtinnenseite anklammert.
Links ist schematisiert die Mitnahme des unteren Rahmens durch die Laschen eines Zahnradpaares dargestellt, wobei die Streckenlängen auf die funktionellen Bedürfnisse abzustimmen sind. Jede der beiden rechten Laschen ist mit einer solchen linksseitig vom Schacht durch Verbindungsstange (480,vgl.Fig.47 unten) verbunden. Auf diese Weise können die Verbindungsstangen mit einem Handgriff einander genähert werden - gestrichelt dargestellt -, wobei die für Mitnahme der Glukometerbewegung verantwortlichen Winkel den Gehäuserand des Glukometers verlassen. (Die Lasche und Rahmen zusammenhaltende Schiene ist gestrichelt angedeutet).

Die Figur 52 zeigt im Maßstab 2 : 1 einen Punktionsgriffel (175) mit spezieller Ausstattung für die Rotationsbewegung einer Schneidespitze in einem um einen kleinen Sektorwinkel drehbaren Testfeld innerhalb eines Teststreifenbandes im Längsschnitt.
Wie der Querschnitt etwa in Griffelmitte unter dem Längsschnitt verdeutlicht, hat der Punktionsgriffel eine ovaläre Gestalt und ist eine Nase der Stange (469) fest mit dem oberen Ende des Stößels (489) verbunden, dessen unterer Abschnitt nach Kalibersprung durch einen Dichtring geführt wird. Das untere dünnere Ende des Stößels ist es nun, das auf die sektorale Keilschräge (490) einer am Griffelende gegen die Torsionsfeder (491) drehenden Scheibe trifft. Der Stift (492) vom Griffel in eine Sektornut der Scheibe begrenzt den Drehraum.
Die dem Rasterbolzen (475) anliegende Keilnase der Stange (469) behindert deren Anhebung im Griffel durch Zug am Handgriff (464) wenig. Erheblichen Widerstand aber bildet die Steilflanke der Absenkung der Stange. Die Stärke dieses Widerstandes kann an der Stellschraube (493), die gegen eine Druckfeder wirkt, reguliert werden. An der Schraube (494) wird das Ausmaß der Stößelabsenkung und damit die Schnittlänge der Schneidespitze festgelegt.
Die Figur 53 gibt im Maßstab 2 : 1 Details über eine Ankoppelung eines Testfeldes an das Punktionsgriffelende oben in einem Vertikal- und unten in einem Längsschnitt (bezogen auf den Längsschnitt der Fig.52).
Die Scheibe (499, ohne Darstellung der Keilschräge für den Stößel) weist Dornen auf, deren Winkelgestaltung den Durchlaß des Teststreifens begünstigen. Bei ihrer Abwärtsfederung mit der Scheibe (499) durch eine Druckfeder im Griffelende besteht eine Rotationshemmung in der seitlichen Kulissenführung (495) und damit eine zentrische Ausrichtung der drehbaren Scheibe des Testfeldes (104, Fig.54 Draufsicht in der Mitte), zwischen deren Wellenprofil die Dornen eingreifen. Drehbar wird die Scheibe erst nach ihrer Anhebung und der Freigabe der Sektorbewegung durch den von der Scheibe in die Kulissenführung des Griffels aufsteigenden Stift.
Diese Anhebung wiederum wird durch das Eindringen eines abgeblasenen Gummikissens (497) während des Teststreifendurchzuges bewirkt. Dabei dient die kräftiger gefederte Schienenführung (498) als Widerlager. Das Gummikissen hat über dem Zentrum des anliegenden Tellers der Schneidespitze - bei Verklebung mit diesem - eine zentrale Lücke für den elektrischen Kontakt. Ein weiteres Loch spitzenwärts ist durch eine Weichplastikmembran (500) verschlossen. Diese wird durch die Heizdrahtschlinge (501, unten auf dem Längsschnitt) über einen Stromstoß vom durch elektrische Leitungen verbundenen Glukometer mit entsprechender Steuervorrichtung eröffnet. Die Luft entweicht dann aus dem Gummikissen und die Schneidespitze dringt in die hochgehobene zwischen die Schiene eindringende Haut. Bei entsprechender mechanischer Anordnung kann auch die Schneidespitze durch Andruck an die Weichplastikmembran dieselbe eröffnen.

Die Figur 54 zeigt im Maßstab 2 : 1 oben im Längsschnitt nochmals eine Darstellung der Verriegelung eines Stiftes (476,vgl.Fig.50) mit der Bodenwand des Glukometers (176). Der Handhebel kann am Haken vom Finger verschoben und die Tülle (483) mit der verriegelnden Nase so verschoben werden, daß letztere durch die Lücke in der Bodenwand geschoben werden kann. Zweckdienlicherweise wird man diese Lücke etwa im Winkel von 45 Grad zur dargestellten Hebellage für eine linksseitige Verriegelung anordnen.
Darunter eine Draufsicht auf ein Stück Teststreifen im Maßstab 2 : 1. Man erkennt die seitliche Randperforationen des Teststreifens (103), welche erlauben, zum Transport auch in der Photoindustrie längst geläufige Verfahren einzusetzen.
Zwei Testfelder (104) liegen auf einer in einer Umrandung drehbaren Scheibe. Die Kreise stellen metallene Kontaktpunkte da (falls meßtechnisch erforderlich). Die zentralen (schwarz ausgefüllten) Kontakte entsprechen der Lücke im (hier entfernten) Gummikissen oder eines sonstigen Füllmaterials, das auch durch Aufspulen eines getrennten Teststreifens von der Schneidespitze (285) - diese freilegend - entfernt werden könnte.. Man erkennt das Wellenprofil der drehbaren Testscheibe, wobei auch der Innenkreis innerhalb des Wellenprofils durch eine zerstörbare Weichplastikmembran geschützt sein könnte. Die Testscheibe kann auch durch einen überstehenden Rand oder Einzelzarken (wie auf der schrägen Darstellung links bei Fig.53) gegen Loslösung gesichert sein und industriell bereits im Sektorwinkel ausgerichtet.
Ein Längsschnitt darunter zeigt eine Schneidespitze, welche unter Spannung gegen einen der Oberwandung eines Testfeldes anliegenden Schenkel nach links abgebogen ist. Die Lage wird durch eine Querachse (rechts in der Vertikalen) gesichert. Wird die Weichplastikmembran(500) zerstört, so schnellt die Schneidespitze (285) in die gestrichelt dargestellte senkrechte Lage für die Punktion.
Erst jetzt erfolgt die Hautansaugung.
Ganz unten im Längsschnitt ein Stück Teststreifen im Maßstab 2 : 1 zur Darstellung einer alternativen Schnitterzeugung. Links werden zwei parallele Keilstößel bei (nicht gezeigtem) Druck auf ihre Druckfedern gegen den Teststreifen gedrückt. Die Distanz der spitzen ist so gewählt, daß eine von ihnen über eine Perforation in deren Anfang zu liegen kommt und beim Eindringen in dieselbe eine Teststreifenverschiebung in eine der Richtungen - bei entsprechend rückgefederter Toleranz - bewirkt.
Rechts davon eine Magnetspule (99) im Querschnitt, welche stoßartig und quer zum Teststreifentransport bei entsprechend rückgefederter Toleranz der Schienenführung (498) die Schneidebewegung ermöglicht.
Zwischen der mittleren Draufsicht der Fig.54 bis zwischen die Abbildungen der Fig.53 sind zwei Teststreifenenden dargestellt , die mittels der Haken (504, 505) miteinander vereinigt sind.
Zur Inbetriebnahme einer Einrichtung nach den Fig.1-6 werden die Verriegelungen der beiden Stifte (476,Fig.50,Fig.54 oben) gelöst und das funktionsbereite Glukometer wird abgehoben. Nach Herausdrehen des (nicht dargestellten) verschraubten Stiftes (466) kann dann der Deckelzylinder nach seitlichem Herausschieben auch der Blattfeder (466,Fig.50) vom Stützzylinder abgenommen werden.
Eine Trommel (433) mit dem einem Teststreifen wird gegen eine Blattfeder am Gehäuse eingeschoben und der Teststreifen aus dem Seitenschlitz der Trommel herausgezogen, nachdem seine Klammer am Ende entfernt wurde. Der Teststreifen wird über die Zahnradpaare (472) geführt, wobei zwischen beiden eine Schleife für den Punktionsgriffel hängen bleibt. Die linke Leertrommel wird aus ihrem Gehäuse herausgeschoben und das freie Teststreifenende in den Achsenschlitz eingesteckt. Die Leertrommel wird in ihr Gehäuse eingeschoben, der Deckelzylinder aufgesetzt, der Griffel eingeschoben und das Glukometer aufgesetzt und verriegelt. Im Normalfall aber wird am Ende eines verbrauchten Teststreifens nach dessen Lösung aus dem Schlitz der Trommelachse ein Haken aufgeschoben und mit ihm der Haken des unverbrauchten Bandes verbunden, ohne daß das Gerät geöffnet werden muß.
Die Saugglocke (1) wird am Griff abgesenkt, die Lage des Testfeldes und seine Nummer auf der Unterseite und diejenige außerhalb der rechten Dichtung zwischen Deckel- und Stützzylinder (jeweils zwischen den Testfeldern aufgedruckt) werden verglichen.
Der Deckel (462) wird auf die Saugglocke aufgeschraubt. Er hat ein düsenartig feines Belüftungsloch, um jetzt den Punktionsgriffel am Handgriff hochziehen zu können. Während des Zusammendrückens des Faltenbalges durch Druck gegen den Stempel wird dieses Loch durch eine Ventilklappe verschlossen, so daß der Überdruck im Inneren eingeleitet ist. Dabei wird die Rollmembran im Innern ausgerichtet und läßt die Kolbenrückführung erst zu. Der Deckel (462) wird abgenommen und der Stützzylinder auf die Haut aufgesetzt. (Er braucht dabei nur an zwei gegenüberliegenden Kanten Platz auf der Körperwölbung zu finden). In fraglichen Fällen erfolgt visuelle Hautkontrolle durch den Tubus. Erst wird jetzt vom Handgriff her Druck gegen die Haut ausgeführt, bis der Signalton oder das Zeichen des Glukometers die Blutsättigung des Testfeldes meldet.
Das Gerät kann jetzt abgekippt oder der Handgriff gezogen werden für die Wiederbelüftung der Saugglocke. Der Glukosewert ist nach der vorgeschriebenen Zeit auf dem Glukometer ablesbar. Der Handgriff wird abgesenkt und der Saugglockendeckel aufgeschraubt. Vor neuer Nutzung wird der Handgriff ganz herausgezogen.

Die Figur 55 zeigt linksseitig im schematischen Längsschnitt durch eine Saugglocke(1) im Maßstab 2 : 1 eine Vorrichtung ähnlich derjenigen der Fig.40 mit der gebogenen Schiene(237) für die Führung der Aufnahmebuchse(231). Ergänzend ist die (gestrichelte) Scheibe (312) um die Achse(313) drehbar, welche mit der Nase oder dem Vorsprung(314) in die angehobene Haut eingreift. Mittels des Elektromagneten(144) bewegt der Stößel(7) das obere Ende des Teststreifens(103) nach rechts, während ein unterer Hebelarm, der in einer Bohrung des Stößels verschieblich ist gegen eine Druckfeder auf eine Leiste auf der Scheibe wirkt und so den Vorsprung in Gegenrichtung zur Punktionsspitzenbewegung und verzögert und verlangsamt bewegt. Dabei wird der Hautspalt über der Punktionsspitze erweitert.
Rechts wird ein Vertikalschnitt gezeigt aber für die Variante, bei welcher der quadratische Stift - etwa über Laserschnitt - längs in zwei gleich Hälften zertrennt wurde und in zwei parallele Plastikstreifen eingebettet ist (der eine in die Scheibe).
Der Doppelstift wird so mit seinem (gestrichelt gezeichneten) Ende ins Elektrolytbad eingeführt und erhält eine gemeinsame Spitze. Erst nachdem diese Spitze für die Punktion seitlich bewegt wurde wird über elektrische Reibungswärme (die Leitungen sind strichpunktiert) über den Schleifkontakt(316) die Wachsplombe(315) über der Achse gelöst und damit eine Drehbewegung der Stifte gegeneinander unter Einfluß der Druckfeder freigegeben. Die Meldung der Blutsättigung des Testfeldes durch das Meßistrument für chemische Analyse im Testfeld des Testreifens an die Steuerzentrale bewirkt Abschaltung des Magneten(auch in analogen Fällen).

Die Figur 56 zeigt ein Beispiel wie es sowohl für eine Bewegung der Punktionsspitze nach dem Schwing- als auch nach dem Rotations-Prinzip einsetzbar ist, wobei ein seitlicher Lappen auf die hochgezogene (hier nicht eingezeichnete) Haut herabreicht.
Der tiefe Einschnitt zwischen dem seitlichen Lappen und dem Teil mit Testfeld (strichpunktierten) Signalleitungen und Punktions-spitze erlaubt ein Nachfedern gegenüber der Bewegung der Punktionsspitze, was vor allem bei einer Drehung des Teststreifens um seine Mittellinie von Bedeutung ist. Es handelt sich um eine Seitenansicht im Maßstab 2 : 1 unter starker Übertreibung der Breite der Punktionsspitze.

Die Figur 57 zeigt oben die Seitenansicht auf einen Teststreifen, welcher seitlich Schlitze aufweist, in welchen der Drahtring(317) Eingeführt ist, der mit seinen drei Stützen auf die Haut herabreicht. Die exzentrische Punktionsspitze kann bei Rotation gedreht werden, während die Stützen stehenbleiben und die Haut festhalten. Zweckmäßig für eine fast zweidimensionale Verpackung ist die Beschränkung auf zwei gegenüberliegende Stützen auf dem Drahtring.

Die Figur 58 zeigt etwa im Maßstab 1,5 : 1 schematisch in Längsschnitten eine Vorrichtung zum halbautomatischen Teststreifenwechsel als Vorstufe eines Vollautomaten.
Die Teststreifen(103) sind im Stapel durch die Brückenstifte oder -leisten(318, unten im Querschnittsdetail) in Abstand gehalten, wobei das Leistenende in einen nach außen dichten Gehäuseschlitz des ovalen Schieberkastens(319) klemmend eingeschoben ist. Dieser Schieberkasten wird läuft innerhalb des Dichtungsringes(320) eines Schachtes, der sich in den Saugglockenraum öffnet.
Über den Galgen(321) und eine Feder ist der Schieberkasten mit dem Schiebedeckel(322) über der Saugglocke verbunden. Das untere Test-streifenende ist gerundet und wird seitlich von einem Lappen überragt welcher auf

In der oberen Darstellung ist dieser Schiebedeckel geschlossen und das Magazin bis zum Anstoß der Aufnahmebuchse(231) genähert. Letztere kann über den Magneten, der sich aus der Batterie speist, über einen Waagebalken abgesenkt und sein verkürzter Randbezirk durch Reibungswärme beheizt werden. In ihrer letzten Absenkungsphase hat die Aufnahmebuche die Brückenleiste durchtrennt.
Nach Öffnen des Schiebedeckels kann nun die Saugglocke auf die Haut gestellt und die (nicht dargestellt Saugpumpe gegenüber dem Sogkanal(32) betätigt werden. (Eine Schwenkbewegung des Schiebedeckels über zwei Führungsbügel in einer Rinne hinter das Saugglockenniveau zurück wurde nicht behandelt; sie kann unterbleiben bei gehöriger Distanz des Deckels vom Saugglockenrand.) Der Elektromagnet wird über Kontaktschluß(323) zwischen Brückenleisten-metalleinlage und einer Schachtfeder so über die Steuerzentrale (184) betätigt, daß die Aufnahmebuchse gegen ihre Druckfeder angehoben wird, und ein unverbrauchter Teststreifen nachrücken kann. Nach Zeitintervall erfolgt die Absenkung bis zur Durchschneidung der Brückenleiste aus der (nicht dargestellten) mechanischen Raststellung in der dargestellten Mittellage.
In der unteren Abbildung ist der Schieberkasten(319) mit dem Schiebedeckel zurückgezogen und die Rohrtülle(324) von unten in die Saugglocke bis zum Anschlag eines Kragens eingeschoben.
In letzterer ist der Sammeltopf(325) verschieblich. Er weist an einer Querstrebe das Drehgelenk für die Zange(326) auf, welche durch Druckwirkung des paarigen gefederten Winkelarms(327) hinter dem Leistenvorsprung(328) des Teststreifens geschlossen ist (vgl.Vertikalschnittsdetail links unten).
Beim Herausziehen des Sammeltopfes aus der Saugglocke wird auch der verbrauchte Teststreifen gezogen und aus dem Zangengriff losgelassen, so daß er in den Sammeltopf fällt. Der Abzug der Schutzkappe über der neuen Punktionsspitze erfolgt an der Lasche (329).

Die Figur 59 zeigt links oben einen Längsschnitt darunter einen Querschnitt durch das Detail einer Saugglocke (1) in Höhe der Tauchmagnete im Maßstab 2 : 1. Rechts ist das Detail des rechten Tauchmagneten für die Ventilbetätigung im Maßstab 4 : 1 im Längsschnitt herausgezeichnet.
Rechts unten wiederum oben im Längs- und darunter im Querschnitt dasselbe Detail im Augenblick der Punktionsspitzenablenkung.
Der Tauchmagnet(144) ist stoßend wirksam und verlagert dabei die Drehachse (339) für die Aufnahmebuchse(231) des Teststreifens (103) ruckartig nach rechts, wobei die Punktionsspoitze in der Bewegung etwas nach links zurückbleibt (vgl.Längsschnittdetail rechts unten) und den Wundspalt spreizt, so daß Blut leichter in den feinen Spalt zwischen dem Teststreifen und damit ins Testfeld gerät (nicht dargestellt, da bei Teststreifen zu Glukometer "Elite" von BAYER marktgängig). Der nach unten konische Sperrstift (340) kann mittels eines Vierkantes an seinem Ende in seiner Gewindebuches herabgedreht werden und dient der Begrenzung der Magnetankerbewegung. Er ist im Maßstab 4 : 1 unten rechts sowohl im Quer- als auch im Längsschnitt abgebildet.
Das Detail links unten zeigt einen Magnetanker mit aufgeschraubter Mutter (341), an welcher der der Abknickwinkel in der Drehachse begrenzt werden kann. Die schwache Blattfeder(342) besorgt die Rückstellung der Aufnahmebuchse in die Senkrechte.
Das Sitzventil(208) für die Belüftung und damit Unterdrucksteuerung in der Saugglocke ist durch Aktivierung des Tauchmagneten(145) über die Leitungen(143) links oben geöffnet, während der Aktivierung des Tauchmagneten (144) aber geschlossen (links unten). Das Detail rechts oben zeigt den Sogkanal (32), der sich in den Luftraum öffnet.

Die Figur 60 bringt Längsschnitte durch eine Punktionsvorrichtung in natürlicher Größe, wobei der als Saugpumpe dienende Faltenbalg die Saugglocke (1) völlig und konzentrisch umschließt.
In der oberen Abbildung entspricht die linke Hälfte dem Stadium der Anspannung der starken Druckfeder (70) unter Verkleinerung des Faltenbalges (46), während die linke Bildhälfte den Zustand nach Entspannung der Druckfeder unter Ausdehnung des Faltenbalges zeigt. Von der Grundplatte (16)gehen insgesamt vier (vgl.Fig.61 auf dem Querschnitt) Stifte aus, welche jeweils von einer Röhre umfaßt werden, die von der Deckelplatte (15) ausgehen. Die Funktion des Auslöseringes(344) wird unten verdeutlicht.
Innerhalb der Saugglocke wird das Stadium der Hautansaugung dargestellt, wie es der Ausdehnung des Faltenbalges entspricht. Der kurze Teststreifen ist von unten in die Klammer der Aufnahmebuchse (231) eingeschoben und hat dort Kontakt mit den Signalleitungen (345) zum Meßgerät für Stoffwechselparameter(vgl.Querschnitt Fig.61)und mit einer Signalleitung, welche den Kontakt der Spitze oder Kanüle(vgl.Fig.63 rechts)an die Steuerzentrale (184,vgl. Querschnitt Fig.61) weitermeldet. Weitere Hautberührungsmelder (sog."Erdkonakte") gehen als spitzer und stumpfer Kegel aus der Darstellung in Fig.61 hervor und wurden hier weggelassen.
Die Drehachse (339) für die Aufnahmebuchse ist am Saugglockendach befestigt. Der Tauchmagnet (144) wirkt mit seinem Anker stoßend gegen die Aufnahmebuchse und damit gegen den Teststreifen und bewirkt die Querbewegung der Punktionsspitze. Eine Nachführung der Testspitze nach der Gefäßeröffnung zur Spalterweiterung ist bei gebremster Energiezufuhr aus der Batterie (83,vgl.Querschnitt Fig.61) über Rückzug der Anschlagplatte(346) mittels des Elektromagneten (347) möglich (vgl.Querschnitt Fig.63).
Die Sogregulierung und Wiederbelüftung der Saugglocke erfolgt über den Belüftungskanal(411) durch das Sitzventil(208) bei Aktivierung des Tauchmagneten (145), dessen Anker dann gegen eine Rückstellfeder am Stößel des Sitzventiles nach links gezogen wird. Die Rückstellfeder kann auch durch diejenige für den Magnetanker ersetzt sein. Der Sogkanal setzt sich in eine Rinne (348,Fig.59 rechts unten) auf der Grundplatte unten fort die radiär verläuft. Das Saugglockendach weist zentral eine Öffnung in den Faltenbalgraum auf, kann aber auch als Ganzes entfallen und durch einen Galgen für die Befestigung der Drehachse ersetzt werden.
Die Saugglocke wird vom Gehäusering (349) für Batterie, Meßgerät und elektronsiche Steuereinheit - etwa für die Ventilsteuerung - zirkulär umgeben. Der Blechring(350) ist über den Dichtungsring unten im Saugglockengehäuse eingeschoben; sein unterer Rand ist für ein besseres Gleiten der Haut nach außen und dann nach oben aufgebogen.
Von den beiden unteren Längsschnitten zeigt der obere das Stadium während der Faltenbalgentfaltung, der untere das der Kompression. Der Auslösering (344) ist enlang der Zunge (351) verschieblich, welche mit der Grundplatte (16) elastisch federnd verbunden ist. Eine knaufartige Endbiegung gleitet während der manuellen Absenkung der Deckelplatte (15) den Keilvorsprung (353) oben an der Lasche (352) an der Deckelplatte.
Die Schnittführung der rechtsseitigen Detaildarstellung liegt von derjenigen links radiär um 23 Winkelgrade versetzt (vgl.Querschnitt Fig.61). Eine kleine Nase an der oberen Innenkante des Auslöseringes gleitet bei der Deckelplattenabsenkung längs des Keilprofiles(354) der entsprechenden Lasche von der Deckelplatte und bewirkt dabei, daß die entsprechende Zunge nach außen abgebogen wird (vgl.Detail Mitte rechts in Fig.61). Dies erfolgt gleichzeitig an den vier gleichmäßig über die Scheibe der Grundplatte verteilten Zungen und bewirkt, daß die knaufartigen Endbiegungen die Keilvorsprünge auf den Laschen verlassen.
Die Sperrung gegen die Ausdehnungsbewegung der starken Druckfeder (70) wird damit aufgehoben, die Saugpumpe also betätigt. Nach Meldung der Hautanhebung in der Steuerzentrale erfolgen programmentsprechende Aktivierungen der Hubmagnete(145,144).
Eine optische Hautkontrolle kann der Sogauslösung vorausgehen. Hierfür können Lichtstrahlen von einer Lichtquelle (17,Pig.59)) unter dem Saugglockendach auf das Zentrum der noch nicht erhobenen Haut projiziert und von einer Meßsonde für die Signalverarbeitung in einem Meßgerät im Ringgehäuse (349) projiziert werden, aber auch durch die Linse oder Öffnung (355) im Saugglockendach durch die Linse (356) zur visuellen Direktkontrolle der Haut. Der Faltenbalg (46) ist an der Grundplatte sowohl als an der Deckelplatte gedichtet befestigt.

Die Figur 61 zeigt oben in natürlicher Größe einen Querschnitt durch eine Punktionseinrichtung nach Fig.60.
Im Zentrum liegt die Saugglocke (1) mit dem Teststreifen in der Aufnahmebuchse zentral, flankiert von den Hubmagneten. Die Lichtquelle (17) und die Linse (356) für die optische Hautkontrolle liegen im Spaltraum zwischen den Hubmagneten. Hinter dem Sitzventil zur Saugglockenbelüftung folgen der Sogkanal und die radiäre Rinnen (348) für die Lufteinleitung. Im Ringgehäuse liegen Batterie(83), Meßgerät(e) mit Display oder Fenster (357) auf der Grundplatte. (Die Ablesung von Meß- oder Eingabewerten könnte auch von einem Display auf der Deckelplatte bei entsprechendem Verbindungskabel zum Meßgerät erfolgen.) Man erkennt die Signalleitungen (345) von den Klemmfedern leitenden (358,Fig.63) des Aufnahmeschachtes. Signalleitungen (strichpunktiert) verbinden auch Meßgeräte(e) und elektronische Steuerzentrale (184), welche nach Öffenen einer Klappe (gestrichelt mit Schraube)in der Grundplatte zur Programmierung zugänglich ist, wobei auch mit Berührungsschaltern etwa von der Deckelplatte aus programmiert werden kann. Die Batterie (83) ist über die zwei als Kreise eingezeichneten Steckbuchsen wiederaufladbar und versorgt die Verbraucher über die Leitungen (143).
Zwischen dem Ringgehäuse und dem Faltenbalg (46) liegt die starke Druckfeder (70). Den äußeren Abschluß bildet der Auslösering(344), dessen Schlitze die Zungen (351) der Grundplatte umfassen und mit der knaufartigen Endbiegungen dem jeweiligen Keilvorsprung (353) der Laschen (352) der Deckelplatte bei Faltenbalgkompression aufliegen. (Das Montageproblem einer anzuschweißenden Rückblende am Auslösering hinter den jeweiligen Zungen wurde nicht dargestellt.) Die Zugfeder (359) hält zwischen den jeweiligen vier Segmenten des Auslöseringes dieselben zusammen.
Auch die von der Deckelplatte ausgehende Röhre (343), welche jeweils einen Stift auf der Grundplatte teilweise umfaßt, ist in Vierzahl vorhanden.

Dem Kompressionszustand des Faltenbalges entsprechend ist unten ein Längsschnitt durch die Einrichtung zu sehen. Ganz rechts unten das Detail einer Vertikalsicht in die Rinne(348), die sich über den Sogkanal und das Sitzventil in die Saugglocke fortsetzt. Wiederum wird wie in Fig.60 links die Schnittebene durch den Keilvorsprung der Lasche der Deckelplatte und die Endbiegung der Zunge von der Grundplatte gelegt, während der Schnitt rechtsseitig sektoral verschoben erfolgt und das Keilprofil (354) zeigt, von dem die Nase des Auslöserringes (344) abgedrängt wird, der die Zungen mit nach außen nimmt.

Das Sitzventil (208) wird über dem Längsschnitt als Detail in geschlossenem Zustand gezeigt, wodurch es durch die kleine Druckfeder im Knick des Sogkanals(32) gehalten wird. Die Öffnung erfolgt durch den Zug des Magnetankers nach links. Die Grundplatte weist der Haut zu eine (nicht dargestellte) leichte Wölbung auf, was die Körperanlage und den Luftabzug bei Federspannung gegen eine Unterlage erleichtert.

Die Figur 62 zeigt in natürlicher Größe eines der vier Segmente des Auslöseringes (344). Der Schlitz, welcher die Zunge (351) umfaßt ist nach links etwas verbreitert und läßt dadurch eine leichte Drehung des Auslöseringes im Uhrzeigersinne zu.
Im dargestellten Funktionsstadium wurde durch Linksdrehung des Auslöseringes, nach Verrasterung der Kompressionsstellung wie oben beschrieben, die Aussparung (360) für den Durchtritt eines Vorsprunges der Lasche beiseitegedreht. Der Auslösering ist also an der lasche verriegelt und kann nicht (etwa unabsichtlich) abgesenkt werden. Dies ist erst nach Zurückdrehen des Auslöseringes im Uhrzeigersinne möglich.
Die gestrichelte Tangente soll die gerade Ansatzlinie der Zunge (351) am Knick zur Grundplatte veranschaulichen.
In einer zweiten Darstellung der Lasche wird die Verbindung der Zunge mit der Grundplatte als Variante zweigeteilt und wiederum geradlinnig bewerktstelligt. Über dem gestrichelten Querschnitt der Basis der Zunge ist deren doppelter Fuß, der sich oben vereinigt (nicht dargestellt)um einen rechten Winkel in die Bildebene geklappt. Die Röhre (343) ist zweimal zur Positionsangabe zum oberen Auslöseringsegment eingezeichnet.
Die Zugfeder(349) kann auch , wie darunter links in einem Detail gezeigt, durch elastische Kettenglieder beispielsweise ersetzt sein. Es werden zwei in einander verklammerte mäanderförmige Kettenglieder -etwa aus Kunststoff- dargestellt.)

Rechts davon, also in der Mitte wird in natürlicher Größe die seitliche Aufrollung einer Zunge (351) gezeigt, aus welcher eine elastische Federzunge herausgestanzt und geformt ist, die rückstellend gegen die Absenkung des Auslöseringes wirkt. Die Lage und Funktion wird Vertikalschnittdetail rechts erläutert.

Die Figur 63 zeigt eine Variante der Ausstattung einer Saugglocke für eine Punktionseinrichtung links im Querschnitt und rechts im Längsschnitt im Maßstab 2 : 1. Der Hub- oder Tauchmagnet(144) trifft mit der Anschlagplatte (361) gegen eine schräge Metallzunge, welche verhältnismäßige kräftigen Widerstand bietet. Diese Metallzunge ist auf dem Schlitten (362) befestigt, der über die Brückenstange (363, oben Längsschnittdetail durch den Tauchmagneten 210) vom Elektro- Tauchmagneten (210) zurückgezogen werden kann. Die am Ende geschlitzte Einstellschraube (364) regelt die Steilheit der Stahl- oder Metallzunge durch Anstoß, je nachdem wie weit sie vor oder zurückgedreht wird. Der Elektromagnet kann als doppeltwirkender in der Gegenrichtung das Sitzventil (365) in den Faltenbalgraum öffnen und so den Luftddruck in der Saugglocke, welcher über die Betätigung des Tauchmagneten (145) durch das Sitzventil (208)über den Sogkanal (32) abgesenkt worden war. Es kommt - etwa über eine Hebelüber-setzung mit Bewegungsumkehr im Bereich der Brückenstange - auch der Einsatz eines in einer Richtung wirkenden Magneten in Frage.
Wird nämlich der Schlitten(362) in seiner Buchse zum Nachschnitt, daß heißt zur Wiederholung einer erfolglosen Querbewegung der Punktionsspitze - zurückgezogen, so ist auch eine Unterdrucksteigerung in der Sauglocke zweckmäßig.
Gesteuert werden die Sitzventile auch unter dem Einfluß der Rückmeldungen von den Berührungsschaltern und dem Punktionsmittel. Auf dem Längsschnitt wird gezeigt, wie links eine Kegelspitze durch ein Loch im Deckel des Blechringes (350) hindurch die angehobene Haut erreicht. Auf der rechten Seite tritt ein Kegelstumpf durch eine Bohrung im Deckel mit der höchste Kuppenerhebung der Haut in Kontakt. Wenn letztere Berührung nicht mehr rückgemeldet wird, muß mit der Öffnung des Sitzventiles (208) sparsam verfahren werden, wie der Steuerzentrale einprogrammiert wird. Die Detailvergrößerung unter der Punktionsspitze auf den Maßstab 4 : 1 (wieder mit wesentlicher Überzeichnung) läßt erkennen, daß als Beispiel hier ein Kanülensegment dem Teststreifen beigeheftet wurde (unter Hitzeanwendung etwa).Das obere stumpfe Kanülenende leitet Blut direkt auf das Testfeld (104).
Der Blechring (350) ist in diesem Falle mit einem Teststreifen ausgestattet der längs eines Deckelschlitzes die Drehachse (339) aufweist. Die Schnittkante der Kanüle wird längs dieses Schlitzes in Schnittrichtung ausgerichtet. Zum Abgriff der Signalleitungen dient die Klemmfeder (358), auf deren Breitseite entsprechende Isolationszonen zwischen den beiden äußeren Kontaktleitern vom Testfeld und dem mittleren in Leitungsverbindung mit der Kanüle vorhanden sind (in Analogie zum üblichen Vorgehen bei der Anwendung von Teststreifen etwa in Glukometern).
Die Aufhängung der Klemmfeder im Saugglockendach erfolgt seitennachgiebig, die Ablenkung nach rechts über den Aufprall der Anschlagplatte (361) auf den Teststreifen. Solche Blechringe mit integrierter Punktionsspitze erlauben die Anwendung der Punktionseinrichtung bei verschiedenen Patienten ohne Ansteckungsgefahr, da sie nach jeder Benutzung ausgewechselt werden. Die in Mehrzahl vorhandene und auf dem Umfang verteilt angeordnete Druckfeder (371) hält den Blechring nach unten, wobei eine obere Randkröpfung ein herausfallen nach unten verhindert.
Die Magnete und die Schlittenbuchse sind unter einander ringförmig verstrebt. Es finden sich oberhalb der Magnete wenigstens zwei Haltezungen (366), welche zwischen keilförmig ansteigenden Plattensegmentschlitze des Saugglockendaches drehend eingeführt werden. Der elektrischen Kontaktherstellung dienen dabei Schleifkontakte, deren drei auf dem Querschnitt mit dem Leitungsende eingezeichnet wurden. Links unten ist ein Fragment einer Kralle dargestellt, welche bei der Einführung und Befestigung der Magnetausrüstung in die Saugglocke dienlich ist.
Rechts unten ist wird skizzenhaft die Möglichkeit demonstriert, zur Herabsetzung der Gerätehöhe Magnete (hier einen) innerhalb des Sograumes außerhalb der Saugglocke(1) zu plazieren. Der mit dem Magnetanker verschraubte Stößel kann bei doppelwirkendem Elektromagneten gegen eine Druckfeder aus der Saugglockenwandung zurückgezogen oder aber manuell herausgeschraubt werden.

Die Figur 64 zeigt in natürlicher Größe einen Querschnitt durch eine Punktionseinrichtung ähnlich wie in Fig.60 bis 63, bei welcher aber die taschengängigere Rechteckform gewählt wurde. Die Saugglocke mit der Magnet- und Punktionsausstattung liegt zentral innerhalb des kastenförmigen Faltenbalges (46), der die Druckfeder (70) umgibt. (Die zweckmäßige Eckenabrundung wurde zeichnerisch nicht vollzogen.)
Der an jeder Längsseite angeordnete und längs der Deckelplatte verschiebliche Schieberiegel (367) wird durch eine Druckfeder zwischen jedem Paar in gesperrter Distanzstellung gehalten, wie auch unten aus einem Längsschnitt Längs des unteren Schieberiegelpaares hervorgeht.
Während der Faltenbalg flächig mit der Deckelplatte verbunden ist, übergreift der Schieberiegel jeweils galgenartig die Oberkante, die hier unterbrochen ist und an der nach unten reichenden Lasche (352) - hier schachtelartiger Rahmen - zwei quere Schlitze (368) an jeder Längsseite aufweist, durch welche hindurch der paarige Querbolzen den Schieberiegel an der Lasche festhält, wie rechts neben dem Längsschnitt auf einem Vertikalschnittsdetail längs der Schnittlinie A - B des Querschnittes deutlich wird.
Von der Grundplatte (16), deren Rand die Lasche über-ragt, reicht die Säule (370) nach oben und endet in einer Querachse, welcher in einem Schrägschlitz des Schieberiegels verläuft.
Während der Deckelplattenabsenkung zur Spannung der Feder (70)und zur Faltenbalgverkleinerung werden die Schieberiegel infolge der Kurvengestaltung im Schrägschlitz nach innen aus bis sie im queren Endschenkel unter Einfluß der Druckfedern in die Ausgangsstellung zurückkehren und Grundplatte und Deckelplatte gegenseitig verriegeln. (Liegt die Grundplatte dabei auf einer Unterlage, so kann die Luft bei der Faltenbalgkompression entweichen, da die Grundplatte in der Querrichtung leicht: gewölbt ist.)
Die Saite (372) verbindet jedes Paar Schieberiegel einer Längsseite miteinander. Die beiden Saiten werden mittig über das Band(371) verbunden, das unterbrochen gezeichnet wurde.
Wird die Saugglocke (1) auf die Haut aufgesetzt, nach dem von unten ein Teststreifen mit Punktionsspitze oder Kanülensegment in seiner leicht abziehbaren Umhüllung in die Aufnahmebuchse eingeschoben wurde, so greift der Benutzer von unten unter das Band und nähert über die Zugwirkung die beiden Schieberiegelpaare einander, wodurch die Querachse der Säule die Saugpumpenauslösung freigibt. Batterie, Steuerzentrale und Meßvorrichtung und Display sind auf zwei über Leitungen miteinander verbundenen (vgl.Querschnitt Fig.61) Behältern beidseits der Saugglocke untergebracht, wie auf dem Querschnitt oben zu sehen ist; es kann auch ein Datenspeicher und ein Drucker vorhanden sein oder über eine Buchse angeschlossen werden. Auch das Ventil und die Magnete funktionieren entsprechend, Überlappender Kantenüberstand (374) sichert vor seitlichem Ausscheren der Funktionsteile während der Faltenbalgkompression. Auch Teleskopglieder können eingesetzt werden, besonders wenn die Höhe der Einrichtung herabgesetzt werden soll.
Die Steuerleitung (375) zur Steuereinheit wird durch die Passage der Querachse auf der Säule (370) an Schleifkontakten der Seitenwandung oder Lasche der Deckelplatte kurzgeschlossen, wenn die Querachse wenigstens auf ihrer Oberfläche metallisch leitend ist. Das Signal kann zur Auslösung des Tauchmagneten (144,Fig.63) benutzt werden. Die Querbewegung der Punktionsspitze kann aber auch durch andere Schalter, etwa einen längenveränderlichen Zugschalter in Abhängigkeit von der Faltenbalgentfaltung und damit an den Unterdruckaufbau in der Saugglocke ausgelöst werden.

Die Figur 65 zeigt in einem Querschnitt im Maßstab 1 : 2 eine Einrichtung wie in Fig.64, jedoch ist die Saugglocke von zwei quadratischen Faltenbälgen mit jeweiliger Druckfeder flankiert. (Rechts ist als Alternative durch Kreise innerhalb des Faltenbalges die Verwendung mehrerer runder Druckfedern in Reihe angedeutet). Der Sogkanal (32) muß paarig in der Grundplatte angelegt sein. Meßgerät und Batterie liegen zwischen den Faltenbälgen. Der Verriegelungsmechanismus wurde hier weggelassen.

Die Figur 66 zeigt in einem Längsschnitt skizzenhaft im Maßstab 1 : 2 die Kombination einer Punktionseinrichtung mit innerhalb des Faltenbalges liegender Saugglocke, einem Elektromagnet und Batterie und Versorgungseinrichtung im Ringgehäuse mit einem handelsüblichen Meßgerät (etwa einem Glukometer).
Die Meßwerte werden über ein Leitungsband einer Art Steckdose auf dem Faltenbalgdach zugeführt und von dort (eventuell auch direkt ohne Steckdose) über ein Verbindungskabel dem Meßgerät zugeführt, das hier beispielsweise in einer Schiene dem Dach des Faltenbalges aufgeschoben ist. Fast selbstverständlich können auch Batterie und die Steuerzentrale außerhalb des Faltenbalges untergebracht werden, etwa in Nachbarschaft des Meßgerätes oder sogar als dessen Bestandteile.
Das Detail rechts unten skizziert in einem Querschnitt einen Elektromagneten, der als Rotor im Zentrum einer Sauggglocke montiert werden kann und dessen zentrale Achse die Aufnahmebuchse mit dem Teststreifen (gestrichelt gezeichnet) trägt. Der Teststreifen kann der Haut gegenüber mit leicht exzentrischer Punktionsspitze oder Kanülensegment ausgestattet sein.

Zur Ventilsteuerung für die Unterdruckregelung ist nachzutragen, daß auch mehr als die drei dargestellten Meßpunkte zur Berührungsbestätigung durch die Hautkuppe herangezogen werden können. Zweckmäßigerweise werden verschieden schnelle Öffnungsrhythmen für das Ventil zum Sogkanal nach außen in der Steuerzentrale programmiert, wobei zuerst eine längerer Öffnungsabstand und Rhythmus zweckmäßig vorgegeben wird, dem dann schnellere Rhythmen mit geringerem Lufteinlaß folgen. Es kann dabei der Zeitabstand herangezogen werden, der zwischen dem Abbruch des Hautkontaktes an einem oder an zwei Meßpunkten festgestellt wird. Bei rascher Veränderung wird sogar - soweit vorhanden - die Öffnung des Ventiles in den Faltenbalg aktiviert werden, um die Punktionsspitze solange sicher unter der Haut zu halten, bis gegebenenfalls das Meßgerät die Blutsättigung des Testfeldes auf dem Teststreifen meldet. Die Verriegelung der Grund- und Deckelplatte durch Schieberiegel (367.Fig.64) gegen Fehlauslösung kann nach innen rückfedernd (Spiegelung der Schlitzführung für die Säulenachsen) erfolgen, wobei die Saiten(372) über Umlenkrollen geführt werden.

Die Figur 67 zeigt oben in einer Aufsicht oben in einem Querschnitt in Höhe der Schnittlinie A - B im Maßstab 3 : 1 - bei starker Übertreibung der Punktionsspitze - die Variante eines Teststreifens nach Fig.11.
Der Teststreifen(103) weist zwei seitliche Lappen auf , welche - in der hier gezeigten Variante mit zwei - in die Hautoberfläche verdrängend eindringende Vorsprünge oder Zähne aufweisen und vom eigentlichen Teststreifen mit dem Testfeld(104) durch einen Spalt getrennt ist. In diesen Spalt dringt in einer Richtung quer zum Teststreifen je ein Arm des Keilschiebers(330) ein und drängt je den anliegenden Lappen und damit auch die Haut seitlich von der Punktionsspitze ab. (Dieses Abdrängungsstadium ist rechtsseitig abgebildet.) Die Abbiegung der seitlichen Lappen kann durch Nachgiebigkeit des Materials oben in der Verbindungsbrücke(333) zwischen Teststreifen mit Testfeld und seitlichem Lappen stattfinden oder aber - insbesondere bei Verbreiterung des Lappens in die Tiefe - wie im Querschnitt dargestellt - durch die Elastizität des Lappens (gestrichelte Linien rechts am rechten Lappen) unterhalb der Verbindungsbrücke. Die Punktionsspitze wird durch den Stössel(7) ebenfalls quer bewegt, so daß die Lappenspreizung den Hautspalt quer zur Schnittrichtung für die Blutgefäßeröffnung für den Blutaustritt in das Testfeld erweitert. Die Signalleitungen wurden weggelassen.

Die Figur 68 zeigt oben in einem Längs- und unten in einem Querschnitt das Detail einer Saugglocke mit einer besonderen Vorrichtung zur Wundspaltspreizung (bei starker Übertreibung der Punktionsspitze) im Maßstab von etwa 1,5 : 1 links im Stadium vor und rechts im Stadium nach der Wundspaltspreizung.
Die Haut (strichpunktiert) wurde über den Sogkanal(32) mittels nicht dargestellter Saugpumpe in die Saugglocke zu einer Kuppe hochgezogen und dabei vom Ende des Teststreifens(103) und der diesen paarig umgebenden Klaue(334) dellenartig von oben her eingestülpt. Die Klaue ist setzt sich in eine Hülse fort, welche je einem Stift(335) aufgesteckt wird. (Die das Herausfallen der Klaue hindernde Konizität bzw. Rastfeder wurde nicht dargestellt.) Der Stift(335) setzt sich in eine obere Querlasche fort, welche in einer Rinne unter der Dachkappe(336) quer oder frontal zur Bildebene gegen die schwache Rückstellfeder(337) verschieblich ist und dabei die Klaue mitnimmt. Die Abspreizung der beiden Klauenhälften erfolgt über den Vorschub des Keilschiebers(332) mittels des Elektrotauchmagneten(144) mit Rückholwirkung, dessen Bewegungsausmaß an der zur Saugglocke gedichteten Stellschraube (338) beschränkt und damit reguliert werden kann. (Zur Regulierung können auch besondere Erregungsarten des Magneten herangezogen werden,vgl.Fig.30.) Das Auseinanderdrängen des Klauenpaares hat eine Ausspannung der Haut zur Folge, welche vom vorspringenden Randbogen der Klaue hinterschnitten und so festgehalten wird. Der vorspringende Rand und die anschließende Rundung übt auch Druck auf die Blutgefäße der Haut in diesem Bereich aus. Der Elektrotauchmagnet(144) wird in vorzugsweise erst aktiviert, wenn der Elektrotauchmagnet(145) über das Gestänge((230) die Aufnahmebuchse (231) mit dem Teststreifen - und damit auch die Punktionsspitzeruckartig in gleicher (oder Gegenrichtung) mit dem Keilschieber (332) bewegt hat und zwar in der Phase der Blutanschoppung in der Haut. Das Blut wird dann durch den Druck der Klaue in die Richtung des Wundspaltes gepreßt und dort über einen kapillaren Spalt vorn im Teststreifen zum Testfeld hin hochgesaugt (Längsschnitt rechts oben). Der Wundspalt wird quer zur Schnittrichtung auseinandergezogen. Der Unterdruck kann in Art der Schilderung zu Fig.31,42 herabgesetzt und die stärkere Hautstrapazierung mit Gefahr des flächigen Blutaustrittes unter die Haut stärker - nämlich auf die Fläche zwischen der Klaue - herabgesetzt werden. Eine Kombination mit anderen Wundspreizverfahren (etwa nach Fig.31,40) kann vorteilhaft sein.
Figur 69 gibt in einem Längsschnitt in natürlicher Größe eine Punktionseinrichtung für den Mehrfachgebrauch auch bei verschiedenen Personen wieder. Der Topf (381), vorzugsweise aus Plastik, beispielsweise Polystyrol, enthält in seiner verdünnten Dachmitte eine rechteckige Trägerlamelle, die hier kein Testfeld trägt, sondern eine Kanüle, deren Lichtung sich in einen gewundenen Kapillarschlauch fortsetzt. Diese Teile wurden bereits einer sterilen Verpackung entnommen und in die Saugglocke eingeschoben, wo der Topf durch Puffer an rückfedernden Stangen festgehalten werden. Zur Unterdruckerzeugung wird der Topf mit dem Rand auf die Haut gesetzt und die Kappe (376) über dem Handgriff gedreht, dessen Mittelstab die Topfdichtung(377) und damit in Kunststofffolien verpacktes pulverförmiges Natriumbikarbonat und Schwefelsäure (etwa 10%ig) vor sich her in den Spieß(378) hineindrückt. Es kommt zu heftiger Co2-Gasentwicklung sobald nach Eröffnung eines Bikarbonatbeutel auch ein Säurebeutel eröffnet ist.
Das Druckgas strömt über einen Kanal in die Gasstrahlpumpe (379), welche die Luft aus der Saugglocke und über eine Lücke im Topf über der Haut absaugt. Letztere steigt dann in die Kanülenspitze auf.
Bei Betätigung des Schaltknopfes setzt die Batterie (83) den Tauchmagneten(144) in Aktivität, der die Kanüle gegen den Anschlag treibt. Das aus der Verletzung aufsteigende Blut sammelt sich im Kapillarschlauch.

Die Figur 70 zeigt im Längsschnitt schematisch eine Saugglocke(1) zum Einmalgebrauch mit umgebendem Vakuumspeicher (383), der industriell evakuiert ist. Die Spannung des gegen den Träger der Kapillare gerichteten Federbogens wird durch die Elektrodrähte (384) gehalten. Das Gehäuse mit der Batterie (83) ist in eine Mittelsenke des Vakuumspeichers drehbar und bringt Kontakte zunächst mit dem der Heizdrahtschlinge über einer Membranlücke (385) im Saugglockendach zur Abschmelzung, nachdem die Schutzmembran entfernt und die Saugglocke auf die Haut gestellt wurde. Die Drehung des Batteriegehäuses bewirkt einen Kontaktschluß mit den Elektrodrähten (383), die zerstört werden, so daß der Federbogen die Punktionsspitze gegen den Anschlag (382) schnellt. Die Nicht nur die Unterdruckeinwirkung auf die Haut soll durch die Erfindung begrenzt werden; auch schon die Punktionswunde und - narbe soll möglichst gering gehalten werden. In EP 0 798 004 A1, veröffentlicht am 1.10.97, hatte ich bei Fig.20 bereits die herstellungs- und anwendungsmäßigen Vorteile erkannt, die darin liegen bei einer querbewegten Punktionsspitze auf eine weitere Schneide, die ja zudem nach der Bewegung ausgerichtet werden müßte, zu vermeiden. Mit Fig.33 wurde auch schon die Hinzufügung einer Metallspitze zum Teststreifen vorgeschlagen, in Fig.8 eine Kanüle speziell umgestaltet. Die Möglichkeiten einer Wundspaltverbreiterung durch eine Nachbewegung der Spitze wurde in Fig.35 erörtert, in Fig.40 wird das Blut über eine Art Kanüle (die aus verschiedenen Materialien bestehen konnte) in das Testfeld geleitet. In Figur 19 von DE 196 13 722.5 wird bereits eine Punktionsspitze dem Tststreifen hinzugefügt; allerdings noch mit einem auch die Testfelder vereinigenden Rahmen. Eine zeichnerisch nicht besonders abgesetzte "Punktionsspitze im plastikummantelten Endteil" eines Teststreifens geht aus Fig.7 DE 197 23 730.4 hervor, die Punktionssspitze in Fig.6 aus DE 197 22 852.6 ist auch zeichnerisch deutlich vom Teststreifen abgegrenzt. Eine Punktions-spitze an einem Haltestahlblech findet sich an anderer Stelle bis zur Anklebung einer Stahlspitze mit DE 197 45 600.6.
Zur Wundspalterweiterung wird der Punktionsstift zusätzlich quer zu seiner Stoßbewegung gekippt, durch Knick im Teststreifen, dessen Führung in einer Kurvenschiene im Saugglockendach oder einfach durch Stöße gegen ein Drehgelenk der Aufnahmebuchse für den Teststreifen und damit weiter weg von der verunreinigenden Haut. Der Bewegungswinkel wird (eventuell veränderlich) festgelegt. Hautabspreizung kann auch von nicht oder gegenbewegten Fortsätzen des Teststreifens bewirkt werden, die neben der Punktions-spitze auf die Haut reichen, durch wenigstens zwei auseinander getriebene zunächst in eine einzige Spitze konvergierende Einzelnadeln und besonders wirksam quer zur Schnittrichtung, etwa durch die Hautdelle untergreifende Backen,
welche den Hautbereich um die Punktionsspitze ausspannen.
Von besonderer Bedeutung ist die Narbenkleinheit eben für Patienten mit häufiger und regelmäßiger Anwendungsnotwendigkeit.
Da das Gerät auch für die Blutgewinnung etwa für die Blutkörperchenzählung und Gerinnungsfaktorenbestimmung anwendbar ist, wird auch die Variante einer Wegwerfsaugglocke mit um ein Gelenk schwenkbarer Kanüle oder Punktionsspitze vorgeschlagen, wie als kleinen Trichter schon Gerhard Wagner für den Injektionsgebrauch beschrieb, nachdem ich meine diesbezügliche Anmeldung in München zurückgezogen hatte.
Besonders günstig hinsichtlich des Herstellungsaufwandes ist die Unterbringung der Saugglocke innerhalb eines Faltenbalges zur Sogerzeugung oder klinisch für den Mehrfachgebrauch die Kombination Wegwerfsaugglocke innerhalb einer Saugglocke mit Vakuumquelle, etwa einer Gasstrahlpumpe, welche mit einer Säure-Bikarbonatmischung oder Druckgaspatrone betrieben wird.
Möglich ist dies durch den Einsatz wenigstens eines Elektromagneten zur seitlichen Punktionsspitzenablenkung, wobei die elektrischen Zuleitungen keine Dichtungsprobleme bereiten.
Wie in Fig.69 kann über Stromkreisschluß in der Signalleitung (386) durch Blut ein Signal für Wiederbelüftung gewonnen werden; hier zum Folienabriß an einer Wandlücke.

Die Figur 71 zeigt im einem Längsschnitt durch den unteren Teil eines Teststreifens etwa im Maßstab von 1 : 15 den bereits angespitzten Stahlstift(1), der in eine Art Röhre(2) aus Kunststoff gesteckt oder mit in die Kunststofform bei der Einbettung der Reaktionsfelder in eine solche Röhre geriet. Der Querschnitt zeigt die Variante einer Hohlrinne mit vorspringenden Kanten, hinter welchen der Stift festgehalten wird. Zwischen den Testfeldern(3) und dem Stift als Nulleiter besteht die Leitungsverbindung(4). Die gestrichelten Quadrate entsprechen Meßwertaufnehmern(5) von Seiten des Meßgerätes, deren Kontakte direkt auf Testfelder und Nulleiter aufgesetzt werden. (Beide können durch metallisierte Kunststoff- oder Metallfolien nach außen abgeschlossen werden. (Das Metall kann im Vakuum durch ein elektrisches Entladungsverfahren den Teststreifen aufgedampft werden.

Die Figur 72 entspricht in ihrer Dsrstellungsweise der Fig.2 und wird ebenfalls im Längsschnitt gezeigt. Die Kunststoffröhre (2) setzt sich in diesem Falle fort. Es wurde von oben ein zweiter kleinerer Stift als Kontaktstift eingeführt. Zur Stromleitung kann sich zwischen Stiftende(1) und oberem Kontaktstift eine Elektrolytflüssigkeit befinden. Anstelle des Stiftes mit der Flüssigkeit können aber auch Drähte eingeführt und mit dem Stahlstift unten in Kontakt gebracht werden. Die gestrichelten Linien in den Testfeldern entsprechen dort eingebrachten Leitungen für die Weiterleitung zum Meßabgriff für das Meßgerät.

Die Figur 73 zeigt einen handelsüblichen Teststreifen in der Ansicht von der Rückseite im Maßstab von etwa 4 : 1, wobei die Stärke(Breite) des Stahlstiftes(1) noch weit übertrieben wurde. Dieser ist von der Rückseite dem Teststreifen so aufgeklebt, daß der Spitzenbereich den Teststreifen überragt. Zur Befestiguhg dient noch verstärkend die Folie(7), wie auf dem Querschnitt unten erkennbar ist, die in der Variante im Längsschnitt nicht nur auf den Endbreit des Testfeldes (gestrichelter Kreis) oder die Schaftlänge des Stiftes beschränkt ist. Es handelt sich hier um eine Metallfolie (Kupfer oder Aluminium) für den Stromabgriff von oben. Einer der Leitungswege ist mit der Kontaktschulter(6) verbunden, der andere endet auf einem metallisierten Fleck(8) auf der Streifenoberseite.

Die Figur 74 zeigt eine Grundanordnung zur Spitzenherstellung an einem Stahlstift(1). Die Wanne(9) ist bis in vorbestimmter Höhe mit 20%iger Schwefelsäure gefüllt. Sie hat den Deckel(10) mit einem Schlitz für die Einführung des Teststreifens (entsprechend Fig.3). Eine vom Deckel herabreichende Federzunge(12) bringt den Stahlstift in Kontakt mit der Leitung zum positiven Pol der Batterie(11), während eine zweite Leitung vom Minuspol der Batterie isoliert unter dem Deckel in das Schwefelsäurebad hängt. Der elektrolytische Zersetzungsprozeß führt zur Anspitzung des Stahlspitzes, dessen Ergebnis dargestellt ist.

Die Figur 75 zeigt oben in einem Maßstab von etwa 1 : 4 , unten in etwa natürlicher Größe - in Wirklichkeit natürlich (abgesehen von den noch kleineren Stahlstiften) noch großzügiger dimensioniert. schematisch in einer Draufsicht und teilweisem Längschnitt (durch die Wanne) ein Fertigungsverfahren unter Anspitzung der Stahlspitze im Säurebad, das unten etwa in natürlicher als Ausschnitt gezeigt wird. Links das Detail eines Teststreifens mit noch nicht geschlossenen Verpackungsfolien im Vertikalschnitt.
Die Band wird gegen den Uhrzeigersinn gedreht ist an der Oberfläche mit Polyäthylen gegen Säure geschätzt und enthält bereits die Stahlstifte, die aus einem Magazinschieber(12) unter Hitzeeinwirkung aufgeschweißt wurden. Die Umrisse - wie sie teilweise aufgezeigt werden, entstehen erst in einer späteren Fertigungsphase durch Ausstanzen aus dem Band. Zuvor werden noch Testfelder und Leitungsfolien aufgestempelt.
Das Vertikalschnittdetail eines Teststreifens links zeigt die Variante einer Stromzufuhr über die Klammer(12) und eine Metallfolie (vgl.Fig.3) auf den Stahlstift. (Hierzu wäre die Oberfläche der Metallfolie oder sonstige Zuleitung im unteren Abschnitt des späteren Teststreifens - etwa durch Polyäthylenüberzug - säurefest gemacht worden, der Stahlstift bis zum Spitzenbereich.
Die Test- oder Reaktionsfelder werden auf einem (nicht dargestellten weiteren Radius des Rades(14)jeweils in Stiftnähe hinzugefügt. Die Verpackungsfolien können ebenfalls schon im Kreisprozeß an das Band herangebracht werden, allerdings nach der Ausstanzung vorzugsweise innwandig - dem Teststreifen zu -klebend im Moment der Abrollung des Restmatrizzenbandes zuerst das eine und dann das andere.
Die Stromzufuhr aus der Batterie(11) zu Stahlstiften und Säurebad entspricht prinzipiell derjenigen in Fig.4. Es wird jedoch zu jedem Stahlstift der Leitungskontakt zum Pluspol der Batterie hergestellt, was lediglich zur Demonstration in vier Fällen durch die gestrichelten Linien zwischen Leitfolie von jeder Spitze zum Zentrum des Rades(14), das sich bis zu dem Band nach peripher erstreckt, eingezeichnet ist.
Links ist, wiederum schematisch, eine Vorrichtung mit dem Schacht (15) für die Zufuhr der Stahlstifte zum Folienband dargestellt; sie ist von der Vertikalen um 90 Grad nach unten herabgekippt wiedergeggeben. Die Stiftreihe wird durch den Schieber(16) bandwärts befördert, der durch das Kupplungsrad(17) vom Elektromotor(18) angetrieben wird. Die Antriebssignale entstammen einer nicht dargestellten Steuerzentrale, welche über zwei Kontakte mit Stiftberührung über Stromschluß den Vorgang überwacht. Die Kunststoffschicht des Folienbandes, welche über die Vorrichtung(21) aufgebracht wurde, wird von der Heizvorrichung(20) - etwa eine Lichtquelle - zur Stiftanklebung aufgeweicht.
In der Vorrichtung(22), die der Vorrichtung mit dem Schacht(15) analog vorgestanzte Schutzfolien führt oder vorzugsweise analog der Vorrichtung(21) einen Kunststoffilm aufträgt, werden Folie und Stahlstift bis zu dessen als Spitze vorgesehenen Teil mit einem Schutzfilm gegen Säure und zur elektrischen Isolation versehen.
Nachdem der Stift im Säurebad - die Rotationsgeschwindigkeit des Rades(14) wurde nebst andere Vorrichtungen entsprechend geregelt - angespitzt wurde erreicht schließlich die Stanze(23) wo der Test-streifen gegen eine (nicht dargestellte) Schnittunterlage ausgestanzt wird. Die Rolle(24) mit dem Band der ersten Verpackungsfolie - zur Darstellung aus der Vertikalen nach oben in die Horizontale gekippt gezeichnet -liegt unter halb des Folienbandes unmittelbar neben der Stanze, wobei der Stempel(25) - zur Deutlichkeit als Oval eingezeichnet) von oben gegen die Rolle drückt, so daß der ausgestanzte Teststreifen auf dem Verpackungsfolienband haftet. Letzteres wird einer -nicht dargestellten - zweiten Rolle mit zweitem Verpackungsband zugeführt und dann einer weiteren Stanze, die einzelnen Teststreifen mit größerem Umriß ausgestanzt.
Die im Vertikalschnitt links dargestellte Aufrollung der vorderen und hinteren Verpackungsfolie von oben und unten ist eine Variante möglicher Schutzmaßnahmen bereits vor dem Säurebad aufgebrachter Stoffe, die weniger zu empfehlen ist.

Die Figur 76 zeigt in einem Längsschnitt durch das Ende eines Teststreifens im Maßstab von etwa 4 : 1 einen angespitzten Stahlstift(1), der quer zum Teststreifenverlauf dort durch ein Loch gesteckt ist (das umgebende Testfeld wurde weggelassen). Der Stift hat eine kopfartige Verbreiterung zur Befestigung unter Anklebung. Die Folie(7) sichert beispielsweise die Stiftbefestigung und kann für Leitungszwecke dienen. Sie wird wiederum zweckmäßig durch einen Filmauftrag ersetzt.
Die Detailskizze darunter zeigt, daß der Stahlstift -hier mit Kopf, aber auch ohne einen solchen - in den Teststreifen selbst mit seinem auch später stumpfen Ende eingebettet sein kann.

In allen Fällen kann Silber oder Gold als Leitbahn auf die Folie aufgebracht in der Art einer gedruckten Schaltung die genannten Metalle Kupfer oder Aluminium ersetzen.
Überhaupt soll der Erfindungsgedanke nicht auf die dargestellten Beispiele eingeengt sein, sondern sich auch auf Kombinationen insbesondere mit bekannten Prinzipien der Stift-, Teststreifen- und Verpackungsproduktion erstrecken.

Die Figur 77 zeigt im Vertikalschnitt oben im Maßstab 2 : 1 einen Arzneiinjektor mit Saugglocke(1) und Gasstrahlpumpe(24).
Batterie(29) und Steuerzentrale (17) befinden sich linksseitig. Auf die Ventilsteuerung soll im übrigen hier nicht eingegangen werden, zumal sie durch die Erfindung vereinfacht wird.
Im oberen Längsschnitt ist die Lage des Schraubgewindes für die Treibsatzpatrone (die auch ohne Arznei und Düse angeschlossen werden kann) angegeben, im unteren die Ausstattung mit den Elektromagneten.
Die Patrone(12) , im Längsschnitt, hat etwa den Maßstab 2 : 1 .
Die Patrone wird mittels einer Bajonettkupplung am düsennahen Ende in die Saugglocke (um 90 Grad gedreht) eingeschraubt. Ihr Innenraum ist durch die Trennwand(820) in zwei getrennte Kompartiments geteilt. Beide Teilräume sind mit je einem Hohlkolben(14) mit Bikarbonatgranulat und mit je einer Säureblase (etwa mit 20%iger Schwefelsäure) ausgestattet und mit Heizdrahtschlingen. Zunächst wird der Treibsatz hinter der Schraubkappe(756) gestartet. Das Druckgas wird über eine Bohrung im Patronenmantel in die Dichtmanschette(821) geleitet und von durch (schematischer Zusammenhang gestrichelt) über die Druckleitung(413) der Gasstrahlpumpe zugeleitet.
Erst wenn die Verhältnisse (etwa hinsichtlich der Hautkontrolle) optimiert sind, wird über Stromzufuhr der Treibsatz hinter der Arznei gestartet und die Arznei durch die (größenmäßig stark übertriebene) Düse in die Haut gespritzt. Der Treibsatz unter der Schraubkappe(756) kann erneuert werden.
Die Ausstattung mit den Elektromagneten (144,145) mit deren Leitungen(143) für die Stromzufuhr aus der Batterie - im unteren Längsschnitt dargestellt - für den diagnostischen Gebrauch, welcher in der Regel der Injektion vorhergeht, entspricht der Darstellung in Fig.59. Da der hintere Treibsatz als Ganzes nach Öffnung der Schraubkappe nachrüstbar ist, kann die Haut zur Injektion in zeitlichem Abstand zur Meßwertgewinnung vorgenommen werden.

Die Figur 78 zeigt im Längsschnitt im Maßstab 1 : 1 eine Punktionseinrichtung mit einer Punktionsspitze an einem Teststreifen ohne Anwendung einer Saugglocke. Links wird die Phase vor und rechts die Phase während der Punktion dargestellt. In Hülse(389) ist der Kolben(390) eingeschoben.
Letztere umschließt das Rohrsegment(391), das den kolbigen Schiebehalter(392) für Aufnahmebuches(231) für den Teststreifen (103) umschließt. Letztere ist um eine Achse(schwarz) schwenkbar.
Das Auf der Hülse verschiebliche Rohrsegment(393) trägt an zwei gegenüberliegenden Stützen je eine um ein Gelenk schwenkbare Klaue(394), welche mittels Blattfeder gegen die gerundete Kuppe des Hülsenendes gedrückt wird. (Die Stützen sind radiär um 90 Winkelgrade gedreht dargestellt).
Über der Hülse ist oben die Kappe(395) verschieblich, welche mit dem in ihr befestigten Einschub für Batterie(83) und Glukometer (12) oder Meßgerät verkürzt dargestellt ist. Unten an ihr ist die kräftige Druckfeder (396) sowohl mit dem mit ihr bajonettverschraubten Einschub als auch mit dem Kolben (390) fest verbunden. Auf dem oberen Stift des Schiebehalters(392) mit der an seinem Ende befestigten Druckfeder ist die Tülle(397) über einen Führungsstift eine Strecke weit verschieblich und stößt mit dem unteren Ende auf einen Querstift des Kolbens. Dessen Verschiebung wird -wie linksseitig dargestellt - den Sperrstift(398) gehemmt, der in ein trichterförmiges Bohrloch des Kolbens eingreift und von der Blattfeder (399) in der Hohlrinne des Ringriegels(400) kolbenwärts bewegt wird. Ein weiterer Sperrstift (401) ist rechtsseitig zu sehen. Er geht von dem Schiebesegment(402) aus und greift in eine Längsnut des Kolbens ein. Die elastische Saite(403) spannt sich zwischen Schiebesegment und Schraubring(404), welcher höhenverstellbar ist.
Der Schwinggalgen(406) verläuft in einem Schlitz des Schieberhalters und wird mit einer Rollenführung auf dem Keilprofil(408) bei der Absenkung des Schieberhalters nach rechts gegen die Blattfeder(407) gedrängt. (Der Höhepunkt dieser Verdrängung unter Anspannung der Blattfeder ist auf der rechten Abbildung erkennbar.) Das Keilprofil, das von der Hülse(389) ausgeht, setzt sich nach oben mit der Leiste(409) in der Hülse fort (vgl.Fig.79, rechts). Die Rundkuppe der Hülse unten weist einen zentralen Schlitz für den Punktionsstift auf und gibt des Schwingung um die Achse der Aufnahmebuchse(231) frei. Die Klemmfedern übernehmen von dort von der Teststreifenoberfläche die Signalimpulse und leiten sie über die Signalleitungen zum Meßinstrument weiter.
Es ist nur eine Signalleitung (216) links eingezeichnet, die über Schleifkontakt - beispielsweise bei als Längsstreifen eingegossener Signalleitung auf den Rundteiloberflächen, wie heute etwa auch bei Schutzblechen an Fahrrädern üblich - über Aufnahmebuchse, Rohrsegment, Kolben und Hülse die Signale zum Einschub in der Kappe und von dort zum Meßinstrument weiter vermitteln. Das Display kann sich beispielsweise auf der Kappenoberfläche befinden. Die - hier mit dem unteren Ende der Blattfeder(zweckmäßiger aber radiär verschoben) - nach unten reichende Zunge des Kolbens greift mit elastisch federnder Kralle in einen Schlitz in der Hülse ein. Die schwache Zugfeder(41=) führt das innere Rohrsegment(391) nach oben zurück.
Zur Vorbereitung werden durch Zug an der Kappe die Innenteile aus der Hülse gezogen und der Teststreifen zwischen die Klammern (Detail in der Mitte Maßstab 2 : 1 ) eingeschoben. Der Kolben mit seinen Innenteilen wird dann wieder entsprechend seiner Nut für die Leiste(409) eingesetzt. Der Ringriegel wird im Gegenuhrzeigersinne bis zum Anschlag gedreht (vgl.Fig.79), Wird die Kuppe der Hülse gegen die Haut gepreßt, so senkt sich die Kappe unter Spannung der Druckfeder (396). Die paarige Kralle(394) wird nach außen gespreizt und spannt wegen zackiger, aufgerauhter Oberfläche die Haut zwischen sich aus, auf welche die Kuppe sich absenkt.
Erst wenn der Keilfortsatz(links) des äußeren Rohrsegments(393)den Teller des Sperrstiftes(399) verdrängt, wird letzterer zurückgezogen und gibt die Bewegung des Kolbens nach unten frei. Diese erfolgt unter Einfluß der kräftigen Druckfeder plötzlich. Dabei wirkt sich die Spannung der inneren Druckfeder im Rohrsegment(397) ohne Widerstand aus und schnellt die Punktionsspitze in die Haut. Die Rollenführung des Schwinggalgens(406) gerät dann auf die Schräge des Keilprofils(408) und erleidet an dessen Nase einen gewissen Widerstand. Wird dieser durch den Druck von oben überwunden, so verläßt die Rollenführung den Gipfel des Keilprofils und der untere Haken des Schwinggalgens schlägt nach links gegen den Punktionsstift. Letzterer bewirkt eine Gefäßplexusverletzung. Nach Signal vom Meßinstrument, das die Blutsättigung des (nicht dargestellten) Testfeldes meldet, wird der manuelle Druck auf die Kappe gelockert. Das Klauenpaar schiebt sich über die Kuppe der Hülse, die Haut abdrängend, auch wird die schwach Zugfeder(410) wirksam und zieht den Punktionsstift zurück. Die Stiftbreite ist wieder stark überzeichnet; auch die Einrichtung kann kleiner gebaut werden.

Die Figur 79 zeigt Querschnitte längs der Schnittlinien A - B und A - C des Längsschnittes der Fig.78. Die Stützen der paarigen Klaue(394) sind lagegemäß dargestellt, mit gestrichelter Kontur der Unterrand eingezeichnet, welcher der Haut aufliegt. Dazwischen liegt ein verdünnter und elastischer Teil, der nachgeben kann, falls die Elastizität der Haut erschöpft ist. Es ist zu erkennen, daß die Spreizung der Haut quer zur Schwingung des Punktionsstiftes erfolgt, wie auch aus der Lage des Schwinggalgens (406) hervorgeht, im Zentrum der Teststreifen und darüber die Achse der Aufnahmebuchse(231).
Der Querschnitt rechts längs der Schnittlinie C - D des Längsschnittes zeigt, daß die Sperrstifte(399,401) als Triplets angelegt und radiär gleich verteilt sind. Der Kopf des Sperrstiftes(399) liegt jeweils der Blattfeder(398) an.
Es ist auch die von der Hülse(389) in Rinnen des Kolbens (390)vorspringende Leiste(409) sichtbar. Durch Drehung des Ringriegels (400) im Uhrzeigersinn verläßt die Blattfeder(398) den Sperrstift(399), so daß der Kolben herausgezogen werden kann.
(Die Trichterbohrung im Kolben muß einen entsprechenden Winkel aufweisen). Auch für das Triplet des Schiebesegments(402) ist entsprechend Raum in der Aushöhlung des Ringriegels.
Die Aufrollung des Ringriegels von der Seite unter dem Querschnitt zeigt die Lage unter dem Schraubring(405). Gestrichelt längs letzterem eine Schraubwindung, mit welcher er auf der Hülse drehbar ist. Es ist auch die elastische Saite(403) zwischen dem Schiebesegment(402) und dem Schraubring eingezeichnet, an welcher Der Ringriegel, der auf der Hülse höhenverschieblich ist, aufgehängt ist. Wird der Kolben mit dem Schwinggalgen nach Überwinden des Keilprofiles durch dessen Rollenführung heftig abgesenkt, so kommt die Bewegung durch Straffung an der elastischen Saite(403) zunächst zum Stillstand, um allmählich auszulaufen.
Dieses Auslaufen entspricht einer Nachbewegung der Punktionsspitze. Die gestrichelte Darstellung einer anderen Stellung der Saite zeigt, daß die Abbremsung durch die Winkelstellung der Saite und durch Randreibung (hier am Ringriegel) beeinflußt werden kann.
Die Letztere und der Schraubring könnten auch als Permanentmagnete ausgebildet sein, die unter dem Schlag nach unten auseinandergerissen werden. Es gibt auch biologische und technische Materialien, welche einer Zugwirkung zunächst höheren Widerstand entgegensetzen. Die Saite könnte aus solchem Material gefertigt oder durch eine Feder ersetzt sein.
Eine Punktionseinrichtung kann natürlich auch hier mittels eines Elektromagnetes die Querbewegung der Punktionsspitze bewirken; auch kann jegliche derartige Einrichtung mit einer Heizvorrichtung ausgestattet sein, um die Hautdurchblutung nach der Gefäßverletzung zu fördern und damit den Blutaustritt.

Die Figur 80 variiert nochmals die Verpackungslösung für einen Teststreifen mit Punktionsspitze, wie sie nicht nur bei einer Einrichtung nach Fig.78 und 79 verwendet werden kann.
Es handelt sich linksseitig um einen Längsschnitt und rechtsseitig um einen Vertikalschnitt, wobei das linke Figurenpaar den Zustand vor und das rechte denjenigen nach Benutzung zeigt.
Der Maßstab ist etwa 2 : 1 bei starker Übertreibung der Stärke des Punktionsstiftes. Der Teststreifen(103) hat ovale Form und liegt in einem ovalären oberen Ausschnitt der Folienscheide(102), wobei für den Schutz des Stiftes noch eine reichlich bemessene Bohrung oder ein Rechteckausschnitt vorgesehen ist. Oberkante der Folienscheide - hier aus einem einzigen Blatt bestehend - und Teststreifenunterkante werden zweckmäßigerweise durch leichte Klebung oder Sollbruchlinie (wenn aus einem Stück aus Kunststoff oder Pappe gefertigt) zusammengehalten. Gestrichelt ist die Kontur der doppelblättrigen verklebten Verpackungshülle dargestellt, deren beide Blätter an den oberen winklig abstehenden Enden erfaßt und auseinandergezogen werden.
Die rechte Darstellung zeigt, daß zur Entnahme des Teststreifens aus dem Schacht der Aufnahmebuchse die Folienscheide umgedreht wurde. Mit ihrem Gabelausschnitt wurde sie so gegen die Punktions-spitze geschoben, so daß diese in eine Spaltverengerung (oder in ein sich verjüngendes Sackloch eindrang und festklemmte. Durch Zug an der Folienscheide kann der Teststreifen aus dem Schacht herausgezogen und dann entsorgt werden.

Die Figur 81 wiederholt im Längsschnitt die Fig.77 und ergänzt deren Beschreibung. Der untere Längsschnitt stellt eine Variante dar, die durch Magnete für eine zusätzliche Rotation und eine Absenkung des Teststreifens außer dessen Abschwenkung nach dem Schwingprinzip ausgestattet ist.
Im oberen Längsschnitt kann der Kuppenstift(414), welcher den Belüftungskanal(411) - hier einen Schlauch - zudrückt, am Handknopf rechts gegen eine Druckfeder zur Wiederbelüftung der Saugglocke zurückgezogen werden.
Bei der Variante im Längsschnitt unten links erfolgt die Wiederbelüftung durch Öffnung des Sitzventiles(208) mittels des Elektrotauchmagneten(145). Der Belüftungskanal endet unten in einer Bohrung neben der Saugglocke. Man erkennt die Einmündung der Druckleitung(413) hinter der Düse der Gasstrahlpumpe und den Sogkanal (32), der in der Saugglocke endet(gestrichelt).
Zu dem Elektrotauchmagneten(145), dessen Ankerfortsatz die Aufnahmebuchse(231) mit dem Teststreifen nach rechts geschleudert hat, ist oben auf dem Saugglockendach der Rotationsmagnet(146) oder Schrittmotor gelagert, dessen Rotoranker fest mit der Aufnahmebuchse verbunden ist und eine Sektordrehung des Punktionsstiftes vermitteln kann. Der Elektromagnet(412) in Verlängerung des Rotorankers, der die Einrichtung vervollständigen kann, hat die Aufnahmebuchse zuvor abgesenkt und den Einstich verstärkt.
Der Punktionsstift zeigt in diesem Falle eine bajonettförmige Knickung. Die Rotation soll möglichst langsam und gedämpft erfolgen, etwa auch durch einen einfachen Magneten mit Federdämpfung, welcher einen Querhebel zum Magnetanker bewegt.
Bereits das Einstechen der Bajonettkrümmung des Stiftes in die haut bewirkt deren Aufspreizung.
Rechts ist im Maßstab 4 : 1 bei Übertreibung des Punktionsstiftes der Teststreifen(103) mit dem Testfeld(104) und den strichpunktierten Signalleitungen abgebildet. Strichpunktiert ist auch die Haut, in welche der Stift eingestochen ist. Bei den dargestellten Längenverhältnissen zur Bajonettkrümmung erfolgt die Hautplexuseröffnung durch Schwingung oder Rotation der Punktionsspitze, wenn diese noch nicht so tief in die Haut (gestrichelte Linie) eingedrungen ist. Ein nachträgliche Absenkung durch den Elektromagneten(412) oder eine entsprechende mechanische Vorrichtung bewirkt dann die Wundspalteröffnung mittels der Stiftkrümmung.
Die Punktionsspitze kann aber - wie rechts im Detail gezeigt - sich gleich an die Bajonettkrümmung nach unten anschließen und die Rotation oder Schwingung oder beides kann nach Eintauchen des Knickes oder der Bajonettkrümmung unter die Haut erfolgen.
Das Blut dringt über einen kapillaren Spalt zwischen Folienblättern auf das Testfeld (wie bei "Elite" BAYER).

Ganz unten ist im Maßstab 1 : 2 das Schema einer Lösung der Sogregulation im Längsschnitt nach Fig.63 in einer Variante wiedergegeben, welche anstelle eines Ventiles zu einem Unterdruckspeicherraum eine Sauggpumpe einsetzt. Deren Funktion wird abgestoppt und bei erneutem Unterdruckbedarf innerhalb der Saugglocke erneut über die Steuerzentrale(186 aktiviert.
Hierzu wurde auf die Pumpendarstellung der Fig.24 zurückgegriffen. Gespeist aus der Batterie(83) veranlaßt die Steuerzentrale bei Signalen aus dem Sauggglockenbereich, welche auf zu starkes Absinken der Hautkuppe hinweisen (vgl.Fig.42) oder bei nicht rechtzeitiger Meldung einer Blutsättigung von Seiten etwa eines Meßgerätes eine gezielte Aktivierung des Rotationsmagneten (146) oder Schrittmotors, welcher die Gewindebuchse(416) bewirkt.
Hierdurch wird die Schraube innerhalb der Gewindebuchse, deren Ende dem Kegelkonus der Pumpenkolbenachse aufliegt nach links zurückbewegt. Der Dichtkolben(169) wird innerhalb des Zylinders (168) durch die starke Druckfeder(70), welche die Deckelplatte(15) anhebt, mit angehoben. Die Saugglocke(1) ist mit der Grundplatte(16) verbunden; ein Galgen verbindet sie mit dem Rotationsmagneten. Die Ausstattung der Saugglocke entspricht ansonsten derjenigen, die zum Längsschnitt darüber besprochen wurde.
Der Kegelstumpf ist auf einem Gewinde des Kolbenstempels mittels einer Gewindespindel drehbar, welche von dem Schrittmotor(417) über die Steuerzentrale gedreht werden kann.
Bei derartiger Antriebsgestaltung kann letztlich auch der Kegelstumpf durch ein einfaches Gewinde ersetzt werden und der Rotationsmagnet (146) entfallen. Es kann mittels des Schrittmotors (417) der Pumpenkolben auch nachträglich und dosiert abgesenkt und der Unterdruck in der Saugglocke so herabgesetzt werden (in Gegendrehung - bei ausreichender Motorkraft oder über Zwischengetriebe - aber auch wieder erhöht).
Das zuletzt gegebene Beispiel soll auch auf die Möglichkeit einer elektrisch gesteuerten Unterdruckregulierung überhaupt hinweisen.

In Anmerkung zu Anspruch 17 wird festgestellt, daß der die beiden Punktionsnadeln auseinandertreibende Keil erst nach gemeinsamer ruckartiger Bewegung quer zur Haut betätigt werden kann, um die Punktionsspitzen auseinander zu treiben. Zweckmäßiger wird das Auseinandertreiben der Spitzen ruckartig, den Hautgefäßplexus verletzend geschehen. Eine dreidimensionale Anordnung von drei Punktionsstiften könnte eine fast beliebig große Öffnung für den Abfluß bedeutenderer Blutmengen garantieren.
Zu Anspruch 17 kann erweiternd festgestellt werden, daß anstelle einer Ventilbetätigung zwischen einem Unterdruckspeicherraum und der Saugglocke auch die Reservekraft einer Saugpumpe zum selben Zweck eingesetzt werden kann. Die Sauggpumpe wird eine Strecke weiter oder erneut betätigt, wenn der Luftdruck in der Saugglocke zu stark abzufallen droht (etwa infolge starker Körperbehaarung).
Zu Anspruch 18 ist daran zu erinnern, daß das Auseinanderziehen der beiden Krallen, es könnten auch mehrere auf dem Umkreis um den Punktionsstift verteilte sein, auch eine - etwa unter Elektromagnetkraft - gesteuerte sein könnte. Auch könnten die Krallen innerhalb einer kappenartigen Umrandung in entsprechendem Abstand von den Rändern angeordnet sein. Auch ohne Unterdruckanwendung würde sich die Haut beim Andruck der Umrandung im Raum innerhalb derselben zu einer Kuppe aufstauen und die Krallen können in deren Zentrum wiederum die Delle bilden, welche zur Hautspannung auseinandergezogen wird. Es könnten auch äußere Krallen, die Faltenhebung der Haut zwischen ihnen durch Bewegung der Hautränder nach innen, dem Punktionsstift entgegen, begünstigen.
Die hier beschriebenen und dargestellten Konstruktions- und Funktionsmerkmale sollen auch in Abwandlungen und in gegenseitigem Austausch und Kombination geschützt sein.

Nachtrag zu den Fig.59-66:
Der Sogkanal(32) ist funktionsnotwendig; in den Fig.60-66 kann stattdessen das Saugglockendach weggelassen werden. In Fig.11 begrenzt die gestrichelte Linie zwischen Hautoberfläche und Punktionsspitze den entstandenen Hautspalt.

## Patentansprüche

1. Punktionsmittel mit Spitze in Verbindung mit einem Träger zur Blut und Gewebsflüssigkeitsgewinnung aus der Haut eines Lebewesens durch plötzliche Bewegung nach Eindringen unter die Hautoberfläche in Richtung etwa längs der Hautoberfläche zur Eröffnung von Blutgefäßen,
**dadurch gekennzeichnet,**
daß das Ende des stiftartigen Punktionsmittels als Spitze ausgebildet ist, wobei eine etwaige zusätzliche Schneide sich höchstens auf die unmittelbare Spitzennähe erstreckt, so daß die Stichwunde eine etwaige Schnittwunde beim Eindringen in die Haut überwiegt, vorzugsweise in Verbindung mit Trägermitteln. welche eine Wundspalteröffnung für den Austritt von Blut- und Gewebsflüssigkeit begünstigen und eine sterile Verpackung vor Benutzung und die Einführung in und aus einer Vorrichtung für die Anwendung erleichtern.

2. Punktionsmittel nach Anspruch 1 ,
dadurch gekennzeichnet,
daß der Schaft des Punktionsstiftes eine bajonettförmige Knickung aufweist.

3. Punktionsmittel nach Anspruch 1 ,
dadurch gekennzeichnet,
daß der Schaft des Punktionsmittels einen Querschnitt mit unterschiedlichen Abmessungen auf zwei rechtwinkelige Bezugsgrößen aufweist.

4. Punktionsmittel nach Anspruch 1 ,
dadurch gekennzeichnet,
daß es Teil eines Griffels als Träger ist, welcher wenigstens ein Mittel zur Aufnahme von Blut und Gewebsflüssigkeit aufweist.

5. Punktionsmittel nach Anspruch 1,
dadurch gekennzeichnet,
daß es an einer Art Teststreifen als Träger befestigt ist, welcher wenigstens ein Mittel zur Aufnahme von Blut und Gewebsflüssigkeit aufweist.

6. Punktionsmittel nach Anspruch 1,
dadurch gekennzeichnet,
daß nach seinem Eindringen unter die Hautoberfläche von seinem Träger ausgehende Vorsprünge bestimmungsgemäß auf die Haut herabreichen und der Wundspalterweiterung dienen, vorzugsweise bewegliche, welche auch dem Träger beigeheftet sein können.

7. Punktionsmittel nach Anspruch 1,
dadurch gekennzeichnet,
daß sie mit einem Teststreifen als Träger vor Gebrauch innerhalb einer Verpackungshülle gelagert sind, wobei Punktionsmittel und Träger von einer zusätzlich von einem verhältnismäßig festen die Punktionsspitze überragenden und schützenden Stützmittel wenigstens teilweise umgeben ist, wobei dieses Stützmittel auch streckenweise Teil der Verpackungshülle sein kann.

8. Punktionsmittel nach Anspruch 1,
dadurch gekennzeichnet,
daß für einen Mehrfachgebrauch mehrere Punktionspitzen mit jeweils wenigstens einem Mittel zur Aufnahme von Blut und Gewebsflüssigkeit auf einem gemeinsamen bandförmigen Träger befestigt sind.

9. Punktionsmittel nach Anspruch 1,
dadurch gekennzeichnet,
daß mehrere Punktionsmittel mit Träger innerhalb eines Wechselbehälters in einer Punktionsvorrichtung gelagert sind.

10. Punktionsmittel nach Anspruch 1,
dadurch gekennzeichnet,
daß wenigstens zwei Stifte mit je einer Spitze vor Gebrauch so zueinander konvergierend gelagert sind, daß sich ihre Spitzen zu möglichst nahe berühren, so daß sie beim Eindringen in die Haut annähernd einen einzigen Stichkanal bilden können.

11. Punktionsmittel nach Anspruch 1,
dadurch gekennzeichnet,
daß ein Tesstreifen als Träger des Punktionsstiftes kapilläre Sammelräume für die Blutaufnahme enthält.

12. Verfahren zur Anwendung eines Punktionsmittels mit Spitze nach Anspruch 1,
**dadurch gekennzeichnet,**
daß sie eine hautschonenden Wundspalterweiterung durch Mittel, welche die Hautspannung beeinflussen ermöglicht wird und durch besondere Mittel taschengängige Formgebung den ambulanten Einsatz erst ermöglichen.

13. Verfahren nach Anspruch 12,
dadurch gekennzeichnet,
daß die Spitze des Punktionsmittels durch wenigstens eine Vorrichtung nach anfänglich plötzlicher Bewegung zur Eröffnung von Blutgefäßen in verlangsamter Geschwindigkeit etwa quer zur Hautoberfläche durch eine Vorrichtung weiterbewegt wird, um den Wundspalt zu erweitern, vorzugsweise in Verbindung mit Mitteln, welche die Haut in der Nähe der Punktionsstelle bei einer queren Mitbewegung zu behindern.

14. Verfahren nach Anspruch 12,
dadurch gekennzeichnet,
daß das Punktionsmittel, nachdem wenigstens seine Spitze wenigstens die Hautoberfläche durchdrungen hat, eine Änderung Winkelstellung abweichend vom Winkel vor der Hautdurchbohrung über eine Vorrichtung erfährt, so daß die Punktionsspitze und der Schaftteil des Punktionsmittels nahe der Hautoverfläche von einer Senkrechtprojektion gesehen auseinandertreten und dabei der Wundspalt aufgespreizt wird.

15. Verfahren nach Anspruch 12,
dadurch gekennzeichnet,
daß die Vorrichtung zur Bewegung eines Punktionsmittels quer zur Hautoberfläche oder Einstichrichtung wenigstens ergänzend so ausgestattet ist, daß sie mit ihrer Spitze konvergierend angeordnete Punktionsstifte, wenigstens zwei, nach dem Einstich in die Haut auseinandertreibt, so daß der Wundspalt erweitert wird.

16. Verfahren nach Anspruch 12,
dadurch gekennzeichnet,
daß die Punktion der Haut innerhalb einer Saugglocke erfolgt, in welche letztere durch Unterdruckerzeugung angehoben wird, und daß die in der Saugglocke auf die Haut im Bereich der Punktionsstelle ausgeübte Zugwirkung durch Regelung der des Unterdruckes den Erfordernissen angepaßt werden kann, welche dadurch erfolgt, daß eine variierbare Streckenverbindung zwischen einem sich zur Sogerzeugung bewegenden Pumpenteil und einem durch ein Antriebsmittel, vorzugsweise einer Feder, bewegten Rahmen hergestellt wird.

17. Verfahren nach Anspruch 12,
dadurch gekennzeichnet,
daß die Punktion der Haut innerhalb einer Saugglocke erfolgt, in welche letztere durch Unterdruckerzeugung angehoben wird, und daß die in der Saugglocke auf die Haut im Bereich der Punktionsstelle ausgeübte Zugwirkung durch Regelung der des Unterdruckes über die regulierbare Ausdehnung eines Saugpumpenraumes, vorzugsweise während der Saugglockenanwendung nachstellbar, den Erfordernissen angepaßt werden kann.

18. Verfahren nach Anspruch 12,
dadurch bekennzeichnet,
daß die Punktion der Haut innerhalb einer Saugglocke erfolgt, in welche letztere durch Unterdruckerzeugung angehoben wird, und daß die in der Saugglocke auf die Haut im Bereich der Punktionsstelle ausgeübte Zugwirkung durch Regelung der des Unterdruckes den Erfordernissen mittels eines Ventiles oder durch steuerbare Verringerung eines Saugpumpenraumes angepaßt werden kann, welches bewirkt, daß nach anfänglich starkem Unterdruck zum Einzug der Haut in die Saugglocke letzterer auf eine zweckdienliche Stärke herabgesetzt wird.

19. Verfahren nach Anspruch 12,
dadurch gekennzeichnet,
daß die Punktion der Haut innerhalb einer Saugglocke erfolgt, in welche letztere durch Unterdruckerzeugung angehoben wird, und daß die in der Saugglocke auf die Haut im Bereich der Punktionsstelle ausgeübte Zugwirkung durch Regelung der des Unterdruckes den Erfordernissen mittels wenigstens eines Ventiles angepaßt werden kann, wobei neben einer Luftzufuhrregelung in Richtung der äußeren Atmosphäre oder eines höheren Luftdruckes eine solche in Richtung eines Unterdruckspeicherraumes oder einer Pumpe besteht, um einen drohenden zu starken Druckanstieg in der Saugglocke vor der beabsichtigten Blutabnahme gegensteuern zu können.

20. Verfahren nach Anspruch 12,
dadurch gekennzeichnet,
daß mit Absenkung des Punktionsmittels in die Delle einer Hautkuppe auch wenigstens zwei Krallen abgesenkt werden, welche mittels einer Vorrichtung von einander entfernt werden und dabei eine Zugwirkung auf die das Punktionsmittel umgebende Haut ausüben.

21. Verfahren nach Anspruch 12,
dadurch gekennzeichnet,
daß die notwendig flache Formgestaltung des Gehäuses für die Vorrichtungen dadurch gesichert wird, daß für Träger des Punktionsmittels für den Einsatz zur Signalübermittlung zu einem Meßinstrument für physikalisch-chemische Analysen Kontakte innerhalb der Aufnahme des punktionsfernen Endes des Punktionsstiftes elektrische Kontakte bestehen, welche über Leitungsverbindungen mit dem Meßinstrument in Verbindung gesetzt werden können, vorzugsweise unter Einsatz eines Zwischenkabels.

22. Verfahren zur Herstellung eines Punktionsmittels nach Anspruch 1 ,
**dadurch gekennzeichnet ,**
daß mehrere Punktionsstifte als Punktionsmittel aus Metall bestehen, vorzugsweise aus Stahl, und in Reihe einem Elektrolytbad zugeführt werden, um eine Punktionsspitze und, soweit beabsichtigt, eine spitzen nahe kleine Schneide zu erzeugen.

23. Verfahren nach Anspruch 22,
dadurch gekennzeichnet,
daß die Punktionsstifte in Transportglieder einer Transportkette aufgenommen werden, vorzugsweise unter Einsatz von Magnetkraft, Wobei die Transportglieder dem Elektrolytbad angenähert und wieder von diesem entfernt werden.

24. Verfahren nach Anspruch 22,
dadurch gekennzeichnet,
daß die Punktionsstifte zunächst einem Trägerband Transportmittel angeheftet werden, vorzugsweise unter Wärmeeinwirkung auf das letztere, um dann in das Elektrolytbad eingetaucht zu werden.

25. Verfahren nach Anspruch 22,
dadurch gekennzeichnet,
daß die Punktionsstifte zwischen zwei Trägerbändern, die miteinander adhäsiv vereinigt werden, teilweise und spitzenabgekehrt eingeschlossen werden.

26. Verfahren nach Anspruch 22,
dadurch gekennzeichnet,
daß die Punktionsstifte direkt aus der Extrusionsdüse oder Strangpresse abgeteilt und einem quer zur Bewegungsrichtung des ausgetretenen Drahtes von Trägermitteln zur weiteren Verarbeitung aufgenommen werden.

27. Verfahren nach Anspruch 22,
dadurch gekennzeichnet,
daß die Punktionsstifte aus der Extrusionsdüse oder Strangpresse zwischen wenigstens zwei gegeneinander laufende und sich teilweise berührende Walzen einzeln geformt werden.

28. Verfahren nach Anspruch 22,
dadurch gekennzeichnet,
daß die Punktionsstifte mit einem Streckenabschnitt aus dem Trägerband, das auch mit zusätzlichen Funktionsmitteln bestückt wurde, in normierte Teststreifen ausgestanzt oder ausgeschnitten werde.

29. Verfahren nach Anspruch 22,
dadurch gekennzeichnet,
daß der jeweils ausgestanzten Teststreifen mit Punktionsstiften von wenigstens einem Verpackungband, welches im zugeführt wurde, aufgenommen und der Verpackungsstreifen von dort in einer weiteren Stufe, vorzugsweise aber sogleich nach Zufuhr eines zweiten Verpackungsstreifens von der Gegenseite her, ausgeschnitten oder ausgestanzt wird.

30. Verfahren nach Anpruch 22.
dadurch gekennzeichnet.
daß die Punktionsstifte zur Befestigung dem Teststreifen beheizt werden, vorzugsweise durch äußere Strahlenzufuhr.

31. Verfahren nach Anspruch 22,
dadurch gekennzeichnet,
daß die Punktionsstifte in polygonale Glieder eines Transportkarussels aufgenommen werden, nach dessen Sektordrehung sie einer besonderen Verarbeitungsreihe je nach Sektorwinkel zugeführt werden.
